# EUROPÄISCHE PATENTSCHRIFT

(11) **EP 1 469 316 B1**
(45) Veröffentlichungstag und Bekanntmachung des Hinweises auf die Patenterteilung: **02.11.2006**
(21) Anmeldenummer: 04014603.7
(22) Anmeldetag: 03.08.2001
(51) Int. Cl.: G01N 33/68, C07K 14/47, C12N 15/00, C07K 16/00, C12N 5/00, A01K 67/027, C12Q 1/68, G01N 33/94

(54) **Screeningverfahren**
Screening method
Procédé de criblage

(30) Priorität: 03.08.2000 DE 10037759
(43) Veröffentlichungstag der Anmeldung: 20.10.2004
(62) Teilanmeldung aus: 01960621.9
(73) Patentinhaber: Grünenthal GmbH, 52078 Aachen (DE)
(72) Erfinder: Weihe, Eberhard, Prof. Dr., 35037 Marburg (DE); Schäfer, Martin K.-H., Dr., 35041 Marburg (DE); Gillen, Clemens, Dr., 52076 Aachen (DE); Wetzels, Ingrid, 52080 Aachen (DE); Wnendt, Stephan, 52066 Aachen (DE)
(74) Vertreter: Graf von Stosch, Andreas

(56) Entgegenhaltungen:
- WO-A-00/43524
- WO-A-00/75118
- WO-A-98/26054
- WO-A-99/12559
- WO-A-99/18193
- CA-A- 2 148 898
- SARAGOVI, H. URI ET AL: "Development of pharmacological agents for targeting neurotrophins and their receptors" TRENDS PHARMACOL. SCI., 21(3), 93-98, März 2000 (2000-03), XP004202571
- GUPTA SHASHI ET AL: "Selective interaction of JNK protein kinase isoforms with transcription factors." EMBO (EUROPEAN MOLECULAR BIOLOGY ORGANIZATION) JOURNAL, Bd. 15, Nr. 11, 1996, Seiten 2760-2770, XP002915273 ISSN: 0261-4189 -& DATABASE SWISS-PROT ID: MK10_HUMAN AC: P53779 "Mitogen-activated protein kinase 10 (JNK3)" XP002206558
- RAUSCH O ET AL: "Nerve injury-associated kinase: a sterile 20-like protein kinase up-regulated in dorsal root ganglia in a rat model of neuropathic pain." NEUROSCIENCE, 101 (3) 767-77., 15. November 2000 (2000-11-15), XP001066240
- BAYTEL D ET AL: "The human Pim-2 proto-oncogene and its testicular expression" BIOCHIMICA ET BIOPHYSICA ACTA . GENE STRUCTURE AND EXPRESSION, ELSEVIER, AMSTERDAM, NL, Bd. 1442, Nr. 2-3, 8. November 1998 (1998-11-08), Seiten 274-285, XP004275262 ISSN: 0167-4781 -& BAYTEL D ET AL: "Erratum to: 'The human Pim-2 proto-oncogene and its testicular expression' - [Biochim. Biophys. Acta 1442 (1998) 274-285]" BIOCHIMICA ET BIOPHYSICA ACTA . GENE STRUCTURE AND EXPRESSION, ELSEVIER, AMSTERDAM, NL, Bd. 1444, Nr. 2, 16. Februar 1999 (1999-02-16), Seiten 312-313, XP004275323 ISSN: 0167-4781
- LUGT VAN DER N M T ET AL: "PROVIRAL TAGGING IN EMU-MYC TRANSGENIC MICE LACKING THE PIM-1 PROTO-ONCOGENE LEADS TO COMPENSATORY ACTIVATION OF PIM-2" EMBO JOURNAL, OXFORD UNIVERSITY PRESS, SURREY, GB, Bd. 14, Nr. 11, 1995, Seiten 2536-2544, XP001074091 ISSN: 0261-4189
- GARRY E.M. ET AL MOL. CELL NEUROSC. 29 September 2005,
- SVENSSON C. ET AL J. NEUROCHEM. Bd. 92, Nr. 6, M{rz 2005, Seiten 1508 - 1520
- ZHUANG ET AL PAIN Bd. 114, Nr. 1-2, M{rz 2005, Seiten 149 - 159
- TSUDA M. ET AL TRENDS. NEUROSCI Bd. 28, Nr. 2, Februar 2005, Seiten 101 - 107
- DRAY A. ET AL J. OROFAC. PAIN Bd. 18, Nr. 4, 2004, Seiten 381 - 385
- MANNING D.C. CURR. PAIN HEADACHE REP. Bd. 8, Nr. 3, 2004, Seiten 192 - 198
- JI R.R. CURR. OPIN. INVESTIG. DRUGS Bd. 5, Nr. 1, 2004, Seiten 71 - 75
- TSUDA ET AL GLIA. Bd. 45, Nr. 1, Januar 2004, Seiten 89 - 95

### SEQUENZPROTOKOLL

<110> GRONENTHAL GmbH
<120> Screeningverfahren
<130> GR01P015WOEPT1
<140>
   <141>
<150> DE 100 37 759.9
   <151> 2000-08-03
<160> 116
<170> PatentIn Ver. 2.1
<210> 1
   <211> 243
   <212> DNA
   <213> Rattus norvegicus
<400> 1
   ctttctaccc tgctgtagct aaccgttaca cactcccttc cactatcccc ttagggacct 60
   cccatttctc agttctgaac ctcaacgatg ccttcttctc tgtccgcaaa gcacctttgt 120
   cttcacctgg actgaacctg gcacccccat ctctacccaa ctcacctaaa ctgtcttacc 180
   tcaggggttc tgggatagcc ctgaccactg atctcctctc tttgtcgatc cccaaaaaaa 240
   aaa                                                               243
<210> 2
   <211> 1268
   <212> DNA
   <213> Rattus norvegicus
<400> 2
   ctttctaccc tgctgtagct aaccgttaca cactcccttc cactatcccc ttagggacct 60
   cccatttctc agttctgaac ctcaacgatg ccttcttctc tgtccgcaaa gcacctttgt 120
   cttcacctgg actgaacctg gcacccccat ctctacccaa ctcacctaga ctgtcttacc 180
   tcaggggttc tgggatagcc ctgaccactg atctcctctc tttgtcgatc cccaaattaa 240
   aactcataca gtatgtagat natcttttcc tctgtggtcc atgcctataa attagccana 300
   tggacacctc tgctcttcta aacttcttct ctagctgggg ctgtacagyc tcaccatcta 360
   aggtccaatt gtctgcccct gtctgttacc ccaactcaca aagccattgc cttggataac 420
   gccttagagt tttacttctg tgaacagaca ccgtgaccaa ggcaactctt ataaagacaa 480
   catttaattg gggctggctt acaggttcag aggttcagtc cattgtcatc aaagtgggag 540
   caaggcagtg ctcaggcagg catggggctg gaggaactga gagttctaca tcttcatctg 600
   aaggctgcta gcagaatact gacttccaga cagctaggat gagggtctta aagcccacat 660
   ccacagtgac acacctactc caacagggcc acaccttcta atagtgccac tcctaggcta 720
   agcctattca aaccatgaca gatagaaaac acctaaccta tcccttctcc ctctatgtaa 780
   cagaaaaata ggatacaccg tgggggtcct atgtcctagg ccactgacta ggatcttcct 840
   ttcaccaact ggcatctatg tcaaggagat tagaccttac cacccaggga taggcagcct 900
   gcaatacact tcagcagctg ccaaactcct tatgcaggaa ttgaaaaacc taccactctc 960
   ttaccactgt cttctcctct cacagcttgc ccacctctta gcgtataaag gcttacagac 1020
   tctacctcct tcttgggttc tctctctcca ggtggtagta gtagaggaag gcacacgcat 1080
   ttccaatcat gcccacccct caacatctca aatcttcttc cccaaccaaa ctcagactat 1140
   gcctgttttg ttcttgattt gaaaactcag aggaaccact gccccatctc ttgcacacac 1200
   aggaaagcat gttacctcag gccatccata ccgggttccc cccgtacttt gcgttgttac 1260
   cactgctt                                                          1268
<210> 3
   <211> 1063
   <212> DNA
   <213> Rattus norvegicus
<400> 3
   ctttctaccc tgctgtagct aaccgttaca cactcccttc cactatcccc ttagggacct 60
   cccatttctc agttctgaac ctcaacgatg ccttcttctc tgtccgcaaa gcacctttgt 120
   cttcacctgg actgaacctg gcacccccat ctctacccaa ctcacctaga ctgtcttacc 180
   tcaggggttc tgggatagcc ctgaccactg atctcctctc tttgtcgatc cccaaattaa 240
   aactcataca gtatgtagat gatcttttcc tctgtggtcc atgcctataa attagccaga 300
   tggacacctc tgctcttcta aacttcttct ctagctgggg ctgtacagyc tcaccatcta 360
   aggtccaatt gtctgcccct gtctgttacc ccaactcaca aagccattgc cttggataac 420
   gccttagagt tttacttctg tgaacagaca ccgtgaccaa ggcaactctt ataaagacaa 480
   catttaattg gggctggctt acaggttcag aggttcagtc cattgtcatc aaagtgggag 540
   caaggcagtg ctcaggcagg catggggctg gaggaactga gagttctaca tcttcatctg 600
   aaggctgcta gcagaatact gacttccaga cagctaggat gagggtctta aagcccacat 660
   ccacagtgac acacctactc caacagggcc acaccttcta atagtgccac tcctaggcta 720
   agcctattca aaccatgaca gatagaaaac acctaaccta tcccttctcc ctctatgtaa 780
   cagaaaaata ggatacaccg tgggggtcct atgtcctagg ccactgacta ggatcttcct 840
   ttcaccaact ggcatctatg tcaaggagat tagaccttac cacccaggga taggcagcct 900
   gcaatacact tcagcagctg ccaaactcct tatgcagttc ttgacctgtc tctcccctga 960
   ccctagctgg ctacatattc tctctccctg aaaccctgcc ccactctcag agccttcaga 1020
   tgtccagctc tgccccctca tgcttaataa atgcagtgac aca                   1063
<210> 4
   <211> 840
   <212> DNA
   <213> Rattus norvegicus
<400> 4
   cctctagatg catgctcgag cggccgccag tgtgatggat atctgcagaa ttcgccctta 60
   ctcatatagg gctcgagcgg ccgcccgggc aggtctagaa ttcagcggcc gctttttttt 120
   tttttttttt tttttkgggg tttgggtttc cattttggtt tacagcagac ctttaatttg 180
   gacgtttgra gggatcaaca tcacttgttt tgttctgrag agaagttcct gatagtgttt 240
   ctcccctctc acctccagat ggactcagtt attaaaattt ggcgctttaa aaagagtgat 300
   gagcagtgct tcaattacag tgacaaagga gaaaaaagta agaaagcacc catggccttg 360
   tcgccaacct taaaggacat tcatattttg ctttaaggct tkgtagttgc cacagtcagg 420
   ttacagagct ccaagggacc attattgatg ttgncattnt aagcgacttc agtttgatgg 480
   agtgattatc ttaattttcc catgaataga cttcacctgc ctcaggtcac tgaacctctc 540
   ctcttcccca gccctcctta aaaatttcta cctaggaacc aatcgaaggg aaaaaaggct 600
   atgactacat ggcattcaga ttcaccccct ctagctatgt tttattatta cttagcttcc 660
   acgtgacctg ggtatagaat caaacanatg ttatggtgaa gcatagcgga aaaggtaaaa 720
   tggccactgg ggcccaaaat gatgcatgtt aaaattctga ccctagtgcg aaggtcttga 780
   aaaaggcttt gtggggtcct tanagcatga aaacagaaac tgcatggatg gattggttcc 840
<210> 5
   <211> 716
   <212> DNA
   <213> Rattus norvegicus
<400> 5
   ggaaccaatc catccatgca gtttctgttt tcatgctnta aggaccccac aaagcctttt 60
   tcaagacctt cgcactaggg tcagaatttt aacatgcatc attttgggcc ccagtggcca 120
   ttttaccttt tccgctatgc ttcaccataa catntgtttg attctatacc caggtcacgt 180
   ggaagctaag taataataaa acatagctag agggggtgaa tctgaatgcc atgtagtcat 240
   agcctttttt cccttcgatt ggttcctagg tagaaatttt taaggagggc tggggaagag 300
   gagaggttca gtgacctgag gcaggtgaag tctattcatg ggaaaattaa gataatcact 360
   ccatcaaact gaagtcgctt anaatgncaa catcaataat ggtcccttgg agctctgtaa 420
   cctgactgtg gcaactacma agccttaaag caaaatatga atgtccttta aggttggcga 480
   caaggccatg ggtgctttct tacttttttc tcctttgtca ctgtaattga agcactgctc 540
   atcactcttt ttaaagcgcc aaattttaat aactgagtcc atctggaggt gagaggggag 600
   aaacactatc aggaacttct ctycagaaca aaacaagtga tgttgatccc tycaaacgtc 660
   caaattaaag gtctgctgta aaccaaaatg gaaacccaaa ccccmaaaaa aaaaaa 716
<210> 6
   <211> 1540
   <212> DNA
   <213> Rattus norvegicus
<400> 6
   gcctgagaac cgtaatcacc aaatgcctgc agcaaaacag aggtgtttgt taagcctttg 60
   gtatcaggaa gcttcgagag aattcatttc ttgtctttga agaggggtgg tttcattaca 120
   aagagatgga aaggcatggt tgaaaattga gctaaaaatt agaattgaga gagagagaga 180
   gagagaatag atgattagaa caataattca taatttttga tctaaacatt tggaggttgt 240
   cttatttgca tttctcttag cttcccctct tagcatcctc atggttttaa gcacagctat 300
   aaatttccat ttattcaaat cacaatgcaa ttgattcttg tgttctccat tagaccttga 360
   atgactcaga ggcaattcag ccatgttttt tcttcgttgt tactctatcg tgtagcactg 420
   tgtgtggtgt ccatgtgtgt gcactgaata atgcaatgaa aggcgcatag tgagatgatg 480
   ctggtttgac attactccga caaaaaagaa aatgtctgtg tagcaatggt cattactatg 540
   aaaatgtggt tgttttttaa gcattccgtg tgtcacagga acgggattta atgagtgtcc 600
   taggacctgg taccgcatag aactacgtgt ctcagaagca caggatgtac tgaattactt 660
   tgccagtnac ctggtcctca aaatccctcc accgagttgc ttattcaaat cctcacgctc 720
   ataaatctcc atcagccatc actagttgaa ttacgtttgc ctagtgtttc tctctaactc 780
   ccgcaaacct gcttcatgta ctctcttcaa gactgatggg aagctaattg cttgctacgt 840
   ttcatgtcct accatgagtc atgctgtccc aatctcatcc tcaaccaccc gactctccaa 900
   cagccaagtc ctagagctcc aacagggaga tggaaattgg ttactgatcg acttaaaatg 960
   gcaggattct ctcacaggga tgtaggctgt gaaaaaaaca gangacagtg ttaacagnga 1020
   attgcctaga tcctattcat taggttctat tcattaggtc tgcagagaaa aagaccggct 1080
   tttgtaatat gntatnagat tcagtttaac tttccatttg tcctcccctc ttcttggtcc 1140
   cccctgtaag gctttaggaa aaaactacct acatattgcc aatctttccc caaagacatc 1200
   tgtttttctc cacaaatcct tacctccgaa aatatatgct aatgaagaca gtgattgttt 1260
   aatgaaaact tcaaatcagg agcccagacc tagatctaac agcctcaggc aaatcacaag 1320
   agcaaatgct gttcagatgt gtaggctcca aatactgtga attcttaggc ggcatcctgg 1380
   agaatcacca aaaatgttga ttcctaactt gaaagtcaac attccagaat agcacttgca 1440
   gatacatacg ganagtctgt tctctggctc ttttcctatg aaggtccttt aactgcttcg 1500
   taataggaaa acattcagtc cccaatgccc aaaaaaaaaa                       1540
<210> 7
   <211> 2025
   <212> DNA
   <213> Rattus norvegicus
<400> 7
   tttttttttt gggcggggag ggggagaggc caagatnttn tctttcaagt ccagggagca 60
   cagaacttgg cacacattat caaaaggtgg gaacgaaact accagggtca gtttctttta 120
   ggagcctagc aactacatat tccattctgc gggtaggaac cagccatctg ttgtggaccc 180
   taatttaaac tcctcccata cccacaggaa attgatacat actgaagttg gaaagtgccg 240
   gtctaggctt tacatgtctg gcttttggta cactttccgg gtgttagtgt gcagtgagtt 300
   ggggtgggag gtgtttcagt ttttgcacgt gctcgtggtt tgtaaatgcg gcccacaatc 360
   aaagacggca atgtctataa ggcaaaaatc taaattgcac tctaatccag gtgctctggc 420
   cttgtgggcc aaaccattcc tgggactcga taggaggcgc tggactgata taagaacacc 480
   tgtcttgggg gtgggggtgg ttgtaaagga accgcagtgg atatctgggg aagatcaaac 540
   caaggacagg ctggggacag cgctggggac agtgcatgct tctttggact ctaratgctt 600
   gcctgccatc ccctccccgg gatcctgaca tcccaggagc gtggtggttt tcagcagttg 660
   atcacagtgt aatactttca gttctattcc agagtcagga tatgtccaag gaatgcgtgt 720
   gtgttgggaa taggggcagt ctccgggcct ggggatggga caggaggagt ttcttggctt 780
   gactggtttc ttaacgctag cacgctttca cccgctggga gagtttcatg aggtcctctt 840
   gtccccgaat atctttgact cgccttattc agtggcctgc gtccagagtg ggatctgatt 900
   atttcgagaa cttagacagt acctatgcaa gaaggaagcc aaacaggtac caaggctgct 960
   ggggtggcct ctctctggag gtgactccct gggtagctga gatgtattga gaccaagact 1020
   ttgaaaggac tcctgagaag gaagggctgg gaagctactc tcttgtgtag gccatgctca 1080
   caggtacacg acaaggggca ttgccaataa ggaccttgga gttcagtggt gtcaggaaaa 1140
   aaaggcgaat gcaagaacgc tgtggacctc cagagccgag gaggggagag gggacccaag 1200
   actggagaga aggcgcctgc tttccagctt tattggccac ctccgctcca gcctctgcca 1260
   accaccgagc ggttgggagg gggaggaggt ggtcaccatg ccattgtggt tcctgagtca 1320
   gccggagccc attgccgccg gggagccaga ctccgtgaaa tgttaattat ctgttagaaa 1380
   aaacagtata ttttatacaa cgttgaaaac aaaacaaaaa aaatctaatt cctgagcagg 1440
   ccctgctatt tgactcactc aatgtacatt tatttatgcc ccataaaagg tacacgggaa 1500
   gccatgcagt ggtctttttt aaaaggcgat aacgtgttct ttacactaaa ccgtaacacc 1560
   tacgcacaca gtgattgagt tttagtcaga aaggtaccca aagtgacatt tcctcccact 1620
   aatttcacat tgcctgtgta gttctgggga gtaacattac tcttcagcag aaaaaaaaat 1680
   gttctatccc gtcctggcaa cgccacatgg cgcttgtatt cccgtcagtt gttttatcag 1740
   ctcaacttcc cccatttctt tctgggataa ctttttctct gatgctagtt tgcattttat 1800
   ggagtgtgga gtcatggcta tttccttgtc ctggtcccat taacttcctg gtactattgt 1860
   ttgaagcctc cagaccctga tgtataatcc aactgtttcc ccactgtgtg ggaagagaaa 1920
   gccgttggcc taggtcttga acaacccaag ctctcctttc ccatgaaggt gctggcaaga 1980
   ctttctggag tgcagttcta gaattcagcg gccgctgaat tctag                 2025
<210> 8
   <211> 2392
   <212> DNA
   <213> Rattus norvegicus
<400> 8
   gtctttgtca tcagaaggaa aatgacgtgt tctatgtgtg ttgacacagt caggtttggt 60
   aacctcatga agagcagaca tgaatgatga acacagaggc ataatatagt aaatctcagg 120
   agccacacag ccaagaaaca cgtggagcag caaatgcctg gaagactgat attagcaacc 180
   acgaggcaca tagtaattgc taatcctaca catgaatacc caaaactact ctgtgaggtc 240
   aagtgctaga cttagaggcc agtggtgtcc ttcttcctct tcattatgtc actgagcaag 300
   tcatctcaga aactccttaa aaaaaatcct gattccaatc acatcacacc actcaactgc 360
   agtgtaaaat cttcctacct gatcttgtga acctgtcctg cactccagaa agtcttgcca 420
   gcaccttcat gggaaaggag agcttgggtt gttcaagacc taggccaacg gctttctctt 480
   cccacacagt ggggaaacag ttggattata catcagggtc tggaggcttc aaacaatagt 540
   accaggaagt taatgggacc aggacaagga aatagccatg actccacact ccataaaatg 600
   caaactagca tcagagaaaa agttatccca gaaagaaatg ggggaagttg agctgataaa 660
   acaactgacg ggaatacaag cgccatgtgg cgttgccagg acgggataga acattttttt 720
   ttctgctgaa gagtaatgtt actccccaga actacacagg caatgtgaaa ttagtgggag 780
   gaaatgtcac tttgggtacc tttctgacta aaactcaatc actgtgtgcg taggtgttac 840
   ggtttagtgt aaagaacacg ttatcgcctt ttaaaaaaga ccactgcatg gcttcccgtg 900
   taccttttat ggggcataaa taaatgtaca ttgagtgagt caaatagcag ggcctgctca 960
   ggaattagat tttttttgtt ttgttttcaa cgttgtataa aatatactgt tttttctaac 1020
   agataattaa catttcacgg agtctggctc cccggcggca atgggctccg gctgactcag 1080
   gaaccacaat ggcatggtga ccacctcctc cccctcccaa ccgctcggtg gttggcagag 1140
   gctggagcgg aggtggccaa taaagctgga aagcaggcgc cttctctcca gtcttgggtc 1200
   ccctctcccc tcctcggctc tggaggtcca cagcgttctt gcattcgcct ttttttcctg 1260
   acaccactga actccaaggt ccttattggc aatgcccctt gtcgtgtacc tgtgagcatg 1320
   gcctacacaa gagagtagct tcccagccct tccttctcag gagtcctttc aaagtcttgg 1380
   tctcaataca tctcagctac ccagggagtc acctccagag agaggccacc ccagcagcct 1440
   tggtacctgt ttggcttcct tcttgcatag gtactgtcta agttctcgaa ataatcagat 1500
   cccactctgg acgcaggcca ctgaataagg cgagtcaaag atattcgggg acaagaggac 1560
   ctcatgaaac tctcccagcg ggtgaaagcg tgctagcgtt aagaaaccag tcaagccaag 1620
   aaactcctcc tgtcccatcc ccaggcccgg agactgcccc tattcccaac acacacgcat 1680
   tccttggaca tatcctgact ctggaataga actgaaagta ttacactgtg atcaactgct 1740
   gaaaaccacc acgctcctgg gatgtcagga tcccggggag gggatggcag gcaagcatyt 1800
   agagtccaaa gaagcatgca ctgtccccag cgctgtcccc agcctgtcct tggtttgatc 1860
   ttccccagat atccactgcg gttcctttac aaccaccccc acccccaaga caggtgttct 1920
   tatatcagtc cagcgcctcc tatcgagtcc caggaatggt ttggcccaca aggccagagc 1980
   acctggatta gagtgcaatt tagatttttg ccttctagac attgccgtct ttgattgtgg 2040
   gccgcattta caaaccacga gcacgtgcaa aaactgaaac acctcccacc ccaactcact 2100
   gcacactaac acccggaaag tgtaccaaaa gccagacatg taaagcctag accggcactt 2160
   tccaacttca gtatgtatca atttcctgtg ggtatgggag gagtttaaat tagggtccac 2220
   aacagatggc tggttcctac ccgcagaatg gaatatgtag ttgctaggct cctaaaagaa 2280
   actgaccctg gtagtttcgt tcccaccttt tgataatgtg tgccaagttc tgtgctccct 2340
   ggacttgaaa ganaanatct tggcctctcc ccctccccgc ccaaaaaaaa aa         2392
<210> 9
   <211> 1721
   <212> DNA
   <213> Rattus norvegicus
<400> 9
   aagcagtggt aacaacgcan antacgcggg gccccccacc tccttcctac ctgtgcaaaa 60
   agtacaacta aaaccaaacc aaaacaaacc aataaacaaa aganaaagaa aactgaaaac 120
   aaaacaaaac aaaacaacaa aaacaaaaat tcaaaccatt cactttacct aacaaggatt 180
   ccaaaacaca ttttttttca ttgacanaat tctgggagta tattttcaca tgctatttca 240
   ttagtttaat tgctctaaaa ctgtaatgac cttgaaataa ttttaaaatg ggtttaaaat 300
   taacttgtca aatttcagtt taagacttta ttgtccgccc acacaatgta actgcttgtc 360
   ccctaagtat taaagcagtt ttacaagcgg tggtgtgcag attctcttcc actaataata 420
   gccttgataa taagtcataa ggaatggtgg ctaatgtgag tttagcagga agccacaaag 480
   aaataaaaca gcagatacct taagtctgat atttctacat taaaattcaa ttaccacctc 540
   taccaccagt tatgagaagc cacgatggat acatactagt ttccacattt gacatcttct 600
   ggttttgctg acaccctcat tgccctaaga tatgatgatg atttcaagat ttcwwmwgyt 660
   yaasamktya ykgtcmkmmc rcatcatcaa tattcacaga tgttagttcc aagccagaag 720
   gaaaaaaaga aaagacataa cgttgcagga atatgatcta atcagttgta caaagttctc 780
   aaaaactaag acttaaaaaa aaaaaaaaag tctgatactc ttagacaagg caaagtggga 840
   ggtgaagact ggtagatcct tggagcttcc tggccagcta accttgccta cctgtagaag 900
   tttaaaatca atgagagaca caatttcaaa caaaaggtag acagaacctg agaaaaaaca 960
   cccaggattt tcctgtaacc ttcatattga tacacatgta ctactctgaa gaaaatacac 1020
   atgcacwggc wcaagcgcgc gcgtgcgcgc gcgcgcacac acwcwcacwc acacacacac 1080
   acacacacac acacacctca aaccacaaat agttacttat acaagagatc cctttttaaa 1140
   gtagaaatgg gaccatactt gacaaaagtg atgcgtttta cataaagata tcgaatttgt 1200
   ttaatgcaag cttagtccga aatggatact taagtcataa atactctttt ggtcatatcc 1260
   ctgtttcata tttgatttct tagtaacatc tatattcaaa aacagtgtcg gaagattttc 1320
   tagaagaach agaaatattt tcgcacaatg tyttatctta gtttwwcatt agaaaagcak 1380
   aagaaggaat atttycyggc ygtcmmcmga gaatgttccm gaaccactaa aaakaaaarc 1440
   tygtgggnaa gggcratwtg ccygtngttc nttaaaaata agtattcngg ggttgcraaa 1500
   aaaanttgtc ctccttgttt cttttttaaa aaattttgaa aacagtaatc ctcatagcct 1560
   ttgcagtttc cttcaacttg caagatattt cacatgttta ccaagcaatn acctaagtga 1620
   tgtcaccata ttntcaccaa aaatacctcc tttaatgctt caggatggtg ccacctatag 1680
   tagaaagcca gtgaagtcan tttgcgttgt taccactgct t                    1721
<210> 10
   <211> 1009
   <212> DNA
   <213> Rattus norvegicus
<400> 10
   aagcagtggt aacaacgcaa antacgcggg ttctgtgtaa agganaagga natgtcggaa 60
   aatggaanac atatgtaaag tattggcaan tgcaggcaac tgggattttg aggcagtcan 120
   aacaactgca gganaaactg cagcanaagc ctccaacttt ataatggaag cacaactggc 180
   tcanatacag atttatcttc aacagctgta tttttggcct tttatgttag tgagtatctt 240
   tcatacctca tggtaattag tgctgactaa atttttgagc ttcttctttt ctttttttgg 300
   gggggggtnc tctttcttta catttcaatt gttattcctt tacccagttt cctggtcata 360
   agccccctat cccattctcc tccccttctt ctataagggt gtkccccctc ctccctgaca 420
   tttcccaaca ctgggactac aatattggat ggattatggg tttctcctcc cattggtgcc 480
   caacaaggcc atcctctgct acatatgcag ctggarccat agggtctgtc tatgtgtagt 540
   ctttgggtag tggtttagtc cctggcagct ctgtttggtt agtattgttg ttattatgaa 600
   atttcaagcc ccttcagctc cttcagtcct tttctaattc ctccaatggg gaccctgttc 660
   tcagttcaat ggtttgctgc tagtgtcaca tctgtatttg acatgctctg gctgagcctc 720
   tcagctatat caggtgcctg tcagcatgaa cttcttggct ttatcaatat tgtctaatta 780
   tggtgcctgt ataaatatgg gctggatccc aaggtgaggt aggctctgaa tggccattcc 840
   ctcaggtgaa agtgctggcc aaggaatatg ggaaaaaraa rgaamactcc ctccattgtt 900
   tgttgggaat gcaaaaatgg gtacaatcaa tctggaaaat cvatcttggg tkttcttcaa 960
   aaaaaattgg aaattaaact tacctgnang aaccaaccta tacctgntn            1009
<210> 11
   <211> 685
   <212> DNA
   <213> Rattus norvegicus
<400> 11
   ntgcttgatg ataggtgggt gaacacaaac acatggaatt agtcttgtgg ttttcttaga 60
   tttttttaat tagggcattt ctgaccatgt tagaattaaa tntatttaaa tgcaattaga 120
   attgtttagg gaagattatt gtgtggcaga tgaattgaga agatatggtg gctgtgctga 180
   aaaaggaatg tgaggaaaca cagaagaaaa gaaagtgatc agtgtgtaca tcaagaagct 240
   caaaaattcc ttcagtctct gctccaaact ttgtctccat atcccctcat atgaatattt 300
   ccccctttta agaaggactg aagcatcccy actttggtta tccttcttct tgagcttcat 360
   gtggtttgtg gattatatct tgggtacttt gagcttttgg gctaatatcc acttatcaat 420
   gagtgcatac caagtgtgtt ctgtgtttgg gttacctgac acaggacaat attttctagc 480
   tcattccatt tgcctatgta tttcatgaag tcattgtttt tgatagctga atagtactcc 540
   attgtgtaga tgttaccaca tccattcctt tgttgaaaga aatctgcatt ctttctaact 600
   tcgcattatt ataaataaar ctactatgaa catattggaa caatgtgtct ttcctggtat 660
   gttggaacac catttgtgca tatgc                                       685
<210> 12
   <211> 3032
   <212> DNA
   <213> Rattus norvegicus
<400> 12
   caatgtaact gcttgtcccc taagtattaa agcagtttta caagcggtgg tgtgcagatt 60
   ctcttccact aataatagcc ttgataataa gtcataagga atggtggcta atgtgagttt 120
   agcaggaagc cacaaagaaa taaaacagca gataccttaa gtctgatatt tctacattaa 180
   aattcaatta ccacctctac caccagttat gagaagccac gatggataca tactagtttc 240
   cacatttgac atcttctggt tttgctgaca ccctcattgc cctaagatat gatgatgatt 300
   tcaagatttc aaatgctcaa caagtcacgg tcataacgat catcaatatt cacagatgtt 360
   agttccaagc cagaaggaaa aaaagaaaag acataacgtt gcaggaatat gatctaatca 420
   gttgtacaaa gttctcaaaa actaagactt aaaaaaaaaa aaaaagtctg atactcttag 480
   acaaggcaaa gtgggaggtg aagactggta gatccttgga gcttcctggc cagctaacct 540
   tgcctacctg tagaagttta aaatcaatga gagacacaat ttcaaacaaa aggtagacag 600
   aacctgagaa aaaacaccca ggattttcct gtaaccttca tattgataca catgtactac 660
   tctgaagaaa atacacatgc acaggcacaa gcgcgcgcgt gcgcgcgcgc gcacacacac 720
   acacacacac acacacacac acacacacac acctcaaacc acaaatagtt acttatacaa 780
   gagatccctt ttaaagtaga aatgggacca tacttgacaa aagtgatgcg tttacataaa 840
   gatatcgaat ttgtttaatg caagcttagt ccgaaatgga tacttaagtc ataaatactc 900
   ttttggtcat atccctgttt catatttgat ttcttagtaa catctataat caaaaacagt 960
   gtcagaagat tatctagaag aacaagaaat atttcgcaca atgtcttatc ttagttttac 1020
   atagaaagca gagaaggaat attttctgct gtcaacaaga atgttccaga ccaaaaaaga 1080
   aaagctcgtg gagggcaata tgcctgtgtt cttaaaaata agtattgggg ttgcaaaaaa 1140
   aacttgtcct ccttgtttct tttttaaaaa attttgaaaa cagtaatcct catagccttt 1200
   gcagtttcct tcaacttgca agatatttca catgtctacc aagcaattac ctaagtgatg 1260
   tcaccatatt ctcaccaaaa atacctcctt taatgcttca ggatggtgcc acctatagta 1320
   gaaagccagt gaagtcactc tgcgttgtta ccactgcttg atgataggtg ggtgaacaca 1380
   aacacatgga attagtcttg tggttttctt agattttttt aattagggca tttctgacca 1440
   tgttagaatt aaatctattt aaatgcaatt agaattgttt agggaagatt attgtgtggc 1500
   agatgaattg agaagatatg gtggctgtgc tgaaaaagga atgtgaggaa acacagaaga 1560
   aaagaaagtg atcagtgtgt acatcaagaa gctcaaaaat tccttcagtc tctgctccaa 1620
   actttgtctc catatcccct catatgaata tttccccctt ttaagaagga ctgaagcatc 1680
   cctactttgg ttatccctct tcttgagctt catgtggttt gtggattata tcttgggtac 1740
   tttgagcttt tgggctaata tccacttatc aatgagtgca taccaagtgt gttctgtgtt 1800
   tgggttacct gacacaggac aatattttct agctcattcc atttgcctat gtatttcatg 1860
   aagtcattgt ttttgatagc tgagtagtac tccattgtgt agatgtacca catccattcc 1920
   tttgttgaag gacatctgca ttctttctag cttcgcatta ttataaataa agctactatg 1980
   aacatagtgg agcatgtgtc tttcttgtat gttggagcat catttgtgca tatgcccagg 2040
   agaggtatag ctgggtcctc aggtagtgta atgtccaatt ttctgaagaa caccaagact 2100
   gatttccaga ttgattgtac cattttgcaa tcccacaaac aatggaggag tgttcctctt 2160
   tctccatatc cttgccagca ctgtcacctg agggaatagc cattcagagc ctacctcacc 2220
   ctgggatcca gcccatattt atacaggcac cataactaga caatattgat aaagccaaga 2280
   agttcatgct gacaggcacc tgatatagct gagaggctca gccagagcat gtcaaataca 2340
   gatatgacgc tagcagcaaa ccattgaact gagaacaggg tccccattgg aggaattaga 2400
   aaaggactga aggagctgaa ggggcttgaa atctcataat aacaacaata ctaaccaaac 2460
   agagctgcca gggactaaac cactacccaa agactacaca tagacagacc ctatggytcc 2520
   agctgcatat gtagcagagg atggccttgt tgggcaccaa tgggaggaga aacccataat 2580
   ccatccaata ttgtagtccc agtgttggga aatgtcaggg aggagggggc acacccttat 2640
   agaagaaggg gaggagaatg ggataggggg cttatgacca ggaaactggg taaaggaata 2700
   acaattgaaa tgtaaagaaa gagnaccccc ccccaaaaaa agaaaagaag aagctcaaaa 2760
   atttagtcag cactaattac catgaggtat gaaagatact cactaacata aaaggccaaa 2820
   aatacagctg ttgaagataa atctgtatnt gagccagttg tgcttccatt ataaagttgg 2880
   aggcttctgc tgcagtttct cctgcagttg ttctgactgc ctcaaaatcc cagttgcctg 2940
   cacttgccaa tactttacat atgtcttcca ttttccgaca tctccttntc ctttacacag 3000
   aacccgcgta ctttgcgttg ttaccactgc tt                              3032
<210> 13
   <211> 860
   <212> DNA
   <213> Rattus norvegicus
<400> 13
   actcactata gggctcgagc ggccgcccgg gcaggtaaat tgagcctgac ttaagaaatg 60
   cttactttac cactgcactg acaaccacaa tcatttggtc atggtcaggt gattgccatg 120
   tgtctctttt ctatttcata tatgaaggaa tgttgacaac atctaatgtg ttaactctag 180
   ccaagccaag tgcctattca ttgttgaata atactgtggg cttttaagaa ttaaacagta 240
   aaagacttca acaagcatga aaataatacg tgcttaaata aacttaaatt ctggaaaaga 300
   aaaatatttt cagagatcag tacatcatcc acaatcattg ttattttcac aaatagataa 360
   aatttctaga ataaattcaa agttttattt gggtggaagt aaattttggg ctgtaactag 420
   aataagagaa ttacctaagg ctcagaagtt atgcaagatt gatctaaaat aatctagata 480
   tgaaagatct gcaagtacat gatgaagcct ttgttaattc atttgctgta acataaagaa 540
   atacaattcc aaggctaaac tgaattccat acaccacaca gtattataga accaaagaaa 600
   cttctcagat tccatagaat caaaggaaaa gaaaaaatac agaatacaat attcaaacat 660
   gcagttctaa gtgttgtgat tttgcccaaa aatgtggggt ttttttgctg cattgatgaa 720
   ttggctcata tcatcctata gccataagag aagagaagag aagagatgct ttctagcaaa 780
   acctaatact gttgctgaat caaaatgtat tctgtacatt aggcctagga cattgaaaca 840
   acatacagtt gcatcaagca                                             860
<210> 14
   <211> 1348
   <212> DNA
   <213> Rattus norvegicus
<400> 14
   tgacagtggc acacaaaagt acagattcct ttcttatgcc atggagggcg ggccccagcc 60
   tctgctgctt ctgccatgat ccagccatgc agccctgcca ggacctgccc tcacatccgt 120
   tgtttctggt agcctttgaa atcaggagct tcncatgata atctgacctt ctaagatacc 180
   aaaggtcact ttccaggaag cttttctgag aaatgagtaa atgtttcctg gtggttttaa 240
   atttccaagt gaggaacgtc attcttgtcc cagttcagcg actagtggta acctccacat 300
   gccagaaagc agccacactg ttgctttcag agcaccagaa attactgagt aaatgctaag 360
   tatttatctt ctgggaagca aaggaattga aagaatatgt taatttttaa aattatatga 420
   atatgctttc agttcttcat aggggattga aaaagaatct tcgacttcat atggaaacac 480
   aaaaatctca gcatagctga acagtcttga atagtaagtg aactgatttc atgttgtaat 540
   gcgaatctgt atattgggcg ggggagaaca cgctgcagaa ctgacagcaa ctttaatgtc 600
   tggtcatgct ggatggggtg gctgcatgta gagcattgca aatgatccat agctatcacc 660
   ctgcccaaaa ttcaattcca gatggattaa gggccttaaa ataagaccat ataccctgaa 720
   tctgatagaa aatagggaat gggtctgaac ttactggcac cagaaaggaa tttccggaaa 780
   ggaagaggac acatagccct ggtgctaaaa cccaacaata aatgggaccg gatgaagcta 840
   aagtttctgt ggagcaaaag acaccatgta gtgaatgttt ttcatgattt tgcgttggga 900
   tctaggcatg tgacatttga aatgttcctt ggtataatta tctggtcttg tccttggtca 960
   agggttcttt ggttggtact gcccgctctg ggtctgggcc agttgtgatt cctggtagaa 1020
   gtgtctgtgg gcaggcaggt gtcacaaagg agttgagatg ggctttaagg aaaagccaaa 1080
   gggagtaata gtactaaggg gagcctagtt ctgcaccgag acagaatccc tggtggatgg 1140
   tggtccaaat gagaaggggg gctcctgtgg gcgtgctgca gcaggactaa aggtgaatta 1200
   aactgaccaa atgcaaaact ctgccttttg ggatgggttc agtgtaacca nttgcctcca 1260
   actccngcca ctgtgcatcc cctgnnatga tnaactgcca cctggagctg tgaaatcaaa 1320
   ataaatgctt ccttccccaa aaaaaaaa                                     1348
<210> 15
   <211> 1949
   <212> DNA
   <213> Rattus norvegicus
<400> 15
   tgacagtggc acacaaaagt acagattcct ttcttatgcc atggagggcg ggccccagcc 60
   tctgctgctt ctgccatgat ccagccatgc agccctgcca ggacctggcc ctcacatccg 120
   ttgtttctgg tagcctttga aatcaggagc ttccacatga taatctgacc ttctaagata 180
   ccaaaggtca ctttccagga agcttttctg agaaatgagt aaatgtttcc tggtggtttt 240
   aaatttccaa gtgaggaacg tcattcttgt cccagttcag cgactagtgg taacctccac 300
   atgccagaaa gcagccacac tgttgctttc agagcaccag aaattactga gtaaatgcta 360
   agtatttatc ttctgggaag caaaggaatt gaaagaatat gttaattttt aaaattatat 420
   gaatatgctt tcagttcttc atagggattg aaaaagaatc ttcgacttca tatggaaaca 480
   caaaaatctc agcatagctg aacagtcttg aatagtaagt gaactgattt catgttgtaa 540
   tgcgaatctg tatattgggc gggggagaac acgctgcaga actgacagca actttaatgt 600
   ctggtcatgc tggatggggt ggctgcatgt agagcattgc aaatgatcca tagctatcac 660
   cctgcccaaa attcaattcc agatggatta agggccttaa aataagacca tataccctga 720
   atctgataga aaatagggaa tgggtctgaa cttactggca ccagaaagga atttccggaa 780
   aggaagagga cacatagccc tggtgctaaa accaacaata aatgggaccg gatgaagcta 840
   aagtttctgt ggagcaaaag acaccatgta gtgaatgttt ttcatgattt tgcgttggga 900
   tctaggcatg tgacatttga aatgttcctt ggtataatta tctggtcttg tccttggtca 960
   agggttcttt ggttggtact gcccgctctg ggtctgggcc agttgtgatt cctggtagaa 1020
   gtgtctgtgg gcaggcaggt gtcacaaagg agttgagatg ggctttaagg aaaagccaaa 1080
   gggagtaata gtactaaggg gagcctagtt ctgcaccgag acagaatccc tggtggatgg 1140
   tggtccaaat gagaaggggg gctcctgtgg gcgtgctgca gcaggactaa aggtgaatta 1200
   gaactgacca gagtgcagag ctctgccttt tgggatgggt tcagtgtaac cagttgcctc 1260
   caactccagc cactgtgcat cccctgccat gatgaactgc cacctggagc tgtgaaatca 1320
   aaataaatgc ttccttccct aagttgcttt tgtcattttg tcacagcaac agaaaatcat 1380
   cataataatc agtgctaagt gtggacacat gctgatgggc tgagctctca ggaaactaag 1440
   gcgggaccag ctctgtggga ggcatcttaa accatatagt aaggccttgc ctcaagccag 1500
   tggatcggta gaagacttgg ggggtatgta ccactccttg gcatataccc aaaggttgca 1560
   gtttcttgca cagaggtggt tactcagctg gctccgttgt cgctctagcc acaaagctag 1620
   gaaatgggag cagcctaagt ggtactcagc tgatgcctgc ataatgaaat gcatatacac 1680
   acaatgtaat actgttcagc tctaacagtg aggttcacag gtaaatgaat gaatggaact 1740
   gggtaaggca ccatgttctc tcttatttgt ggatccgagc tccagacctc caaagtgagt 1800
   ataaaaccaa agtaacctca ggagccatgt ggaggaagga gttcacaagg gggatgatgg 1860
   gacaggtgct atgaaggaag agacgggaaa actgggggag aggttagaaa cggaggaagg 1920
   aaacataaca ctaagaatgt ttgtaaaag                                   1949
<210> 16
   <211> 923
   <212> DNA
   <213> Rattus norvegicus
<400> 16
   tggattggtc tggtgtatgg aagtgtggac agcatcttcc tgctctccag cgaatggttt 60
   tcttggctaa gaattttgtg cccttatttt atagacatgt ttttatattt tttgactatg 120
   tacaaaggga ttagacaggt caagtgataa ccatgtttat gtgatctact tttgaatatt 180
   ataattttaa atagtgctac aaatggaaat ttggtctaga agtattttca aatgtaaaat 240
   aaaaatttca tatttaggat tagctttttt aactttattt tgtaactttt ttttcttttt 300
   ttgaggtgtg tgttatgtga ccaaggctgg cttagagctc acagtgcccc cttttacttt 360
   cagtgctgtg gttatggatg aaccccagac atagactatc acacctgact taataatttg 420
   taaatatttg gggcccatga ttttattttt taagataggt ctcaaggagg ctggagagat 480
   ggctcagagg ttaagaccat tggttgctct tccagaggac catggtttaa ttcccagcac 540
   ccacatggta gctcacacct gtctgtaact ccagttccag ggcttctgac accctcacac 600
   agacattcat gcaggcaaaa tatcaatgaa cattttaaaa ggatgggtct cacaccgnaa 660
   tgaagacact ggcttgaaac tcatcatgta gtctagatct ggcacagttc accctctcag 720
   gtgctgggat tgcagtgccc cacatctggc tctagggatt ggattgtttt agggcctatg 780
   gtgttcacag ggcctaggtt atttgtgtta aaaaaaaaaa aaaaaaaaaa gcggccgctg 840
   aattctagac ctgcccgggc ggccgctcga gccctatagt gagtcgtatt aggaaggggt 900
   tatcacttga cctgtctaat ccc 923
<210> 17
   <211> 741
   <212> DNA
   <213> Rattus norvegicus
<400> 17
   cttaaattaa aaaatcatat aaatgaaaag agggatcctc agattccttt gaataggtgg 60
   aggaatatct aatttccctg tgtgcagaag aggaattttt gctgtccctc caggaaaagg 120
   tgatagattt ttgatgtttt aaagtataag ggtgaaatag gatctcccct ggactgtatg 180
   gaatgtgaat agcttctctt cctcttatgt ctggagtact attgagcaga agttgaaggt 240
   tgattgatac cttggaaatt atagaacatt ccagccacac actacttcag caactccaaa 300
   ctgaaaatca cagtaaagaa tacmccctga atatccatgg taggcagtca acagtcttag 360
   aagtcttgta cgaggctcta atgcagtagt cttaggttca gtatcttatt caaatatatg 420
   tccatctaca cattcctctg catgtgaaag gacacctctg tgtaagggaa cacaatgaag 480
   atagtagtct gatttgtgct ggcttttaga ctcttgtttg agaactctgt ccagatttct 540
   ctgagatctc ttcgtcagaa cagtgkwtct cagrmcaata aacttakawa ttktagagag 600
   gaaatgsatt tkgcatatgg ggraatstgt sgtaattcct tggccttctg gagtcctcct 660
   aattgtaaaa gtctaaatct tacttggtaa gatggtggag gtagggaact tgcacagaaa 720
   gtaggagtgt tgtgtcagat c 741
<210> 18
   <211> 109
   <212> DNA
   <213> Rattus norvegicus
<400> 18
   tggattggtc agaacaaagc agttaaagtc tacagattag tgactcgtaa ctcctatgan 60
   aganagatgt ttgaccgagc cagcttgaaa ctgggcacaa aaaaaaaaa 109
<210> 19
   <211> 531
   <212> DNA
   <213> Rattus norvegicus
<400> 19
   tcataggtgg caanccatat cctcattaaa tncaataatc caggtggcag naggggtcag 60
   tggatgagat gtttgctgcc aagttttgca gcntgagttt agtcctcagg gcccacacga 120
   gaggagagca ctctctctaa aggtgtcctc tgacctctgt ctgtgatcct tgtcacatcc 180
   ttgtccacac acattcacac agaaaaataa atgcattgat taacttaatc taaatcataa 240
   tgacccataa ggaatctgta agtacagtct taaaatctaa tttactgcca attgtggcat 300
   tattcctcct tcaaatcaaa aaattcccat gtactttctt gctctctcaa gttcactatc 360
   aatgaatttt tggaagatcg tctataactt ttccttttct gtaggctcaa gctcgttgcc 420
   acwggattgg tcagaacaaa gcagttaaag tctacagatt agtgactcgt aactcctatg 480
   agagagagat gtttgaccga gccagcttga aactgggcac aaaaaaaaaa a          531
<210> 20
   <211> 829
   <212> DNA
   <213> Rattus norvegicus
<400> 20
   gttttattca tcccccagtg ttctctgttg tcttcctccc tcgttggccc ctttcttctt 60
   ccaaccagac ccctctatct ttagttcttt ctgttttgtt tttagtgata caataaattt 120
   aatgggtgag gagtgatcta caggatcata agcaacttat cagtagctat accagagaag 180
   aaaatgtttc ctcctccctc agcaacctta acttgcttat aatcttcagg caggagtgga 240
   gcctctgtga attgcttcaa gcagcttggt ttgcctgtag atccttgggg agggctgcag 300
   ccatattagc tcctccccct tccttgagta aatgttaatg gggtctgttg tatgtaatga 360
   gtcttctgca ctaactgtac ccactctgca ctcaaaagtg catcaccatg tcaagcacag 420
   cagacagtgt ctgcaccact gtgttctgtc cagagttctt agctgcgagc acagcatcaa 480
   tatctgcaga agttactcat tttatctctc cattgcactg tacatgccag gccaggtctg 540
   tgccgctgag ctatgctcct agcccatcta ctcccttgag atggaggcct taccaggttg 600
   gcctckaggc tgagcttctg tctcagcctc ttgagtggtt acggcctcag gtctacatcc 660
   acatggccag catttctaac tgactagctg tgcccttgag gtgagcagaa tgggcgagtg 720
   ctgaggagat agactgcatg tcctgtggac tccaggaaag ggttggcagg aactcggaat 780
   agaccanaaa gctccacagc ctcctgctcc cacctctcac agtgtaatg            829
<210> 21
   <211> 1508
   <212> DNA
   <213> Rattus norvegicus
<400> 21
   aatttgtatg atttaatgat tttaagcaca agtacntcga gcacatttaa attttagcaa 60
   tatgaacatc tgactttcag cacattaaat gtccctttca tggggtcttc aggttcaatt 120
   ggtaaanatt ccttaatttt gtaaaaaatg taattaaagc aggcactaat catgttttga 180
   ggcttttgcg gtgaaattta ggatgcaaaa gcctgtttac ttacttacta tcccatcatg 240
   aaagcccatt ggcacaaggc ttagaattct taagaggctg gatgggaaaa ctgctcacaa 300
   tcctgagccc cctaggaagg ggtcaggtga gtaacattca atacctcctc ttaacttaag 360
   acagctatat ttatccctac catgcaatac tgcagattca agttaaactt atctgttaac 420
   agcagtctgc tgtttcctgc tccgagatga aatgaagcaa ctcttctgct tatgaagaga 480
   aacctgtctg aggcaaacga aagattggca tactgcaccg cctctcaagc cactcgatcc 540
   cactcctctc agtaagctca gcttcttttt actccaggat taatgtagaa tttttacttc 600
   attttattct gcttttgtaa aagcagttga gatgaacgtt tgatcttcac tggttttcat 660
   caattcttta cttttgtgga taaagcttgg taagccatta acttttaaag gtagatttcc 720
   ataactttta aaggtagatt tccataactt tatactggtt gctctcattt gcttaattat 780
   tgcatatgca gacttcatca gcagtaagac tctcaggctc ctgctcattc cccatggaga 840
   catgacatta tcttttctgc ctttacttat cactcattca ccaaggagct gttccctctg 900
   catctaggtc atcacactgc caggctggtt atgactcaga tggtgttatc tgaaaaaggt 960
   ctacagaaaa aaaaggtttc ccctccctca tcaacaaaag cccaccctnt aaaagacatt 1020
   caggctgaac taccacaatt cttacattat aaattgcaaa tacaaagtat acaatataca 1080
   aaaaataagc ataccttaca tcttgtttag tcacaaggaa actcatttct cctacactag 1140
   gaaagacttg cttatacaaa actgagatgg aatctcttag ttgataatat aaaagctatc 1200
   accagaagaa tcccttcagk gtyakycwwg ccacttcctt tgctctgaag ttcctatagt 1260
   agttattaag anacattaag tcacctgaaa aaaaatttca aaagagaacc acacactaca 1320
   atntatatcc ggtagcttnt agagaatccc cacactgtaa cttccacacc actttagtga 1380
   tcagaccaac ccccaaaccc tacacacttt cacgggaaca gacgactgtt gcgatttact 1440
   ttccttgccc acaatattgg attagtggga acacccaagg caatcttcct tactgcccaa 1500
   aaaaaaaa 1508
<210> 22
   <211> 4662
   <212> DNA
   <213> Homo sapiens
<400> 22
   gccggggagg cattgaggca gccagggcag gggcttctgc tgagggggca ggcggagctt 60
   gaggaaaccg cagataagtt ttttttctct ttgaaagata gagattaata caactactta 120
   aaaaatatag tcaataggtt actaagatat tgcttagcgt taagttttta acgtaatttt 180
   aatagcttaa gattttaaga gaaaatatga agacttagaa gagtagcatg aggaaggaaa 240
   agataaaagg tttctaaaac atgacggagg ttgagatgaa gcttcttcat ggagtaaaaa 300
   atgtatttaa aagaaaattg agagaaagga ctacagagcc ccgaattaat accaatagaa 360
   gggcaatgct tttagattaa aatgaaggtg acttaaacag cttaaagttt agtttaaaag 420
   ttgtaggtga ttaaaataat ttgaaggcga tcttttaaaa agagattaaa ccgaaggtga 480
   ttaaaagacc ttgaaatcca tgacgcaggg agaattgcgt catttaaagc ctagttaacg 540
   catttactaa acgcagacga aaatggaaag attaattggg agtggtagga tgaaacaatt 600
   tggagaagat agaagtttga agtggaaaac tggaagacag aagtacggga aggcgaagaa 660
   aagaatagag aagataggga aattagaaga taaaaacata cttttagaag aaaaaagata 720
   aatttaaacc tgaaaagtag gaagcagaag aaaaaagaca agctaggaaa caaaaagcta 780
   agggcaaaat gtacaaactt agaagaaaat tggaagatag aaacaagata gaaaatgaaa 840
   atattgtcaa gagtttcaga tagaaaatga aaaacaagct aagacaagta ttggagaagt 900
   atagaagata gaaaattata aagccaaaaa ttggataaaa tagcactgaa aaaatgagga 960
   aattattggt aaccaattta ttttaaaagc ccatgcaatt taatttctgg tggtgcagaa 1020
   gttagaaggt aaagcttgag aagatgaggg tgtttacgta gaccagaacc aatttagaag 1080
   aatacttgaa gctagaaggg gaagttggtt aaaaatcaca tcaaaaagct actaaaagga 1140
   ctggtgtaat ttaaaaaaaa actaaggcag aaggcttttg gaagagttag aagaatttgg 1200
   aaggccttaa atatagtagc ttagtttgaa aaatgtgaag gactttcgta acggaagtaa 1260
   ttcaagatca agagtaatta ccaacttaat gtttttgcat tggactttga gttaagatta 1320
   tttttttaaa tcctgaggac tagcattaat tgacagctga cccaggtgct acacagaagt 1380
   ggattcagtg aatctaggaa gacagcagca gacaggattc caggaaccag tgtttgatga 1440
   agctaggact gaggagcaag cgagcaagca gcagttcgtg gtgaagatag gaaaagagtc 1500
   caggagccag tgcgatttgg tgaaggaagc taggaagaag gaaggagcgc taacgatttg 1560
   gtggtgaagc taggaaaaag gattccagga aggagcgagt gcaatttggt gatgaaggta 1620
   gcaggcggct tggcttggca accacacgga ggaggcgagc aggcgttgtg cgtagaggat 1680
   cctagaccag catgccagtg tgccaaggcc acagggaaag cgagtggttg gtaaaaatcc 1740
   gtgaggtcgg caatatgttg tttttctgga acttacttat ggtaaccttt tatttatttt 1800
   ctaatataat gggggagttt cgtactgagg tgtaaaggga tttatatggg gacgtaggcc 1860
   gatttccggg tgttgtaggt ttctcttttt caggcttata ctcatgaatc ttgtctgaag 1920
   cttttgaggg cagactgcca agtcctggag aaatagtaga tggcaagttt gtgggttttt 1980
   tttttttaca cgaatttgag gaaaaccaaa tgaatttgat agccaaattg agacaatttc 2040
   agcaaatctg taagcagttt gtatgtttag ttggggtaat gaagtatttc agttttgtga 2100
   atagatgacc tgtttttact tcctcaccct gaattcgttt tgtaaatgta gagtttggat 2160
   gtgtaactga ggcggggggg agttttcagt attttttttt gtgggggtgg gggcaaaata 2220
   tgttttcagt tctttttccc ttaggtctgt ctagaatcct aaaggcaaat gactcaaggt 2280
   gtaacagaaa acaagaaaat ccaatatcag gataatcaga ccaccacagg tttacagttt 2340
   atagaaacta gagcagttct cacgttgagg tctgtggaag agatgtccat tggagaaatg 2400
   gctggtagtt actctttttt ccccccaccc ccttaatcag actttaaaag tgcttaaccc 2460
   cttaaacttg ttatttttta cttgaagcat tttgggatgg tcttaacagg gaagagagag 2520
   ggtgggggag aaaatgtttt tttctaagat tttccacaga tgctatagta ctattgacaa 2580
   actgggttag agaaggagtg taccgctgtg ctgttggcac gaacaccttc agggactgga 2640
   gctgctttta tccttggaag agtattccca gttgaagctg aaaagtacag cacagtgcag 2700
   ctttggttca tattcagtca tctcaggaga acttcagaag agcttgagta ggccaaatgt 2760
   tgaagttaag ttttccaata atgtgacttc ttaaaagttt tattaaaggg gaggggcaaa 2820
   tattggcaat tagttggcag tggcctgtta cggttgggat tggtggggtg ggtttaggta 2880
   attgtttagt ttatgattgc agataaactc atgccagaga acttaaagtc ttagaatgga 2940
   aaaagtaaag aaatatcaac ttccaagttg gcaagtaact cccaatgatt tagttttttt 3000
   ccccccagtt tgaattggga agctggggga agttaaatat gagccactgg gtgtaccagt 3060
   gcattaattt gggcaaggaa agtgtcataa tttgatactg tatctgtttt ccttcaaagt 3120
   atagagcttt tggggaagga aagtattgaa ctgggggttg gtctggccta ctgggctgac 3180
   attaactaca attatgggaa atgcaaaagt tgtttggata tggtagtgtg tggttctctt 3240
   ttggaatttt tttcaggtga tttaataata atttaaaact actatagaaa ctgcagagca 3300
   aaggaagtgg cttaatgatc ctgaagggat ttcttctgat ggtagctttt gtattatcaa 3360
   gtaagattct attttcagtt gtgtgtaagc aagttttttt ttagtgtagg agaaatactt 3420
   ttccattgtt taactgcaaa acaagatgtt aaggtatgct tcaaaaattt tgtaaattgt 3480
   ttattttaaa cttatctgtt tgtaaattgt aactgattaa gaattgtgat agttcagctt 3540
   gaatgtctct tagagggtgg gcttttgttg atgagggagg ggaaactttt tttttttcta 3600
   tagacttttt tcagataaca tcttctgagt cataaccagc ctggcagtat gatggcctag 3660
   atgcagagaa aacagctcct tggtgaattg ataagtaaag gcagaaaaga ttatatgtca 3720
   tacctccatt ggggaataag cataaccctg agattcttac tactgatgag aacattatct 3780
   gcatatgcca aaaaaatttt aagcaaatga aagctaccaa tttaaagtta cggaatctac 3840
   cattttaaag ttaattgctt gtcaagctat aaccacaaaa ataatgaatt gatgagaaat 3900
   acaatgaaga ggcaatgtcc atctcaaaat actgctttta caaaagcaga ataaaagcga 3960
   aaagaaatga aaatgttaca ctacattaat cctggaataa aagaagccga aataaatgag 4020
   agatgagttg ggatcaagtg gattgaggag gctgtgctgt gtgccaatgt ttcgtttgcc 4080
   tcagacaggt atctcttcgt tatcagaaga gttgcttcat ttcatctggg agcagaaaac 4140
   agcaggcagc tgttaacaga taagtttaac ttgcatctgc agtattgcat gttagggata 4200
   agtgcttatt tttaagagct gtggagttct taaatatcaa ccatggcact ttctcctgac 4260
   cccttcccta ggggatttca ggattgagaa atttttccat cgagcctttt taaaattgta 4320
   ggacttgttc ctgtgggctt cagtgatggg atagtacact tcactcagag gcatttgcat 4380
   ctttaaataa tttcttaaaa gcctctaaag tgatcagtgc cttgatgcca actaaggaaa 4440
   tttgtttagc attgaatctc tgaaggctct atgaaaggaa tagcatgatg tgctgttaga 4500
   atcagatgtt actgctaaaa tttacatgtt gtgatgtaaa ttgtgtagaa aaccattaaa 4560
   tcattcaaaa taataaacta tttttattag agaatgtata cttttagaaa gctgtctcct 4620
   tatttaaata aaatagtgtt tgtctgtaaa aaaaaaaaaa aa 4662
<210> 23
   <211> 2210
   <212> DNA
   <213> Mus musculus
<400> 23
   aacttatgga tcaggccaaa ttggccgagt tcgaattttt tgagttaatt aaattcatcc 60
   cccccccccc ccgaggcatt caggcagcga gagcagagca gcgtagagca gcacagctga 120
   gctcgtgagg caggagactc agcccgagga aatcgcagat aagtttttaa ttaaaaagat 180
   tgagcagtaa aaagaattag aactctaaac ttaagctaat agagtagctt atcgaaatat 240
   tacttagtct taataatcta agaagatctt aagagataac atgaaggctt atttaaacag 300
   tttgaaaaag gaaatgagga gaaaagtatt tgtactgtat aatggaggct gaccagagca 360
   gtttaggaga ttgtaaaggg aggttttgtg aagttctaaa aggttctagt ttgaaggtcg 420
   gccttgtaga ttaaaacgaa ggttacctaa atagaatcta agtggcattt aaaacagtaa 480
   agttgtagag aatagtttga aaatgaggtg tagttttaaa agattgagaa aagtaggtta 540
   agttgacggc cgttataaaa atccttcgac tggcgcatgt acgtttgaag gcatgagttg 600
   gaaacaggga agatggaagt gttaggctag ccgggcgatg gtggcgcacg cctttaatcc 660
   tagcacttgg gaggcagagg caggcggatt tctgagttcg aggccagcct ggtctacaga 720
   gtgagttcca ggacagccag ggctacacag agaaaccctg tcttgaaaaa acaaaaaggt 780
   taggctagta tttggagaaa gaagattaga aaatggaagt gaaagacgaa gaagacatac 840
   aggaaggtga agaaaaagct gttagagaag ataggaaaat agaagacaaa gcatctttag 900
   aagacagaaa aggtacttaa aggcacaggt agtaggaagc cgaagaatag aagatagaaa 960
   gaagcaagat agaaaaacaa aatggaagtt aagacaactt tggatgccag cattcaagat 1020
   aggcaaagaa gataagattg aggccaaaag gttggataag atataaagtc agaaggaaat 1080
   tatctttaaa gccataagtt caaatttctg atggagcgag cagtttagaa gagtctttag 1140
   acagccacat acaagattga agctagcaat caaagctact aggactgaag taaaaagtta 1200
   aggcagaatg cctttgaaga gttagaagaa tattaaaagc cttaacttgt agcttaattt 1260
   tgcttgatga caaaaggact tttgataaca gtttcaagat tgtcagcatt ttgcattgga 1320
   cttgagctga ggtgctttta aaatcctaac gactagcatt ggcagctgac ccaggtctac 1380
   acagaagtgc attcagtgaa ctaggaagac aggagcggca gacaggagtc ccgaagccag 1440
   tttggtgaag ctaggaagga ctgaggagcc agcagcagca gtgcatggtg aagatagccc 1500
   aggaaagagt gcggttcggt ggaggaagct aggaagaagg agccatacgg atgtggtggt 1560
   gaagctggga aagggttcca ggatggtgga gcgagagcga gttggtgatg aagctagctg 1620
   gcggcttggc ttgtcaactg cgcggaggag gcgagcaggc attgtggaga ggatagatag 1680
   cggctcctag accagcatgc cagtgtgcaa gaaaggctgc agggagagca tgcggtgcgg 1740
   taacattcct tgaggtcggc aacatggtgg tggttttctg taacttggat ggtaacttgt 1800
   ttactttgtc ttaatagtta tgggggagtt gtaggcttct gtgtaaagag atatatctgg 1860
   ggctgtatgt aggcctttgc gggtgttgta ggtttttctt tttcagggtt atgtcctctt 1920
   gcatcttgtc agaagctttt gagggctgac tgccaaggcc cagaaagaag aatggtagat 1980
   ggcaagttgt ctttaaccgc tcagagggga atgaatggta gagccagcac aacctcccag 2040
   ttttgtaaga cgttgtagtt tgaacagatg acctaccaca agcctcactc ctgtgtaggg 2100
   gaggtaattg ggcaaagtgc ttttggggga atgggggcaa aatatatttt gagttctttt 2160
   ccccttaggt ctgtctagaa tcctaaaggc agatgactca agggaaccag 2210
<210> 24
   <211> 2168
   <212> DNA
   <213> Mus musculus
<400> 24
   ggaaaaaagg aaatccactc tcaggataag cagagctcgc caggtttaca gtttgtagga 60
   agtagaggat ggatgctagc tttcacactg agtgtggagg agctggccat ggcggaattg 120
   ctggtagttt actctttccc cctcccttaa tgagatttgt aaaatcctaa acacttttac 180
   ttgaaatatt tgggagtggt cttaacaggg aggagtgggt gggggaaacg ttttttttct 240
   aagattttcc acagatgcta tagttgtgtt gacacactgg gttagagaag gcgtgtactg 300
   ctatgctgtt ggcacgacac cttcagggac tggagctgcc ttttgtcctt ggaagagttt 360
   tcccagttgc cgctgaagtc agcacagtgc ggctttggtt cacagtcacc tcaggagaac 420
   ctcaggagct tggctaggcc agaggttgaa gttaagtttt acagcaccgt gatttaaaat 480
   atttcattaa aggggagggg taaaacttag ttggctgtgg cgttgtgttt gggtgggtgg 540
   gggtgttagg taattgttta gtttatgatt tcagataatc ataccagaga acttaaatat 600
   ttggaaaaac aggaaatctc agctttcaag ttggcaagta actcccaatc cagtttttgc 660
   ttcttttttc ctttttcttt ttttgaggcg ggcagctaag gaaggttggt tcctctgccg 720
   gtccctcgaa agcgtagggc ttgggggttg gtctggtcca ctgggatgat gtgatgctac 780
   agtggggact cttctgaagc tgttggatga atatagattg tagtgtgtgg ttctcttttg 840
   aaattttttt caggtgactt aatgtatctt aataactact ataggaacaa aggaagtggc 900
   tttaatgacc ctgaaggaat ttcttctggt gatagctttt atattatcaa gtaagagata 960
   ctatctcagt tttgtataag caagtctttt tcctagtgta ggagaaatga ttttccttgt 1020
   gactaaacaa gatgtaaagg tatgcttttt ttcttcttgt gcattgtata cttgtgttta 1080
   tttgtaactt ataatttaag aattatgata attcagcctg aatgtctttt agagggtggg 1140
   cttttgttga tgagggaggg gaaacctttt tttttctgta gacctttttc agataacacc 1200
   atctgagtca taaccagcct ggcagtgtga tgacgtagat gcagagggag cagctccttg 1260
   gtgaatgagt gataagtaaa ggcagaaaaa ataatgtcat gtctccatgg ggaatgagca 1320
   tgagccagag attgttccta ctgatgaaaa gctgcatatg caaaaattta agcaaatgaa 1380
   agcaaccagt ataaagttat ggcaatacct ttaaaagtta tggcttatct accaagcttt 1440
   atccacaaaa gtaaagaatt gatgaaaaac agtgaagatc aaatgttcat ctcaaaactg 1500
   cttttacaaa agcagaatag aaatgaagtg aaaatgctgc attaagcctg gagtaaaaag 1560
   aagctgagct tgttgagatg agtgggatcg agcggctgcg aggcggtgca gtgtgccaat 1620
   gtttcgtttg cctcagacag gtttctcttc ataagcagaa gagttgcttc attccatctc 1680
   ggagcaggaa acagcagact gctgttgaca gataagtgta acttggatct gcagtattgc 1740
   atgttaggga tagataagtg ccttttttct ctttttccaa aaagacctgt agagctgttg 1800
   aatgtttgca gctggcccct cttaggcagt tcagaatttt gagtagtttt cccatccagc 1860
   ctcttaaaaa ttcctaagcc ttgcaccgat gggctttcat gatgggatag ctaataggct 1920
   tttgcatcgt aaacttcaac acaaaagcct acatgattaa tgcctacttt aattacattg 1980
   cttacaagat taaggaatct ttatcttgaa gaccccatga aagggatcat tatgtgctga 2040
   aaattagatg ttcatattgc taaaatttaa atgtgctcca atgtacttgt gcttaaaatc 2100
   attaaattat acaaattaat aaaatacttc actagagaat gtatgtattt agaagcaagg 2160
   atccgaat 2168
<210> 25
   <211> 1995
   <212> DNA
   <213> Rattus norvegicus
<400> 25
   agcgagcaca ggcgcctggt gaggcaggag actcagcctg aggcattcgc agataagttt 60
   ctatttaaaa agatttaaaa ggaaactaga actctaaact taatagagta gcttactgag 120
   ataataatat tttaatatta agattttaaa aggaaagtat ggaggtttat ttaaacaatt 180
   tgaaaaagga aaagagaaaa atttttgtac caccagatgg aggctgacct gaggcgttta 240
   ttcagagtag gttagaagat tgtaaaaggg agcaattgtg gagtttcaaa ggttctagct 300
   tgagggtcag ccttgttagg tgtactttta aacgaaggtt acctaaacag aatctaaatg 360
   gctttttaaa attgtaaagt tgtagataat agtttgaaaa tgagatgtag tttaaaaaga 420
   ttgaaaaaag taagttgaag ggccgttata agagtcgact gtcgcatgta cgaaggcatg 480
   agttggaaac ggggaatgga agtggtaggg ttagtatttt gagaaagaag attagaaaat 540
   ggaagtggaa aaatgaagac aagttcagga aggtgaagaa aagctgtaga gaagatagga 600
   aaatagaaga ccgaagaatc attagaagac agaaaaggta tttaaacaca caggtaggaa 660
   gaaaactaga agatagaaaa aaagcaagat agaaagacaa aaggaaaaaa aaaagaacaa 720
   aaaaagcaag atagaaagac aaaaggaagt tcagacaact ttggatgcca gcattcaaga 780
   taggcaaaga agataagatc aagaccaaaa ggttggataa ggttaaatct cagaaataaa 840
   gtcagaagga aattattttt aaagccataa attcaagttt ctgatggagc ccagcagttt 900
   agaagtgtct gtagacagcc attttacaag agtgaagcta gcagtcaaag ctacaactga 960
   agtaattaaa aaggcagaat gcgtttgaaa agttagaaga atattagaag ccttaacttg 1020
   tagcttaatt ttgttttgag aacaaaagga cttttgataa caatttcaag attgtcagca 1080
   ttttgcattg gacttgagct gaggtgcttt aaaagtgtta acgactagca ttggcagccg 1140
   accaaggtct acacagaagt ggattcagtg aacctagaaa gacaggagtc ccgaagccag 1200
   tttggtgaag ctaggaagga ctgagcagcc agcagcagca ggtcatggtg aagatagccc 1260
   aggaaacagt gcggttcgtt ggaggaagct aggaagaagg agccgaaatg atgtggtgaa 1320
   gctagaaaag aggattccgg gaaggtggag ccagttcgag ttggtgatga agctagctgg 1380
   cgtcttggct tgtcaacgcg gaggaggcga gcaggcattg cgttgtggag agaatagata 1440
   gcggctccta gaccagcatg tcaatgtgca agaaaggctg cagggaaagc atgtggtgcg 1500
   gtaacatccc gtgaggtcgg caacgcggtg gtggtttctg taacttgagg atggtaactt 1560
   gtttattttg ccttggtatg gggagtttgg taggcttctg tctgtgaaga gagtcatctg 1620
   gggctgtagg ccgctgcggg tgttggaggt ttttcttttt cagggttgtg tcctcttgca 1680
   tcttgtctga agcttttgag ggcagactgc caaggcccag aagaatggta gatggcaagt 1740
   tgtcttaacc cgatctgagg gaaaatgaat ggtagagcca acagtctcgc cagttttgta 1800
   agacgtttta gttctgtgaa cagctaacct actgcagtct tctgcaagtc ttactactgt 1860
   gtaggggagg taattgggca aagtgcttgg ggggtggggt gggacaaaat atattttgag 1920
   ttcttttccc cttaggtctg tctagaatcc taaaggcaga tgactcaagg gaaccaggaa 1980
   aaaaaaaaaa aaaaa                                                  1995
<210> 26
   <211> 1996
   <212> DNA
   <213> Rattus norvegicus
<400> 26
   gaagattttc cacagatgct atagttctgt tggcacactg ggttagagaa ggcgtgtact 60
   gctatgctgt tggcacgaca ccttcaggga ctggagctgc cctttatcct tggaagagtt 120
   tccccagtgg ccgctgaagt acagcacagt gcagctttgg ttcacagtca cctcaggaga 180
   acctcaggag cttgagtagg ccagatgtta aagttaagtt ttnnnnnnnn nnnnnnnnnn 240
   nnnnnnnnnn nnnnnnnnnn nnnnnnnnnn nnnnnnnnnn nnnnnnnnnn nnnnnnnnnn 300
   nnnnnnnnnn nnnnnnnnnn nnnnnnnnnn nnnnnnnnnn nnnnnnnnnn nnnnnnnnnn 360
   nnnnnnnnnn nnnnnnnnnn nnnnnnnnnn nnnnnnnnnn nnnnnnnnnn nnnnnnnnnn 420
   nnnnnnnnnn nnnnnnnnnn nnnnnnnnnn nnnnnnnnnn nnnnnnnnnn nnnnnnnnnn 480
   nnnnnnnnnn nnnnnnnnnn nnnnnnnnnn nnnnnnnnnn nnnnnnnnnn nnnnnnnnnn 540
   nnnnnnnnnn nnnnnnnnnn nnnnnnnnnn nnnnnnnnat tgtagtgtgt ggttctcttt 600
   tgaaattttt tttcaggtga cttaatgtnt cttaataact actataggaa cttcagagca 660
   aaggaagtgg cwwgrmtgac cctgaaggga tttcttctgg tgatagcttt tatattatca 720
   agtaagagat tccatctcag ttttgtataa gcaagtcttt cctagtgtag gagaaatgag 780
   tttccttgtg actaaacaag atgtaaggta tgcttatttt ttgtatattg tatactttgt 840
   atttgcaatt tataatgtaa gaattgtggt agttcagcct gaatgtcttt tanagggtgg 900
   gcttttgttg atgagggagg ggaaaccttt tttttctgta gacctttttc agataacacc 960
   atctgagtca taaccagcct ggcagtgtga tgacctagat gcagagggaa cagctccttg 1020
   gtgaatgagt gataagtaaa ggcagaaaag ataatgtcat gtctccatgg ggaatgagca 1080
   ggagcctgag agtcttactg ctgatgaagt ctgcatatgc aataattaag caaatgagag 1140
   caaccagtat aaagttatgg aaatctacct ttaaaagtta tggaaatcta cctttaaaag 1200
   ttaatggctt accaagcttt atccacaaaa gtaaagaatt gatgaaaacc agtgaagatc 1260
   aaacgttcat ctcaactgct tttacaaaag cagaataaaa tgaagtaaaa attctacatt 1320
   aatcctggag taaaaagaag ctgagcttac tgagaggagt gggatcgagt ggcttgagag 1380
   gcggtgcagt atgccaatct ttcgtttgcc tcagacaggt ttctcttcat aagcagaaga 1440
   gttgcttcat ttcatctcgg agcaggaaac agcagactgc tgttaacaga taagtttaac 1500
   ttgaatctgc agtattgcat ggtagggata aatatagctg tcttaagtta agaggaggta 1560
   ttgaatgtta ctcacctgac cccttcctag ggggctcagg attgtgagca gttttcccat 1620
   ccagcctctt aagaattcta agccttgtgc caatgggctt tcatgatggg atagtaagta 1680
   agtaaacagg cttttgcatc ctaaatttca ccgcaaaagc ctcaaaacat gattagtgcc 1740
   tgctttaatt acatttttta caaaattaag gaatctttac caattgaacc tgaagacccc 1800
   atgaaaggga catttaatgt gctgaaagtc agatgttcat attgctaaaa tttaaatgtg 1860
   ctcgatgtac ttgtgcttaa aatcattaaa tcatacaaat taataaaata cttcactaga 1920
   gaatgtatgt atttagaagg ctgtctcctt atttaaataa agttttgttt gttgtctgta 1980
   aaaaaaaaaa aaaaaa                                                1996
<210> 27
   <211> 1339
   <212> DNA
   <213> Rattus norvegicus
<400> 27
   tttttttttt tttttttttt atatacttat atattatata tttgtgttat tatgttatat 60
   aataataaat taattacata tattatatat attaacaatc aattacaaag acaggtatat 120
   aggaggactt ggaagacaga aagggaaagg gaaaataata tatctcaraa aataaaaaat 180
   aaaaatttta aactatataa atgaggggag tgatgcctaa tatggagtct ttwgtcgctg 240
   ggcgatgcgt tggaccctcc tgtgagacct cagcctcctc ctcctttctt tgctccattg 300
   gccactacaa gcccagagct gcaatgccaa ccaagatntc caagactgcg agccaacata 360
   cacttttcct ctttgcaaac tggcttcata aggtgacaga aaactgatgg atactttcta 420
   agtctcagta agaatgaaga aaatggtgag cagggccaag cacctgccta gcatgcgtgg 480
   ggttccaagt tcaagccctg gcacagcaag aaaatcgaag cacagtttct gatctgcctt 540
   gcagcaatga gaccctcayt tcaaggcctt gccctgtagc cttaagagaa gaacaatgaa 600
   ggatccacca tggcccagca tggacgttgt tcagtcacat tttcactgat gttcatggtt 660
   tcttcatacc catgtaatga gaccttcata aagtcaaatg gggcagtgta tagagaccat 720
   ctaggggttc ctgatggggt tctcaaagag agcgtgagag tgcagcaacc cttctcccag 780
   accttaactg ctttatctgt atcctttaaa ataaaagttg tagttctcgg ttgtgtgaac 840
   catttcggca aactgacgga acccgagtgg gcgagagcag tggagatcag ttatttgcaa 900
   gaacttgagc tttcctatgc ccgcctttgc tttgaataat gacatggaga ccctttatat 960
   ttataataaa ttccaggcag taagatgggc aaactctgag ctactctaac ccgactgctg 1020
   ccctggcctg cttcccagcg gtgtagccct tttcctgaca tctcagcctc tctctggcac 1080
   ctcctccttc atttgtgtcc tgacagagat ggtgactccc tggatctcat gtaagaccct 1140
   ctctctcctc atccaggcat ggctgtccgg ccttatgctc tcttgggagc tataggctat 1200
   tcagctcttt atataaccaa tcagaaggcg atggggaaca atgctttaca aaatactgag 1260
   tcataccaag gtccagactg catccaggtc tctgggtaca ggaatcagcc tatgaataca 1320
   ccatgcacaa aaaaaaaaa                                              1339
<210> 28
   <211> 177
   <212> DNA
   <213> Rattus norvegicus
<400> 28
   ggaaccaatt tcatagtaac acaaacttnt cgataacctt aaacgtgaca tttgtgctag 60
   tgttttacac tatgggtgat aaaagtttag aatctcagat ttcatcttta aagaactaaa 120
   agcaaccctt gcttcaagtg tcacttctgc aattgtacgg accacaaaaa aaaaaaa    177
<210> 29
   <211> 1102
   <212> DNA
   <213> Rattus norvegicus
<400> 29
   ctgacagtaa ttctttaaat ggtgtaattt attcatttaa agcatgtcaa gataaattaa 60
   aagaacattt gtctgaaaat gctgccagga gcctattgtg taactgtagg tattttgtat 120
   aataatcctg aattgtaaat tgacccatgt ttggtaagat tgtgtcagaa ttgantngta 180
   gagttttttt ttttttngaa cngcaataat taattccatg attgccacat tcngtcacca 240
   tgagatgtgt tattaccata aggctgaaat aatcttagga ggtggatttg atggtggggt 300
   ggcagctaca tgtttntttc ccaaccatgc ttgacagtta aagtatttgt gttttgaata 360
   cataaaccct ttgatanttc ntagccggaa atcagtttca taaagctatt ttngactata 420
   gtttgtgtaa aataaaagtg ttnggctnng gcaacagctt tccaagntga gctccctttc 480
   gagcaagata tttttcagtg cttttattta ctatgcactt agactatgca ctttttntga 540
   aatatttttg taacactttt ttgtattttt gccatttgaa anggctgtgg tgtcattggt 600
   ctctcattaa gntgcaggtt cgaaaccgct gttagctttg tgaatcngaa tctagagatg 660
   tgtccgtcct gtgatgttgt cgtcccatct tcacctggag ctggaatcca ttgatnttct 720
   agctaccatt cattttcttc acagttcaca aagaagagtg tgaaattcag tgaatgcagt 780
   tactaacctg tctggaaatg aatctcattt caccaaatca attgtttttc cactaaaatg 840
   atggtgtatg ttgaagacat cactgagatc ggaagacctc accactaatg gctccacagt 900
   ggctggctca gctgaacttt aggtttccac ttcttccttt gttcacccat tgggtgatgc 960
   agctttttaa ctgttgggag atgaccattn taactactgt tgagtgtttc tgttttggga 1020
   aggtaaaata agaaaataaa aaggaatata tatgaaggga gaggctggtt gtntntcccg 1080
   tgtggttgta caaaaaaaaa aa                                          1102
<210> 30
   <211> 478
   <212> DNA
   <213> Rattus norvegicus
<400> 30
   gtcagtatgg aaaaattggt gcaggggtaa aggctaggga aaaacntctc taagcccagc 60
   ctggactgtc ttgtcacctg ctacagcctc ggggacccct aaacacgtag cctacaacac 120
   gtattactta tcacagaagc tttcatccac ggacatacca caggtgtctg tgatttaaaa 180
   actaccattt ttataantgc ccctggaggt gatattttcc tctgtgcaaa gggctctgag 240
   gtctgggtgg gttaagccat gtntttaagg tggggcagct gctcttccac agactggaga 300
   cctaagacca ggtctctttg cttacagtag gtaggcactt tgcaatgata ccgagctgcc 360
   tttctgtggc aactcagtta atgaaataac catgaattaa tcctgtagct tttcgggtct 420
   tatatcaact atctccagtc acacacagac acacacagac acacacakac actcacac   478
<210> 31
   <211> 964
   <212> DNA
   <213> Rattus norvegicus
<400> 31
   gtcagtatgg aaaaattggt gcaggggtaa aggctaggga aaaacntctc taagcccagc 60
   ctggactgtc ttgtcacctg ctacagcctc ggggacccct aaacacgtag cctacaacac 120
   gtattactta tcacagaagc tttcatccac ggacatacca caggtgtctg tgatttaaga 180
   actaccattt ttataagtgc ccctggaggt gatattttcc tctgtgcaaa gggctctgag 240
   gtctgggtgg gttaagccat gtgtttaagg tggggcagct gctcttccac agactggaga 300
   cctaagacca ggtctctttg cttacagtag gtaggcactt tgcaatgata ccgagctgcc 360
   tttctgtggc aactcagtta atgaaataac catgagttag tcctgtagct tttcgggtct 420
   tatatcaact atctccagtc acacacagac acacacagac acacacagac acacacacac 480
   agacacacac actcacatac agacacacac agacacacac actcacacac acacacactc 540
   acacatacac cactacacac aaagacatgt atacatttca cttatgaatg catatttaat 600
   cacacagtgt ccacccttcc cccctctacc cccacacaca accattcacc cagttattca 660
   cacatcggcc cacccatcta tgtatccatc tcccagccca tctatgtatc catctccctg 720
   cccatctatg tatccatctc cctgcccatc tatgtatcca tctccctgcc cgtccctgcc 780
   cactcatctg ctcctccatc cacctgtgcg tctggccacc cactacccat ctagtcatct 840
   accttcccat ccatctaccc acccacctgt tcacataccc atcttcccac tcatcctgca 900
   tctaatanac gctttcagga ttccctctgt gccccaggga cactattaag tcctgtgcat 960
   gtgt 964
<210> 32
   <211> 616
   <212> DNA
   <213> Rattus norvegicus
<400> 32
   tttttttttt tgtggaaaga aaacatcaga aacctttgag gtcccaaatg ccattcatcg 60
   gagggaggga agcacatgcc aacgacaggg ctccatgtac actccgcgtc tctgaaacag 120
   gacatcttcc agcttcctca ccaaagaggt gcgtcctctc agcttgaccc ccaaccccca 180
   ccctggagcc aaatcacaga gagacataga gacagagaca gacagacagg gggagagaga 240
   gagagagaga gagagagaga gagagagaga aacytctttc ccactgttaa cttgatctat 300
   tgggcagagt ctcagagaaa agccaagtca gctgagtttc aggcaaagac tgagaaaatg 360
   tgggcacctg aaggtaaatt aaatatttat aaagacaaca acatcatcac agatagaaat 420
   gctgatttat taataattcc tacwagaagc cagagagagt cactagcgta agtcattgcc 480
   ccatctctta ggcatgacat ggcccattac ttccagatcc aaacagattt gctaacatga 540
   aattggctgg gattgtgart taattaaggt tgggttggta cacatttccc ttgatcttgg 600
   tgggcaatgt kacacn 616
<210> 33
   <211> 945
   <212> DNA
   <213> Rattus norvegicus
<400> 33
   gtccttataa gagttgcctt ggtcatggca tctgttcaca gtagtaaaac cctaantaag 60
   acgactatga tcacacacgt ctccacggtg tggcagatcc tatttccacc gtaactgacc 120
   cctgtctctt ctttctccct gaaggggatg aggacgattg ttggcacagg ggtcagatcc 180
   actatgtcca agatcgagat gttcctggcc tctgggcaga cagcctgctc tactctgaga 240
   ggcttggagc agcactggct gcagcaggag ctgaaaggga gctccacccc catgctctgc 300
   atttccttga cagtccacag cagcaatctc tgtgtcacat tgccaccaag atcaagggaa 360
   atgtgtacca acccaacctt aattaactca caatcccagc caatttcatg ttagcagatc 420
   tgtttggatc tggaagtaat gggccatgtc atgcctaaga gatggggcaa tgacttacgc 480
   tagtgactct ctctggcttc ttgtaggaat tattaataaa tcagcatttc tatctgtgat 540
   gatgttgttg tctttataaa tatttaattt accttcaggt gcccacattt tctcagtctt 600
   tgcctgaaac tcagctgact tggcttttnt ctgagactct gcccaataga tcaagttaac 660
   agtgggaaag aggtttctct ctctctctct ctctctctct ctctctctct ctcccccngt 720
   ctgtctgtct ctgtctcnat gtctctctgt gatttggctc cagggtgggg gttgggggtc 780
   aagctgagag gacgcacctc tttggtgagg aagctggaag atgtcctgtt tcagagacgc 840
   ggagtgtaca tggagccctg tcgttggcat gtgcttccct ccctccgatg aatggcattt 900
   gggacctcaa aggtttctga tgttttcttt ccacaaaaaa aaaaa                 945
<210> 34
   <211> 236
   <212> DNA
   <213> Rattus norvegicus
<400> 34
   ttggtaaagg ggcagaaggg taaagggtan anatgganan atacaattaa tactaaagtc 60
   ctttgaaaaa gctatatgca tattcacaat tgtanaaact tcatttaaaa atatgtatgt 120
   atcaananta caaacatagt ttttctatgc attgaaaaaa atgtctctct tgtattggac 180
   atttgcanaa aaaaaatgtc tggtcanaag tgctgttacg ccacaaaaaa aaaaaa      236
<210> 35
   <211> 1497
   <212> DNA
   <213> Rattus norvegicus
<400> 35
   tctcagcata gctgaacagt cttgaatagt aagtgaactg atttcatgtt gtaatgcgaa 60
   tctgtatatt gggcggggga gaacacgctg cagaactgac agcaacttta atgtctggtc 120
   atgctggatg gggtggctgc atgtagagca ttgcaaatga tccatagcta tcaccctgcc 180
   caaaattcaa ttccagatgg attaagggcc ttaaaataag accatatacc ctgaatctga 240
   tagaaaatag ggaatgggtc tgaacttact ggcaccagaa aggaatttcc ggaaaggaag 300
   aggacacata gccctggtgc taaaacccaa caataaatgg gaccggatga agctaaagtt 360
   tctgtggagc aaaagacacc atgtagtgaa tgtttttcat gattttgcgt tgggatctag 420
   gcatgtgaca tttgaaatgt tccttggtat aattatctgg tcttgtcctt ggtcaagggt 480
   tctttggttg gtactgcccg ctctgggtct gggccagttg tgattcctgg tagaagtgtc 540
   tgtgggcagg caggtgtcac aaaggagttg agatgggctt taaggaaaag ccaaagggag 600
   taatagtact aaggggagcc tagttctgca ccgagacaga atccctggtg gatggtggtc 660
   caaatgagaa ggggggctcc tgtgggcgtg ctgcagcagg actaaaggtg aattagaact 720
   gaccagagtg cagagctctg ccttttggga tgggttcagt gtaaccagtt gcctccaact 780
   ccagccactg tgcatcccct gccatgatga actgccacct ggagctgtga aatcaaaata 840
   aatgcttcct tccctaagtt gcttttgtca ttttgycmsm scamcagaaa wtcatcataa 900
   taatccagtg cntaagtgkg rmcmcatgct gatgggctga gctctcagga aaytwaggcg 960
   ggnaccagct ctgtgggagg catcttaaac catatagtaa ggccttgcct caagccagtg 1020
   gatcggtaga agacttgggg ggtatkkwcc actcctttgg catatacccc aaanggttgc 1080
   aagtttcttt gcacaagaag gtgggttacc tcarctggct tccgttgkcc gsttctarcc 1140
   cacaaaagct tagggaaaat ggggaagcca gncctaartg ggtanctcar cttgatgncc 1200
   ctgcattaat ggaaaatggc atwtmcacac aatgtaatac tgttcagctc taacagtgag 1260
   gttcacaggt aaatgaatga atggaactgg gtaaggcacc atgttctctc ttatttgtgg 1320
   atccgagctc cagacctcca aagtgagtat aaaaccaaag taacctcagg agccatgtgg 1380
   aggaaggagt tcacaagggg gatgatggga caggtgctat gaaggaagag acgggaaaac 1440
   tgggggagag gttagaaacg gaggaaggaa acataacact aagaatgttt gtaaaag 1497
<210> 36
   <211> 2964
   <212> DNA
   <213> Homo sapiens
<400> 36
   ttgggctgtt ttaagtgcaa gagctgtggg aagctcctga atgccgagta catcagcaag 60
   gatgggctgc cctactgcga agctgactat cacgccaagt tcggcatccg ctgtgacagc 120
   tgtgagaaat acatcacggg gcgcgtgctg gaggccggag agaagcacta ccacccttcc 180
   tgcgcgctat gtgtcaggtg cggccagatg tttgcagaag gcgaagagat gtatcttcaa 240
   ggttcctcca tctggcatcc ggcgtgtcga caagcagcca gaactgaaga cagaaacaag 300
   gaaaccagaa cttcctcaga gagcatcatt tctgtccctg cttccagcac ctcagggtct 360
   ccgagccgtg tgatttatgc caagcttggt ggtgagatcc tggactacag ggacttggca 420
   gcccttccta aaagtaaggc catctatgac atcgaccgcc ccgacatgat ctcctactca 480
   ccctacatca gccactctgc aggggacagg cagagctacg gcgagtcccc tcagttgctc 540
   tcgccaacgc cgaccgaggg ggatcaggat gaccggtcct acaagcagtg ccggacctcc 600
   agcccaagct ccactgggtc ggttagcctc gggcgctaca ctccgacctc acggtcacca 660
   cagcactaca gccgtccagc tggtactgtg agtgtgggta ccagtagctg cctctccctg 720
   tcccaacacc caagccctac atccgtgttc agacatcatt acatccccta cttccgaggc 780
   agtgaaagtg gccggagcac ccccagcctc tccgtgctct ctgacagcaa gccgcccccc 840
   tccacctacc agcaggcacc tcgccacttc cacgtcccag ctgccaggcg atcggatggg 900
   gaggatggaa gcttggacca ggataacagg aagaagagca gctggctgat gctcaagggg 960
   gatgcagaca caaggaccaa ttctccagac ctggacaccc agtccttgtc ccacagcagc 1020
   gggaccgaca gagaccctct ccaaaggatg gcaggggaca gctttcactc acatctatcc 1080
   gtatgactcc ctcatcgtca caaaccgaat tcgcgtgaaa ctgcccaaag acgtggaccg 1140
   gacgagactg gagagacact tgtcgcccga ggagttccag gaagtgtttg ggatgagcat 1200
   cgaggagttt gaccgcctgg ccctctggaa gaggaatgac cttaagaaga aagccctttt 1260
   gttctgacgg ctgccagcct gccccactgg tgtgtgccgg gcgccgaggc caggggcccc 1320
   tggcgagaac cgcacacacc cctcccacac accttgctct ggcttctctg tgtccatggg 1380
   gtgggcggga gggggtcccc cagcaggtgc ggcccctgca cctgccggcg acactcctgc 1440
   cggtagttta gggccgagac ggctagcttc acgccaccct tccccgctgt ggcttggtgt 1500
   cagggagagg ctgtagagtg gctgtgtcgg gcatcagatg gagcacacag gtggctgcag 1560
   cccagcccac cttcccagcg ttctcgaggt gccctggccc cggtgctggg cacgtggggg 1620
   acagaggtgg ccgggacgtg agctgtgagg cttgttgatg atggttgctg acaccatcat 1680
   cgggggtggg cacacggccc ttcggagcct gggcagcctg gcctcacagg cagactcgca 1740
   gacggggcag tgagcgtctg ggacagtgcc aagagtgggg tgtgtgattt ttgcaggcgt 1800
   ctgtgatggg tctctttagg gacaaatgtc aacaagggac aagacagggc accttccgcc 1860
   agcgcccctc catgcgctgc gttcctcctc cagatcgacc cctagatgcc tacacaatat 1920
   ctttaaagta acacagaagt ttttatttta ttaaaaagta tagcctactt aaacgcgaga 1980
   tgacatatat agagtttaat tttacggtcc ctccgcaggg gagcggcctc cagccttatt 2040
   ctccaccgct cggatctgtg tggtttcagt ctgttcttgg tgtggtcctc atacacagag 2100
   ctccctgtct agtttctttt ctttttcttt tttctttttc tcgtcacaca ggtaacctta 2160
   aagacagacc cctctaaaga acgctttgta aatacatgtg aggtatagcc accactgttt 2220
   tccttgctgt tatttttcca agtcttgggg agaaaacatc ctcctctgat ggccaaagcc 2280
   ctggaatcaa gggtttccac gtaccctgcc taatacccga cgtagctctt gatgcaccgt 2340
   ccttgtgctg tggctggcgg tgtctcagcc tgaaacataa accccacatg ccccaggagc 2400
   gatgtgctcc ctgaaacaga caaccacacg ctgttgggga gagaaggatg gagataggat 2460
   ggagatagga tggagatgtg gctcttctca tctttgaagc cagcagggca tcccggagca 2520
   ggaggctggc cgggctcccc aagcgaaggc gttggtgtct gtcattaggt gtgtgttagg 2580
   gtgcagcacc ggccgtcaca ggatgctgat aagcgcgctg agaggtggat gaaacaccaa 2640
   agtctgtttc cccgtccgca gtgggtgttg cctctttgtg tgtgtcccga tgttcctgcc 2700
   tgtgagtcgg ccttactccg tttccttagc gcccatgaca cgccaagtcc cgtttcgcac 2760
   tcggcttctc acccgcccag ctcggctagg gagggggagt ttttagcacc taatatgctt 2820
   cctgccattg cgcaatctga gcctgagcaa ctggaaaccc ccatttctca ttagtgcaat 2880
   gtcatatctg atcccaggaa gcctggaaaa taaaagacga tgcattataa cttgcggaag 2940
   tacaagaaaa aaaaaaaaaa aaaa                                        2964
<210> 37
   <211> 2351
   <212> DNA
   <213> Mus musculus
<400> 37
   cgggtgctag aggccggaga gaagcactac cacccttcat gtgcgctatg cgtcaggtgc 60
   ggccagatgt tctcagaggg cgaggagatg tacctgcaag gctcctccat ctggcatccg 120
   gcatgtcgac aggcagccag gactgaagac aagagcaagg aaaccaggac ttcttcggag 180
   agcattgtct ccgtgcctgc ctccagcacg tcagggtccc cgagccgtgt gatctacgca 240
   aagcttggcg atgagatcct ggactacagg gacttggctg ctcttcccaa aaacaaagcg 300
   atctacaaca tcgaccgccc cgacatgatc tcctattcgc cctacattag ccactcnnnn 360
   nnnnnnnnnn nnnnnnnnnn nnnnnnnnnn nnnnnnnnnn nnnnnnnnnn nnnnnnnnnn 420
   nnnnnnnnnn nnnnnnnnnn nnnnnnnnnn nnnnnnnnnn nnnnnnnnnn nnnnnnnnnn 480
   nnnnnnnnnn nnnnnnnnnn nnnnnnnnnn nnnnnnnnnn nnnnnnnnnn nnnnnnnnnn 540
   nnnnnnnnnn nnnnnnnnnn nnnnnnnnnn nnnnnnnnnn nnnnnnnnnn nnnnnnnnnn 600
   nnnnnnaaac agcacgctgc caggcgattg gacgtggagg acagcagctt cgaccaggac 660
   agcaggaaga agacaacctg gctgcttctc aagggggatg cagacaccag aactaactct 720
   ccagacctgg acagtcagtc gctgtccctc agcagcggga ctgaccagga acctctccaa 780
   aggatggcag gggacagcct ctactcacag tataagatat atccctatga ttccctcatc 840
   gtaacaaacc gaattcgtgt gaaactgccc aaagacgtgg accggacacg gctggagaga 900
   cacctatcac ctgaggagtt ccaagaagtg tttgggatga gcatcgagga gtttgaccgc 960
   ctggccctgt ggaagaggaa tgacctcaag aagaaagcct tgctgttctg acagctgcca 1020
   acatcttacc aggagcctgc tgggggggca gggctgaagg accccaccag tcccctgaaa 1080
   atcccctcct gcaccttgct ctgcttctct gtgtcagtag ggcaggcagg gtgcctgcag 1140
   gaggcagggt ggggccaggc cctgaggagc atccagcagg catggtccct tgtatagcaa 1200
   ggatgctctt gcctgtagtt tagggccagg tgccagcttg acccactcct tcccgccaca 1260
   actgggcatc agggcagtgt tgcagccagc tatcctagga gatagggaca gagcagtcag 1320
   ccagcccagc acctttccgg ggcacctggt gtgcccacac ctgtgctagg cactggggca 1380
   cagagtgtct gggacgtgag tgaaagactt gtcaggggca gactctgaca cagcaggaat 1440
   ggtaagtgtg agacttctcg gagcgtgagg aggtaccaag tagccttgca gactgaggca 1500
   ggggagccag gccaaagtct gcgaggcagt gggacggagg ttttctacag ttattgtgat 1560
   agatctttta tggggaatga atgttgacct gacagggtgc cccccgctaa tgctctgtgt 1620
   attgtgcccc tcttccagat caccccctta gttgcctaaa tgatatcttt caagttacac 1680
   agaagttttt attttattaa aaagtatcgc ttccttacac atatgaagac atatatgcag 1740
   agtttagttt tacggtccct gagaagggga ccgctccggc ttacacctcc ccacccatcc 1800
   caccgtgcag gcctctgtgc ttccagtcct ccgcggctcc tcacagacag gagccggtct 1860
   gtttcccttc gtccctgctc ctttgtatgt gtcacgtgac acagccattg actgcagcgg 1920
   cagagatgga cagctagagg tggtgcctcc tccaacctgg gtgtctagaa gtgggggcta 1980
   actggggccc tggtagcggg tatggaagag cacccctgtg cagaatgggt ggacactgtg 2040
   gtcctgggtg actctagctg tccctcctcc gggttgccgt cactattctc acatcccggg 2100
   acatgtgtga gtctcatctg tgctggggtt ctcacagcct ggtgggcggg agggaacttg 2160
   ggtacttatc tggttctgca ttagagtctg taacggaaat ggaaaccact ctccatcctg 2220
   agggtgtagg tggccgggac cggaaacacg ggccgggcac aacaaacaca acacaacaca 2280
   gagaacagca caaaacaaaa aaaggggggt aaggcgccca tgagcaaaaa agggggaggg 2340
   acgtaacatt t                                                      2351
<210> 38
   <211> 712
   <212> DNA
   <213> Rattus norvegicus
<400> 38
   tggggcacag agtgtctggg atgtgagtga aaggcttgtc agtggcagac cctgacacag 60
   tagtaatggt aagtgcgaga ctcctcagtg cacgaggagg catcgggcag actggcaggc 120
   tgaggcaggg gagccaggcc agtgtttgag aggcagtggg gctgtgtttt ttgcagttat 180
   tgtgatggat cttttctggg gaatgaatgt cgacctgaca gggtgccccc cactagtgct 240
   gtgcatttgt attgtgccct ttttccggat caccccctta gttgcctaaa tgatatcttt 300
   caagttacac agaagtttnt atttatttaa aaagtatcgc ttccttacac ataagaagac 360
   atatatgcag agtttagttt tacggtcccc gagaagggga gcgcctccgc cttacacccc 420
   caccaacccc cgcccgccca tcatgcaggc ctntgtgctt ccagtcctcg gcgttcctca 480
   cagacaagag ccagtntgtt tccctccgcc cctgctcctt tgtatgtgtc acacagacag 540
   cctcaaagac actggctcag cagtgcagtt tgtaaataca tgtgaaatgt atccatcttt 600
   tcccctctct gtcattttcc caagtcccaa agagaagagc gccccctgat ggccaaagcc 660
   ctagaataaa ggtttcccca ctgtggccta gtctcaaaaa aaaaaaaaaa aa         712
<210> 39
   <211> 197
   <212> DNA
   <213> Rattus norvegicus
<400> 39
   tttttttttt ttgtgtaagg aagcgatact ttttaataaa ataaaaactt ctgtgtaact 60
   tgaaaaatat catttaggca actaaggggg tgatccggaa aaanggcaca atacaaatgc 120
   acagcactag tggggggcac cctgtcaggt cgacattcat tccccagaaa agatccatca 180
   caataactgc gaaaaca                                                197
<210> 40
   <211> 947
   <212> DNA
   <213> Rattus norvegicus
<400> 40
   ggaacagaag tcatagggtc catggctcca gcatggactt ccttcaggca ggatagaggg 60
   ttcgctgcag atgagctctt tttgcctgaa accttctgct ttgcttctct gcctctcaca 120
   tgctgtctct gcccttccac ctggaggcag cgaaagtggc cgtagcactc caagtctctc 180
   agtgcactcc gacagcaggc ctccttcctc tacctatcag caggctccga gacacttcca 240
   cgtcccagcc gccaggcgac tggacgtgga ggacagcagt ttcgaccagg atagcaggaa 300
   gaagacaacg tggctgctcc tcaagggaga tgcagacacc agaactaact ctccggactt 360
   ggacagtcag tctctgtccc tcagcagtgg gggaggagca tccagcaggc atggtccctt 420
   gtgtagcaag gatgctcctg cctgtagttt agggccacgt gccagcttga cccactcctt 480
   cccgccacag ctgggcatcg gggcagtgtt gcagctatcc taggagacgg ggcagagcat 540
   tcagccagcc cagcaccttc ccagggcacc tggtgtgctc acacctgtgc taggcactgg 600
   ggcacagagt gtctgggatg tgagtgaaag gcttgtcagt ggcagaccct gacacagtag 660
   taatggtaag tgcaagactc ctcagtgcac gaggaggcat cgggcagact ggcaggctga 720
   ggcaggggag ccaggccagt gtctgagagg cagtggggct gtgtttttcg cagttattgt 780
   gatggatctt ttctggggaa tgaatgtcga cctgacaggg tgccccccac tagtgctgtg 840
   catttgtatt gtgccctttt tccggatcac ccccttagtt gcctaaatga tatttttcaa 900
   gttacacaga agtttttatt ttattaaaaa gtatcgcttc cttacac               947
<210> 41
   <211> 2818
   <212> DNA
   <213> Rattus norvegicus
<400> 41
   cccccgcttc cctaagatct gtgccggtgt ctctgtttac aataaacccc ttcctgagcc 60
   agctcctggg tggacttccc ctggaagagg agtcagcccc acaaaggana naatcccgct 120
   ttcatgcctt ttcgttcatc tggctagata gagttctaga attcagcggc cgctgaattc 180
   tagaactcta tctagccaga tgaacgaaaa ggcatgaaag cgggattctc tcctttgtgg 240
   ggctgactcc tcttccaggg gaagtccacc caggagctgg ctcaggaagg ggtttattgt 300
   aaacagagac accggcacag atcttaggga agcgggggat ccttcagaaa tcattgctgt 360
   ctggctttgg aatgtattga ttttgctcct agacagttcg cctccctaac tcctctcggc 420
   ttaagtaagc aatacaaaac ctgtttgcct gttttgatct catctcagct ggtggcaagg 480
   ggcaagggcc tcacaggctc ccatgctgtc tggaggaagg gaccgggttt acaactcatc 540
   tgcagagagc cacaaacaca gaaactgctt gtcctgggga actgggtcag ccctggagaa 600
   catgagtgtt ttcgacagag tagacaccag caagaggtcc gggcttccga aaagactttc 660
   tacttgacat tagggaacag agtctcactt ccgtggctcc ccaccttcac tccctacttc 720
   cttacaggtg tgaacctgaa gcagaagcat cagtcccctt agctggcata aagatggggg 780
   ctattcattc tgtctaggca gctgttgcaa agtttgtgac atactgggtc cagagtgaca 840
   gctcagttaa gtttcgcaga cagagcctgg aatggtagac tcggacaacg gtgaaacagc 900
   aataatgttg aaaacagagg ccactgacaa acaaccaaac atgaccaggt gagctgttca 960
   ctcaggatca cccaggatgc tagtggctta ttctgaacgc ctttctgttg cctctctgag 1020
   aactgtgccc tcctggagat cacacctgct ttttgcacat tcttccttct cattttgcca 1080
   accatcgagc tgtggtgtag aaagccatgc atgcctctgt gaacctctgt tcgtgcaaac 1140
   gccatgtgtg ggtgccaaga aatggcactc cacctacagg acatgcccct ctggaaggcg 1200
   gggggggggg gggtatggag ccaggaggcc acaggttacc tgtcatcctt actttaaaca 1260
   ccaatccgat gttctgttgg atgaggccac ggaagcctgg gctcttcatc tacaaaactg 1320
   gagtggggtg gaccaagtga ggctggtgaa actctgtgat gctatgaaat gaccctcaat 1380
   actgtggcca ctgggcttct gttttgactc tataatctga gcatcccttt agactggctg 1440
   tagatataat agaatttgta ctttctccaa aattaaatta ctggcagtaa acgatcaatc 1500
   aatccattga ttaactactt attgatctgc tgctgacgtg gctctgagac agtttggctg 1560
   tacttgggaa cacaatcctt tggtaattgg cttatcatgc tgggtgcaca gcctctgtta 1620
   aaaggcatct gggtcttggt aaatggcttt ttatctgtgt tatttatgtg tccaacattt 1680
   tatgtgtgtg ccgagttcag aggtaagccc acatgctacc acagactcag ccagggaaat 1740
   ccagtacaat gggtccaagc acttaattca ttaatttatt tttgagacag ccacgtgtag 1800
   cccagggtgg ctttaaactc acaatgtagc agaggctggc tttgaacttt ttatcctccc 1860
   gattctaatt cccatgctag aattagaggc ttttgccacc acacccagct aactgagggt 1920
   actggagaag tcactttact tcttgtctca gctgccaccc cccaaaaaaa caaaggttgt 1980
   ggctccacag cctttaaggg gctcacattc cttacctcag attttggctt tctctcccca 2040
   cagtctcact taaggagatg ggatatttcc atatcaaaag ctacttcagg ctcaggttgg 2100
   caaggctagg tccctgggga aaccgtggac tatattgata ctttgaagtg ttgacattaa 2160
   gggcatcccc tatactgggc acaggccata gggaatcacc ctgccccatt cccaaatgat 2220
   aaacacggag gaaggaattt gggccacaat tcttccctgc aaacatcctc tgtgttacta 2280
   cccttctgtc acattggcat gtcaggaaaa caagctcatt cactagtgac atctgcagaa 2340
   tcaccccaga agactgcccc cttgtgagct ctaaagggtg acggagcttg gaagacctat 2400
   ggtgttggac tgtctgtgtc agacatccat cttgggggca aaggagttag gaaccacatg 2460
   ctacctgagc acagaaatgc aactttcctc ctgtcctgta gcgtargcag gtgtacctct 2520
   tattggatga tcaacagtgg cctcagcctg tagccttgtg gaccctttct gtccccctct 2580
   atagggtanc catacctccc tctgaaggtt gaatccaaat atgtctcant ccttgnttca 2640
   aaacttaccc angggagtcc acactccngt ctatancgct ntgaccatcc tacagccatc 2700
   cccattgtcc accccactyc wgttytgyas myraaggtng tcgtggggta aacacatcan 2760
   tgaatgaatg agtttggatc tgaatganaa agganaacta atttcccatc ctacatta 2818
<210> 42
   <211> 4672
   <212> DNA
   <213> Rattus norvegicus
<400> 42
   gaactctatc tagccagatg aacgaaaagg catgaaagcg ggattctctc ctttgtgggg 60
   ctgactcctc ttccagggga agtccaccca ggagctggct caggaagggg tttattgtaa 120
   acagagacac cggcacagat cttagggaag cgggggatcc ttcagaaatc attgctgtct 180
   ggctttggaa tgtattgatt ttgctcctag acagttcgcc tccctaactc ctctcggctt 240
   aagtaagcaa tacaaaacct gtttgcctgt tttgatctca tctcagctgg tggcaagggg 300
   caagggcctc acaggctccc atgctgtctg gaggaaggga ccgggtttac aactcatctg 360
   cagagagcca caaacacaga aactgcttgt cctggggaac tgggtcagcc ctggagaaca 420
   tgagtgtttt cgacagagta gacaccagca agaggtccgg gcttccgaaa agactttcta 480
   cttgacatta gggaacagag tctcacttcc gtggctcccc accttcactc cctacttcct 540
   tacaggtgtg aacctgaagc agaagcatca gtccccttag ctggcataaa gatgggggct 600
   attcattctg tctaggcagc tgttgcaaag tttgtgacat actgggtcca gagtgacagc 660
   tcagttaagt ttcgcagaca gagcctggaa tggtagactc ggacaacggt gaaacagcaa 720
   taatgttgaa aacagaggcc actgacaaac aaccaaacat gaccaggtga gctgttcact 780
   caggatcacc caggatgcta gtggcttatt ctgaacgcct ttctgttgcc tctctgagaa 840
   ctgtgccctc ctggagatca cacctgcttt ttgcacattc ttccttctca ttttgccaac 900
   catcgagctg tggtgtagaa agccatgcat gcctctgtga acctctgttc gtgcaaacgc 960
   catgtgtggg tgccaagaaa tggcactcca cctacaggac atgcccctct ggaaggcggg 1020
   gggggggggg gtatggagcc aggaggccac aggttacctg tcatccttac tttaaacacc 1080
   aatccgatgt tctgttggat gaggccacgg aagcctgggc tcttcatcta caaaactgga 1140
   gtggggtgga ccaagtgagg ctggtgaaac tctgtgatgc tatgaaatga ccctcaatac 1200
   tgtggccact gggcttctgt tttgactcta taatctgagc atccctttag actggctgta 1260
   gatataatag aatttgtact ttctccaaaa ttaaattact ggcagtaaac gatcaatcaa 1320
   tccattgatt aactacttat tgatctgctg ctgacgtggc tctgagacag tttggctgta 1380
   cttgggaaca caatcctttg gtaattggct tatcatgctg ggtgcacagc ctctgttaaa 1440
   aggcatctgg gtcttggtaa atggcttttt atctgtgtta tttatgtgtc caacatttta 1500
   tgtgtgtgcc gagttcagag gtaagcccac atgctaccac agactcagcc agggaaatcc 1560
   agtacaatgg gtccaagcac ttaattcatt aatttatttt tgagacagcc acgtgtagcc 1620
   cagggtggct ttaaactcac aatgtagcag aggctggctt tgaacttttt atcctcccga 1680
   ttctaattcc catgctagaa ttagaggctt ttgccaccac acccagctaa ctgagggtac 1740
   tggagaagtc actttacttc ttgtctcagc tgccaccccc caaaaaaaca aaggttgtgg 1800
   ctccacagcc tttaaggggc tcacattcct tacctcagat tttggctttc tctccccaca 1860
   gtctcactta aggagatggg atatttccat atcaaaagct acttcaggct caggttggca 1920
   aggctaggtc cctggggaaa ccgtggacta tattgatact ttgaagtgtt gacattaagg 1980
   gcatccccta tactgggcac aggccatagg gaatcaccct gccccattcc caaatgataa 2040
   acacggagga aggaatttgg gccacaattc ttccctgcaa acatcctctg tgttactacc 2100
   cttctgtcac attggcatgt caggaaaaca agctcattca ctagtgacat ctgcagaatc 2160
   accccagaag actgccccct tgtgagctct aaagggtgac ggagcttgga agacctatgg 2220
   tgttggactg tctgtgtcag acatccatct tgggggcaaa ggagttagga accacatgct 2280
   acctgagcac agaaatgcaa ctttcctcct gtcctgtagc gtaggcaggt gtacctctta 2340
   ttggatgatc aacagtggcc tcagcctgta gccttgtgga ccctttctgt ccccctctat 2400
   agggtagcca tacctccctc tgaaggttga atccaaatat gtctcactcc ttgtttcaaa 2460
   acttacccag ggagtccaca ctccaggcta taatgcttga ccagcctaca gccatcccca 2520
   ttgtcctcca gttctgttct gcacccaaag gttgtcgtgg ggtaaacaca tcaatgaatg 2580
   aatgagtttg gatctgaatg acaaaggaga actaagttcc atcctacata atttaaacgc 2640
   atacaagagg gtctggggag atggcttagt caataaagta catgctgcac acgcgtgggt 2700
   accggaatca gaatcccgta aaagctacct cataaaagct ggggaggcag agacaccgaa 2760
   ggaccccagg gcttactggt cagatgaatg gatgagctcc aggtccattg agagaccctg 2820
   tcttaaaaac taactcggaa ggctcatggc tcagtgggta aaggtacttg ccaccaggcc 2880
   tggcaacctg agttcaatgc tcagaatgca cacggtggaa ggagagaaca tactcaggcc 2940
   acagtccact tgtccatgac gctgtcactg caccagttcc tgtttctcaa acaaaccagt 3000
   gttttaaccc aggctcctcg cctgtcccag ggtctctgct tgtgttcctt gagcctagaa 3060
   agccctcgtc agtcacctcg tcctgtaagg tctctccgaa tcttcagacc tagtttcttc 3120
   cctggccagt tacagccctg gaccagtctt gtccctctta cttcctccct gtcaggcttg 3180
   cttttgtcag gcttggtttt catcctatcc ctgaggaggc caccagccca tgagcactag 3240
   gaaccggttt gtcgtcttct ccattctgtc cccagggcct gggcagtact tggctctaga 3300
   atgggattag atggctattg aaggaatgaa cgacccagca ggtggcacat ccttcactct 3360
   cgctcttgct actcaggcag atgggaacct cagaggcctc tccggatcag atgccactgc 3420
   cagcaaaact ttgttcccca gagccagtgt gaggatcagt cccagcccat ctgccctcct 3480
   tgccgcccgc ctctgccagg ctcccagatc gcagaagccc cccattccgt gaggaccagt 3540
   tttttgaggc ctgtgtttca cagcaggaag ctttttggtt ttctcctttt aagggaaatt 3600
   tttgctctgg gactggctgt aattgttgaa tttgcatctg gtccacctgc tgtgacaggc 3660
   cccagggcac ttaggccttt gcaaatcaat aggaataaac aaacaaatac attccgagga 3720
   aaggggtctc cagaggaggg gtactgaagg ggaagggaga gagcacagct cgagcacggc 3780
   tcaccagtct gcagttgggg acctgcctcc tccccaccag acccgactcc aggccgcctg 3840
   ctgtgccaca ggacccgcct tccttcacta cagagaaacc aagactataa tttcagtgct 3900
   aggctgaatg gcttcatttc tttttattaa ctccagccct ggggttttag gcatatcggc 3960
   tgttgtttgt gctatatgga tcctctcccc tccattctcc aggctttgag aatttgaagc 4020
   agcagaaaca gccagcaaga gatggcacca agtggcagaa gaggttgcat ggagacctag 4080
   tcaccctgtg cgggagccga aggagcagga ggagggtggt gaggacagga cgcctagaac 4140
   ccagcgctac ctagataccc gcacaggtgc cttcccggct cacctgctgc tgtcctacaa 4200
   ttaggaagaa atctggccag cctcccaggt acccactgag ctatagcgta atgaggcaaa 4260
   aagcaaccag tgttgacaag gttacctgtg agtcttcatg cacatgccag gttttgtggg 4320
   gagctcacca ggctccaggg ctccacagct gctggcatgg aaggggccgc tacagaataa 4380
   agggctgtac tctgggaggc attttacaga gtcttgggag ccatgacatc atgactaagg 4440
   gcaggctgca actacaggag gacgggcacc agatcctccg tcttaccctt ggctccgttc 4500
   ctttcctggc atgtagcagg catgttgaag gtgtgtgtgt gtgtgtgtgt gtgtgtgtgt 4560
   gtgtgtgtgt gtgtgtttgt gtgtgtgtgt aatgcacatg tatgtattca tgtgtggata 4620
   tgtatggaag tcagaagtcg ttgttgatgg tctttttcta ccattcgttg aa         4672
<210> 43
   <211> 539
   <212> DNA
   <213> Rattus norvegicus
<400> 43
   catcaccact accaccccac ttctcttttc ctcttctctc ctctcctttc ttccccaccc 60
   caactccctg agcccagact agttccaggt tacagggctc aaacaatctt ccaacctcac 120
   cttctctagc agagataaca actccacacc actgtgccta ttgaatttta atccagaatg 180
   tctgtatttt aatttagatc tctctataaa gatagcacct tccaatctgg tggatgtttt 240
   ggtttgaagg ttttgaggga acaggggtga tttgatagat atttaaatgg tattctatac 300
   aacaagtaag aaaacacaca taattctctg tggtccatga aaccctctgc taaattggac 360
   acacctaact aatacaaaat atcaaaataa cttcttaaaa cataccatac aacaatgaaa 420
   tataatgcat tgaacatcac gaaagaccac aggaactaca aagatacttg gagactaagc 480
   aatabctttt gagggatggt ggaaaggtaa aggggagggg gacaatagaa tccaagctt  539
<210> 44
   <211> 1759
   <212> DNA
   <213> Rattus norvegicus
<400> 44
   gtgctctatt gactctatcg aagtctggga tgggcaggta tctcgacagc tttgagatgt 60
   ctcacgtata aactgtggtc tctttctact aatgtggcat ttgatgggag aaggcagaat 120
   gggtccttgt gactggcctg gctggtgtcc agttcccttg tgagcaggcc aggcagtaga 180
   agcctgggaa ggagcagctg actaggagag aagtgaaggt tggtggtgag accctaaggc 240
   ttcaggtttc tggcacatag catgcagtgc aagattgaga ccctgggtcc cacatccaac 300
   aggagttgga gggtgaaggc tgacagccat tagaggagta aaaatatggg ttaaaaatta 360
   attaagttgc ggggccacca aactccattc tgcttgaccc tgtcacatct gggtgattct 420
   gtgttctcac ctgctaaaag tggtttagga ggacacagcc aagaagcaga agtgtaaaca 480
   ggtctctaat agccttggaa gtaccaccca gaacagggca ctggctgcta ccttggtctt 540
   tgagcgctag ctcctcctcc ctgaagatac ccttcaggta tggtgtctgc ctgcctagag 600
   gccctatcag acagaggaca aaatctagkt caccagacag gaccstgggg ttggagatgc 660
   agagggttgc tgctgagtcc tgtgaactgg aattgcatgc gtaaccagcc gctagagtta 720
   acgacttttc agggagctga ggttctctag gcaggaacta ctyaggtcac tctggagtgg 780
   gaccaatggg ggtaggagca cgccctgggt gggggtatgt gccccaggat aaaggactga 840
   atgccacagg ccgggagggg cggtgcttct tgtgcaccac gtggatctct ggagcagtca 900
   gcttgtgctc tgtttgcgga acgttggtca gtaggaggct ccgggtgccg gagggtctgt 960
   gttcctgggt gctactcctc cctgcctgta tccttcgggc gttgaactcg ggtgctgcag 1020
   cagagcaatg aaggatctgc ggttcggtga gtgatcctat cagggctgcg gtaagcagca 1080
   agatctgttt tacctggaaa agcttctggt ttccgcaggg aactgggagt taaacccaga 1140
   gtgagcttag aggaaaggag agggagagag gggatccctg aaagtcaggc gaggaccctc 1200
   ccctacctga ggcgcatcag gcttttctta ataaacgttt tatcgtcttg tcacaccttc 1260
   tggtgcttct gttttcctca cacggaacat tttgtatttc tctttgtcca gattgctcat 1320
   tttccggtca atttaggctg tcttgagatc tcttctgcca cactattaat ccaaaacgct 1380
   tcaatttaac ctcaggtata tattttagac aagggcagaa agcagctgca ttcttgccaa 1440
   aatgtctcaa gaatggtctt aaggccactc accagtatcc ttcccttctg aaacttgagc 1500
   tgggctctca tggtttctat cgctctcagc tgctgtcttc cgygctccta ctcagatgca 1560
   cattaaatcc ccttagagag ttcaaacgct tttctagcct gaagttgcag agttcacatt 1620
   cggccgacaa cagcatggcc aggcctgtca cagcaatacc ccgttccctg gtctcagctt 1680
   ctgtcttagt cactgttctt tctattcctg tgaagagaca gcatgaccat aggaagtctg 1740
   acaaaagaaa gcatttctt                                              1759
<210> 45
   <211> 827
   <212> DNA
   <213> Rattus norvegicus
<400> 45
   gktttcgcag cttggccatt tctgcttcta tgttaggact ccagtggcat aaatgtagat 60
   ggtttattgt ggacctagca atttattagc cttttttttc tgttattcgg attgtgtaaa 120
   atctagtgct ctagcaccag attcactaat cctacagttg tatttttcac tccagttcta 180
   gggaatccat tgtcttcatc cagcctttaa gatattatgc aggaggtaca tagacatttg 240
   tacaggcaaa acatgtgaat gtataagata aaaactttca aataaatatt aaacaaatta 300
   aaagtaaatg tatgttcccc atgataatga gtataaaaga agctccaagt ttgattgact 360
   agccttaaca tgatttacac acctccagaa ttcccaggga ctaaactacc aaccaaagag 420
   tacacatgag ggacccacga ctccaacttc atatgtagca gaggatggcc tatctgccat 480
   cagtgggagg ggaggccctt gatcctgtga gggcttgatg ccccagtgga agggaatgct 540
   agggtggtga ggcaggagtg tgtgagtggg tgatcagggt gggagagcac cctcagaaac 600
   aggggggaga gggatgggat agggagtttg cagaggggaa actgggaagg gggataacat 660
   tcgaaatgta aataaataaa ataacccata aaaaaaataa aaaaaaaaaa aaaaaaaaaa 720
   aaaaaaaaag cggccgctga attctacctg cccgggcggc cgctcgagcc ctatagtgag 780
   taagggcgaa ttccagcaca ctggcggccg ttactagtgg atccgag 827
<210> 46
   <211> 1081
   <212> DNA
   <213> Rattus norvegicus
<400> 46
   actcactata gggctcgagc gggccgcccg ggcaggtgtg tcacctgatt cttcccccca 60
   tttcacatat caaaatatat tctcactagt ccatcatgga ttataaaaaa gacaggccct 120
   caatggaaat acatagaata tgtttagaaa tagatatgct ctgaccaatt atcacagaac 180
   aagtcaaagg gtggatttac tctttgagga acactgtatc ccagtgtgaa aganagtatc 240
   ctgtttagaa tttgacgctc acagtgttcg ggcttgtgac taccgtaaat taaatgaagc 300
   cacatgcccc cagtgcacag agtaggcatc caaaaataga acgagggaag agagctgatt 360
   tgaaggaggg acgagttant cagaagtggc cataagaagc cagggagcct gagaagtgcc 420
   ttgggatgtg caatttcaag atgcaagcaa ataacagaag tcccagaaca cgctcagtta 480
   ttccggactg agtgtccctc ttgttatgtg attatttctg gaatttttct ccaccggctt 540
   catttacaag ggaaagggaa agatgactgg agaagaacgg aggaagtaaa acttactgag 600
   ttcagaaaaa attatttcta tcccagattc tatcatccag agaacagcta ataaaaagaa 660
   atctctaata gcaaattttc cacaatccat cacagccaac agacaattgt aatttagcat 720
   gaaggttttt cttgcacaga gaactagcaa ggctatccat cacctttctg tcagtgacat 780
   cataaacagg cccctctcta atatttgttc atcctttgtg atccactgaa ataggtataa 840
   gtatattact tatagaaata cagaagaagc aagtcccact agagtatgaa aggtaaattt 900
   cagagagaca attattacac ctagaataac atacaaaatt atctcttctt tctctgaaat 960
   gggagaaatc aaattgtacg cttttcaagt agaaggagaa cattaacctg acgggccatg 1020
   tgttaacagt gctacagaat caaaatgcca ttgaacacac tgtgtccctg cggacttgat 1080
   c 1081
<210> 47
   <211> 195
   <212> DNA
   <213> Rattus norvegicus
<400> 47
   ggtactaang caaaanttgg tactananag tggactattg cagtgagaga cctgactgaa 60
   ccaacttang agaanaaaga gtttactttg ggctcacagt ttcanagggt gagtccatct 120
   caatagttcc ttgtgttgct cctaggaaac ttcctgtgtg taaattctta aattttgctg 180
   aaaaaaaaaa aaagg 195
<210> 48
   <211> 538
   <212> DNA
   <213> Rattus norvegicus
<400> 48
   tgacagtggc acacaaaagt acagattcct ttcttatgcc atggagggcg ggccccagcc 60
   tctgctgctt ctgccatgat ccagccatgc agccctgcca ggacctggcc ctcacatccg 120
   ttgtttctgg tagcctttga aatcaggagc ttccacatga taatctgacc ttctaaaata 180
   ccaaaggtca ctttccagga agcttttctg aaaaatgagt aaatgtttcc tggtggtttt 240
   aaatttccaa gtgaggaacg tcattcttgt cccagttcag cgactagtgg taacctccac 300
   atgccagaaa gcagccacac tgttgctttc agagcaccag aaattactga gtaaatgcta 360
   agtatttatc ttctgggaag caaaggaatt gaaagaatat gttaattttt aaaattatat 420
   gaatatgctt tcagttcttc atagggattg aaaaaaaatc ttcgacttca tatgggaaac 480
   acaaaaatct ctagcatanc tgaaacartc ytkraatagt aarttaactg aataccng 538
<210> 49
   <211> 547
   <212> DNA
   <213> Rattus norvegicus
<400> 49
   ccccttctca tttggaccac catccaccag ggattctgtc tcggtgcaaa actaggctcc 60
   ccttagtact attactccct ttggcttttc cttaaagccc atctcaactc ctttgtgaca 120
   cctgcctgcc cacagacact tctaccagga atcacaactg gcccagaccc agagcgggca 180
   gtaccaacca aagaaccctt gaccaaggac aagaccagat aattatacca aggaacattt 240
   caaatgtcac atgcctagat cccaacgcaa aatcatgaaa aacattcact acatggtgtc 300
   ttttgctcca caaaaacttt agcttcatcc ggtcccattt attgttggtt ttagcaccag 360
   ggctatgtgt cctcttcctt tccgggaaat tcctttctgg tgccagtaag ttcagaccca 420
   ttccctattt tctatcaaat tcagggtata tggtcttatt ttaaggccct taatccatct 480
   ggaattgaat tttgggcagg gtgataacta tgggatcatt ttgcaatgct ctactatncc 540
   ataacan 547
<210> 50
   <211> 250
   <212> DNA
   <213> Rattus norvegicus
<400> 50
   tcgatacagg caaatacaga tgataaaatg taggcaacaa ctaaattaac catgttttct 60
   tatatctctt cagttctaag gttacctttg tgagttataa cagcgcctgg atggagactg 120
   acacattagc ctgaaaagct acaccagtag actgattcaa tcaagatatt tgtgtattgt 180
   gttctatgtc cactttctgt cctattctta cacccctgta tctctaaaac actggaagag 240
   ctagtaaatt 250
<210> 51
   <211> 90
   <212> DNA
   <213> Rattus norvegicus
<400> 51
   gatcgcattg ggtatgtggt atcagtgtag gagacaagaa tagcaatggg tacttattta 60
   gtgcccttct tcttaatctg tgaccaatcc                                  90
<210> 52
   <211> 559
   <212> DNA
   <213> Rattus norvegicus
<400> 52
   gatcgcattg caccagacaa acttgaacat cccagtgttc ataaaaacaa atatcctttt 60
   tttctaacag aaatcatgta gtagttcgac caatccagct tcatttggac ttcctaagtc 120
   taagagttcc aaaacagcac matgggkgga catatcacaa tacatttgtt gaatggtcct 180
   ctagagagat gaatggaaac cctgcttcag ttttggtaaa aacagatttg aaagatgctt 240
   agaaacttac aactatttat tcctaagaaa cgcttttctt ccacagtaat ttcatctaca 300
   tatggaactg tgttataaga agcagctgtg tattctwaat accttggata atttatggaa 360
   aaccccaaag gaagtgttct aattacttac aggcatttct tatagtagat attatttaat 420
   gnccagaggg agtaaatttt taacaagtca aagtagcttc agtttatatt aaggaattat 480
   tctattcatt attaatnnaa tactgaggag ggcaatgggg agatggcacc atgtatgana 540
   tgattagagg gcaagccca                                              559
<210> 53
   <211> 445
   <212> DNA
   <213> Homo sapiens
<400> 53
   gatctaaccg gcaggcatac gtgcagatag attattgggt atataatata taaatacatc 60
   tttaaaacaa agcaaaactg cataccaaaa ctaagtatct ccaattacaa agttactcat 120
   gaaactgaaa aaaggaagga cacattcaga agacaacaca actgggaagg taaggagatc 180
   acacatcttg agaacattcc atttcggtta gaacacaagt ctaaggtcgc cactccctgt 240
   tcccctgtga cttgtcatgg tcaaatcccc accatttccc atcattctcg gcacctgtac 300
   ttgccaacct tggcacattc ctgtttcagt cccaacccgg ttttctcaga ttacctcaca 360
   cagggtacca acgagtcctg tctcccaagc ttgcccctta tcttccacca aagcataggg 420
   tttgtctgct gtctcctacc gagtg                                       445
<210> 54
   <211> 1773
   <212> DNA
   <213> Homo sapiens
<400> 54
   atctcagatc ttcactatgg caacttatgc aagaaactgt tgaattagac ccgtttccta 60
   tagatgagaa accatacaag ctgtggtatt tatgagcctc catttcttat actactgcag 120
   tgaaccaaca ttggatgtga aaattgcctt ttgtcaggga ttcgataaac aagtggatgt 180
   gtcatatatt gccaaacatt acaacatgag caaaagcaaa gttgacaacc agttctacag 240
   tgtggaagtg ggagactcaa ccttcacagt tctcaagcgc taccagaatc taaagcctat 300
   tggctctggg gctcagggca tagtttgtgc cgcgtatgat gctgtccttg acagaaatgt 360
   ggccattaag aagctcagca gaccctttca gaaccaaaca catgccaaga gagcgtaccg 420
   ggagctggtc ctcatgaagt gtgtgaacca taaaaacatt attagtttat taaatgtctt 480
   cacaccccag aaaacgctgg aggagttcca agatgtttac ttagtaatgg aactgatgga 540
   tgccaactta tgtcaagtga ttcagatgga attagaccat gagcgaatgt cttacctgct 600
   gtaccaaatg ttgtgtggca ttaagcacct ccattctgct ggaattattc acagggattt 660
   aaaaccaagt aacattgtag tcaagtctga ttgcacattg aaaatcctgg actttggact 720
   ggccaggaca gcaggcacaa gcttcatgat gactccatat gtggtgacac gttattacag 780
   agcccctgag gtcatcctgg ggatgggcta caaggagaac gtggatatat ggtctgtggg 840
   atgcattatg ggagaaatgg ttcgccacaa aatcctcttt ccaggaaggg actatattga 900
   ccagtggaat aaggtaattg aacaactagg aacaccatgt ccagaattca tgaagaaatt 960
   gcaacccaca gtaagaaact atgtggagaa tcggcccaag tatgcgggac tcaccttccc 1020
   caaactcttc ccagattccc tcttcccagc ggactccgag cacaataaac tcaaagccag 1080
   ccaagccagg gacttgttgt caaagatgct agtgattgac ccagcaaaaa gaatatcagt 1140
   ggacgacgcc ttacagcatc cctacatcaa cgtctggtat gacccagccg aagtggaggc 1200
   gcctccacct cagatatatg acaagcagtt ggatgaaaga gaacacacaa ttgaagaatg 1260
   gaaagaactt atctacaagg aagtaatgaa ttcagaagaa aagactaaaa atggtgtagt 1320
   aaaaggacag ccttctcctt cagcacaggt gcagcagtga acagcagtga gagtctccct 1380
   ccatcctcgt ctgtcaatga catctcctcc atgtccaccg accagaccct ggcatctgac 1440
   actgacagca gcctggaagc ctcggcagga cccctgggtt gttgcaggtg actagccgcc 1500
   tgcctgcgaa acccagcgtt cttcaggaga tgatgtgatg gaacacacac acacgcagac 1560
   acacacacac acacaaatgc agacacacaa catcaagaaa acagcaaggg agagaatcca 1620
   agcctaaaat taaataaatc tttcagcctg cttcttcccc agggttctgt attgcagcta 1680
   agctcaaatg tatatttaac ttctagttgc tcttgctttg gtcttcttcc aatgatgctt 1740
   actacagaaa gcaaatcaga cacaattaga gaa                              1773
<210> 55
   <211> 422
   <212> PRT
   <213> Homo sapiens
<400> 55
   Met Ser Leu His Phe Leu Tyr Tyr Cys Ser Glu Pro Thr Leu Asp Val
   1               5                   10                  15
   Lys Ile Ala Phe Cys Gln Gly Phe Asp Lys Gln Val Asp Val Ser Tyr
               20                  25                  30
   Ile Ala Lys His Tyr Asn Met Ser Lys Ser Lys Val Asp Asn Gln Phe
           35                  40                  45
   Tyr Ser Val Glu Val Gly Asp Ser Thr Phe Thr Val Leu Lys Arg Tyr
   50 55 60
   Gln Asn Leu Lys Pro Ile Gly Ser Gly Ala Gln Gly Ile Val Cys Ala
   65 70 75 80
   Ala Tyr Asp Ala Val Leu Asp Arg Asn Val Ala Ile Lys Lys Leu Ser
   85 90 95
   Arg Pro Phe Gln Asn Gln Thr His Ala Lys Arg Ala Tyr Arg Glu Leu
   100 105 110
   Val Leu Met Lys Cys Val Asn His Lys Asn Ile Ile Ser Leu Leu Asn
   115 120 125
   Val Phe Thr Pro Gln Lys Thr Leu Glu Glu Phe Gln Asp Val Tyr Leu
   130 135 140
   Val Met Glu Leu Met Asp Ala Asn Leu Cys Gln Val Ile Gln Met Glu
   145 150 155 160
   Leu Asp His Glu Arg Met Ser Tyr Leu Leu Tyr Gln Met Leu Cys Gly
   165 170 175
   Ile Lys His Leu His Ser Ala Gly Ile Ile His Arg Asp Leu Lys Pro
   180 185 190
   Ser Asn Ile Val Val Lys Ser Asp Cys Thr Leu Lys Ile Leu Asp Phe
   195 200 205
   Gly Leu Ala Arg Thr Ala Gly Thr Ser Phe Met Met Thr Pro Tyr Val
   210 215 220
   Val Thr Arg Tyr Tyr Arg Ala Pro Glu Val Ile Leu Gly Met Gly Tyr
   225 230 235 240
   Lys Glu Asn Val Asp Ile Trp Ser Val Gly Cys Ile Met Gly Glu Met
   245 250 255
   Val Arg His Lys Ile Leu Phe Pro Gly Arg Asp Tyr Ile Asp Gln Trp
   260 265 270
   Asn Lys Val Ile Glu Gln Leu Gly Thr Pro Cys Pro Glu Phe Met Lys
   275 280 285
   Lys Leu Gln Pro Thr Val Arg Asn Tyr Val Glu Asn Arg Pro Lys Tyr
   290 295 300
   Ala Gly Leu Thr Phe Pro Lys Leu Phe Pro Asp Ser Leu Phe Pro Ala
   305 310 315 320
   Asp Ser Glu His Asn Lys Leu Lys Ala Ser Gln Ala Arg Asp Leu Leu
   325 330 335
   Ser Lys Met Leu Val Ile Asp Pro Ala Lys Arg Ile Ser Val Asp Asp
   340 345 350
   Ala Leu Gln His Pro Tyr Ile Asn Val Trp Tyr Asp Pro Ala Glu Val
   355 360 365
   Glu Ala Pro Pro Pro Gln Ile Tyr Asp Lys Gln Leu Asp Glu Arg Glu
   370 375 380
   His Thr Ile Glu Glu Trp Lys Glu Leu Ile Tyr Lys Glu Val Met Asn
   385                 390                 395                 400
   Ser Glu Glu Lys Thr Lys Asn Gly Val Val Lys Gly Gln Pro Ser Pro
                   405                 410                 415
   Ser Ala Gln Val Gln Gln
               420
<210> 56
   <211> 1505
   <212> DNA
   <213> Homo sapiens
<400> 56
   ttatgcaaga aactgttgaa ttagacccgt ttcctataga tgagaaacca tacaagctgt 60
   ggtatttatg agcctccatt tcttatacta ctgcagtgaa ccaacattgg atgtgaaaat 120
   tgccttttgt cagggattcg ataaacaagt ggatgtgtca tatattgcca aacattacaa 180
   catgagcaaa agcaaagttg acaaccagtt ctacagtgtg gaagtgggag actcaacctt 240
   cacagttctc aagcgctacc agaatctaaa gcctattggc tctggggctc agggcatagt 300
   ttgtgccgcg tatgatgctg tccttgacag aaatgtggcc attaagaagc tcagcagacc 360
   ctttcagaac caaacacatg ccaagagagc gtaccgggag ctggtcctca tgaagtgtgt 420
   gaaccataaa aacattatta gtttattaaa tgtcttcaca ccccagaaaa cgctggagga 480
   gttccaagat gtttacttag taatggaact gatggatgcc aacttatgtc aagtgattca 540
   gatggaatta gaccatgagc gaatgtctta cctgctgtac caaatgttgt gtggcattaa 600
   gcacctccat tctgctggaa ttattcacag ggatttaaaa ccaagtaaca ttgtagtcaa 660
   gtctgattgc acattgaaaa tcctggactt tggactggcc aggacagcag gcacaagctt 720
   catgatgact ccatatgtgg tgacacgtta ttacagagcc cctgaggtca tcctggggat 780
   gggctacaag gagaacgtgg atatatggtc tgtgggatgc attatgggag aaatggttcg 840
   ccacaaaatc ctctttccag gaagggacta tattgaccag tggaataagg taattgaaca 900
   actaggaaca ccatgtccag aattcatgaa gaaattgcaa cccacagtaa gaaactatgt 960
   ggagaatcgg cccaagtatg cgggactcac cttccccaaa ctcttcccag attccctctt 1020
   cccagcggac tccgagcaca ataaactcaa agccagccaa gccagggact tgttgtcaaa 1080
   gatgctagtg attgacccag caaaaagaat atcagtggac gacgccttac agcatcccta 1140
   catcaacgtc tggtatgacc cagccgaagt ggaggcgcct ccacctcaga tatatgacaa 1200
   gcagttggat gaaagagaac acacaattga agaatggaaa gaacttatct acaaggaagt 1260
   aatgaattca gaagaaaaga ctaaaaatgg tgtagtaaaa ggacagcctt ctccttcagg 1320
   tgcagcagtg aacagcagtg agagtctccc tccatcctcg tctgtcaatg acatctcctc 1380
   catgtccacc gaccagaccc tggcatctga cactgacagc agcctggaag cctcggcagg 1440
   acccctgggt tgttgcaggt gactagccgc ctgcctgcga aacccagcgt tcttcaggag 1500
   atgat                                                             1505
<210> 57
   <211> 464
   <212> PRT
   <213> Homo sapiens
<400> 57
   Met Ser Leu His Phe Leu Tyr Tyr Cys Ser Glu Pro Thr Leu Asp Val
   1               5                   10                  15
   Lys Ile Ala Phe Cys Gln Gly Phe Asp Lys Gln Val Asp Val Ser Tyr
               20                  25                  30
   Ile Ala Lys His Tyr Asn Met Ser Lys Ser Lys Val Asp Asn Gln Phe
           35                  40                  45
   Tyr Ser Val Glu Val Gly Asp Ser Thr Phe Thr Val Leu Lys Arg Tyr
       50                  55                  60
   Gln Asn Leu Lys Pro Ile Gly Ser Gly Ala Gln Gly Ile Val Cys Ala
   65                  70                  75                  80
   Ala Tyr Asp Ala Val Leu Asp Arg Asn Val Ala Ile Lys Lys Leu Ser
                   85                  90                  95
   Arg Pro Phe Gln Asn Gln Thr His Ala Lys Arg Ala Tyr Arg Glu Leu
               100                 105                 110
   Val Leu Met Lys Cys Val Asn His Lys Asn Ile Ile Ser Leu Leu Asn
           115                 120                 125
   Val Phe Thr Pro Gln Lys Thr Leu Glu Glu Phe Gln Asp Val Tyr Leu
       130                 135                 140
   Val Met Glu Leu Met Asp Ala Asn Leu Cys Gln Val Ile Gln Met Glu
   145                 150                 155                 160
   Leu Asp His Glu Arg Met Ser Tyr Leu Leu Tyr Gln Met Leu Cys Gly
                   165                 170                 175
   Ile Lys His Leu His Ser Ala Gly Ile Ile His Arg Asp Leu Lys Pro
               180                 185                 190
   Ser Asn Ile Val Val Lys Ser Asp Cys Thr Leu Lys Ile Leu Asp Phe
           195                 200                 205
   Gly Leu Ala Arg Thr Ala Gly Thr Ser Phe Met Met Thr Pro Tyr Val
       210                 215                 220
   Val Thr Arg Tyr Tyr Arg Ala Pro Glu Val Ile Leu Gly Met Gly Tyr
   225                 230                 235                 240
   Lys Glu Asn Val Asp Ile Trp Ser Val Gly Cys Ile Met Gly Glu Met
                   245                 250                 255
   Val Arg His Lys Ile Leu Phe Pro Gly Arg Asp Tyr Ile Asp Gln Trp
               260                 265                 270
   Asn Lys Val Ile Glu Gln Leu Gly Thr Pro Cys Pro Glu Phe Met Lys
           275                 280                 285
   Lys Leu Gln Pro Thr Val Arg Asn Tyr Val Glu Asn Arg Pro Lys Tyr
       290                 295                 300
   Ala Gly Leu Thr Phe Pro Lys Leu Phe Pro Asp Ser Leu Phe Pro Ala
   305                 310                 315                 320
   Asp Ser Glu His Asn Lys Leu Lys Ala Ser Gln Ala Arg Asp Leu Leu
                   325                 330                 335
   Ser Lys Met Leu Val Ile Asp Pro Ala Lys Arg Ile Ser Val Asp Asp
               340                 345                 350
   Ala Leu Gln His Pro Tyr Ile Asn Val Trp Tyr Asp Pro Ala Glu Val
           355                 360                 365
   Glu Ala Pro Pro Pro Gln Ile Tyr Asp Lys Gln Leu Asp Glu Arg Glu
       370                 375                 380
   His Thr Ile Glu Glu Trp Lys Glu Leu Ile Tyr Lys Glu Val Met Asn
   385                 390                 395                 400
   Ser Glu Glu Lys Thr Lys Asn Gly Val Val Lys Gly Gln Pro Ser Pro
   405 410 415
   Ser Gly Ala Ala Val Asn Ser Ser Glu Ser Leu Pro Pro Ser Ser Ser
   420 425 430
   Val Asn Asp Ile Ser Ser Met Ser Thr Asp Gln Thr Leu Ala Ser Asp
   435 440 445
   Thr Asp Ser Ser Leu Glu Ala Ser Ala Gly Pro Leu Gly Cys Cys Arg
   450 455 460
<210> 58
   <211> 1240
   <212> DNA
   <213> Mus musculus
<400> 58
   tacaggtaaa acaagggatt cgataaacac gtggatgtgt catctattgc caaacattac 60
   aacatgagca aaagcaaggt ggacaaccag ttctacagtg tggaagtggg ggactcaacc 120
   ttcaccgttc ttaagcgcta ccagaacctg aagccaattg gctctggggc tcagggaata 180
   gtctgtgctg cgtacgacgc tgtccttgac agaaatgtgg ccattaagaa gctcagcaga 240
   cccttccaga accaaactca cgccaagagg gcttaccggg agctggtcct catgaagtgt 300
   gtgaaccata aaaacattat tagcttatta aatgttttta caccccagaa aacactggag 360
   gagttccaag atgtctactt agtgatggaa ctgatggacg ccaacctgtg tcaggtgatt 420
   cagatggagc tggaccacga gcggatgtct tacttgctgt accagatgct gtgtggcatc 480
   aagcacctcc actccgctgg gatcatccac agggacttaa aacccagtaa cattgtagtc 540
   aagtctgatt gcacactgaa aatcctcgac ttcggactgg ccaggacagc gggtacaagc 600
   ttcatgatga ctccgtatgt ggtgacgcga tattacagag cccctgaggt catcctgggc 660
   atgggctaca aggagaacgt ggacatatgg tctgtgggat gcatcatggg agaaatggtt 720
   cgccacaaaa tcctctttcc cggaagggac tatattgacc agtggaacaa agtcatcgag 780
   cagctaggaa ctccgtgtcc agagttcatg aagaaattgc agcccacagt cagaaactac 840
   gtggagaatc ggcccaagta cgcaggactc accttcccca agctctttcc agattccctc 900
   ttcccagcgg attctgagca caataaactt aaagccagcc aagccaggga tttgttgtct 960
   aagatgttag tgattgaccc agcgaagagg atatcggtgg acgacgcact gcagcatccg 1020
   tacatcaacg tttggtacga cccggctgaa gtggaggcgc ctccgcctca gatatatgat 1080
   aagcagctgg atgaaaggga gcacaccatc gaagaatgga aagaacttat ctacaaggag 1140
   gtaatgaact cagaagagaa gactaagaat ggcgtagtca aaggccagcc ctcgccttca 1200
   gcacaggtgc agcagtgaac agcagtgaga gtctccctcc                       1240
<210> 59
   <211> 384
   <212> PRT
   <213> Mus musculus
<400> 59
   Met Ser Lys Ser Lys Val Asp Asn Gln Phe Tyr Ser Val Glu Val Gly
   1               5                   10                  15
   Asp Ser Thr Phe Thr Val Leu Lys Arg Tyr Gln Asn Leu Lys Pro Ile
               20                  25                  30
   Gly Ser Gly Ala Gln Gly Ile Val Cys Ala Ala Tyr Asp Ala Val Leu
           35                  40                  45
   Asp Arg Asn Val Ala Ile Lys Lys Leu Ser Arg Pro Phe Gln Asn Gln
           50                  55                  60
   Thr His Ala Lys Arg Ala Tyr Arg Glu Leu Val Leu Met Lys Cys Val
   65                  70                  75                  80
   Asn His Lys Asn Ile Ile Ser Leu Leu Asn Val Phe Thr Pro Gln Lys
                   85                  90                  95
   Thr Leu Glu Glu Phe Gln Asp Val Tyr Leu Val Met Glu Leu Met Asp
               100                 105                 110
   Ala Asn Leu Cys Gln Val Ile Gln Met Glu Leu Asp His Glu Arg Met
           115                 120                 125
   Ser Tyr Leu Leu Tyr Gln Met Leu Cys Gly Ile Lys His Leu His Ser
       130                 135                 140
   Ala Gly Ile Ile His Arg Asp Leu Lys Pro Ser Asn Ile Val Val Lys
   145                 150                 155                 160
   Ser Asp Cys Thr Leu Lys Ile Leu Asp Phe Gly Leu Ala Arg Thr Ala
                   165                 170                 175
   Gly Thr Ser Phe Met Met Thr Pro Tyr Val Val Thr Arg Tyr Tyr Arg
               180                 185                 190
   Ala Pro Glu Val Ile Leu Gly Met Gly Tyr Lys Glu Asn Val Asp Ile
           195                 200                 205
   Trp Ser Val Gly Cys Ile Met Gly Glu Met Val Arg His Lys Ile Leu
       210                 215                 220
   Phe Pro Gly Arg Asp Tyr Ile Asp Gln Trp Asn Lys Val Ile Glu Gln
   225                 230                 235                 240
   Leu Gly Thr Pro Cys Pro Glu Phe Met Lys Lys Leu Gln Pro Thr Val
                   245                 250                 255
   Arg Asn Tyr Val Glu Asn Arg Pro Lys Tyr Ala Gly Leu Thr Phe Pro
               260                 265                 270
   Lys Leu Phe Pro Asp Ser Leu Phe Pro Ala Asp Ser Glu His Asn Lys
           275                 280                 285
   Leu Lys Ala Ser Gln Ala Arg Asp Leu Leu Ser Lys Met Leu Val Ile
       290                 295                 300
   Asp Pro Ala Lys Arg Ile Ser Val Asp Asp Ala Leu Gln His Pro Tyr
   305                 310                 315                 320
   Ile Asn Val Trp Tyr Asp Pro Ala Glu Val Glu Ala Pro Pro Pro Gln
                   325                 330                 335
   Ile Tyr Asp Lys Gln Leu Asp Glu Arg Glu His Thr Ile Glu Glu Trp
               340                 345                 350
   Lys Glu Leu Ile Tyr Lys Glu Val Met Asn Ser Glu Glu Lys Thr Lys
           355                 360                 365
   Asn Gly Val Val Lys Gly Gln Pro Ser Pro Ser Ala Gln Val Gln Gln
       370                 375                 380
<210> 60
   <211> 1975
   <212> DNA
   <213> Rattus norvegicus
<400> 60
   ccctccttat tccggtttgg aatgtggcta atgaaagccc agtaggagga tttctggggc 60
   aaacaggtgg accaggatcc tggttctcag gcacggaatg gctattgtga gagcgccacc 120
   agcaggacca tcgcagatct tggttatggc tgctcacgca agaggctgtt gatgtagacc 180
   ccctttcccg tagatgagaa atcacacgag cagtggtatt tatgagcctc catttcttat 240
   actactgcag tgaaccaacc ttggatgtga aaattgcctt ttgtcaggtg tgtgttcctt 300
   acaggtaaaa caaagggatt cgacaaacac gtggatgtgt cttctgttgt caaacattac 360
   aacatgagca aaagcaaggt agataaccag ttctacagtg tggaagtggg agactcaacc 420
   ttcacagttc taaagcgcta ccagaacctg aagccgatcg gctctggggc tcagggaata 480
   gtttgtgctg cgtatgacgc tgtcctcgac agaaatgtgg ccattaagaa gctcagcaga 540
   cccttccaga accaaactca tgccaagagg gcttaccggg agctggtcct catgaagtgt 600
   gtgaaccata aaaacattat tagcttatta aatgtcttta caccccagaa aacactggag 660
   gagttccaag atgtttactt agtgatggaa ctgatggacg ccaacttgtg tcaggtgatt 720
   cagatggagc tggaccacga gcggatgtcg tacttgctgt accagatgct gtcggcgatc 780
   aaacacctcc actccgctgg gatcatccac aggacttaaa aacccagtaa catcgtagtc 840
   aagtctgatt gcacactgaa aatcctggac tttggactgg ccaggacagc gggcacaagc 900
   ttcatgatga ctccgtatgt ggtgacgaga tattacagag cccccgaggt catcctgggc 960
   atgggctaca aggagaacgt ggacatatgg tctgtgggct gcatcatggg agaaatggtt 1020
   cgtcacaaaa tcctctttcc cggaagggac tatattgacc agtggaacaa agtcatagag 1080
   cagctaggaa ctccgtgtcc agaattcatg aagaaattgc agcccaccgt cagaaactac 1140
   gtggagaacc ggcccaagta tgcaggcctc accttcccca agctctttcc agattccctc 1200
   ttcccagcgg attccgagca caataaactt aaagccagcc aagccaggga cttgttgtca 1260
   aagatgttag tgattgaccc agcgaagagg atatcggtgg atgacgcatt gcagcatccg 1320
   tacatcaacg tttggtacga ccctgctgaa gtggaggcgc ctccgcctca gatatatgac 1380
   aagcaattgg atgaaaggga gcacaccatc gaagaatgga aagaactcat ctacaaggaa 1440
   gtaatgaact cagaagagaa gactaagaac ggcgtagtca aaggccagcc ctcaccttca 1500
   ggtgcagcag tgaacagcag tgagagtctc cctccatcct catctgtcaa cgacatctcc 1560
   tccatgtcca ccgaccagac cctcgcatcc gacactgaca gcagcctgga agcctcggcg 1620
   ggaccgctgg gttgttgcag gtgactagcc gcctgcctgc gaaacccagc gttcttcagg 1680
   agatgacgcc atgatagaac acagcgcaca tgcacacaca cagagcttgt acacacacac 1740
   acacacacac acacacgcac gcacgcacgc acgcaagcac gcacgcacgc acaaatgcac 1800
   tcacgcaatg tcaagaaaaa aaaaagtagc gagagagagc gagagagcca acgtaaaact 1860
   aagttaaatc tttctgcgtg cttctccaga gttctgtatc gcagctgagc tgaaatgtat 1920
   acttaacttc tagtcgcgct cgctcgactt tggtctccct ccggcagtgc ttact 1975
<210> 61
   <211> 426
   <212> PRT
   <213> Rattus norvegicus
<400> 61
   Met Ser Lys Ser Lys Val Asp Asn Gln Phe Tyr Ser Val Glu Val Gly
   1               5                   10                  15
   Asp Ser Thr Phe Thr Val Leu Lys Arg Tyr Gln Asn Leu Lys Pro Ile
               20                  25                  30
   Gly Ser Gly Ala Gln Gly Ile Val Cys Ala Ala Tyr Asp Ala Val Leu
           35                  40                  45
   Asp Arg Asn Val Ala Ile Lys Lys Leu Ser Arg Pro Phe Gln Asn Gln
       50                  55                  60
   Thr His Ala Lys Arg Ala Tyr Arg Glu Leu Val Leu Met Lys Cys Val
   65 70 75 80
   Asn His Lys Asn Ile Ile Ser Leu Leu Asn Val Phe Thr Pro Gln Lys
   85 90 95
   Thr Leu Glu Glu Phe Gln Asp Val Tyr Leu Val Met Glu Leu Met Asp
   100 105 110
   Ala Asn Leu Cys Gln Val Ile Gln Met Glu Leu Asp His Glu Arg Met
   115 120 125
   Ser Tyr Leu Leu Tyr Gln Met Leu Ser Ala Ile Lys His Leu His Ser
   130 135 140
   Ala Gly Ile Ile His Arg Asp Leu Lys Pro Ser Asn Ile Val Val Lys
   145 150 155 160
   Ser Asp Cys Thr Leu Lys Ile Leu Asp Phe Gly Leu Ala Arg Thr Ala
   165 170 175
   Gly Thr Ser Phe Met Met Thr Pro Tyr Val Val Thr Arg Tyr Tyr Arg
   180 185 190
   Ala Pro Glu Val Ile Leu Gly Met Gly Tyr Lys Glu Asn Val Asp Ile
   195 200 205
   Trp Ser Val Gly Cys Ile Met Gly Glu Met Val Arg His Lys Ile Leu
   210 215 220
   Phe Pro Gly Arg Asp Tyr Ile Asp Gln Trp Asn Lys Val Ile Glu Gln
   225 230 235 240
   Leu Gly Thr Pro Cys Pro Glu Phe Met Lys Lys Leu Gln Pro Thr Val
   245 250 255
   Arg Asn Tyr Val Glu Asn Arg Pro Lys Tyr Ala Gly Leu Thr Phe Pro
   260 265 270
   Lys Leu Phe Pro Asp Ser Leu Phe Pro Ala Asp Ser Glu His Asn Lys
   275 280 285
   Leu Lys Ala Ser Gln Ala Arg Asp Leu Leu Ser Lys Met Leu Val Ile
   290 295 300
   Asp Pro Ala Lys Arg Ile Ser Val Asp Asp Ala Leu Gln His Pro Tyr
   305 310 315 320
   Ile Asn Val Trp Tyr Asp Pro Ala Glu Val Glu Ala Pro Pro Pro Gln
   325 330 335
   Ile Tyr Asp Lys Gln Leu Asp Glu Arg Glu His Thr Ile Glu Glu Trp
   340 345 350
   Lys Glu Leu Ile Tyr Lys Glu Val Met Asn Ser Glu Glu Lys Thr Lys
   355 360 365
   Asn Gly Val Val Lys Gly Gln Pro Ser Pro Ser Gly Ala Ala Val Asn
   370 375 380
   Ser Ser Glu Ser Leu Pro Pro Ser Ser Ser Val Asn Asp Ile Ser Ser
   385 390 395 400
   Met Ser Thr Asp Gln Thr Leu Ala Ser Asp Thr Asp Ser Ser Leu Glu
                   405                 410                 415
   Ala Ser Ala Gly Pro Leu Gly Cys Cys Arg
               420                 425
<210> 62
   <211> 1093
   <212> DNA
   <213> Homo sapiens
<400> 62
   ccctggctcc ccagctgcgc tgccccgggc gtccacgccc tgcgggctta gcgggttcag 60
   tgggctcaat ctgcgcagcg ccacctccat gttgaccaag cctctacagg ggcctcccgc 120
   gccccccggg actcccacgc cgccgccagg aggcaaggat cgggaagcgt tcgaggccga 180
   gtatcgactc ggccccctcc tgggtaaggg gggctttggc accgtcttcg caggacaccg 240
   cctcacagat cgactccagg tggccatcaa agtgattccc cggaatcgtg tgctgggctg 300
   gtcccccttg tcagactcag tcacatgccc actcgaagtc gcactgctat ggaaagtggg 360
   tgcaggtggt gggcaccctg gcgtgatccg cctgcttgac tggtttgaga cacaggaggg 420
   cttcatgctg gtcctcgagc ggcctttgcc cgcccaggat ctctttgact atatcacaga 480
   gaagggccca ctgggtgaag gcccaagccg ctgcttcttt ggccaagtag tggcagccat 540
   ccagcactgc cattcccgtg gagttgtcca tcgtgacatc aaggatgaga acatcctgat 600
   agacctacgc cgtggctgtg ccaaactcat tgattttggt tctggtgccc tgcttcatga 660
   tgaaccctac actgactttg atgggacaag ggtgtacagc cccccagagt ggatctctcg 720
   acaccagtac catgcactcc cggccactgt ctggtcactg ggcatcctcc tctatgacat 780
   ggtgtgtggg gacattccct ttgagaggga ccaggagatt ctggaagctg agctccactt 840
   cccagcccat gtctccccag actgctgtgc cctaatccgc cggtgcctgg cccccaaacc 900
   ttcttcccga ccctcactgg aagagatcct gctggacccc tggatgcaaa caccagccga 960
   ggatgtaccc ctcaacccct ccaaaggagg ccctgcccct ttggcctggt ccttgctacc 1020
   ctaagcctgg cctggcctgg cctggccccc aatggtcaga agagccatcc catggccctg 1080
   tcacagggat aga                                                    1093
<210> 63
   <211> 311
   <212> PRT
   <213> Homo sapiens
<400> 63
   Met Leu Thr Lys Pro Leu Gln Gly Pro Pro Ala Pro Pro Gly Thr Pro
   1               5                   10                  15
   Thr Pro Pro Pro Gly Gly Lys Asp Arg Glu Ala Phe Glu Ala Glu Tyr
               20                  25                  30
   Arg Leu Gly Pro Leu Leu Gly Lys Gly Gly Phe Gly Thr Val Phe Ala
           35                  40                  45
   Gly His Arg Leu Thr Asp Arg Leu Gln Val Ala Ile Lys Val Ile Pro
       50                  55                  60
   Arg Asn Arg Val Leu Gly Trp Ser Pro Leu Ser Asp Ser Val Thr Cys
   65                  70                  75                  80
   Pro Leu Glu Val Ala Leu Leu Trp Lys Val Gly Ala Gly Gly Gly His
                   85                  90                  95
   Pro Gly Val Ile Arg Leu Leu Asp Trp Phe Glu Thr Gln Glu Gly Phe
               100                 105                 110
   Met Leu Val Leu Glu Arg Pro Leu Pro Ala Gln Asp Leu Phe Asp Tyr
   115 120 125
   Ile Thr Glu Lys Gly Pro Leu Gly Glu Gly Pro Ser Arg Cys Phe Phe
   130 135 140
   Gly Gln Val Val Ala Ala Ile Gln His Cys His Ser Arg Gly Val Val
   145 150 155 160
   His Arg Asp Ile Lys Asp Glu Asn Ile Leu Ile Asp Leu Arg Arg Gly
   165 170 175
   Cys Ala Lys Leu Ile Asp Phe Gly Ser Gly Ala Leu Leu His Asp Glu
   180 185 190
   Pro Tyr Thr Asp Phe Asp Gly Thr Arg Val Tyr Ser Pro Pro Glu Trp
   195 200 205
   Ile Ser Arg His Gln Tyr His Ala Leu Pro Ala Thr Val Trp Ser Leu
   210 215 220
   Gly Ile Leu Leu Tyr Asp Met Val Cys Gly Asp Ile Pro Phe Glu Arg
   225 230 235 240
   Asp Gln Glu Ile Leu Glu Ala Glu Leu His Phe Pro Ala His Val Ser
   245 250 255
   Pro Asp Cys Cys Ala Leu Ile Arg Arg Cys Leu Ala Pro Lys Pro Ser
   260 265 270
   Ser Arg Pro Ser Leu Glu Glu Ile Leu Leu Asp Pro Trp Met Gln Thr
   275 280 285
   Pro Ala Glu Asp Val Pro Leu Asn Pro Ser Lys Gly Gly Pro Ala Pro
   290 295 300
   Leu Ala Trp Ser Leu Leu Pro
   305 310
<210> 64
   <211> 20.39
   <212> DNA
   <213> Mus musculus
<400> 64
   agtagcgttg gggctggcgc gcgcgacgaa tcttaacgct gcgccgtctg caggcgcttc 60
   cgggccgcca gattccctgc cttccaccct ggcgcccccc agccctggct ctccggccgc 120
   tctgccccgg gcatccacgc cctgcgggct cagcggattc agcgggctca atatacgcag 180
   cacctcctcc atgttgacca agcctctgca ggggcatcct tcgccccctg tgacccccac 240
   gcagcctcca ggaggcaagg atcgggcagc tttcgaggcc gaataccgac ttggccccct 300
   cctgggtaag ggaggctttg gcaccgtctt cgcgggacac cgcgtcacgg atagacgtca 360
   ggtggccatc aaagtaatct cccggaaccg tgtgctaggc tggtccaccg tgtcagactc 420
   agtcacctgc ccacttgagg ttgcgctgct gtggaaggtg ggtgaaggca atggccatcc 480
   gggtgtgata cgccttcttg actggttcga aacacccgaa ggcttcatgc tggtccttga 540
   gcggcctatg cctgctcagg atctcttcga ctatatcaca gagaaggggc cgctgggtga 600
   aagctgtagc cgcagcttct ttacccaagt cgtggcagct gtccagcact gccacgcccg 660
   tggagttgtc catcgggata tcaaggatga gaacatcctg atcgacctat gccggggttc 720
   cattaaactc attgattttg gttccggcgc cctgcttcac gatgagccgt acactgactt 780
   tgatgggaca agagtgtata gccctccaga gtggatctcg cgacaccagt accatgccct 840
   gccagcgacc gtctggtcac taggtgtcct actctatgac atggtctgtg gggacattcc 900
   cttcgagaga gaccaggaga ttctggaggc tgagctgcac ttccctgctc atgtctcccc 960
   cgattgctgt gccctaatcc gccggtgcct ggcccctaaa ccctgctccc gaccctcact 1020
   ggaggagatt ctgctggacc cctggatgca atcaccagct gaagaaaagc ccatcaactc 1080
   ctccaaagga agccccaccc ccttgccctg gtccctgctt ccctagaccc agccaggctc 1140
   tctggttgga atcccatggc acgctacagg gatagatgga catctgttga cctggttata 1200
   caggttgtta aaaatccagt attactaagg tcagggaata gggatcaggg gtcagaagaa 1260
   gatgatagcc aagtctgccc agttcctatc caatctggaa ggagctctcc tcccagaacc 1320
   gtggtcccta atttgggagg gggagttact tgtttcttgt tctgttaaag atgtttattt 1380
   ggttggattc cttccctctg ggccctaaga ttcttaatct gacattgtag tccctacact 1440
   ggcacttcct atactaccac aaaccacgta cacacttagt tcaaatgctc tcacttgggc 1500
   aagggtgctt tccttaaaat tgggcccatc cccagcagct ctttaggcaa agggccttcc 1560
   ctgggatctc attctgttgc tgcctgcctg ccttagccca gacttggtga ttctggggga 1620
   ggtactgctc attaattagt taatatccca cgcttccttc ctttttcttc ctttgtttgg 1680
   tgagggaccc tactgttacc caagtgctct tattccggtg agaagaacct cacttccatg 1740
   atttgggaag gaatgggaga tggcatcacc ggacaccacc agagactagg atgggatgga 1800
   tggtttgggg gggatgagat ggaggaaata agtcttattg ttgttctcct ggggcccccc 1860
   ctttccaact tttgctgcct cctcctgagc caggattgtc cagtggttga aatgtaaata 1920
   attctgtact ggaggtggag agcggcaagt gccctcctgt cccttcctcc cccctgcctg 1980
   gattatttaa aaagccatgt gtggaaacct atttaataaa taatgaagtc acaaaaaag 2039
<210> 65
   <211> 370
   <212> PRT
   <213> Mus musculus
<400> 65
   Met Ala Arg Ala Thr Asn Leu Asn Ala Ala Pro Ser Ala Gly Ala Ser
   1               5                   10                  15
   Gly Pro Pro Asp Ser Leu Pro Ser Thr Leu Ala Pro Pro Ser Pro Gly
               20                  25                  30
   Ser Pro Ala Ala Leu Pro Arg Ala Ser Thr Pro Cys Gly Leu Ser Gly
           35                  40                  45
   Phe Ser Gly Leu Asn Ile Arg Ser Thr Ser Ser Met Leu Thr Lys Pro
       50                  55                  60
   Leu Gln Gly His Pro Ser Pro Pro Val Thr Pro Thr Gln Pro Pro Gly
   65                  70                  75                  80
   Gly Lys Asp Arg Ala Ala Phe Glu Ala Glu Tyr Arg Leu Gly Pro Leu
                   85                  90                  95
   Leu Gly Lys Gly Gly Phe Gly Thr Val Phe Ala Gly His Arg Val Thr
               100                 105                 110
   Asp Arg Arg Gln Val Ala Ile Lys Val Ile Ser Arg Asn Arg Val Leu
           115                 120                 125
   Gly Trp Ser Thr Val Ser Asp Ser Val Thr Cys Pro Leu Glu Val Ala
       130                 135                 140
   Leu Leu Trp Lys Val Gly Glu Gly Asn Gly His Pro Gly Val Ile Arg
   145                 150                 155                 160
   Leu Leu Asp Trp Phe Glu Thr Pro Glu Gly Phe Met Leu Val Leu Glu
                   165                 170                 175
   Arg Pro Met Pro Ala Gln Asp Leu Phe Asp Tyr Ile Thr Glu Lys Gly
               180                 185                 190
   Pro Leu Gly Glu Ser Cys Ser Arg Ser Phe Phe Thr Gln Val Val Ala
           195                 200                 205
   Ala Val Gln His Cys His Ala Arg Gly Val Val His Arg Asp Ile Lys
   210 215 220
   Asp Glu Asn Ile Leu Ile Asp Leu Cys Arg Gly Ser Ile Lys Leu Ile
   225 230 235 240
   Asp Phe Gly Ser Gly Ala Leu Leu His Asp Glu Pro Tyr Thr Asp Phe
   245 250 255
   Asp Gly Thr Arg Val Tyr Ser Pro Pro Glu Trp Ile Ser Arg His Gln
   260 265 270
   Tyr His Ala Leu Pro Ala Thr Val Trp Ser Leu Gly Val Leu Leu Tyr
   275 280 285
   Asp Met Val Cys Gly Asp Ile Pro Phe Glu Arg Asp Gln Glu Ile Leu
   290 295 300
   Glu Ala Glu Leu His Phe Pro Ala His Val Ser Pro Asp Cys Cys Ala
   305 310 315 320
   Leu Ile Arg Arg Cys Leu Ala Pro Lys Pro Cys Ser Arg Pro Ser Leu
   325 330 335
   Glu Glu Ile Leu Leu Asp Pro Trp Met Gln Ser Pro Ala Glu Glu Lys
   340 345 350
   Pro Ile Asn Ser Ser Lys Gly Ser Pro Thr Pro Leu Pro Trp Ser Leu
   355 360 365
   Leu Pro
   370
<210> 66
   <211> 345
   <212> PRT
   <213> Mus musculus
<400> 66
   Met Ala Pro Pro Ser Pro Gly Ser Pro Ala Ala Leu Pro Arg Ala Ser
    1               5                  10                  15
   Thr Pro Cys Gly Leu Ser Gly Phe Ser Gly Leu Asn Ile Arg Ser Thr
               20                  25                  30
   Ser Ser Met Leu Thr Lys Pro Leu Gln Gly His Pro Ser Pro Pro Val
           35                  40                  45
   Thr Pro Thr Gln Pro Pro Gly Gly Lys Asp Arg Ala Ala Phe Glu Ala
       50                  55                  60
   Glu Tyr Arg Leu Gly Pro Leu Leu Gly Lys Gly Gly Phe Gly Thr Val
   65                  70                  75                  80
   Phe Ala Gly His Arg Val Thr Asp Arg Arg Gln Val Ala Ile Lys Val
                   85                  90                  95
   Ile Ser Arg Asn Arg Val Leu Gly Trp Ser Thr Val Ser Asp Ser Val
               100                 105                 110
   Thr Cys Pro Leu Glu Val Ala Leu Leu Trp Lys Val Gly Glu Gly Asn
   115 120 125
   Gly His Pro Gly Val Ile Arg Leu Leu Asp Trp Phe Glu Thr Pro Glu
   130 135 140
   Gly Phe Met Leu Val Leu Glu Arg Pro Met Pro Ala Gln Asp Leu Phe
   145 150 155 160
   Asp Tyr Ile Thr Glu Lys Gly Pro Leu Gly Glu Ser Cys Ser Arg Ser
   165 170 175
   Phe Phe Thr Gln Val Val Ala Ala Val Gln His Cys His Ala Arg Gly
   180 185 190
   Val Val His Arg Asp Ile Lys Asp Glu Asn Ile Leu Ile Asp Leu Cys
   195 200 205
   Arg Gly Ser Ile Lys Leu Ile Asp Phe Gly Ser Gly Ala Leu Leu His
   210 215 220
   Asp Glu Pro Tyr Thr Asp Phe Asp Gly Thr Arg Val Tyr Ser Pro Pro
   225 230 235 240
   Glu Trp Ile Ser Arg His Gln Tyr His Ala Leu Pro Ala Thr Val Trp
   245 250 255
   Ser Leu Gly Val Leu Leu Tyr Asp Met Val Cys Gly Asp Ile Pro Phe
   260 265 270
   Glu Arg Asp Gln Glu Ile Leu Glu Ala Glu Leu His Phe Pro Ala His
   275 280 285
   Val Ser Pro Asp Cys Cys Ala Leu Ile Arg Arg Cys Leu Ala Pro Lys
   290 295 300
   Pro Cys Ser Arg Pro Ser Leu Glu Glu Ile Leu Leu Asp Pro Trp Met
   305 310 315 320
   Gln Ser Pro Ala Glu Glu Lys Pro Ile Asn Ser Ser Lys Gly Ser Pro
   325 330 335
   Thr Pro Leu Pro Trp Ser Leu Leu Pro
   340 345
<210> 67
   <211> 311
   <212> PRT
   <213> Mus musculus
<400> 67
   Met Leu Thr Lys Pro Leu Gln Gly His Pro Ser Pro Pro Val Thr Pro
    1               5                  10                  15
   Thr Gln Pro Pro Gly Gly Lys Asp Arg Ala Ala Phe Glu Ala Glu Tyr
               20                  25                  30
   Arg Leu Gly Pro Leu Leu Gly Lys Gly Gly Phe Gly Thr Val Phe Ala
           35                  40                  45
   Gly His Arg Val Thr Asp Arg Arg Gln Val Ala Ile Lys Val Ile Ser
           50                  55                  60
   Arg Asn Arg Val Leu Gly Trp Ser Thr Val Ser Asp Ser Val Thr Cys
   65                  70                  75                  80
   Pro Leu Glu Val Ala Leu Leu Trp Lys Val Gly Glu Gly Asn Gly His
                   85                  90                  95
   Pro Gly Val Ile Arg Leu Leu Asp Trp Phe Glu Thr Pro Glu Gly Phe
               100                 105                 110
   Met Leu Val Leu Glu Arg Pro Met Pro Ala Gln Asp Leu Phe Asp Tyr
           115                 120                 125
   Ile Thr Glu Lys Gly Pro Leu Gly Glu Ser Cys Ser Arg Ser Phe Phe
       130                 135                 140
   Thr Gln Val Val Ala Ala Val Gln His Cys His Ala Arg Gly Val Val
   145                 150                 155                 160
   His Arg Asp Ile Lys Asp Glu Asn Ile Leu Ile Asp Leu Cys Arg Gly
                   165                 170                 175
   Ser Ile Lys Leu Ile Asp Phe Gly Ser Gly Ala Leu Leu His Asp Glu
               180                 185                 190
   Pro Tyr Thr Asp Phe Asp Gly Thr Arg Val Tyr Ser Pro Pro Glu Trp
           195                 200                 205
   Ile Ser Arg His Gln Tyr His Ala Leu Pro Ala Thr Val Trp Ser Leu
       210                 215                 220
   Gly Val Leu Leu Tyr Asp Met Val Cys Gly Asp Ile Pro Phe Glu Arg
   225                 230                 235                 240
   Asp Gln Glu Ile Leu Glu Ala Glu Leu His Phe Pro Ala His Val Ser
                   245                 250                 255
   Pro Asp Cys Cys Ala Leu Ile Arg Arg Cys Leu Ala Pro Lys Pro Cys
               260                 265                 270
   Ser Arg Pro Ser Leu Glu Glu Ile Leu Leu Asp Pro Trp Met Gln Ser
           275                 280                 285
   Pro Ala Glu Glu Lys Pro Ile Asn Ser Ser Lys Gly Ser Pro Thr Pro
       290                 295                 300
   Leu Pro Trp Ser Leu Leu Pro
   305                 310
<210> 68
   <211> 1793
   <212> DNA
   <213> Rattus norvegicus
<400> 68
   gcgcgacgaa tcttaacgct gcgccgtctg caggcgcttc cgggccacca gattccctgc 60
   cttccaccct ggcgcccccc agccctggct ctccggccgc tctgccccgg gcatccacgc 120
   cctgcgggct cagcggattc agcgggctca atctgcgcag cacctcctcc atgttgacca 180
   agcctctgca ggggcttcct tcgccccctg tgacccccac gcagcctcca ggaggcaagg 240
   atcgggcagc tttcgaggcc gaataccgac ttggccctct cctgggtaag ggaggctttg 300
   gcaccgtctt cgcgggacac cgcgtcacgg atagacgtca ggtggccatc aaagtaatct 360
   cccggaaccg tgtgctaggc tggtccactg tgtcagactc agtcacctgc ccacttgagg 420
   tggcactgct gtggaaggtg ggtgaaggta atggtcatcc gggtgtgatc cgcctgcttg 480
   actggtttga gacaccagaa ggcttcatgc tggtccttga gcggcctatg cctgcccagg 540
   atctcttcga ctacatcaca gagaagggtc cgctgggtga aagctgtagc cgcagcttct 600
   tcacccaagt agtggcagct gtccagcact gccacgcccg tggcgttgtc catcgtgaca 660
   tcaaggatga gaacatcctg atagacctgt gccggggttc tgttaaactc attgatttcg 720
   gttctggtgc gctgcttcat gatgaaccat acacggactt tgatgggaca agggtgtaca 780
   gccctcccga gtggatctcg agacaccagt accatgccct tccagctact gtctggtcac 840
   tgagtgtcct actctatgac atggtgtgtg gggacattcc cttcgagaga gatcaggaga 900
   ttctggaggc tgagcttcac ttccctgctc acgtgtcccc agattgctgt gccctaatcc 960
   gacggtgcct ggcccctaaa ccctgttccc gaccctcact ggaggagata ctgctggacc 1020
   cctggatgca aacaccagct gaagaaaaac ccatcaaccc ctccaaagga agccccaccc 1080
   ccttgccctg gtctctgggt ccctaggccc agccaggctc tctggttgga atcccatggt 1140
   catgatacag ggatagatgg acatctgttg acctggttgt acaggttgct aaaaatccag 1200
   tattactaag gtcagggaat agggattgag ggtcagaaga tagccaagtc tgcccagttc 1260
   ctatcccaat cccgggaagg agctctcctc ccagaacctg tggtccctaa tttgggaggg 1320
   ggaagttact tgtttcttgt tctgttaagg atgtttattt ggttggattc cttcccttct 1380
   ggtccctaag attcttattc tgacattgta gtccctacac tggcaccccc tatactacta 1440
   ccacaaactt agacacttag ttcaaatgct ctcacttggg caagggtgct ttccttaaga 1500
   ttacccccac ccccagcagc tctttagcaa aagggcctta ccctgggatt tcattcagtt 1560
   gctgctttgc ctgccttagc ccagaagtga ttattctggg gaggtaacgc cccattaatt 1620
   agaatcccag tgctcccccc tttttttccc tttgtttggt gaggggaccc tactgttatc 1680
   ccaagtgctc ttattctggt gagaagaacc ttacttccat aatttgggaa ggaatgggag 1740
   atggacatca ccggacacca ccagagatta ggatgggatg gatggttttg ggg 1793
<210> 69
   <211> 367
   <212> PRT
   <213> Rattus norvegicus
<400> 69
   Ala Thr Asn Leu Asn Ala Ala Pro Ser Ala Gly Ala Ser Gly Pro Pro
    1               5                  10                  15
   Asp Ser Leu Pro Ser Thr Leu Ala Pro Pro Ser Pro Gly Ser Pro Ala
               20                  25                  30
   Ala Leu Pro Arg Ala Ser Thr Pro Cys Gly Leu Ser Gly Phe Ser Gly
           35                  40                  45
   Leu Asn Leu Arg Ser Thr Ser Ser Met Leu Thr Lys Pro Leu Gln Gly
       50                  55                  60
   Leu Pro Ser Pro Pro Val Thr Pro Thr Gln Pro Pro Gly Gly Lys Asp
   65                  70                  75                  80
   Arg Ala Ala Phe Glu Ala Glu Tyr Arg Leu Gly Pro Leu Leu Gly Lys
                   85                  90                  95
   Gly Gly Phe Gly Thr Val Phe Ala Gly His Arg Val Thr Asp Arg Arg
               100                 105                 110
   Gln Val Ala Ile Lys Val Ile Ser Arg Asn Arg Val Leu Gly Trp Ser
           115                 120                 125
   Thr Val Ser Asp Ser Val Thr Cys Pro Leu Glu Val Ala Leu Leu Trp
       130                 135                 140
   Lys Val Gly Glu Gly Asn Gly His Pro Gly Val Ile Arg Leu Leu Asp
   145                 150                 155                 160
   Trp Phe Glu Thr Pro Glu Gly Phe Met Leu Val Leu Glu Arg Pro Met
                   165                 170                 175
   Pro Ala Gln Asp Leu Phe Asp Tyr Ile Thr Glu Lys Gly Pro Leu Gly
               180                 185                 190
   Glu Ser Cys Ser Arg Ser Phe Phe Thr Gln Val Val Ala Ala Val Gln
           195                 200                 205
   His Cys His Ala Arg Gly Val Val His Arg Asp Ile Lys Asp Glu Asn
       210                 215                 220
   Ile Leu Ile Asp Leu Cys Arg Gly Ser Val Lys Leu Ile Asp Phe Gly
   225                 230                 235                 240
   Ser Gly Ala Leu Leu His Asp Glu Pro Tyr Thr Asp Phe Asp Gly Thr
                   245                 250                 255
   Arg Val Tyr Ser Pro Pro Glu Trp Ile Ser Arg His Gln Tyr His Ala
               260                 265                 270
   Leu Pro Ala Thr Val Trp Ser Leu Ser Val Leu Leu Tyr Asp Met Val
           275                 280                 285
   Cys Gly Asp Ile Pro Phe Glu Arg Asp Gln Glu Ile Leu Glu Ala Glu
       290                 295                 300
   Leu His Phe Pro Ala His Val Ser Pro Asp Cys Cys Ala Leu Ile Arg
   305                 310                 315                 320
   Arg Cys Leu Ala Pro Lys Pro Cys Ser Arg Pro Ser Leu Glu Glu Ile
                   325                 330                 335
   Leu Leu Asp Pro Trp Met Gln Thr Pro Ala Glu Glu Lys Pro Ile Asn
               340                 345                 350
   Pro Ser Lys Gly Ser Pro Thr Pro Leu Pro Trp Ser Leu Gly Pro
           355                 360                 365
<210> 70
   <211> 6648
   <212> DNA
   <213> Mus musculus
<400> 70
   atggcgacac ggagcagcag gagggagtcg agactccctt tcctattcgc cctggtcgcg 60
   ctgctacccc gcggggctct cggcgggggc tggacgcaga gattgcacgg tggcccggcg 120
   cccttgcccc aggaccgggg cttcttcgtg gtgcagggcg acccgcgcga tctccggcta 180
   ggaacgcacg gggatgcccc gggggcgagc ccggcggcca ggaagccact caggacccgg 240
   cggagcgctg ctctgcagcc ccagcctatc caggtgtatg gacaggtcag tctgaatgac 300
   tcccacaatc agatggtggt gcactgggcc ggagagaaaa gcaacgtgat cgtggcatta 360
   gcccgagaca gcctggcgtt ggcaaggccc aagagcagtg atgtgtatgt gtcttacgac 420
   tatggaaaat cattcagtaa gatttcagag aagctgaact ttggcgtggg caacaacagc 480
   gaagctgtga tctcccagtt ctaccacagc cctgccgaca acaagaggta catctttgtg 540
   gatgcttacg cccaatacct ctggatcacg tttgacttct gcagcactat ccatggcttc 600
   tccatcccat tccgggcggc tgatctcctc ctccatagca aggcctccaa cctcctcttg 660
   ggctttgaca ggtctcaccc caacaagcag ctatggaagt cagatgattt tggccagacc 720
   tggatcatga ttcaggaaca cgtgaagtcc ttctcttggg gaatcgaccc ctatgatcag 780
   ccaaacgcta tctacattga gcgacatgaa ccctttgggt tctccacagt gctccgcagt 840
   acagacttct tccagtcccg ggaaaaccag gaagtgatcc tggaggaagt gagagacttt 900
   cagcttcggg acaagtacat gtttgctaca aaggtggtgc atcttccggg cagccagcag 960
   cagtcatctg tccagctctg ggtctccttt ggccggaagc ctatgagagc agcccagttt 1020
   gtcacaaagc atcctattaa tgaatattac atcgctgatg ccgccgagga ccaggtgttt 1080
   gtgtgtgtta gccacagtaa caacagcact aacctataca tctcagaggc ggaggggctg 1140
   aagttctccc tctccttgga gaatgtgctg tattacagcc caggaggggc cggcagtgac 1200
   actctggtga ggtattttgc aaatgagccg tttgccgact ttcaccgcgt ggaaggctta 1260
   caaggggtct atattgccac cctgattaat ggttctatga atgaggagaa catgaggtcg 1320
   gtcatcacct ttgacaaagg gggaacctgg gaatttcttc aggccccagc cttcacagga 1380
   tatggagaga aaatcaattg tgagctttct cagggttgtt ccctccacct ggcccagcgc 1440
   ctcagtcaac tgctcaacct ccaacttcgg aggatgccta tcctatccaa ggagtcagcc 1500
   cccggtctca tcattgctac cggctctgtg ggaaagaact tggccagcaa gaccaatgtg 1560
   tacatctcta gcagtgctgg agccaggtgg agagaggctc ttcccggacc tcattactat 1620
   acttggggtg accatggagg catcatcatg gcgattgccc agggcatgga gaccaacgag 1680
   ctgaagtaca gcaccaatga aggggagacc tggaagacgt tcgtcttctc ggagaagcct 1740
   gtgtttgtgt atgggcttct cacagaacct ggtgagaaga gcaccgtctt caccatcttc 1800
   ggctcgaaca aggagagtgt ccacagctgg ctcatcctgc aagtcaatgc cactgatgcc 1860
   ttgggggttc cttgcacaga aaacgactac aagctgtggt ccccatctga tgagcgagga 1920
   aatgagtgtt tgctaggaca caagacagtt ttcaaaagga ggacccctca cgccacatgc 1980
   tttaatgggg aagactttga ccggccagtg gttgtgtcca actgctcctg cacccgggag 2040
   gactacgaat gtgactttgg cttcaagatg agcgaagatc tctccttaga ggtttgtgtt 2100
   ccagaccctg agttttctgg gaagccctac agccctcctg tgccttgtcc tgtgggttct 2160
   tcatacagga gaacgagagg ctatcggaag atttctgggg atacgtgcag tggaggagac 2220
   gttgaagcac ggctggaagg ggagctggtc ccctgtcccc ttgcagaaga gaatgagttc 2280
   atcctatacg ccatgcgcaa gtccatctac cgctacgact tggcctcggg agccacagag 2340
   cagcttcctc tctgggcttt gcgggcggct gtggccctgg actttgacta tgaacgcaac 2400
   tgcttgtatt ggtctgacct ggcactggac accatccagc gtctctgctt gaacgggagc 2460
   acagggcagg aggtgatcat caattccggc ctggagaccg tggaggcttt ggccttcgaa 2520
   cccctcagcc aattacttta ctgggtggat gccggcttta aaaagatcga ggtagctaat 2580
   ccagatggtg acttccgact gacaatcgtc aattcatctg tgcttgatcg acccagggct 2640
   ctagtccttg taccccaaga aggggtgatg ttctggactg actgggggga cctgaagcct 2700
   gggatttacc ggagctacat ggacggctct gctgcctacc gccttgtgtc agaggacgtg 2760
   aagtggccca atggcatctc tgtggacagc cagtggatct actggacgga tgcttacctg 2820
   gactgtatcg agcgcatcac tttcagtggc cagcagggtt cagtcatcct tgacagcctc 2880
   ccacacccct acgccatcgc tgtctttaag aatgagatct actgggacga ctggtcacag 2940
   ctcagcatct tcagagcttc gaagcacagc agatcccagg tggagattct agcaagccag 3000
   ctcacgggcc tcatggacat gaaggttttc tacaagggaa agaatgctgg aagcaatgct 3060
   tgcgtccctc agccctgcag tctactgtgc ctccctaagg ccaacaacag caaaagctgc 3120
   aggtgtccag aaggtgtggc cagcagtgtc ctcccctctg gggacctgat gtgtgactgc 3180
   cctcagggct atcaaaggaa gaacaacacc tgtgtcaaag aagagaacac ctgtctccgc 3240
   aaccagtacc gctgcagcaa cggcaactgt atcaacagca tttggtggtg tgatttcgac 3300
   aatgactgtg gagacatgag tgacgagaga aattgcccca cgacggtctg tgatgcagac 3360
   actcagttcc ggtgccagga gtcggggact tgcattccac tctcctataa gtgtgacctt 3420
   gaagatgact gtggagataa cagtgatgag agtcactgtg agatgcacca gtgtcgtagc 3480
   gacgagttca actgcagctc tgggatgtgc atccggtcct cctgggtgtg tgatggagac 3540
   aacgactgta gggactggtc tgatgaagcc aactgtactg ccatctatca tacctgtgag 3600
   gcctccaact tccagtgcca caatgggcac tgcatccccc agcgatgggc atgtgacggt 3660
   gatgcggatt gccaggatgg ctctgatgaa gatcccgtca gctgtgagaa aaagtgcaac 3720
   ggattccact gcccaaacgg cacgtgcatc ccgtccagca agcattgtga tgggctgagg 3780
   gactgccccg acggctccga tgatgagcac tgtgagccct tctgcacgcg cttcatggac 3840
   ttcgtgtgta agaaccgtca gcagtgtctg ttccactcca tggtgtgtga cgggatcgtc 3900
   cagtgccgag atggctctga tgaggatgcg gccttcgctg gatgctcgca agaccccgag 3960
   ttccacaagg agtgcgatga gttcggcttc cagtgtcaga acggagtgtg catcagcttg 4020
   atttggaaat gcgatgggat ggatgactgc ggggactact ctgacgaggc caactgtgaa 4080
   aaccccacag aagccccaaa ctgttcccgt tactttcagt tccactgtga gaatggccac 4140
   tgcatcccca acaggtggaa atgtgacaga gagaacgact gtggggactg gtctgatgag 4200
   aaggattgtg gagattcaca tgttctgccc tccccgaccc ccgggccctc cacgtgtttg 4260
   cccaattact tcgcgtgcag cagcggggcc tgtgtgatgg gcacctgggt gtgcgacggc 4320
   tacagggact gtgcagatgg ctctgatgag gaggcctgcc cctctctagc aaactccact 4380
   gccgcctcca ctcccaccca gctcgggcag tgcgaccgat ttgagttcga gtgtcaccaa 4440
   ccaaagaaat gtatccccaa ctggaagcgc tgtgacggcc accaagattg ccaagacggc 4500
   caggatgaag ccaactgccc cactcacagc accttgacct gtacgagcag ggagttcaag 4560
   tgtgaggacg gcgaggcatg catcgtgctc tcagagcgct gtgatggctt cctggattgc 4620
   tccgatgaga gcgatgagaa ggcctgcagc gatgagttaa ctgtatacaa agtgcagaat 4680
   ctccagtgga cagctgattt ctccggggat gtgactttga cctggatgcg gccaaagaaa 4740
   atgccatctg cttcttgtgt atacaatgtc tactacaggg tggttggaga gagcatatgg 4800
   aagaccctgg aaacccacag caataaaaca agcactgtgc tcaaggtcct gaagccagac 4860
   accacgtacc aggttaaggt ccaggtgcac tgcctcaaca aggtgcacaa caccaatgac 4920
   tttgtgaccc tcaggactcc agaagggttg ccagatgccc ctcggaatct ccagctctcc 4980
   ctgaacaggg aagaggaagg tgtgattctc gggcactggg ctcctcccgt ccacacccat 5040
   ggcctcatcc gtgagtacat tgtggaatac agcaggagtg ggtccaagat gtgggcctcc 5100
   cagagagctg ctagcaactc tacagaaata aagaacttac tgctcaatgc cctctacact 5160
   gtcagagtgg ctgcggtgac tagtcgtggg ataggaaact ggagtgattc taagtccatc 5220
   accaccataa aaggaaaagt gatccaagca ccaaatatcc acattgacag ctacgatgaa 5280
   aattctctga gctttaccct gaccatggat ggtgacatca aggtcaatgg ctacgtggtg 5340
   aacctttcct ggtcatttga tgcccacaaa caagaaaaga aaaccctgag cttccgcgga 5400
   gggagtgcac tgtcacacaa agttagcaac ctgacagctc atacatccta tgagatttct 5460
   gcctgggcca agacagactt gggagatagc ccactggcct ttgagcacat cttgaccaga 5520
   ggcagcagtc caccagctcc tagtctcaaa gccaaggcca tcaatcagac tgcagtggag 5580
   tgcatctgga ctggccccaa gaatgtggtg tatggtattt tctatgccac atccttcctt 5640
   gacctctacc gaaaccctaa gagcgtaact acttcgctcc acaataagac agtcatcgtc 5700
   agcaaggatg agcagtatct gtttctggtc cgggtgttga tcccttacca agggccgtct 5760
   tctgactacg ttgtagtgaa gatgatccca gacagcaggc tcccacctcg ccatctgcac 5820
   gcggtccaca taggcaaaac ctcagcactc atcaaatggg aatcgcccta tgactctcct 5880
   gaccaggact tgttttacgc cattgcagtg aaagatctga tcaggaagac ggacagaagc 5940
   tacaaagtca gatcccgcaa cagtactgtg gagtacagcc taagcaagct ggaacctgga 6000
   gggaaatacc acatcattgt ccagctggga aacatgagca aggattccag cataaaaatc 6060
   accacagttt cgttatcagc acctgatgcc ttaaaaatca taacggaaaa cgaccacgtt 6120
   ctcctctttt ggaaaagcct agcattgaag gaaaagcagt tcaatgaaac caggggctat 6180
   gagatccaca tgtctgacag tgctgtgaat ctcaccgctt acctcgggaa tactacggac 6240
   aatttcttta aagtgtccaa cctgaagatg ggtcataact acactttcac ggtccaagcg 6300
   cggtgtctct ttggtagcca gatctgtggg gagcccgccg tgctgctgta tgatgaactg 6360
   agctcaggtg cagatgcggc agtgatccac gctgcccggt ctactgatgt tgcagctgtg 6420
   gtggttccca tcctgttcct gatcctgctg agcctgggtg tcggcttcgc catcctctac 6480
   acgaagcacc gtcgcttaca gagcagcttc agcgctttcg ccaatagcca ctacagctcc 6540
   aggctgggct cggccatctt ctcctcaggg gatgacctag gagaggacga tgaagatgct 6600
   cccatgatta ctggattctc agacgatgtc cccatggtga tagcgtga 6648
<210> 71
   <211> 2215
   <212> PRT
   <213> Mus musculus
<400> 71
   Met Ala Thr Arg Ser Ser Arg Arg Glu Ser Arg Leu Pro Phe Leu Phe
   1               5                   10                  15
   Ala Leu Val Ala Leu Leu Pro Arg Gly Ala Leu Gly Gly Gly Trp Thr
               20                  25                  30
   Gln Arg Leu His Gly Gly Pro Ala Pro Leu Pro Gln Asp Arg Gly Phe
           35                  40                  45
   Phe Val Val Gln Gly Asp Pro Arg Asp Leu Arg Leu Gly Thr His Gly
       50                  55                  60
   Asp Ala Pro Gly Ala Ser Pro Ala Ala Arg Lys Pro Leu Arg Thr Arg
   65                  70                  75                  80
   Arg Ser Ala Ala Leu Gln Pro Gln Pro Ile Gln Val Tyr Gly Gln Val
                   85                  90                  95
   Ser Leu Asn Asp Ser His Asn Gln Met Val Val His Trp Ala Gly Glu
               100                 105                 110
   Lys Ser Asn Val Ile Val Ala Leu Ala Arg Asp Ser Leu Ala Leu Ala
           115                 120                 125
   Arg Pro Lys Ser Ser Asp Val Tyr Val Ser Tyr Asp Tyr Gly Lys Ser
       130                 135                 140
   Phe Ser Lys Ile Ser Glu Lys Leu Asn Phe Gly Val Gly Asn Asn Ser
   145                 150                 155                 160
   Glu Ala Val Ile Ser Gln Phe Tyr His Ser Pro Ala Asp Asn Lys Arg
                   165                 170                 175
   Tyr Ile Phe Val Asp Ala Tyr Ala Gln Tyr Leu Trp Ile Thr Phe Asp
               180                 185                 190
   Phe Cys Ser Thr Ile His Gly Phe Ser Ile Pro Phe Arg Ala Ala Asp
           195                 200                 205
   Leu Leu Leu His Ser Lys Ala Ser Asn Leu Leu Leu Gly Phe Asp Arg
       210                 215                 220
   Ser His Pro Asn Lys Gln Leu Trp Lys Ser Asp Asp Phe Gly Gln Thr
   225                 230                 235                 240
   Trp Ile Met Ile Gln Glu His Val Lys Ser Phe Ser Trp Gly Ile Asp
                   245                 250                 255
   Pro Tyr Asp Gln Pro Asn Ala Ile Tyr Ile Glu Arg His Glu Pro Phe
               260                 265                 270
   Gly Phe Ser Thr Val Leu Arg Ser Thr Asp Phe Phe Gln Ser Arg Glu
           275                 280                 285
   Asn Gln Glu Val Ile Leu Glu Glu Val Arg Asp Phe Gln Leu Arg Asp
       290                 295                 300
   Lys Tyr Met Phe Ala Thr Lys Val Val His Leu Pro Gly Ser Gln Gln
   305                 310                 315                 320
   Gln Ser Ser Val Gln Leu Trp Val Ser Phe Gly Arg Lys Pro Met Arg
                   325                 330                 335
   Ala Ala Gln Phe Val Thr Lys His Pro Ile Asn Glu Tyr Tyr Ile Ala
               340                 345                 350
   Asp Ala Ala Glu Asp Gln Val Phe Val Cys Val Ser His Ser Asn Asn
           355                 360                 365
   Ser Thr Asn Leu Tyr Ile Ser Glu Ala Glu Gly Leu Lys Phe Ser Leu
       370                 375                 380
   Ser Leu Glu Asn Val Leu Tyr Tyr Ser Pro Gly Gly Ala Gly Ser Asp
   385                 390                 395                 400
   Thr Leu Val Arg Tyr Phe Ala Asn Glu Pro Phe Ala Asp Phe His Arg
                   405                 410                 415
   Val Glu Gly Leu Gln Gly Val Tyr Ile Ala Thr Leu Ile Asn Gly Ser
               420                 425                 430
   Met Asn Glu Glu Asn Met Arg Ser Val Ile Thr Phe Asp Lys Gly Gly
           435                 440                 445
   Thr Trp Glu Phe Leu Gln Ala Pro Ala Phe Thr Gly Tyr Gly Glu Lys
       450                 455                 460
   Ile Asn Cys Glu Leu Ser Gln Gly Cys Ser Leu His Leu Ala Gln Arg
   465 470 475 480
   Leu Ser Gln Leu Leu Asn Leu Gln Leu Arg Arg Met Pro Ile Leu Ser
   485 490 495
   Lys Glu Ser Ala Pro Gly Leu Ile Ile Ala Thr Gly Ser Val Gly Lys
   500 505 510
   Asn Leu Ala Ser Lys Thr Asn Val Tyr Ile Ser Ser Ser Ala Gly Ala
   515 520 525
   Arg Trp Arg Glu Ala Leu Pro Gly Pro His Tyr Tyr Thr Trp Gly Asp
   530 535 540
   His Gly Gly Ile Ile Met Ala Ile Ala Gln Gly Met Glu Thr Asn Glu
   545 550 555 560
   Leu Lys Tyr Ser Thr Asn Glu Gly Glu Thr Trp Lys Thr Phe Val Phe
   565 570 575
   Ser Glu Lys Pro Val Phe Val Tyr Gly Leu Leu Thr Glu Pro Gly Glu
   580 585 590
   Lys Ser Thr Val Phe Thr Ile Phe Gly Ser Asn Lys Glu Ser Val His
   595 600 605
   Ser Trp Leu Ile Leu Gln Val Asn Ala Thr Asp Ala Leu Gly Val Pro
   610 615 620
   Cys Thr Glu Asn Asp Tyr Lys Leu Trp Ser Pro Ser Asp Glu Arg Gly
   625 630 635 640
   Asn Glu Cys Leu Leu Gly His Lys Thr Val Phe Lys Arg Arg Thr Pro
   645 650 655
   His Ala Thr Cys Phe Asn Gly Glu Asp Phe Asp Arg Pro Val Val Val
   660 665 670
   Ser Asn Cys Ser Cys Thr Arg Glu Asp Tyr Glu Cys Asp Phe Gly Phe
   675 680 685
   Lys Met Ser Glu Asp Leu Ser Leu Glu Val Cys Val Pro Asp Pro Glu
   690 695 700
   Phe Ser Gly Lys Pro Tyr Ser Pro Pro Val Pro Cys Pro Val Gly Ser
   705 710 715 720
   Ser Tyr Arg Arg Thr Arg Gly Tyr Arg Lys Ile Ser Gly Asp Thr Cys
   725 730 735
   Ser Gly Gly Asp Val Glu Ala Arg Leu Glu Gly Glu Leu Val Pro Cys
   740 745 750
   Pro Leu Ala Glu Glu Asn Glu Phe Ile Leu Tyr Ala Met Arg Lys Ser
   755 760 765
   Ile Tyr Arg Tyr Asp Leu Ala Ser Gly Ala Thr Glu Gln Leu Pro Leu
   770 775 780
   Trp Gly Leu Arg Ala Ala Val Ala Leu Asp Phe Asp Tyr Glu Arg Asn
   785 790 795 800
   Cys Leu Tyr Trp Ser Asp Leu Ala Leu Asp Thr Ile Gln Arg Leu Cys
                   805                 810                 815
   Leu Asn Gly Ser Thr Gly Gln Glu Val Ile Ile Asn Ser Gly Leu Glu
               820                 825                 830
   Thr Val Glu Ala Leu Ala Phe Glu Pro Leu Ser Gln Leu Leu Tyr Trp
           835                 840                 845
   Val Asp Ala Gly Phe Lys Lys Ile Glu Val Ala Asn Pro Asp Gly Asp
       850                 855                 860
   Phe Arg Leu Thr Ile Val Asn Ser Ser Val Leu Asp Arg Pro Arg Ala
   865                 870                 875                 880
   Leu Val Leu Val Pro Gln Glu Gly Val Met Phe Trp Thr Asp Trp Gly
                   885                 890                 895
   Asp Leu Lys Pro Gly Ile Tyr Arg Ser Tyr Met Asp Gly Ser Ala Ala
               900                 905                 910
   Tyr Arg Leu Val Ser Glu Asp Val Lys Trp Pro Asn Gly Ile Ser Val
           915                 920                 925
   Asp Ser Gln Trp Ile Tyr Trp Thr Asp Ala Tyr Leu Asp Cys Ile Glu
       930                 935                 940
   Arg Ile Thr Phe Ser Gly Gln Gln Gly Ser Val Ile Leu Asp Ser Leu
   945                 950                 955                 960
   Pro His Pro Tyr Ala Ile Ala Val Phe Lys Asn Glu Ile Tyr Trp Asp
                   965                 970                 975
   Asp Trp Ser Gln Leu Ser Ile Phe Arg Ala Ser Lys His Ser Arg Ser
               980                 985                 990
   Gln Val Glu Ile Leu Ala Ser Gln Leu Thr Gly Leu Met Asp Met Lys
           995                 1000                1005
   Val Phe Tyr Lys Gly Lys Asn Ala Gly Ser Asn Ala Cys Val Pro Gln
       1010                1015                1020
   Pro Cys Ser Leu Leu Cys Leu Pro Lys Ala Asn Asn Ser Lys Ser Cys
   1025                1030                1035                1040
   Arg Cys Pro Glu Gly Val Ala Ser Ser Val Leu Pro Ser Gly Asp Leu
                   1045                1050                1055
   Met Cys Asp Cys Pro Gln Gly Tyr Gln Arg Lys Asn Asn Thr Cys Val
               1060                1065                1070
   Lys Glu Glu Asn Thr Cys Leu Arg Asn Gln Tyr Arg Cys Ser Asn Gly
           1075                1080                1085
   Asn Cys Ile Asn Ser Ile Trp Trp Cys Asp Phe Asp Asn Asp Cys Gly
       1090                1095                1100
   Asp Met Ser Asp Glu Arg Asn Cys Pro Thr Thr Val Cys Asp Ala Asp
   1105                1110                1115                1120
   Thr Gln Phe Arg Cys Gln Glu Ser Gly Thr Cys Ile Pro Leu Ser Tyr
                   1125                1130                1135
   Lys Cys Asp Leu Glu Asp Asp Cys Gly Asp Asn Ser Asp Glu Ser His
   1140 1145 1150
   Cys Glu Met His Gln Cys Arg Ser Asp Glu Phe Asn Cys Ser Ser Gly
   1155 1160 1165
   Met Cys Ile Arg Ser Ser Trp Val Cys Asp Gly Asp Asn Asp Cys Arg
   1170 1175 1180
   Asp Trp Ser Asp Glu Ala Asn Cys Thr Ala Ile Tyr His Thr Cys Glu
   1185 1190 1195 1200
   Ala Ser Asn Phe Gln Cys His Asn Gly His Cys Ile Pro Gln Arg Trp
   1205 1210 1215
   Ala Cys Asp Gly Asp Ala Asp Cys Gln Asp Gly Ser Asp Glu Asp Pro
   1220 1225 1230
   Val Ser Cys Glu Lys Lys Cys Asn Gly Phe His Cys Pro Asn Gly Thr
   1235 1240 1245
   Cys Ile Pro Ser Ser Lys His Cys Asp Gly Leu Arg Asp Cys Pro Asp
   1250 1255 1260
   Gly Ser Asp Asp Glu His Cys Glu Pro Phe Cys Thr Arg Phe Met Asp
   1265 1270 1275 1280
   Phe Val Cys Lys Asn Arg Gln Gln Cys Leu Phe His Ser Met Val Cys
   1285 1290 1295
   Asp Gly Ile Val Gln Cys Arg Asp Gly Ser Asp Glu Asp Ala Ala Phe
   1300 1305 1310
   Ala Gly Cys Ser Gln Asp Pro Glu Phe His Lys Glu Cys Asp Glu Phe
   1315 1320 1325
   Gly Phe Gln Cys Gln Asn Gly Val Cys Ile Ser Leu Ile Trp Lys Cys
   1330 1335 1340
   Asp Gly Met Asp Asp Cys Gly Asp Tyr Ser Asp Glu Ala Asn Cys Glu
   1345 1350 1355 1360
   Asn Pro Thr Glu Ala Pro Asn Cys Ser Arg Tyr Phe Gln Phe His Cys
   1365 1370 1375
   Glu Asn Gly His Cys Ile Pro Asn Arg Trp Lys Cys Asp Arg Glu Asn
   1380 1385 1390
   Asp Cys Gly Asp Trp Ser Asp Glu Lys Asp Cys Gly Asp Ser His Val
   1395 1400 1405
   Leu Pro Ser Pro Thr Pro Gly Pro Ser Thr Cys Leu Pro Asn Tyr Phe
   1410 1415 1420
   Ala Cys Ser Ser Gly Ala Cys Val Met Gly Thr Trp Val Cys Asp Gly
   1425 1430 1435 1440
   Tyr Arg Asp Cys Ala Asp Gly Ser Asp Glu Glu Ala Cys Pro Ser Leu
   1445 1450 1455
   Ala Asn Ser Thr Ala Ala Ser Thr Pro Thr Gln Leu Gly Gln Cys Asp
   1460 1465 1470
   Arg Phe Glu Phe Glu Cys His Gln Pro Lys Lys Cys Ile Pro Asn Trp
   1475 1480 1485
   Lys Arg Cys Asp Gly His Gln Asp Cys Gln Asp Gly Gln Asp Glu Ala
   1490 1495 1500
   Asn Cys Pro Thr His Ser Thr Leu Thr Cys Thr Ser Arg Glu Phe Lys
   1505 1510 1515 1520
   Cys Glu Asp Gly Glu Ala Cys Ile Val Leu Ser Glu Arg Cys Asp Gly
   1525 1530 1535
   Phe Leu Asp Cys Ser Asp Glu Ser Asp Glu Lys Ala Cys Ser Asp Glu
   1540 1545 1550
   Leu Thr Val Tyr Lys Val Gln Asn Leu Gln Trp Thr Ala Asp Phe Ser
   1555 1560 1565
   Gly Asp Val Thr Leu Thr Trp Met Arg Pro Lys Lys Met Pro Ser Ala
   1570 1575 1580
   Ser Cys Val Tyr Asn Val Tyr Tyr Arg Val Val Gly Glu Ser Ile Trp
   1585 1590 1595 1600
   Lys Thr Leu Glu Thr His Ser Asn Lys Thr Ser Thr Val Leu Lys Val
   1605 1610 1615
   Leu Lys Pro Asp Thr Thr Tyr Gln Val Lys Val Gln Val His Cys Leu
   1620 1625 1630
   Asn Lys Val His Asn Thr Asn Asp Phe Val Thr Leu Arg Thr Pro Glu
   1635 1640 1645
   Gly Leu Pro Asp Ala Pro Arg Asn Leu Gln Leu Ser Leu Asn Arg Glu
   1650 1655 1660
   Glu Glu Gly Val Ile Leu Gly His Trp Ala Pro Pro Val His Thr His
   1665 1670 1675 1680
   Gly Leu Ile Arg Glu Tyr Ile Val Glu Tyr Ser Arg Ser Gly Ser Lys
   1685 1690 1695
   Met Trp Ala Ser Gln Arg Ala Ala Ser Asn Ser Thr Glu Ile Lys Asn
   1700 1705 1710
   Leu Leu Leu Asn Ala Leu Tyr Thr Val Arg Val Ala Ala Val Thr Ser
   1715 1720 1725
   Arg Gly Ile Gly Asn Trp Ser Asp Ser Lys Ser Ile Thr Thr Ile Lys
   1730 1735 1740
   Gly Lys Val Ile Gln Ala Pro Asn Ile His Ile Asp Ser Tyr Asp Glu
   1745 1750 1755 1760
   Asn Ser Leu Ser Phe Thr Leu Thr Met Asp Gly Asp Ile Lys Val Asn
   1765 1770 1775
   Gly Tyr Val Val Asn Leu Phe Trp Ser Phe Asp Ala His Lys Gln Glu
   1780 1785 1790
   Lys Lys Thr Leu Ser Phe Arg Gly Gly Ser Ala Leu Ser His Lys Val
   1795 1800 1805
   Ser Asn Leu Thr Ala His Thr Ser Tyr Glu Ile Ser Ala Trp Ala Lys
   1810 1815 1820
   Thr Asp Leu Gly Asp Ser Pro Leu Ala Phe Glu His Ile Leu Thr Arg
   1825 1830 1835 1840
   Gly Ser Ser Pro Pro Ala Pro Ser Leu Lys Ala Lys Ala Ile Asn Gln
   1845 1850 1855
   Thr Ala Val Glu Cys Ile Trp Thr Gly Pro Lys Asn Val Val Tyr Gly
   1860 1865 1870
   Ile Phe Tyr Ala Thr Ser Phe Leu Asp Leu Tyr Arg Asn Pro Lys Ser
   1875 1880 1885
   Val Thr Thr Ser Leu His Asn Lys Thr Val Ile Val Ser Lys Asp Glu
   1890 1895 1900
   Gln Tyr Leu Phe Leu Val Arg Val Leu Ile Pro Tyr Gln Gly Pro Ser
   1905 1910 1915 1920
   Ser Asp Tyr Val Val Val Lys Met Ile Pro Asp Ser Arg Leu Pro Pro
   1925 1930 1935
   Arg His Leu His Ala Val His Ile Gly Lys Thr Ser Ala Leu Ile Lys
   1940 1945 1950
   Trp Glu Ser Pro Tyr Asp Ser Pro Asp Gln Asp Leu Phe Tyr Ala Ile
   1955 1960 1965
   Ala Val Lys Asp Leu Ile Arg Lys Thr Asp Arg Ser Tyr Lys Val Arg
   1970 1975 1980
   Ser Arg Asn Ser Thr Val Glu Tyr Ser Leu Ser Lys Leu Glu Pro Gly
   1985 1990 1995 2000
   Gly Lys Tyr His Ile Ile Val Gln Leu Gly Asn Met Ser Lys Asp Ser
   2005 2010 2015
   Ser Ile Lys Ile Thr Thr Val Ser Leu Ser Ala Pro Asp Ala Leu Lys
   2020 2025 2030
   Ile Ile Thr Glu Asn Asp His Val Leu Leu Phe Trp Lys Ser Leu Ala
   2035 2040 2045
   Leu Lys Glu Lys Gln Phe Asn Glu Thr Arg Gly Tyr Glu Ile His Met
   2050 2055 2060
   Ser Asp Ser Ala Val Asn Leu Thr Ala Tyr Leu Gly Asn Thr Thr Asp
   2065 2070 2075 2080
   Asn Phe Phe Lys Val Ser Asn Leu Lys Met Gly His Asn Tyr Thr Phe
   2085 2090 2095
   Thr Val Gln Ala Arg Cys Leu Phe Gly Ser Gln Ile Cys Gly Glu Pro
   2100 2105 2110
   Ala Val Leu Leu Tyr Asp Glu Leu Ser Ser Gly Ala Asp Ala Ala Val
   2115 2120 2125
   Ile His Ala Ala Arg Ser Thr Asp Val Ala Ala Val Val Val Pro Ile
   2130 2135 2140
   Leu Phe Leu Ile Leu Leu Ser Leu Gly Val Gly Phe Ala Ile Leu Tyr
   2145 2150 2155 2160
   Thr Lys His Arg Arg Leu Gln Ser Ser Phe Ser Ala Phe Ala Asn Ser
   2165 2170 2175
   His Tyr Ser Ser Arg Leu Gly Ser Ala Ile Phe Ser Ser Gly Asp Asp
   2180 2185 2190
   Leu Gly Glu Asp Asp Glu Asp Ala Pro Met Ile Thr Gly Phe Ser Asp
   2195 2200 2205
   Asp Val Pro Met Val Ile Ala
   2210 2215
<210> 72
   <211> 6840
   <212> DNA
   <213> Homo sapiens
<400> 72
   ccggcccagc ggctctcctg gcctcgcgct gcacattctc tcctggcggc ggcgccacct 60
   gcagtagcgt tcgcccgaac atggcgacac ggagcagcag gagggagtcg cgactcccgt 120
   tcctattcac cctggtcgca ctgctgccgc ccggagctct ctgcgaagtc tggacgcaga 180
   ggctgcacgg cggcagcgcg cccttgcccc aggaccgggg cttcctcgtg gtgcagggcg 240
   acccgcgcga gctgcggctg tgggcgcgcg gggatgccag gggggcgagc cgcgcggacg 300
   agaagccgct ccggaggaaa cggagcgctg ccctgcagcc cgagcccatc aaggtgtacg 360
   gacaggttag tctgaatgat tcccacaatc agatggtggt gcactgggct ggagagaaaa 420
   gcaacgtgat cgtggccttg gcccgagata gcctggcatt ggcgaggccc aagagcagtg 480
   atgtgtacgt gtcttacgac tatggaaaat cattcaagaa aatttcagac aagttaaact 540
   ttggcttggg aaataggagt gaagctgtta tcgcccagtt ctaccacagc cctgcggaca 600
   acaagcggta catctttgca gacgcttatg cccagtacct ctggatcacg tttgacttct 660
   gcaacactct tcaaggcttt tccatcccat ttcgggcagc tgatctcctc ctacacagta 720
   aggcctccaa ccttctcttg ggctttgaca ggtcccaccc caacaagcag ctgtggaagt 780
   cagatgactt tggccagacc tggatcatga ttcaggaaca tgtcaagtcc ttttcttggg 840
   gaattgatcc ctatgacaaa ccaaatacca tctacattga acgacacgaa ccctctggct 900
   actccactgt cttccgaagt acagatttct tccagtcccg ggaaaaccag gaagtgatcc 960
   ttgaggaagt gagagatttt cagcttcggg acaagtacat gtttgctaca aaggtggtgc 1020
   atctcttggg cagtgaacag cagtcttctg tccagctctg ggtctccttt ggccggaagc 1080
   ccatgagagc agcccagttt gtcacaagac atcctattaa tgaatattac atcgcagatg 1140
   cctccgagga ccaggtgttt gtgtgtgtca gccacagtaa caaccgcacc aatttataca 1200
   tctcagaggc agaggggctg aagttctccc tgtccttgga gaacgtgctc tattacagcc 1260
   caggaggggc cggcagtgac accttggtga ggtattttgc aaatgaacca tttgctgact 1320
   tccaccgagt ggaaggattg caaggagtct acattgctac tctgattaat ggttctatga 1380
   atgaggagaa catgagatcg gtcatcacct ttgacaaagg gggaacctgg gagtttcttc 1440
   aggctccagc cttcacggga tatggagaga aaatcaattg tgagctttcc cagggctgtt 1500
   cccttcatct ggctcagcgc ctcagtcagc tcctcaacct ccagctccgg agaatgccca 1560
   tcctgtccaa ggagtcggct ccaggcctca tcatcgccac tggctcagtg ggaaagaact 1620
   tggctagcaa gacaaacgtg tacatctcta gcagtgctgg agccaggtgg cgagaggcac 1680
   ttcctggacc tcactactac acatggggag accacggcgg aatcatcacg gccattgccc 1740
   agggcatgga aaccaacgag ctaaaataca gtaccaatga aggggagacc tggaaaacat 1800
   tcatcttctc tgagaagcca gtgtttgtgt atggcctcct cacagaacct ggggagaaga 1860
   gcactgtctt caccatcttt ggctcgaaca aagagaatgt ccacagctgg ctgatcctcc 1920
   aggtcaatgc cacggatgcc ttgggagttc cctgcacaga gaatgactac aagctgtggt 1980
   caccatctga tgagcggggg aatgagtgtt tgctgggaca caagactgtt ttcaaacggc 2040
   ggacccccca tgccacatgc ttcaatggag aggactttga caggccggtg gtcgtgtcca 2100
   actgctcctg cacccgggag gactatgagt gtgacttcgg tttcaagatg agtgaagatt 2160
   tgtcattaga ggtttgtgtt ccagatccgg aattttctgg aaagtcatac tcccctcctg 2220
   tgccttgccc tgtgggttct acttacagga gaacgagagg ctaccggaag atttctgggg 2280
   acacttgtag cggaggagat gttgaagcgc gactggaagg agagctggtc ccctgtcccc 2340
   tggcagaaga gaacgagttc attctgtatg ctgtgaggaa atccatctac cgctatgacc 2400
   tggcctcggg agccaccgag cagttgcctc tcaccgggct acgggcagca gtggccctgg 2460
   actttgacta tgagcacaac tgtttgtatt ggtccgacct ggccttggac gtcatccagc 2520
   gcctctgttt gaatggaagc acagggcaag aggtgatcat caattctggc ctggagacag 2580
   tagaagcttt ggcttttgaa cccctcagcc agctgcttta ctgggtagat gcaggcttca 2640
   aaaagattga ggtagctaat ccagatggcg acttccgact cacaatcgtc aattcctctg 2700
   tgcttgatcg tcccagggct ctggtcctcg tgccccaaga gggggtgatg ttctggacag 2760
   actggggaga cctgaagcct gggatttatc ggagcaatat ggatggttct gctgcctatc 2820
   acctggtgtc tgaggatgtg aagtggccca atggcatctc tgtggacgac cagtggattt 2880
   actggacgga tgcctacctg gagtgcatag agcggatcac gttcagtggc cagcagcgct 2940
   ctgtcattct ggacaacctc ccgcacccct atgccattgc tgtctttaag aatgaaatct 3000
   actgggatga ctggtcacag ctcagcatat tccgagcttc caaatacagt gggtcccaga 3060
   tggagattct ggcaaaccag ctcacggggc tcatggacat gaagattttc tacaagggga 3120
   agaacactgg aagcaatgcc tgtgtgccca ggccatgcag cctgctgtgc ctgcccaagg 3180
   ccaacaacag tagaagctgc aggtgtccag aggatgtgtc cagcagtgtg cttccatcag 3240
   gggacctgat gtgtgactgc cctcagggct atcagctcaa gaacaatacc tgtgtcaaag 3300
   aagagaacac ctgtcttcgc aaccagtatc gctgcagcaa cgggaactgt atcaacagca 3360
   tttggtggtg tgactttgac aacgactgtg gagacatgag cgatgagaga aactgcccta 3420
   ccaccatctg tgacctggac acccagtttc gttgccagga gtctgggact tgtatcccac 3480
   tgtcctataa atgtgacctt gaggatgact gtggagacaa cagtgatgaa agtcattgtg 3540
   aaatgcacca gtgccggagt gacgagtaca actgcagttc cggcatgtgc atccgctcct 3600
   cctgggtatg tgacggggac aacgactgca gggactggtc tgatgaagcc aactgtaccg 3660
   ccatctatca cacctgtgag gcctccaact tccagtgccg aaacgggcac tgcatccccc 3720
   agcggtgggc gtgtgacggg gatacggact gccaggatgg ttccgatgag gatccagtca 3780
   actgtgagaa gaagtgcaat ggattccgct gcccaaacgg cacttgcatc ccatccagca 3840
   aacattgtga tggtctgcgt gattgctctg atggctccga tgaacagcac tgcgagcccc 3900
   tctgtacgca cttcatggac tttgtgtgta agaaccgcca gcagtgcctg ttccactcca 3960
   tggtctgtga cggaatcatc cagtgccgcg acgggtccga tgaggatgcg gcgtttgcag 4020
   gatgctccca agatcctgag ttccacaagg tatgtgatga gttcggtttc cagtgtcaga 4080
   atggagtgtg catcagtttg atttggaagt gcgacgggat ggatgattgc ggcgattatt 4140
   ctgatgaagc caactgcgaa aaccccacag aagccccaaa ctgctcccgc tacttccagt 4200
   ttcggtgtga gaatggccac tgcatcccca acagatggaa atgtgacagg gagaacgact 4260
   gtggggactg gtctgatgag aaggattgtg gagattcaca tattcttccc ttctcgactc 4320
   ctgggccctc cacgtgtctg cccaattact accgctgcag cagtgggacc tgcgtgatgg 4380
   acacctgggt gtgcgacggg taccgagatt gtgcagatgg ctctgacgag gaagcctgcc 4440
   ccttgcttgc aaacgtcact gctgcctcca ctcccaccca acttgggcga tgtgaccgat 4500
   ttgagttcga atgccaccaa ccgaagacgt gtattcccaa ctggaagcgc tgtgacggcc 4560
   accaagattg ccaggatggc cgggacgagg ccaattgccc cacacacagc accttgactt 4620
   gcatgagcag ggagttccag tgcgaggacg gggaggcctg cattgtgctc tcggagcgct 4680
   gcgacggctt cctggactgc tcggacgaga gcgatgaaaa ggcctgcagt gatgagttga 4740
   ctgtgtacaa agtacagaat cttcagtgga cagctgactt ctctggggat gtgactttga 4800
   cctggatgag gcccaaaaaa atgccctctg catcttgtgt atataatgtc tactacaggg 4860
   tggttggaga gagcatatgg aagactctgg agacccacag caataagaca aacactgtat 4920
   taaaagtctt gaaaccagat accacgtatc aggttaaagt acaggttcag tgtctcagca 4980
   aggcacacaa caccaatgac tttgtgaccc tgaggacccc agagggattg ccagatgccc 5040
   ctcgaaatct ccagctgtca ctccccaggg aagcagaagg tgtgattgta ggccactggg 5100
   ctcctcccat ccacacccat ggcctcatcc gtgagtacat tgtagaatac agcaggagtg 5160
   gttccaagat gtgggcctcc cagagggctg ctagtaactt tacagaaatc aagaacttat 5220
   tggtcaacac tctatacacc gtcagagtgg ctgcggtgac tagtcgtgga ataggaaact 5280
   ggagcgattc taaatccatt accaccataa aaggaaaagt gatcccacca ccagatatcc 5340
   acattgacag ctatggtgaa aattatctaa gcttcaccct gaccatggag agtgatatca 5400
   aggtgaatgg ctatgtggtg aaccttttct gggcatttga cacccacaag caagagagga 5460
   gaactttgaa cttccgagga agcatattgt cacacaaagt tggcaatctg acagctcata 5520
   catcctatga gatttctgcc tgggccaaga ctgacttggg ggatagccct ctggcatttg 5580
   agcatgttat gaccagaggg gttcgcccac ctgcacctag cctcaaggcc aaagccatca 5640
   accagactgc agtggaatgt acctggaccg gcccccggaa tgtggtttat ggtattttct 5700
   atgccacgtc ctttcttgac ctctatcgca acccgaagag cttgactact tcactccaca 5760
   acaagacggt cattgtcagt aaggatgagc agtatttgtt tctggtccgt gtagtggtac 5820
   cctaccaggg gccatcctct gactacgttg tagtgaagat gatcccggac agcaggcttc 5880
   caccccgtca cctgcatgtg gttcatacgg gcaaaacctc cgtggtcatc aagtgggaat 5940
   caccgtatga ctctcctgac caggacttgt tgtatgcaat tgcagtcaaa gatctcataa 6000
   gaaagactga caggagctac aaagtaaaat cccgtaacag cactgtggaa tacaccctta 6060
   acaagttgga gcctggcggg aaataccaca tcattgtcca actggggaac atgagcaaag 6120
   attccagcat aaaaattacc acagtttcat tatcagcacc tgatgcctta aaaatcataa 6180
   cagaaaatga tcatgttctt ctgttttgga aaagcctggc tttaaaggaa aagcatttta 6240
   atgaaagcag gggctatgag atacacatgt ttgatagtgc catgaatatc acagcttacc 6300
   ttgggaatac tactgacaat ttctttaaaa tttccaacct gaagatgggt cataattaca 6360
   cgttcaccgt ccaagcaaga tgcctttttg gcaaccagat ctgtggggag cctgccatcc 6420
   tgctgtacga tgagctgggg tctggtgcag atgcatctgc aacgcaggct gccagatcta 6480
   cggatgttgc tgctgtggtg gtgcccatct tattcctgat actgctgagc ctgggggtgg 6540
   ggtttgccat cctgtacacg aagcaccgga ggctgcagag cagcttcacc gccttcgcca 6600
   acagccacta cagctccagg ctggggtccg caatcttctc ctctggggat gacctggggg 6660
   aagatgatga agatgcccct atgataactg gattttcaga tgacgtcccc atggtgatag 6720
   cctgaaagag ctttcctcac tagaaaccaa atggtgtaaa tattttattt gataaagata 6780
   gttgatggtt tattttaaaa gatgcacttt gagttgcaat atgttatttt tatatgggcc 6840
<210> 73
   <211> 2214
   <212> PRT
   <213> Homo sapiens
<400> 73
   Met Ala Thr Arg Ser Ser Arg Arg Glu Ser Arg Leu Pro Phe Leu Phe
    1               5                  10                  15
   Thr Leu Val Ala Leu Leu Pro Pro Gly Ala Leu Cys Glu Val Trp Thr
               20                  25                  30
   Gln Arg Leu His Gly Gly Ser Ala Pro Leu Pro Gln Asp Arg Gly Phe
           35                  40                  45
   Leu Val Val Gln Gly Asp Pro Arg Glu Leu Arg Leu Trp Ala Arg Gly
       50                  55                  60
   Asp Ala Arg Gly Ala Ser Arg Ala Asp Glu Lys Pro Leu Arg Arg Lys
   65                  70                  75                  80
   Arg Ser Ala Ala Leu Gln Pro Glu Pro Ile Lys Val Tyr Gly Gln Val
                   85                  90                  95
   Ser Leu Asn Asp Ser His Asn Gln Met Val Val His Trp Ala Gly Glu
               100                 105                 110
   Lys Ser Asn Val Ile Val Ala Leu Ala Arg Asp Ser Leu Ala Leu Ala
           115                 120                 125
   Arg Pro Lys Ser Ser Asp Val Tyr Val Ser Tyr Asp Tyr Gly Lys Ser
       130                 135                 140
   Phe Lys Lys Ile Ser Asp Lys Leu Asn Phe Gly Leu Gly Asn Arg Ser
   145                 150                 155                 160
   Glu Ala Val Ile Ala Gln Phe Tyr His Ser Pro Ala Asp Asn Lys Arg
                   165                 170                 175
   Tyr Ile Phe Ala Asp Ala Tyr Ala Gln Tyr Leu Trp Ile Thr Phe Asp
               180                 185                 190
   Phe Cys Asn Thr Leu Gln Gly Phe Ser Ile Pro Phe Arg Ala Ala Asp
           195                 200                 205
   Leu Leu Leu His Ser Lys Ala Ser Asn Leu Leu Leu Gly Phe Asp Arg
       210                 215                 220
   Ser His Pro Asn Lys Gln Leu Trp Lys Ser Asp Asp Phe Gly Gln Thr
   225 230 235 240
   Trp Ile Met Ile Gln Glu His Val Lys Ser Phe Ser Trp Gly Ile Asp
   245 250 255
   Pro Tyr Asp Lys Pro Asn Thr Ile Tyr Ile Glu Arg His Glu Pro Ser
   260 265 270
   Gly Tyr Ser Thr Val Phe Arg Ser Thr Asp Phe Phe Gln Ser Arg Glu
   275 280 285
   Asn Gln Glu Val Ile Leu Glu Glu Val Arg Asp Phe Gln Leu Arg Asp
   290 295 300
   Lys Tyr Met Phe Ala Thr Lys Val Val His Leu Leu Gly Ser Glu Gln
   305 310 315 320
   Gln Ser Ser Val Gln Leu Trp Val Ser Phe Gly Arg Lys Pro Met Arg
   325 330 335
   Ala Ala Gln Phe Val Thr Arg His Pro Ile Asn Glu Tyr Tyr Ile Ala
   340 345 350
   Asp Ala Ser Glu Asp Gln Val Phe Val Cys Val Ser His Ser Asn Asn
   355 360 365
   Arg Thr Asn Leu Tyr Ile Ser Glu Ala Glu Gly Leu Lys Phe Ser Leu
   370 375 380
   Ser Leu Glu Asn Val Leu Tyr Tyr Ser Pro Gly Gly Ala Gly Ser Asp
   385 390 395 400
   Thr Leu Val Arg Tyr Phe Ala Asn Glu Pro Phe Ala Asp Phe His Arg
   405 410 415
   Val Glu Gly Leu Gln Gly Val Tyr Ile Ala Thr Leu Ile Asn Gly Ser
   420 425 430
   Met Asn Glu Glu Asn Met Arg Ser Val Ile Thr Phe Asp Lys Gly Gly
   435 440 445
   Thr Trp Glu Phe Leu Gln Ala Pro Ala Phe Thr Gly Tyr Gly Glu Lys
   450 455 460
   Ile Asn Cys Glu Leu Ser Gln Gly Cys Ser Leu His Leu Ala Gln Arg
   465 470 475 480
   Leu Ser Gln Leu Leu Asn Leu Gln Leu Arg Arg Met Pro Ile Leu Ser
   485 490 495
   Lys Glu Ser Ala Pro Gly Leu Ile Ile Ala Thr Gly Ser Val Gly Lys
   500 505 510
   Asn Leu Ala Ser Lys Thr Asn Val Tyr Ile Ser Ser Ser Ala Gly Ala
   515 520 525
   Arg Trp Arg Glu Ala Leu Pro Gly Pro His Tyr Tyr Thr Trp Gly Asp
   530 535 540
   His Gly Gly Ile Ile Thr Ala Ile Ala Gln Gly Met Glu Thr Asn Glu
   545 550 555 560
   Leu Lys Tyr Ser Thr Asn Glu Gly Glu Thr Trp Lys Thr Phe Ile Phe
   565 570 575
   Ser Glu Lys Pro Val Phe Val Tyr Gly Leu Leu Thr Glu Pro Gly Glu
   580 585 590
   Lys Ser Thr Val Phe Thr Ile Phe Gly Ser Asn Lys Glu Asn Val His
   595 600 605
   Ser Trp Leu Ile Leu Gln Val Asn Ala Thr Asp Ala Leu Gly Val Pro
   610 615 620
   Cys Thr Glu Asn Asp Tyr Lys Leu Trp Ser Pro Ser Asp Glu Arg Gly
   625 630 635 640
   Asn Glu Cys Leu Leu Gly His Lys Thr Val Phe Lys Arg Arg Thr Pro
   645 650 655
   His Ala Thr Cys Phe Asn Gly Glu Asp Phe Asp Arg Pro Val Val Val
   660 665 670
   Ser Asn Cys Ser Cys Thr Arg Glu Asp Tyr Glu Cys Asp Phe Gly Phe
   675 680 685
   Lys Met Ser Glu Asp Leu Ser Leu Glu Val Cys Val Pro Asp Pro Glu
   690 695 700
   Phe Ser Gly Lys Ser Tyr Ser Pro Pro Val Pro Cys Pro Val Gly Ser
   705 710 715 720
   Thr Tyr Arg Arg Thr Arg Gly Tyr Arg Lys Ile Ser Gly Asp Thr Cys
   725 730 735
   Ser Gly Gly Asp Val Glu Ala Arg Leu Glu Gly Glu Leu Val Pro Cys
   740 745 750
   Pro Leu Ala Glu Glu Asn Glu Phe Ile Leu Tyr Ala Val Arg Lys Ser
   755 760 765
   Ile Tyr Arg Tyr Asp Leu Ala Ser Gly Ala Thr Glu Gln Leu Pro Leu
   770 775 780
   Thr Gly Leu Arg Ala Ala Val Ala Leu Asp Phe Asp Tyr Glu His Asn
   785 790 795 800
   Cys Leu Tyr Trp Ser Asp Leu Ala Leu Asp Val Ile Gln Arg Leu Cys
   805 810 815
   Leu Asn Gly Ser Thr Gly Gln Glu Val Ile Ile Asn Ser Gly Leu Glu
   820 825 830
   Thr Val Glu Ala Leu Ala Phe Glu Pro Leu Ser Gln Leu Leu Tyr Trp
   835 840 845
   Val Asp Ala Gly Phe Lys Lys Ile Glu Val Ala Asn Pro Asp Gly Asp
   850 855 860
   Phe Arg Leu Thr Ile Val Asn Ser Ser Val Leu Asp Arg Pro Arg Ala
   865 870 875 880
   Leu Val Leu Val Pro Gln Glu Gly Val Met Phe Trp Thr Asp Trp Gly
   885 890 895
   Asp Leu Lys Pro Gly Ile Tyr Arg Ser Asn Met Asp Gly Ser Ala Ala
   900 905 910
   Tyr His Leu Val Ser Glu Asp Val Lys Trp Pro Asn Gly Ile Ser Val
   915 920 925
   Asp Asp Gln Trp Ile Tyr Trp Thr Asp Ala Tyr Leu Glu Cys Ile Glu
   930 935 940
   Arg Ile Thr Phe Ser Gly Gln Gln Arg Ser Val Ile Leu Asp Asn Leu
   945 950 955 960
   Pro His Pro Tyr Ala Ile Ala Val Phe Lys Asn Glu Ile Tyr Trp Asp
   965 970 975
   Asp Trp Ser Gln Leu Ser Ile Phe Arg Ala Ser Lys Tyr Ser Gly Ser
   980 985 990
   Gln Met Glu Ile Leu Ala Asn Gln Leu Thr Gly Leu Met Asp Met Lys
   995 1000 1005
   Ile Phe Tyr Lys Gly Lys Asn Thr Gly Ser Asn Ala Cys Val Pro Arg
   1010 1015 1020
   Pro Cys Ser Leu Leu Cys Leu Pro Lys Ala Asn Asn Ser Arg Ser Cys
   1025 1030 1035 1040
   Arg Cys Pro Glu Asp Val Ser Ser Ser Val Leu Pro Ser Gly Asp Leu
   1045 1050 1055
   Met Cys Asp Cys Pro Gln Gly Tyr Gln Leu Lys Asn Asn Thr Cys Val
   1060 1065 1070
   Lys Glu Glu Asn Thr Cys Leu Arg Asn Gln Tyr Arg Cys Ser Asn Gly
   1075 1080 1085
   Asn Cys Ile Asn Ser Ile Trp Trp Cys Asp Phe Asp Asn Asp Cys Gly
   1090 1095 1100
   Asp Met Ser Asp Glu Arg Asn Cys Pro Thr Thr Ile Cys Asp Leu Asp
   1105 1110 1115 1120
   Thr Gln Phe Arg Cys Gln Glu Ser Gly Thr Cys Ile Pro Leu Ser Tyr
   1125 1130 1135
   Lys Cys Asp Leu Glu Asp Asp Cys Gly Asp Asn Ser Asp Glu Ser His
   1140 1145 1150
   Cys Glu Met His Gln Cys Arg Ser Asp Glu Tyr Asn Cys Ser Ser Gly
   1155 1160 1165
   Met Cys Ile Arg Ser Ser Trp Val Cys Asp Gly Asp Asn Asp Cys Arg
   1170 1175 1180
   Asp Trp Ser Asp Glu Ala Asn Cys Thr Ala Ile Tyr His Thr Cys Glu
   1185 1190 1195 1200
   Ala Ser Asn Phe Gln Cys Arg Asn Gly His Cys Ile Pro Gln Arg Trp
   1205 1210 1215
   Ala Cys Asp Gly Asp Thr Asp Cys Gln Asp Gly Ser Asp Glu Asp Pro
   1220 1225 1230
   Val Asn Cys Glu Lys Lys Cys Asn Gly Phe Arg Cys Pro Asn Gly Thr
   1235 1240 1245
   Cys Ile Pro Ser Ser Lys His Cys Asp Gly Leu Arg Asp Cys Ser Asp
   1250 1255 1260
   Gly Ser Asp Glu Gln His Cys Glu Pro Leu Cys Thr His Phe Met Asp
   1265 1270 1275 1280
   Phe Val Cys Lys Asn Arg Gln Gln Cys Leu Phe His Ser Met Val Cys
   1285 1290 1295
   Asp Gly Ile Ile Gln Cys Arg Asp Gly Ser Asp Glu Asp Ala Ala Phe
   1300 1305 1310
   Ala Gly Cys Ser Gln Asp Pro Glu Phe His Lys Val Cys Asp Glu Phe
   1315 1320 1325
   Gly Phe Gln Cys Gln Asn Gly Val Cys Ile Ser Leu Ile Trp Lys Cys
   1330 1335 1340
   Asp Gly Met Asp Asp Cys Gly Asp Tyr Ser Asp Glu Ala Asn Cys Glu
   1345 1350 1355 1360
   Asn Pro Thr Glu Ala Pro Asn Cys Ser Arg Tyr Phe Gln Phe Arg Cys
   1365 1370 1375
   Glu Asn Gly His Cys Ile Pro Asn Arg Trp Lys Cys Asp Arg Glu Asn
   1380 1385 1390
   Asp Cys Gly Asp Trp Ser Asp Glu Lys Asp Cys Gly Asp Ser His Ile
   1395 1400 1405
   Leu Pro Phe Ser Thr Pro Gly Pro Ser Thr Cys Leu Pro Asn Tyr Tyr
   1410 1415 1420
   Arg Cys Ser Ser Gly Thr Cys Val Met Asp Thr Trp Val Cys Asp Gly
   1425 1430 1435 1440
   Tyr Arg Asp Cys Ala Asp Gly Ser Asp Glu Glu Ala Cys Pro Leu Leu
   1445 1450 1455
   Ala Asn Val Thr Ala Ala Ser Thr Pro Thr Gln Leu Gly Arg Cys Asp
   1460 1465 1470
   Arg Phe Glu Phe Glu Cys His Gln Pro Lys Thr Cys Ile Pro Asn Trp
   1475 1480 1485
   Lys Arg Cys Asp Gly His Gln Asp Cys Gln Asp Gly Arg Asp Glu Ala
   1490 1495 1500
   Asn Cys Pro Thr His Ser Thr Leu Thr Cys Met Ser Arg Glu Phe Gln
   1505 1510 1515 1520
   Cys Glu Asp Gly Glu Ala Cys Ile Val Leu Ser Glu Arg Cys Asp Gly
   1525 1530 1535
   Phe Leu Asp Cys Ser Asp Glu Ser Asp Glu Lys Ala Cys Ser Asp Glu
   1540 1545 1550
   Leu Thr Val Tyr Lys Val Gln Asn Leu Gln Trp Thr Ala Asp Phe Ser
   1555 1560 1565
   Gly Asp Val Thr Leu Thr Trp Met Arg Pro Lys Lys Met Pro Ser Ala
   1570 1575 1580
   Ser Cys Val Tyr Asn Val Tyr Tyr Arg Val Val Gly Glu Ser Ile Trp
   1585 1590 1595 1600
   Lys Thr Leu Glu Thr His Ser Asn Lys Thr Asn Thr Val Leu Lys Val
   1605 1610 1615
   Leu Lys Pro Asp Thr Thr Tyr Gln Val Lys Val Gln Val Gln Cys Leu
   1620 1625 1630
   Ser Lys Ala His Asn Thr Asn Asp Phe Val Thr Leu Arg Thr Pro Glu
   1635 1640 1645
   Gly Leu Pro Asp Ala Pro Arg Asn Leu Gln Leu Ser Leu Pro Arg Glu
   1650 1655 1660
   Ala Glu Gly Val Ile Val Gly His Trp Ala Pro Pro Ile His Thr His
   1665 1670 1675 1680
   Gly Leu Ile Arg Glu Tyr Ile Val Glu Tyr Ser Arg Ser Gly Ser Lys
   1685 1690 1695
   Met Trp Ala Ser Gln Arg Ala Ala Ser Asn Phe Thr Glu Ile Lys Asn
   1700 1705 1710
   Leu Leu Val Asn Thr Leu Tyr Thr Val Arg Val Ala Ala Val Thr Ser
   1715 1720 1725
   Arg Gly Ile Gly Asn Trp Ser Asp Ser Lys Ser Ile Thr Thr Ile Lys
   1730 1735 1740
   Gly Lys Val Ile Pro Pro Pro Asp Ile His Ile Asp Ser Tyr Gly Glu
   1745 1750 1755 1760
   Asn Tyr Leu Ser Phe Thr Leu Thr Met Glu Ser Asp Ile Lys Val Asn
   1765 1770 1775
   Gly Tyr Val Val Asn Leu Phe Trp Ala Phe Asp Thr His Lys Gln Glu
   1780 1785 1790
   Arg Arg Thr Leu Asn Phe Arg Gly Ser Ile Leu Ser His Lys Val Gly
   1795 1800 1805
   Asn Leu Thr Ala His Thr Ser Tyr Glu Ile Ser Ala Trp Ala Lys Thr
   1810 1815 1820
   Asp Leu Gly Asp Ser Pro Leu Ala Phe Glu His Val Met Thr Arg Gly
   1825 1830 1835 1840
   Val Arg Pro Pro Ala Pro Ser Leu Lys Ala Lys Ala Ile Asn Gln Thr
   1845 1850 1855
   Ala Val Glu Cys Thr Trp Thr Gly Pro Arg Asn Val Val Tyr Gly Ile
   1860 1865 1870
   Phe Tyr Ala Thr Ser Phe Leu Asp Leu Tyr Arg Asn Pro Lys Ser Leu
   1875 1880 1885
   Thr Thr Ser Leu His Asn Lys Thr Val Ile Val Ser Lys Asp Glu Gln
   1890 1895 1900
   Tyr Leu Phe Leu Val Arg Val Val Val Pro Tyr Gln Gly Pro Ser Ser
   1905 1910 1915 1920
   Asp Tyr Val Val Val Lys Met Ile Pro Asp Ser Arg Leu Pro Pro Arg
   1925 1930 1935
   His Leu His Val Val His Thr Gly Lys Thr Ser Val Val Ile Lys Trp
   1940 1945 1950
   Glu Ser Pro Tyr Asp Ser Pro Asp Gln Asp Leu Leu Tyr Ala Ile Ala
   1955 1960 1965
   Val Lys Asp Leu Ile Arg Lys Thr Asp Arg Ser Tyr Lys Val Lys Ser
   1970 1975 1980
   Arg Asn Ser Thr Val Glu Tyr Thr Leu Asn Lys Leu Glu Pro Gly Gly
   1985 1990 1995 2000
   Lys Tyr His Ile Ile Val Gln Leu Gly Asn Met Ser Lys Asp Ser Ser
   2005 2010 2015
   Ile Lys Ile Thr Thr Val Ser Leu Ser Ala Pro Asp Ala Leu Lys Ile
   2020 2025 2030
   Ile Thr Glu Asn Asp His Val Leu Leu Phe Trp Lys Ser Leu Ala Leu
   2035 2040 2045
   Lys Glu Lys His Phe Asn Glu Ser Arg Gly Tyr Glu Ile His Met Phe
   2050 2055 2060
   Asp Ser Ala Met Asn Ile Thr Ala Tyr Leu Gly Asn Thr Thr Asp Asn
   2065 2070 2075 2080
   Phe Phe Lys Ile Ser Asn Leu Lys Met Gly His Asn Tyr Thr Phe Thr
   2085 2090 2095
   Val Gln Ala Arg Cys Leu Phe Gly Asn Gln Ile Cys Gly Glu Pro Ala
   2100 2105 2110
   Ile Leu Leu Tyr Asp Glu Leu Gly Ser Gly Ala Asp Ala Ser Ala Thr
   2115 2120 2125
   Gln Ala Ala Arg Ser Thr Asp Val Ala Ala Val Val Val Pro Ile Leu
   2130 2135 2140
   Phe Leu Ile Leu Leu Ser Leu Gly Val Gly Phe Ala Ile Leu Tyr Thr
   2145 2150 2155 2160
   Lys His Arg Arg Leu Gln Ser Ser Phe Thr Ala Phe Ala Asn Ser His
   2165 2170 2175
   Tyr Ser Ser Arg Leu Gly Ser Ala Ile Phe Ser Ser Gly Asp Asp Leu
   2180 2185 2190
   Gly Glu Asp Asp Glu Asp Ala Pro Met Ile Thr Gly Phe Ser Asp Asp
   2195 2200 2205
   Val Pro Met Val Ile Ala
   2210
<210> 74
   <211> 1039
   <212> DNA
   <213> Rattus norvegicus
<400> 74
   cccccgggct gcaggaattc cgtttctagt tcccggccgg tgagccactc cggcttttcg 60
   cggtctactt gtcccagcgt cggctctgga ctgtctatcg ctctctgacg ttcagtcccg 120
   ggtcgctgct tgtgcgcgca cgccgcctca cacgacaaac ttatggcgga gcgcggggaa 180
   ctcgatctga cgggcgccaa acagaacacc ggcgtgtggc tggtgaaggt tcctaaatat 240
   ttgtcccagc aatgggcaaa agcccctgga agaggtgaag ttgggaaact gaggattgcc 300
   aagaatcaag gaaggactga ggtatcattt actttgaatg aggatcttgc gaatattcat 360
   gatatcggtg ggaaaccagc ttcagtcagt gctcctagag aacatccatt tgtcttgcaa 420
   agtgttggag gacagacatt aacagtgttc accgagagct cctcagataa actgtcattg 480
   gaaggaatag tggttcagag agcggaatgc cgacctgctg ccagtgaaaa ctacatgaaa 540
   ctaaagagat tgcaaataga agaatcttcc aaacctgtaa ggctctcaca acagctggac 600
   aaagttgtaa ccaccaatta caagcctgtc gctaaccatc agtacaatat tgaatatgaa 660
   aggaagaaga aagaagatgg aaagcgagct cgggcagaca aacaacacgt cctagacatg 720
   ctgttctcag cctttgagaa acatcagtac tataatctta aggacttggt ggacatcaca 780
   aagcaacctg tgggatacct gaaagaaatc ttgaaagaaa tcggcattca gaatgtaaaa 840
   ggaattcaca agaatacgtg ggagctgaag ccagagtaca ggcattacca aacagaggag 900
   aagagcgact gagagaagcg tgtgcaacag ttacaggcag atctctgcca ccacacgctg 960
   actgcctgcc caccctgaca ggacaaagac aactgagtct gtctgccatt cctcagtaaa 1020
   ctgttaaagc tgggactcc                                              1039
<210> 75
   <211> 249
   <212> PRT
   <213> Rattus norvegicus
<400> 75
   Met Ala Glu Arg Gly Glu Leu Asp Leu Thr Gly Ala Lys Gln Asn Thr
   1               5                   10                  15
   Gly Val Trp Leu Val Lys Val Pro Lys Tyr Leu Ser Gln Gln Trp Ala
               20                  25                  30
   Lys Ala Pro Gly Arg Gly Glu Val Gly Lys Leu Arg Ile Ala Lys Asn
           35                  40                  45
   Gln Gly Arg Thr Glu Val Ser Phe Thr Leu Asn Glu Asp Leu Ala Asn
       50                  55                  60
   Ile His Asp Ile Gly Gly Lys Pro Ala Ser Val Ser Ala Pro Arg Glu
   65                  70                  75                  80
   His Pro Phe Val Leu Gln Ser Val Gly Gly Gln Thr Leu Thr Val Phe
                   85                  90                  95
   Thr Glu Ser Ser Ser Asp Lys Leu Ser Leu Glu Gly Ile Val Val Gln
               100                 105                 110
   Arg Ala Glu Cys Arg Pro Ala Ala Ser Glu Asn Tyr Met Lys Leu Lys
           115                 120                 125
   Arg Leu Gln Ile Glu Glu Ser Ser Lys Pro Val Arg Leu Ser Gln Gln
       130                 135                 140
   Leu Asp Lys Val Val Thr Thr Asn Tyr Lys Pro Val Ala Asn His Gln
   145                 150                 155                 160
   Tyr Asn Ile Glu Tyr Glu Arg Lys Lys Lys Glu Asp Gly Lys Arg Ala
                   165                 170                 175
   Arg Ala Asp Lys Gln His Val Leu Asp Met Leu Phe Ser Ala Phe Glu
               180                 185                 190
   Lys His Gln Tyr Tyr Asn Leu Lys Asp Leu Val Asp Ile Thr Lys Gln
           195                 200                 205
   Pro Val Gly Tyr Leu Lys Glu Ile Leu Lys Glu Ile Gly Ile Gln Asn
       210                 215                 220
   Val Lys Gly Ile His Lys Asn Thr Trp Glu Leu Lys Pro Glu Tyr Arg
   225                 230                 235                 240
   His Tyr Gln Thr Glu Glu Lys Ser Asp
                   245
<210> 76
   <211> 750
   <212> DNA
   <213> Homo sapiens
<400> 76
   atggccgagc gcggggaact cgacttgacc ggcgccaaac agaacacagg agtgtggcta 60
   gtcaaggttc ctaaatattt gtcacagcaa tgggctaaag cctctggaag aggtgaagtt 120
   gggaaactgc ggattgccaa gactcaagga aggactgagg tgtcatttac tttgaatgag 180
   gatcttgcaa atattcatga tattggtgga aaaccagctt cagtcagtgc tcctagagaa 240
   catccatttg tcttgcaaag tgttggagga cagacattaa cagtatttac tgagagctca 300
   tcagataagc tgtcattgga aggaatagtg gtacaaagag ctgaatgccg accagctgcc 360
   agtgaaaact acatgcgatt aaaaagattg caaatagaag agtcttccaa accagtgagg 420
   ctatcacaac agctggacaa agttgtaaca accaattaca aacctgttgc taatcatcaa 480
   tacaatatcg aatatgaaag gaaaaagaaa gaagacggaa agcgagctcg agctgataaa 540
   caacatgttt tagacatgct attttcagcc tttgagaaac atcaatacta taatcttaag 600
   gacttggtgg acatcacaaa acaacctgtg gtgtacctga aggaaatctt aaaagaaatt 660
   ggtgttcaga atgtaaaagg gatccacaaa aacacatggg agctgaagcc agagtacaga 720
   cactatcaag gagaagaaaa gagtgactaa                                 750
<210> 77
   <211> 249
   <212> PRT
   <213> Homo sapiens
<400> 77
   Met Ala Glu Arg Gly Glu Leu Asp Leu Thr Gly Ala Lys Gln Asn Thr
   1               5                   10                  15
   Gly Val Trp Leu Val Lys Val Pro Lys Tyr Leu Ser Gln Gln Trp Ala
               20                  25                  30
   Lys Ala Ser Gly Arg Gly Glu Val Gly Lys Leu Arg Ile Ala Lys Thr
           35                  40                  45
   Gln Gly Arg Thr Glu Val Ser Phe Thr Leu Asn Glu Asp Leu Ala Asn
       50                  55                  60
   Ile His Asp Ile Gly Gly Lys Pro Ala Ser Val Ser Ala Pro Arg Glu
   65                  70                  75                  80
   His Pro Phe Val Leu Gln Ser Val Gly Gly Gln Thr Leu Thr Val Phe
                   85                  90                  95
   Thr Glu Ser Ser Ser Asp Lys Leu Ser Leu Glu Gly Ile Val Val Gln
   100 105 110
   Arg Ala Glu Cys Arg Pro Ala Ala Ser Glu Asn Tyr Met Arg Leu Lys
   115 120 125
   Arg Leu Gln Ile Glu Glu Ser Ser Lys Pro Val Arg Leu Ser Gln Gln
   130 135 140
   Leu Asp Lys Val Val Thr Thr Asn Tyr Lys Pro Val Ala Asn His Gln
   145 150 155 160
   Tyr Asn Ile Glu Tyr Glu Arg Lys Lys Lys Glu Asp Gly Lys Arg Ala
   165 170 175
   Arg Ala Asp Lys Gln His Val Leu Asp Met Leu Phe Ser Ala Phe Glu
   180 185 190
   Lys His Gln Tyr Tyr Asn Leu Lys Asp Leu Val Asp Ile Thr Lys Gln
   195 200 205
   Pro Val Val Tyr Leu Lys Glu Ile Leu Lys Glu Ile Gly Val Gln Asn
   210 215 220
   Val Lys Gly Ile His Lys Asn Thr Trp Glu Leu Lys Pro Glu Tyr Arg
   225 230 235 240
   His Tyr Gln Gly Glu Glu Lys Ser Asp
   245
<210> 78
   <211> 1568
   <212> DNA
   <213> Rattus norvegicus
<400> 78
   ggacagcgca tggcggccac agaggatgac agactggcgg ggagcggaga aggagaacgg 60
   ctggatttcc tgcgggaccg acacgtgcgg ttcttccagc gctgcctcca ggtcttgccg 120
   gagcggtatt cttcgctgga gaccagcagg ctgacaattg cattttttgc actctctggg 180
   ctggatatgt tggactcctt ggatgtggtg aacaaagacg atataataga gtggatttat 240
   tccttgcagg ttcttcccac agaagacagg tcaaatctgg atcgctgtgg tttccgaggt 300
   tcttcatatt tgggtattcc attcaaccca tcaaagaatc caggcacagc tcatccttat 360
   gacagtggac acatagcgat gacttacact ggtctttcct gtttaattat tcttggagat 420
   gatttaagcc gagtagataa agaagcttgc ttagcaggct tgagagcact tcagctggaa 480
   gatgggagct tctgtgctgt tcctgaaggc agtgagaatg acatgaggtt tgtgtactgt 540
   gcttcctgca tttgctatat gctcaacaac tggtcaggca tggatatgaa gaaagccatc 600
   agctacatta gaagaagtat gtcctatgac aatggcctgg cacagggggc aggacttgag 660
   tctcatggag gatccacctt ttgtggcatt gcgtcactgt gcctgatggg taaactggaa 720
   gaagtttttt cagagaaaga actgaaccgg ataaagaggt ggtgcataat gaggcagcag 780
   aacgggtacc acggaagacc taacaagcct gtcgacacct gttactcttt ctgggtggga 840
   gcaacactaa agcttttgaa aattttccag tacactaact ttgagaagaa taggaattac 900
   atcttatcaa ctcaggatcg ccttgttggg ggatttgcta aatggccaga cagtcatcca 960
   gatgctttgc atgcgtactt cgggatctgt ggcctgtcac taatggagga gagtggaatt 1020
   tgtaaagttc atcctgctct gaatgtaagc acacgaactt ctgagcgcct ccgagatctc 1080
   catcaaagct ggaagaccaa ggactctaaa cagtgctcag acaatgtcca tatttccagt 1140
   tgactaaccc tggggtaaag ggtgtgtagc atacgtgtag ctcaaggtta aaagccatgt 1200
   gtaaccaagt gtgctcttct ttaagggtta gtcgtaaaag tcagaagcgt gtactgctag 1260
   ttcttcagga tatgctctta ggccagtgac cactgtcatg gatttcaaga aaatccttgt 1320
   tgacgtgtgg acatcagcag aactctggta tggttcttaa ctgttacact gtgtttctga 1380
   gacctttcat ggggcagata tgtttgtagg ttatcttctt aaaaccctca gtacaagttc 1440
   tggcttacaa aatgtacgta aaccttcaaa acaagtttac actccatatg gcattgataa 1500
   tcttcaggtg agcatttaac gatcacttaa aaatcgctac tgctgatggg aagaaatttg 1560
   tttatccg 1568
<210> 79
   <211> 377
   <212> PRT
   <213> Rattus norvegicus
<400> 79
   Met Ala Ala Thr Glu Asp Asp Arg Leu Ala Gly Ser Gly Glu Gly Glu
    1               5                  10                  15
   Arg Leu Asp Phe Leu Arg Asp Arg His Val Arg Phe Phe Gln Arg Cys
               20                  25                  30
   Leu Gln Val Leu Pro Glu Arg Tyr Ser Ser Leu Glu Thr Ser Arg Leu
           35                  40                  45
   Thr Ile Ala Phe Phe Ala Leu Ser Gly Leu Asp Met Leu Asp Ser Leu
       50                  55                  60
   Asp Val Val Asn Lys Asp Asp Ile Ile Glu Trp Ile Tyr Ser Leu Gln
   65                  70                  75                  80
   Val Leu Pro Thr Glu Asp Arg Ser Asn Leu Asp Arg Cys Gly Phe Arg
                   85                  90                  95
   Gly Ser Ser Tyr Leu Gly Ile Pro Phe Asn Pro Ser Lys Asn Pro Gly
               100                 105                 110
   Thr Ala His Pro Tyr Asp Ser Gly His Ile Ala Met Thr Tyr Thr Gly
           115                 120                 125
   Leu Ser Cys Leu Ile Ile Leu Gly Asp Asp Leu Ser Arg Val Asp Lys
       130                 135                 140
   Glu Ala Cys Leu Ala Gly Leu Arg Ala Leu Gln Leu Glu Asp Gly Ser
   145                 150                 155                 160
   Phe Cys Ala Val Pro Glu Gly Ser Glu Asn Asp Met Arg Phe Val Tyr
                   165                 170                 175
   Cys Ala Ser Cys Ile Cys Tyr Met Leu Asn Asn Trp Ser Gly Met Asp
               180                 185                 190
   Met Lys Lys Ala Ile Ser Tyr Ile Arg Arg Ser Met Ser Tyr Asp Asn
           195                 200                 205
   Gly Leu Ala Gln Gly Ala Gly Leu Glu Ser His Gly Gly Ser Thr Phe
       210                 215                 220
   Cys Gly Ile Ala Ser Leu Cys Leu Met Gly Lys Leu Glu Glu Val Phe
   225                 230                 235                 240
   Ser Glu Lys Glu Leu Asn Arg Ile Lys Arg Trp Cys Ile Met Arg Gln
                   245                 250                 255
   Gln Asn Gly Tyr His Gly Arg Pro Asn Lys Pro Val Asp Thr Cys Tyr
               260                 265                 270
   Ser Phe Trp Val Gly Ala Thr Leu Lys Leu Leu Lys Ile Phe Gln Tyr
           275                 280                 285
   Thr Asn Phe Glu Lys Asn Arg Asn Tyr Ile Leu Ser Thr Gln Asp Arg
   290 295 300
   Leu Val Gly Gly Phe Ala Lys Trp Pro Asp Ser His Pro Asp Ala Leu
   305 310 315 320
   His Ala Tyr Phe Gly Ile Cys Gly Leu Ser Leu Met Glu Glu Ser Gly
   325 330 335
   Ile Cys Lys Val His Pro Ala Leu Asn Val Ser Thr Arg Thr Ser Glu
   340 345 350
   Arg Leu Arg Asp Leu His Gln Ser Trp Lys Thr Lys Asp Ser Lys Gln
   355 360 365
   Cys Ser Asp Asn Val His Ile Ser Ser
   370 375
<210> 80
   <211> 1969
   <212> DNA
   <213> Homo sapiens
<400> 80
   gaataaaatg aacaattcag ttcctcagtc acatgagctg tgtgtcaaat gcacaacagc 60
   cgtatgtggc tcgtggcccc tgtaccggac actcccatcc ctgcagagtt actggacagt 120
   gctgatctag ggattctgtt acaaaatcca tgaaagtgtt cagcacaatg ccgggcccat 180
   ataaacgtca gtagttgttg ttattataat tagtcttgac ccaacggcaa attcactttg 240
   agaccttaga taaatcactc tacctctctg agcctggttt ccttgcccta aaaggatggc 300
   aaggggctgg gcatggtggc cactgaggat gagaggctag cagggagcgg tgagggagag 360
   cggctggatt tcttacggga tcggcacgtg cgatttttcc agcgctgcct ccaggttttg 420
   ccggagcgct attcttcact cgagacaagc aggttgacaa ttgcattttt tgcactctcc 480
   gggctggata tgttggattc cttagatgtg gtgaacaaag atgatataat agagtggatt 540
   tattccctgc aggtccttcc cacagaagac agatcaaatc taaatcgctg tggtttccga 600
   ggctcttcat acctgggtat tccgttcaat ccatcaaagg ctcctggaac agctcatcct 660
   tatgatagtg gccacattgc aatgacctac actggcctct catgcttagt tattcttgga 720
   gacgacttaa gccgagtaaa taaagaagct tgcttagcgg gcttgagagc ccttcagctg 780
   gaagatggga gtttttgtgc agtacctgaa ggcagtgaaa atgacatgcg atttgtgtac 840
   tgtgcttcct gtatttgcta tatgctcaac aactggtcag gcatggatat gaaaaaagcc 900
   atcacctata ttagaaggag tatgtcctat gacaatggac tggcacaggg agctggactt 960
   gaatctcatg gaggatcaac tttttgtggc attgcctcac tatgtctgat gggtaaacta 1020
   gaagaagttt tttcagaaaa agaattgaac aggataaaga ggtggtgtat aatgaggcaa 1080
   caaaatggtt atcatggaag acctaataag cctgtagaca cctgttattc tttttgggtg 1140
   ggagcaactc tgaagcttct aaaaattttc caatacacta actttgagaa aaatagaaat 1200
   tacatcttat caactcaaga tcgccttgta gggggatttg ccaagtggcc agacagtcat 1260
   ccagatgctt tgcatgcata ctttgggatc tgtggcctgt cactaatgga ggaaagtgga 1320
   atttgtaaag ttcatcctgc tctgaatgta agcacacgga cttctgaacg ccttctagat 1380
   ctccatcaaa gctggaaaac caaggactct aaacaatgct cagagaatgt acatatctcc 1440
   acatgactga ttttagattg ggagggtggg ggggatttgt agcataactg tagctcaagt 1500
   ttaaaagcca tgtataacca agtgtgctct ttttttaaaa ggtagagtct tacaatcaaa 1560
   tctcctgctg atttcacttt gggatatggt cttgagccag taatctttat actgggtttc 1620
   aagaaaatct ttgttgaagt ttgaaccaca actttgtcgt ggttcttaaa tgtttatact 1680
   gtatttctaa gaagttgttt gaggcaaatt aactgtatgt gtgtaggtta tctttttaaa 1740
   aactcttcag tgcaaattgt atcttattat aaaatggaca caaattttca agtttacact 1800
   tcatatagca ttgataatct tcaggtgaac acttagtgat catttaaaaa gctcactgct 1860
   gatcgtagaa aatttgcttt aattaattaa gtatctggga ttattctttg aaaacagatg 1920
   accataattt tttttaaaga agagtgactt attttgtctt attcttaag 1969
<210> 81
   <211> 377
   <212> PRT
   <213> Homo sapiens
<400> 81
   Met Val Ala Thr Glu Asp Glu Arg Leu Ala Gly Ser Gly Glu Gly Glu
   1               5                   10                  15
   Arg Leu Asp Phe Leu Arg Asp Arg His Val Arg Phe Phe Gln Arg Cys
               20                  25                  30
   Leu Gln Val Leu Pro Glu Arg Tyr Ser Ser Leu Glu Thr Ser Arg Leu
           35                  40                  45
   Thr Ile Ala Phe Phe Ala Leu Ser Gly Leu Asp Met Leu Asp Ser Leu
       50                  55                  60
   Asp Val Val Asn Lys Asp Asp Ile Ile Glu Trp Ile Tyr Ser Leu Gln
   65                  70                  75                  80
   Val Leu Pro Thr Glu Asp Arg Ser Asn Leu Asn Arg Cys Gly Phe Arg
                   85                  90                  95
   Gly Ser Ser Tyr Leu Gly Ile Pro Phe Asn Pro Ser Lys Ala Pro Gly
               100                 105                 110
   Thr Ala His Pro Tyr Asp Ser Gly His Ile Ala Met Thr Tyr Thr Gly
           115                 120                 125
   Leu Ser Cys Leu Val Ile Leu Gly Asp Asp Leu Ser Arg Val Asn Lys
       130                 135                 140
   Glu Ala Cys Leu Ala Gly Leu Arg Ala Leu Gln Leu Glu Asp Gly Ser
   145                 150                 155                 160
   Phe Cys Ala Val Pro Glu Gly Ser Glu Asn Asp Met Arg Phe Val Tyr
                   165                 170                 175
   Cys Ala Ser Cys Ile Cys Tyr Met Leu Asn Asn Trp Ser Gly Met Asp
               180                 185                 190
   Met Lys Lys Ala Ile Thr Tyr Ile Arg Arg Ser Met Ser Tyr Asp Asn
           195                 200                 205
   Gly Leu Ala Gln Gly Ala Gly Leu Glu Ser His Gly Gly Ser Thr Phe
       210                 215                 220
   Cys Gly Ile Ala Ser Leu Cys Leu Met Gly Lys Leu Glu Glu Val Phe
   225                 230                 235                 240
   Ser Glu Lys Glu Leu Asn Arg Ile Lys Arg Trp Cys Ile Met Arg Gln
                   245                 250                 255
   Gln Asn Gly Tyr His Gly Arg Pro Asn Lys Pro Val Asp Thr Cys Tyr
               260                 265                 270
   Ser Phe Trp Val Gly Ala Thr Leu Lys Leu Leu Lys Ile Phe Gln Tyr
           275                 280                 285
   Thr Asn Phe Glu Lys Asn Arg Asn Tyr Ile Leu Ser Thr Gln Asp Arg
       290                 295                 300
   Leu Val Gly Gly Phe Ala Lys Trp Pro Asp Ser His Pro Asp Ala Leu
   305                 310                 315                 320
   His Ala Tyr Phe Gly Ile Cys Gly Leu Ser Leu Met Glu Glu Ser Gly
                   325                 330                 335
   Ile Cys Lys Val His Pro Ala Leu Asn Val Ser Thr Arg Thr Ser Glu
               340                 345                 350
   Arg Leu Leu Asp Leu His Gln Ser Trp Lys Thr Lys Asp Ser Lys Gln
           355                 360                 365
   Cys Ser Glu Asn Val His Ile Ser Thr
       370                 375
<210> 82
   <211> 2056
   <212> DNA
   <213> Mus musculus
<400> 82
   gctaaactat cccgcaaaga tttttctttc ctccctaaac cctccttttt gctctccttt 60
   tctataccct taactgcaaa caaaccatta aacgacccct ctcctgggcc tccgacggca 120
   ggagtccgcg gacctcccag gccgacagcc ctccctctac ccgcgaggtt ccgggccggg 180
   cgagagggcg cgagcacagc cgaggacatg gaggtgactg cggaccagcc gcgctgggtg 240
   agccaccatc accccgcggt cctcaacggt cagcacccag acacgcacca cccgggcctc 300
   ggccattcgt acatggaagc tcagtatccg ctgacggaag aggtggacgt actttttaac 360
   atcgatggtc aaggcaacca cgtcccgtcc tactacggaa actccgtcag ggctacggtg 420
   cagaggtatc ctccgaccca ccacgggagc caggtatgcc gcccgcctct gctgcacgga 480
   tctctgccct ggctggatgg cggcaaagcc ctgagcagcc accacaccgc ctcgccctgg 540
   aacctcagcc ccttctccaa gacgtccatc caccacggct ctccggggcc tctgtccgtt 600
   taccctccgg cttcatcctc ttctctggcg gccggccact ccagtcctca tctcttcacc 660
   ttcccgccca ccccgccgaa agacgtctcc ccagacccgt cgctgtccac cccgggatcc 720
   gccgggtcgg ccaggcaaga tgagaaagag tgcctcaagt atcaggtgca gctgccagat 780
   agcatgaagc tggagacgtc tcactctcga ggcagcatga ccaccctggg tggggcctca 840
   tcctcagccc accaccccat taccacctat ccgccctatg tgcccgagta cagctctgga 900
   ctcttcccac ccagcagcct gctgggagga tcccctaccg ggttcggatg taagtcgagg 960
   cccaaggcac gatccagcac agaaggcagg gagtgtgtga actgcggggc aacctctacc 1020
   ccactgtggc ggcgagatgg taccgggcac tacctttgca atgcctgcgg actctaccat 1080
   aaaatgaatg ggcagaaccg gccccttatc aagcccaagc gaaggctgtc ggcagcaagg 1140
   agagcaggga catcctgcgc gaactgtcag accaccacca ccaccctctg gaggaggaac 1200
   gctaatgggg acccggtctg caatgcctgt gggctgtact acaagcttca taatattaac 1260
   agacccctga ctatgaagaa agaaggcatc cagacccgaa accggaagat gtctagcaaa 1320
   tcgaaaaagt gcaaaaaggt gcatgacgcg ctggaggact tccccaagag cagctccttc 1380
   aacccggccg ctctctccag acacatgtca tccctgagcc acatctctcc cttcagccac 1440
   tccagccaca tgctgaccac accgacgccc atgcatccgc cctccggcct ctccttcgga 1500
   cctcaccacc cttccagcat ggtcaccgcc atgggttaga gaggcagagc cctgctccac 1560
   atgcgtgagg agtctccaag tgtgcgaaga gttcctccga ccccttctac ttgcgttttt 1620
   cgcaggagca gtatcatgaa gcccgaaagc gacagatctg tgtttttgaa ggcagaaagc 1680
   aaaatgtttg cttctttttt caaaggagct cgaggtggtg tctgcattcc aaccactgaa 1740
   tccggatccc atttgtgaat aagccattca gactcatatt ccctatttaa cagggtctct 1800
   agtgctgtga aaaaaatatt gctgaacatt gcatataact tatattgtaa gaaatactgt 1860
   acatttgagg aagactttat tgtacctgga tagctgtaag aaaggcatga aggacgccaa 1920
   gagttttaag gaatataggg gnnttaaagt atggagatac agaagaaacc actaagtctg 1980
   atgtccaaat gggcacactg tcagttttgt ttcccttcag ttgtttgatg catttaaaaa 2040
   aaaaaaaaag aaagaa                                                 2056
<210> 83
   <211> 443
   <212> PRT
   <213> Mus musculus
<400> 83
   Met Glu Val Thr Ala Asp Gln Pro Arg Trp Val Ser His His His Pro
   1               5                   10                  15
   Ala Val Leu Asn Gly Gln His Pro Asp Thr His His Pro Gly Leu Gly
               20                  25                  30
   His Ser Tyr Met Glu Ala Gln Tyr Pro Leu Thr Glu Glu Val Asp Val
           35                  40                  45
   Leu Phe Asn Ile Asp Gly Gln Gly Asn His Val Pro Ser Tyr Tyr Gly
       50                  55                  60
   Asn Ser Val Arg Ala Thr Val Gln Arg Tyr Pro Pro Thr His His Gly
   65                  70                  75                  80
   Ser Gln Val Cys Arg Pro Pro Leu Leu His Gly Ser Leu Pro Trp Leu
                   85                  90                  95
   Asp Gly Gly Lys Ala Leu Ser Ser His His Thr Ala Ser Pro Trp Asn
               100                 105                 110
   Leu Ser Pro Phe Ser Lys Thr Ser Ile His His Gly Ser Pro Gly Pro
           115                 120                 125
   Leu Ser Val Tyr Pro Pro Ala Ser Ser Ser Ser Leu Ala Ala Gly His
       130                 135                 140
   Ser Ser Pro His Leu Phe Thr Phe Pro Pro Thr Pro Pro Lys Asp Val
   145                 150                 155                 160
   Ser Pro Asp Pro Ser Leu Ser Thr Pro Gly Ser Ala Gly Ser Ala Arg
                   165                 170                 175
   Gln Asp Glu Lys Glu Cys Leu Lys Tyr Gln Val Gln Leu Pro Asp Ser
               180                 185                 190
   Met Lys Leu Glu Thr Ser His Ser Arg Gly Ser Met Thr Thr Leu Gly
           195                 200                 205
   Gly Ala Ser Ser Ser Ala His His Pro Ile Thr Thr Tyr Pro Pro Tyr
       210                 215                 220
   Val Pro Glu Tyr Ser Ser Gly Leu Phe Pro Pro Ser Ser Leu Leu Gly
   225                 230                 235                 240
   Gly Ser Pro Thr Gly Phe Gly Cys Lys Ser Arg Pro Lys Ala Arg Ser
                   245                 250                 255
   Ser Thr Glu Gly Arg Glu Cys Val Asn Cys Gly Ala Thr Ser Thr Pro
               260                 265                 270
   Leu Trp Arg Arg Asp Gly Thr Gly His Tyr Leu Cys Asn Ala Cys Gly
           275                 280                 285
   Leu Tyr His Lys Met Asn Gly Gln Asn Arg Pro Leu Ile Lys Pro Lys
       290                 295                 300
   Arg Arg Leu Ser Ala Ala Arg Arg Ala Gly Thr Ser Cys Ala Asn Cys
   305                 310                 315                 320
   Gln Thr Thr Thr Thr Thr Leu Trp Arg Arg Asn Ala Asn Gly Asp Pro
                   325                 330                 335
   Val Cys Asn Ala Cys Gly Leu Tyr Tyr Lys Leu His Asn Ile Asn Arg
               340                 345                 350
   Pro Leu Thr Met Lys Lys Glu Gly Ile Gln Thr Arg Asn Arg Lys Met
           355                 360                 365
   Ser Ser Lys Ser Lys Lys Cys Lys Lys Val His Asp Ala Leu Glu Asp
       370                 375                 380
   Phe Pro Lys Ser Ser Ser Phe Asn Pro Ala Ala Leu Ser Arg His Met
   385                 390                 395                 400
   Ser Ser Leu Ser His Ile Ser Pro Phe Ser His Ser Ser His Met Leu
                   405                 410                 415
   Thr Thr Pro Thr Pro Met His Pro Pro Ser Gly Leu Ser Phe Gly Pro
               420                 425                 430
   His His Pro Ser Ser Met Val Thr Ala Met Gly
           435                 440
<210> 84
   <211> 2365
   <212> DNA
   <213> Homo sapiens
<400> 84
   tcccagcctt cccatccccc caccgaaagc aaatcattca acgacccccg accctccgac 60
   ggcaggagcc ccccgacctc ccaggcggac cgcccttccc tccccgcgcg ggttccgggc 120
   ccggcgagag ggcgcgacga cagccgaggc catggaggtg acggcggacc agccgcgctg 180
   ggtgagccac caccaccccg ccgtgctcaa cgggcagcac ccggacacgc accacccggg 240
   cctcagccac tcctacatgg acgcggcgca gtacccgctg ccggaggagg tggatgtgct 300
   ttttaacatc gacggtcaag gcaaccacgt cccgccctac tacggaaact cggtcagggc 360
   cacggtgcag aggtaccctc cgacccacca cgggagccag gtgtgccgcc cgcctctgct 420
   tcatggatcc ctaccctggc tggacggcgg caaagccctg ggcagccacc acaccgcctc 480
   cccctggaat ctcagcccct tctccaagac gtccatccac cacggctccc cggggcccct 540
   ctccgtctac cccccggcct cgtcctcctc cttgtcgggg ggccacgcca gcccgcacct 600
   cttcaccttc ccgcccaccc cgccgaagga cgtctccccg gacccatcgc tgtccacccc 660
   aggctcggcc ggctcggccc ggcaggacga gaaagagtgc ctcaagtacc aggtgcccct 720
   gcccgacagc atgaagctgg agtcgtccca ctcccgtggc agcatgaccg ccctgggtgg 780
   agcctcctcg tcgacccacc accccatcac cacctacccg ccctacgtgc ccgagtacag 840
   ctccggactc ttccccccca gcagcctgct gggcggctcc cccaccggct tcggatgcaa 900
   gtccaggccc aaggcccggt ccagcacagg cagggagtgt gtgaactgtg gggcaacctc 960
   gaccccactg tggcggcgag atggcacggg acactacctg tgcaacgcct gcgggctcta 1020
   tcacaaaatg aacggacaga accggcccct cattaagccc aagcgaaggc tgtctgcagc 1080
   caggagagca gggacgtcct gtgcgaactg tcagaccacc acaaccacac tctggaggag 1140
   gaatgccaat ggggaccctg tctgcaatgc ctgtgggctc tactacaagc ttcacaatat 1200
   taacagaccc ctgactatga agaaggaagg catccagacc agaaaccgaa aaatgtctag 1260
   caaatccaaa aagtgcaaaa aagtgcatga ctcactggag gacttcccca agaacagctc 1320
   gtttaacccg gccgccctct ccagacacat gtcctccctg agccacatct cgcccttcag 1380
   ccactccagc cacatgctga ccacgcccac gccgatgcac ccgccatcca gcctgtcctt 1440
   tggaccacac cacccctcca gcatggtcac cgccatgggt tagagccctg ctcgatgctc 1500
   acagggcccc cagcgagagt ccctgcagtc cctttcgact tgcatttttg caggagcagt 1560
   atcatgaagc ctaaacgcga tggatatatg tttttgaagg cagaaagcaa aattatgttt 1620
   gccactttgc aaaggagctc actgtggtgt ctgtgttcca accactgaat ctggacccca 1680
   tctgtgaata agccattctg actcatatcc cctatttaac agggtctcta gtgctgtgaa 1740
   aaaaaaaaat cctgaacatt gcatataact tatattgtaa gaaatactgt acaatgactt 1800
   tattgcatct gggtagctgt aaggcatgaa ggatgccaag aagtttaagg aatatgggag 1860
   aaatagtgtg gaaattaaga agaaactagg tctgatattc aaatggacaa actgccagtt 1920
   ttgtttcctt tcactggcca cagttgtttg atgcattaaa agaaaataaa aaaaagaaaa 1980
   aagagaaaag aaaaaaaaag aaaaaagttg taggcgaatc atttgttcaa agctgttggc 2040
   cctctgcaaa ggaaatacca gttctgggca atcagtgtta ccgttcacca gttgccattg 2100
   agggtttcag agagcctttt tctaggccta catgctttgt gaacaagtcc ctgtaattgt 2160
   tgtttgtatg tataattcaa agcaccaaaa taagaaaaga tgtagattta tttcatcata 2220
   ttatacagac cgaactgttg tataaattta tttactgcta gtcttaagaa ctgctttctt 2280
   tcgtttgttt gtttcaatat tttccttctc tctcaatttt cggttgaata aactagatta 2340
   cattcagttg gcaaaaaaaa aaaaa                                        2365
<210> 85
   <211> 443
   <212> PRT
   <213> Homo sapiens
<400> 85
   Met Glu Val Thr Ala Asp Gln Pro Arg Trp Val Ser His His His Pro
   1               5                   10                  15
   Ala Val Leu Asn Gly Gln His Pro Asp Thr His His Pro Gly Leu Ser
               20                  25                  30
   His Ser Tyr Met Asp Ala Ala Gln Tyr Pro Leu Pro Glu Glu Val Asp
           35                  40                  45
   Val Leu Phe Asn Ile Asp Gly Gln Gly Asn His Val Pro Pro Tyr Tyr
       50                  55                  60
   Gly Asn Ser Val Arg Ala Thr Val Gln Arg Tyr Pro Pro Thr His His
   65                  70                  75                  80
   Gly Ser Gln Val Cys Arg Pro Pro Leu Leu His Gly Ser Leu Pro Trp
                   85                  90                  95
   Leu Asp Gly Gly Lys Ala Leu Gly Ser His His Thr Ala Ser Pro Trp
               100                 105                 110
   Asn Leu Ser Pro Phe Ser Lys Thr Ser Ile His His Gly Ser Pro Gly
           115                 120                 125
   Pro Leu Ser Val Tyr Pro Pro Ala Ser Ser Ser Ser Leu Ser Gly Gly
       130                 135                 140
   His Ala Ser Pro His Leu Phe Thr Phe Pro Pro Thr Pro Pro Lys Asp
   145                 150                 155                 160
   Val Ser Pro Asp Pro Ser Leu Ser Thr Pro Gly Ser Ala Gly Ser Ala
                   165                 170                 175
   Arg Gln Asp Glu Lys Glu Cys Leu Lys Tyr Gln Val Pro Leu Pro Asp
               180                 185                 190
   Ser Met Lys Leu Glu Ser Ser His Ser Arg Gly Ser Met Thr Ala Leu
           195                 200                 205
   Gly Gly Ala Ser Ser Ser Thr His His Pro Ile Thr Thr Tyr Pro Pro
       210                 215                 220
   Tyr Val Pro Glu Tyr Ser Ser Gly Leu Phe Pro Pro Ser Ser Leu Leu
   225                 230                 235                 240
   Gly Gly Ser Pro Thr Gly Phe Gly Cys Lys Ser Arg Pro Lys Ala Arg
                   245                 250                 255
   Ser Ser Thr Gly Arg Glu Cys Val Asn Cys Gly Ala Thr Ser Thr Pro
               260                 265                 270
   Leu Trp Arg Arg Asp Gly Thr Gly His Tyr Leu Cys Asn Ala Cys Gly
           275                 280                 285
   Leu Tyr His Lys Met Asn Gly Gln Asn Arg Pro Leu Ile Lys Pro Lys
       290                 295                 300
   Arg Arg Leu Ser Ala Ala Arg Arg Ala Gly Thr Ser Cys Ala Asn Cys
   305                 310                 315                 320
   Gln Thr Thr Thr Thr Thr Leu Trp Arg Arg Asn Ala Asn Gly Asp Pro
                   325                 330                 335
   Val Cys Asn Ala Cys Gly Leu Tyr Tyr Lys Leu His Asn Ile Asn Arg
               340                 345                 350
   Pro Leu Thr Met Lys Lys Glu Gly Ile Gln Thr Arg Asn Arg Lys Met
           355                 360                 365
   Ser Ser Lys Ser Lys Lys Cys Lys Lys Val His Asp Ser Leu Glu Asp
       370                 375                 380
   Phe Pro Lys Asn Ser Ser Phe Asn Pro Ala Ala Leu Ser Arg His Met
   385                 390                 395                 400
   Ser Ser Leu Ser His Ile Ser Pro Phe Ser His Ser Ser His Met Leu
                   405                 410                 415
   Thr Thr Pro Thr Pro Met His Pro Pro Ser Ser Leu Ser Phe Gly Pro
               420                 425                 430
   His His Pro Ser Ser Met Val Thr Ala Met Gly
           435                 440
<210> 86
   <211> 664
   <212> DNA
   <213> Rattus norvegicus
<400> 86
   tttgctaagc tatcccgcaa agaattttct ttcctcccta aaccctctcc tttgctcacc 60
   ttttctatcc acttcactac aaacaaacct ttaagcgact ccctctcctg ggcctccgac 120
   ggcaggactc cgcggacctc ccaggccgac agccctccct ctacccgcga ggttccgggc 180
   ccggcgagag ggcgcgagca cagcccggga catggaggtg actacggacc agccgcgctg 240
   ggtgagccac catcacccgg cggtcctcaa cgggcagcac ccagacacgc accacccggg 300
   cctcggccat tcgtacatgg acccggcaca gtaccccctg acggaagagg tggacgtact 360
   ttttaacatc gatggtcaag gcaaccacgt cccgtcctac tacggaaact cggtcagggc 420
   tacggtgcag aggtatcctc cgacccacca cgggagccag gtatgccgcc cgcctctgct 480
   gcacggatct ctgccctggc tggatggcgg caaagccctg agcagccacc acaccgcctc 540
   gccctggaac ctcagcccct tctccaagac gtccatccac cacggctccc cggggcctct 600
   gtccgtctac cctccggctt catcctcttc tctggctgcg ggccactcca gtccgcatct 660
   cttc                                                              664
<210> 87
   <211> 3277
   <212> DNA
   <213> Homo sapiens
<400> 87
   gccggcgctt cccggccggt gtcgctccgc ggcgggccat ggccaacgtc tctaagaagg 60
   tgtcctggtc cggccgggac cgggacgacg aggaggcggc gccgctgctg cggaggacgg 120
   cgcggcccgg cggggggacg ccgctgctga acggggctgg gcccggggct gcgcgccagt 180
   caccacgttc tgcgcttttc cgagtcggac atatgagcag cgtggagctg gatgatgaac 240
   ttttggaccc ggatatggac cctccacatc ccttccccaa ggagatccca cacaacgaga 300
   agctcctgtc cctcaagtat gagagcttgg actatgacaa cagtgagaac cagctgttcc 360
   tggaggagga gcggcggatc aatcacacgg ccttccggac ggtggagatc aagcgctggg 420
   tcatctgcgc cctcattggg atcctcacgg gcctcgtggc ctgcttcatt gacatcgtgg 480
   tggaaaacct ggctggcctc aagtacaggg tcatcaaggg caatatcgac aagttcacag 540
   agaagggcgg actgtccttc tccctgttgc tgtgggccac gctgaacgcc gccttcgtgc 600
   tcgtgggctc tgtgattgtg gctttcatag agccggtggc tgctggcagc ggaatccccc 660
   agatcaagtg cttcctcaac ggggtgaaga tcccccacgt ggtgcggctc aagacgttgg 720
   tgatcaaagt gtccggtgtg atcctgtccg tggtcggggg cctggccgtg ggaaaggaag 780
   ggccgatgat ccactcaggt tcagtgattg ccgccgggat ctctcaggga aggtcaacgt 840
   cactgaaacg agatttcaag atcttcgagt acttccgcag agacacagag aagcgggact 900
   tcgtctccgc aggggctgcg gccggagtgt cagcggcgtt tggagccccc gtgggtgggg 960
   tcctgttcag cttggaggag ggtgcgtcct tctggaacca gttcctgacc tggaggatct 1020
   tctttgcttc catgatctcc acgttcaccc tgaattttgt tctgagcatt taccacggga 1080
   acatgtggga cctgtccagc ccaggcctca tcaacttcgg aaggtttgac tcggagaaaa 1140
   tggcctacac gatccacgag atcccggtct tcatcgccat gggcgtggtg ggcggtgtgc 1200
   ttggagctgt gttcaatgcc ttgaactact ggctgaccat gtttcgaatc aggtacatcc 1260
   accggccctg cctgcaggtg attgaggccg tgctggtggc cgccgtcacg gccacagttg 1320
   ccttcgtgct gatctactcg tcgcgggatt gccagcccct gcaggggggc tccatgtcct 1380
   acccgctgca gctcttttgt gcagatggcg agtacaactc catggctgcg gccttcttca 1440
   acaccccgga gaagagcgtg gtgagcctct tccacgaccc gccaggctcc tacaaccccc 1500
   tgaccctcgg cctgttcacg ctggtctact tcttcctggc ctgctggacc tacgggctca 1560
   cggtgtctgc cggggtcttc atcccgtccc tgctcatcgg ggctgcctgg ggccggctct 1620
   ttgggatctc cctgtcctac ctcacggggg cggcgatctg ggcggacccc ggcaaatacg 1680
   ccctgatggg agctgctgcc cagctgggcg ggattgtgcg gatgacactg agcctgaccg 1740
   tcatcatgat ggaggccacc agcaacgtga cctacggctt ccccatcatg ctggtgctca 1800
   tgaccgccaa gatcgtgggc gacgtcttca ttgagggcct gtacgacatg cacattcagc 1860
   tgcagagtgt gcccttcctg cactgggagg ccccggtcac ctcacactca ctcactgcca 1920
   gggaggtgat gagcacacca gtgacctgcc tgaggcggcg tgagaaggtc ggcgtcattg 1980
   tggacgtgct gagcgacacg gcgtccaatc acaacggctt ccccgtggtg gagcatgccg 2040
   atgacaccca gcctgcccgg ctccagggcc tgatcctgcg ctcccagctc atcgttctcc 2100
   taaagcacaa ggtgtttgtg gagcggtcca acctgggcct ggtacagcgg cgcctgaggc 2160
   tgaaggactt ccgagacgcc tacccgcgct tcccacccat ccagtccatc cacgtgtccc 2220
   aggacgagcg ggagtgcacc atggacctct ccgagttcat gaacccctcc ccctacacgg 2280
   tgccccagga ggcgtcgctc ccacgggtgt tcaagctgtt ccgggccctg ggcctgcggc 2340
   acctggtggt ggtggacaac cgcaatcagg ttgtcggggt ggtgaccagg aaggacctcg 2400
   ccaggtaccg cctgggaaag agaggcttgg aggagctctc gctggcccag acgtgaggcc 2460
   cagccctgcc cataatgggc actggcgctg gcaccccggc ccttctgcat ttcctcccgg 2520
   agtcactggt ttctcggccc aaaccatgct ccccagcagt ggcaatggcg agcaccctgc 2580
   agctgggcgg gcaggcggca ggcgcggaac tgaccctctc gcgggactga ccctgttgtg 2640
   ggcagtggtc tccccccttg gcgcctcctt gcgcaggccc agcctccact ctcctcgtct 2700
   aggtttcttt acctccaggg atcagctgtg tgtgtgtgac ctccctaccg ggctatcggc 2760
   ctcttgggag ccagcggcag ggccggcacc tgcgtgcctg tgcccgtgtg cgtgagacag 2820
   agcccttgcc cctgctgctg ccccgagggc tgccctgccc tggaagggcc cctctgcctc 2880
   cacaccagtg gagtcttcga gacttgggag ctgcttggcc tcattttcag ccatgagcag 2940
   acggcctgtg gtccctgggc ctgaggcacg gactcgtagc accagggttt ggaggctgcg 3000
   accgccccgg agagcagctt cacactggcg ccacagagga gccccacgtg cactccccgg 3060
   cctgcatccg gcttgggtac acaggcccag aggactgggg tgactcacgg gccctgtgct 3120
   gtgatgttga gagctgagaa aaacctccaa ggccctgagc cccatgccca gccctgcctt 3180
   ggtcccccaa tccccagagc ttggagtctg ggccccacac ccagccctgc cttggtccct 3240
   gagcctcaaa gcgtggaatt gctgccctgt ggacact                          3277
<210> 88
   <211> 805
   <212> PRT
   <213> Homo sapiens
<400> 88
<210> 89
   <211> 2750
   <212> DNA
   <213> Rattus norvegicus
<400> 89
   ggggcgcggg tcacgggaac gctgccgggc tgccggctgt tcttgtggag tttggtcctc 60
   agtgggccat ggccaacgtt tctaagaaag tgtcttggtc cggccgagat cgcgatgacg 120
   aggagggggc gccgctgctt cgaaggacgg ggcaacctga cgaggagacg ccgctgctga 180
   acggagccgg gccgggcgcg cgccagtctc attctgcact tttccgaatt ggacagatga 240
   acaacgtgga gctggatgat gaactcctgg acccggaagt ggaccctcct cacaccttcc 300
   ccaaggagat tccacacaac gagaagctcc tctccctcaa gtatgagagc ctggactatg 360
   acaatagtga gaatcagctc ttcctggagg aggaaagacg aatcaaccac acggctttcc 420
   ggacagtgga gatcaagcgc tgggttatct gtgccctcat tggaatcctc acaggcctag 480
   tagcctgctt cattgacatt gtagtggaga acctggcagg cctcaagtac cgagtcatca 540
   aggacaacat cgacaagttc acagagaagg gcggcctgtc cttctccctc ctgctgtggg 600
   ccacactgaa ctctgccttc gtgctcgtgg ggtctgtgat tgtggccttc atagagccag 660
   ttgctgctgg cagcggaatc cctcagatca agtgcttcct caatggggtg aagatccccc 720
   acgtggtgcg gctcaagacg ctggtgatca aggtgtctgg cgtgattctg tctgtggtag 780
   ggggactggc tgtgggaaag gaagggccaa tgatccactc aggatccgtg attgctgcag 840
   ggatttcaca gggaaggtcg acgtcactca agcgagattt taagatcttt gaatatttcc 900
   gcagagatac agagaagcgg gattttgtct cagctggagc tgcagctgga gtgtctgctg 960
   cgtttggagc acctgtgggt ggggtcctgt tcagcctgga agagggcgcc tccttctgga 1020
   atcagttcct gacatggaga attttctttg cttccatgat ttcgaccttt acactgaatt 1080
   ttgttctgag catctaccat ggaaacatgt gggacctgtc cagccctggc ctcataaatt 1140
   ttggaagatt cgactcagag aaaatggcct acacaatcca tgagattcct gtcttcatcg 1200
   ccatgggtgt ggtgggtggc attcttggag ccgtgttcaa tgccttgaat tactggctaa 1260
   ctatgtttcg aatcaggtac atccaccggc cctgcctcca agtgattgag gccatgctgg 1320
   tggcagctgt cacagccaca gttgcatttg tcttgattta ctcgtctcga gattgccagc 1380
   ccctgcaggg gagctccatg tcctacccac tccagctctt ctgtgcagat ggcgaataca 1440
   actcaatggc cgcagccttc tttaacaccc ctgagaagag cgtcgtcagc ctgttccacg 1500
   acccaccagg ctcctataat cccatgactc tcggcctgtt caccctggtc tacttcttcc 1560
   tggcctgctg gacctatggc ctcacagtat ctgctggtgt cttcatccca tccctgctca 1620
   ttggggctgc ctggggccga ctctttggca tctccatgtc ctacctcaca ggagcagcga 1680
   tctgggcaga tccgggtaaa tacgccctga tgggagctgc tgctcagctt ggtgggatcg 1740
   tgaggatgac ccttagcctg acagtcatca tgatggaggc caccagcaac gtgacctacg 1800
   gttttcccat catgttggtg ctgatgactg ccaagattgt gggtgatgtc ttcattgagg 1860
   gcctctatga catgcacatc cagctgcaaa gtgtgccctt cctacactgg gaagccccgg 1920
   tcacctcaca ttcgctcact gccagggaag taatgagcac gcctgtgacc tgcctgagga 1980
   ggagagagaa ggttggcatc atcgtggatg tcctaagtga cacagcgtct aatcacaatg 2040
   ggttccctgt ggtggaggat gtaggagaca cccagccagc cagactccaa ggcctaatcc 2100
   tgcgttccca gctcatcgtg ctcctgaagc acaaggtgtt tgtggagagg tccaacatgg 2160
   gtttggtgca gcggagactg aggctgaaag actttcgcga tgcctaccca cgcttccccc 2220
   caatccagtc catccacgta tcccaggatg agcgggagtg caccatggac ctttctgagt 2280
   tcatgaaccc ttctccctac actgtgccac aggaggcatc tcttcctcga gtgttcaagc 2340
   tgttccgggc tctgggcctg aggcacctgg tcgtagtaga caaccacaat caggtggtcg 2400
   ggctggtgac caggaaggac ctagcaagat accgcctagg aaaaggaggc ctagaagagc 2460
   tttcactggc ccagacgtga gggctggccc ccacccttgg gcagcggcac cccggcccct 2520
   ctgcacctcc tcccagggtc cctggtctca gccaaagcct tgccctgggc agtgcagcaa 2580
   caggagcaaa tgccctcccc gggcttggct ggtgtggggc ccagaccctt tgtcctgggc 2640
   agttggttta catcatcagc atttccctat tccctgaacc tgcagtcctc agacttgtcc 2700
   cactcctggg tcccttctcc caggatgtaa agtgtgtttt cacacccctt 2750
<210> 90
   <211> 803
   <212> PRT
   <213> Rattus norvegicus
<400> 90
   Met Ala Asn Val Ser Lys Lys Val Ser Trp Ser Gly Arg Asp Arg Asp
    1               5                  10                  15
   Asp Glu Glu Gly Ala Pro Leu Leu Arg Arg Thr Gly Gln Pro Asp Glu
               20                  25                  30
   Glu Thr Pro Leu Leu Asn Gly Ala Gly Pro Gly Ala Arg Gln Ser His
           35                  40                  45
   Ser Ala Leu Phe Arg Ile Gly Gln Met Asn Asn Val Glu Leu Asp Asp
       50                  55                  60
   Glu Leu Leu Asp Pro Glu Val Asp Pro Pro His Thr Phe Pro Lys Glu
   65                  70                  75                  80
   Ile Pro His Asn Glu Lys Leu Leu Ser Leu Lys Tyr Glu Ser Leu Asp
                   85                  90                  95
   Tyr Asp Asn Ser Glu Asn Gln Leu Phe Leu Glu Glu Glu Arg Arg Ile
               100                 105                 110
   Asn His Thr Ala Phe Arg Thr Val Glu Ile Lys Arg Trp Val Ile Cys
           115                 120                 125
   Ala Leu Ile Gly Ile Leu Thr Gly Leu Val Ala Cys Phe Ile Asp Ile
       130                 135                 140
   Val Val Glu Asn Leu Ala Gly Leu Lys Tyr Arg Val Ile Lys Asp Asn
   145                 150                 155                 160
   Ile Asp Lys Phe Thr Glu Lys Gly Gly Leu Ser Phe Ser Leu Leu Leu
                   165                 170                 175
   Trp Ala Thr Leu Asn Ser Ala Phe Val Leu Val Gly Ser Val Ile Val
               180                 185                 190
   Ala Phe Ile Glu Pro Val Ala Ala Gly Ser Gly Ile Pro Gln Ile Lys
   195 200 205
   Cys Phe Leu Asn Gly Val Lys Ile Pro His Val Val Arg Leu Lys Thr
   210 215 220
   Leu Val Ile Lys Val Ser Gly Val Ile Leu Ser Val Val Gly Gly Leu
   225 230 235 240
   Ala Val Gly Lys Glu Gly Pro Met Ile His Ser Gly Ser Val Ile Ala
   245 250 255
   Ala Gly Ile Ser Gln Gly Arg Ser Thr Ser Leu Lys Arg Asp Phe Lys
   260 265 270
   Ile Phe Glu Tyr Phe Arg Arg Asp Thr Glu Lys Arg Asp Phe Val Ser
   275 280 285
   Ala Gly Ala Ala Ala Gly Val Ser Ala Ala Phe Gly Ala Pro Val Gly
   290 295 300
   Gly Val Leu Phe Ser Leu Glu Glu Gly Ala Ser Phe Trp Asn Gln Phe
   305 310 315 320
   Leu Thr Trp Arg Ile Phe Phe Ala Ser Met Ile Ser Thr Phe Thr Leu
   325 330 335
   Asn Phe Val Leu Ser Ile Tyr His Gly Asn Met Trp Asp Leu Ser Ser
   340 345 350
   Pro Gly Leu Ile Asn Phe Gly Arg Phe Asp Ser Glu Lys Met Ala Tyr
   355 360 365
   Thr Ile His Glu Ile Pro Val Phe Ile Ala Met Gly Val Val Gly Gly
   370 375 380
   Ile Leu Gly Ala Val Phe Asn Ala Leu Asn Tyr Trp Leu Thr Met Phe
   385 390 395 400
   Arg Ile Arg Tyr Ile His Arg Pro Cys Leu Gln Val Ile Glu Ala Met
   405 410 415
   Leu Val Ala Ala Val Thr Ala Thr Val Ala Phe Val Leu Ile Tyr Ser
   420 425 430
   Ser Arg Asp Cys Gln Pro Leu Gln Gly Ser Ser Met Ser Tyr Pro Leu
   435 440 445
   Gln Leu Phe Cys Ala Asp Gly Glu Tyr Asn Ser Met Ala Ala Ala Phe
   450 455 460
   Phe Asn Thr Pro Glu Lys Ser Val Val Ser Leu Phe His Asp Pro Pro
   465 470 475 480
   Gly Ser Tyr Asn Pro Met Thr Leu Gly Leu Phe Thr Leu Val Tyr Phe
   485 490 495
   Phe Leu Ala Cys Trp Thr Tyr Gly Leu Thr Val Ser Ala Gly Val Phe
   500 505 510
   Ile Pro Ser Leu Leu Ile Gly Ala Ala Trp Gly Arg Leu Phe Gly Ile
   515 520 525
   Ser Met Ser Tyr Leu Thr Gly Ala Ala Ile Trp Ala Asp Pro Gly Lys
   530 535 540
   Tyr Ala Leu Met Gly Ala Ala Ala Gln Leu Gly Gly Ile Val Arg Met
   545 550 555 560
   Thr Leu Ser Leu Thr Val Ile Met Met Glu Ala Thr Ser Asn Val Thr
   565 570 575
   Tyr Gly Phe Pro Ile Met Leu Val Leu Met Thr Ala Lys Ile Val Gly
   580 585 590
   Asp Val Phe Ile Glu Gly Leu Tyr Asp Met His Ile Gln Leu Gln Ser
   595 600 605
   Val Pro Phe Leu His Trp Glu Ala Pro Val Thr Ser His Ser Leu Thr
   610 615 620
   Ala Arg Glu Val Met Ser Thr Pro Val Thr Cys Leu Arg Arg Arg Glu
   625 630 635 640
   Lys Val Gly Ile Ile Val Asp Val Leu Ser Asp Thr Ala Ser Asn His
   645 650 655
   Asn Gly Phe Pro Val Val Glu Asp Val Gly Asp Thr Gln Pro Ala Arg
   660 665 670
   Leu Gln Gly Leu Ile Leu Arg Ser Gln Leu Ile Val Leu Leu Lys His
   675 680 685
   Lys Val Phe Val Glu Arg Ser Asn Met Gly Leu Val Gln Arg Arg Leu
   690 695 700
   Arg Leu Lys Asp Phe Arg Asp Ala Tyr Pro Arg Phe Pro Pro Ile Gln
   705 710 715 720
   Ser Ile His Val Ser Gln Asp Glu Arg Glu Cys Thr Met Asp Leu Ser
   725 730 735
   Glu Phe Met Asn Pro Ser Pro Tyr Thr Val Pro Gln Glu Ala Ser Leu
   740 745 750
   Pro Arg Val Phe Lys Leu Phe Arg Ala Leu Gly Leu Arg His Leu Val
   755 760 765
   Val Val Asp Asn His Asn Gln Val Val Gly Leu Val Thr Arg Lys Asp
   770 775 780
   Leu Ala Arg Tyr Arg Leu Gly Lys Gly Gly Leu Glu Glu Leu Ser Leu
   785 790 795 800
   Ala Gln Thr
<210> 91
   <211> 1761
   <212> DNA
   <213> Mus musculus
<400> 91
   tgggttatat cttgctaccc gttgatttct ttctcagcat gacttccatg ctctgttgga 60
   aggcttagcc ctgtgtcaga tacttgaggg attagcagga ttgtgtgaga gatgctcact 120
   tcaatgcctg tacttagagg tgaccgtgga atagcagcca tgggccaatg ggggaatctg 180
   gccagaatag gattggccaa agtttcccac tgcaatctca aggcctctga ctctttcctc 240
   tcctgcagcc agccaggctc caaggcttga tcctgcgttc ccagcttatc gttctcttga 300
   agcataaggt acacccagcc gcccttgggt gggagtgtcc tgggcaccgt ccttcacagt 360
   ggcagacaca taactagctg ccgccttcag gtgtttgtgg agaggtccaa catgggtttg 420
   gtgcagcggc gactgaggct gaaggacttc cgcgatgcct atccacgctt ccccccaatc 480
   cagtccatcc atgtatccca agatgagcgt gaatgcacca tggacctttc tgaattcatg 540
   aacccttctc cctacactgt gcctcaggta cttacagacc cagaggagtt ggaagcaggg 600
   ctggggtatg gtttgatgcc ttataaactt tgaaaaggtg gtaaggagcc tctggaatct 660
   tgtggagtaa ttgctgccag gcagaggagg ccttcaggga tcgtgcaagt agagcttgcg 720
   tcctgaggtg gccatctaac cagaatatat tagggacagc ctgagagaac cccattagca 780
   ttacacaggt ccttagagtc aggcatggag gggaagagac agctcagtac ccttgaaatg 840
   cttggtttgg gagtttgaat ctcttcccat gctgggtaaa gcgggctaaa ggtggctgat 900
   tactttgcag gaggcatctc ttcctcgagt gttcaagctc ttccgggctc tgggcctgag 960
   gcacctggta gtagtagaca accacaatca ggtgagactg ctgtctgtcc tgctggagat 1020
   tacgggagct ggttcacact ctacttccta tattcttgct aggttgtggg tggacattgg 1080
   gtcttgtctg tctcaggaca gtaagcctgg gtcctagcct attactgtac acctacttgg 1140
   aactaatatg tggagtatgc tatcacatgg agtagaagtt ctgtagaccc tatggggact 1200
   ctgtgacctg gtattgttct tgcaggtggt cgggctggtg accaggaagg acctagcaag 1260
   ataccgccta ggaaagggag gcctagaaga gctttcgctg gcccagacgt gagggctggc 1320
   ccccagcctt ggacatgcag taccccagcc cctctgcacc tcctcccagg gtccctgatc 1380
   tcagccaaag ccttgccccc taggcagtgc agcaacagga gcaaatgccc tccctagctt 1440
   ggctggtgtg ggggccagac cctttgtcct gggcagttgg tttcagtcat cagcactttc 1500
   cctattccct gaccctccag gcctcagact tgccccactc ctggttcctt ctcccaggat 1560
   gaagtgtgtg ttatcacacc ccttgtggct cacttctgag agccaataga gtgtgtgtga 1620
   gaaagacaga cgtgagctac tacttactgc tgtctctgct cagactctga gcccttctca 1680
   ccctgtgtct gcattgctca cacagccagg ggcttcagta gcagggcatg gggagctata 1740
   cagcctggag caggctctgt c                                          1761
<210> 92
   <211> 803
   <212> PRT
   <213> Mus musculus
<400> 92
   Met Ala Asn Val Ser Lys Lys Val Ser Trp Ser Gly Arg Asp Arg Asp
    1               5                  10                  15
   Asp Glu Glu Gly Ala Pro Leu Leu Arg Arg Thr Gly Gln Pro Asp Glu
               20                  25                  30
   Glu Thr Pro Leu Leu Asn Gly Ala Gly Pro Gly Ala Arg Gln Ser His
           35                  40                  45
   Ser Ala Leu Phe Arg Ile Gly Gln Met Asn Asn Val Glu Leu Asp Asp
       50                  55                  60
   Glu Leu Leu Asp Pro Glu Val Asp Pro Pro His Thr Phe Pro Lys Glu
   65                  70                  75                  80
   Ile Pro His Asn Glu Lys Leu Leu Ser Leu Lys Tyr Glu Ser Leu Asp
                   85                  90                  95
   Tyr Asp Asn Ser Glu Asn Gln Leu Phe Leu Glu Glu Glu Arg Arg Ile
               100                 105                 110
   Asn His Thr Ala Phe Arg Thr Val Glu Ile Lys Arg Trp Val Ile Cys
           115                 120                 125
   Ala Leu Ile Gly Ile Leu Thr Gly Leu Val Ala Cys Phe Ile Asp Ile
       130                 135                 140
   Val Val Glu Asn Leu Ala Gly Leu Lys Tyr Arg Val Ile Lys Asp Asn
   145 150 155 160
   Ile Asp Lys Phe Thr Glu Lys Gly Gly Leu Ser Phe Ser Leu Leu Leu
   165 170 175
   Trp Ala Thr Leu Asn Ser Ala Phe Val Leu Val Gly Ser Val Ile Val
   180 185 190
   Ala Phe Ile Glu Pro Val Ala Ala Gly Ser Gly Ile Pro Gln Ile Lys
   195 200 205
   Cys Phe Leu Asn Gly Val Lys Ile Pro His Val Val Arg Leu Lys Thr
   210 215 220
   Leu Val Ile Lys Val Ser Gly Val Ile Leu Ser Val Val Gly Gly Leu
   225 230 235 240
   Ala Val Gly Lys Glu Gly Pro Met Ile His Ser Gly Ser Val Ile Ala
   245 250 255
   Ala Gly Ile Ser Gln Gly Arg Ser Thr Ser Leu Lys Arg Asp Phe Lys
   260 265 270
   Ile Phe Glu Tyr Phe Arg Arg Asp Thr Glu Lys Arg Asp Phe Val Ser
   275 280 285
   Ala Gly Ala Ala Ala Gly Val Ser Ala Ala Phe Gly Ala Pro Val Gly
   290 295 300
   Gly Val Leu Phe Ser Leu Glu Glu Gly Ala Ser Phe Trp Asn Gln Phe
   305 310 315 320
   Leu Thr Trp Arg Ile Phe Phe Ala Ser Met Ile Ser Thr Phe Thr Leu
   325 330 335
   Asn Phe Val Leu Ser Ile Tyr His Gly Asn Met Trp Asp Leu Ser Ser
   340 345 350
   Pro Gly Leu Ile Asn Phe Gly Arg Phe Asp Ser Glu Lys Met Ala Tyr
   355 360 365
   Thr Ile His Glu Ile Pro Val Phe Ile Ala Met Gly Val Val Gly Gly
   370 375 380
   Ile Leu Gly Ala Val Phe Asn Ala Leu Asn Tyr Trp Leu Thr Met Phe
   385 390 395 400
   Arg Ile Arg Tyr Ile His Arg Pro Cys Leu Gln Val Ile Glu Ala Met
   405 410 415
   Leu Val Ala Ala Val Thr Ala Thr Val Ala Phe Val Leu Ile Tyr Ser
   420 425 430
   Ser Arg Asp Cys Gln Pro Leu Gln Gly Ser Ser Met Ser Tyr Pro Leu
   435 440 445
   Gln Leu Phe Cys Ala Asp Gly Glu Tyr Asn Ser Met Ala Ala Ala Phe
   450 455 460
   Phe Asn Thr Pro Glu Lys Ser Val Val Ser Leu Phe His Asp Pro Pro
   465 470 475 480
   Gly Ser Tyr Asn Pro Met Thr Leu Gly Leu Phe Thr Leu Val Tyr Phe
   485 490 495
   Phe Leu Ala Cys Trp Thr Tyr Gly Leu Thr Val Ser Ala Gly Val Phe
   500 505 510
   Ile Pro Ser Leu Leu Ile Gly Ala Ala Trp Gly Arg Leu Phe Gly Ile
   515 520 525
   Ser Leu Ser Tyr Leu Thr Gly Ala Ala Ile Trp Ala Asp Pro Gly Lys
   530 535 540
   Tyr Ala Leu Met Gly Ala Ala Ala Gln Leu Gly Gly Ile Val Arg Met
   545 550 555 560
   Thr Leu Ser Leu Thr Val Ile Met Met Glu Ala Thr Ser Asn Val Thr
   565 570 575
   Tyr Gly Phe Pro Ile Met Leu Val Leu Met Thr Ala Lys Ile Val Gly
   580 585 590
   Asp Val Phe Ile Glu Gly Leu Tyr Asp Met His Ile Gln Leu Gln Ser
   595 600 605
   Val Pro Phe Leu His Trp Glu Ala Pro Val Thr Ser His Ser Leu Thr
   610 615 620
   Ala Arg Glu Val Met Ser Thr Pro Val Thr Cys Leu Arg Arg Arg Glu
   625 630 635 640
   Lys Val Gly Ile Ile Val Asp Val Leu Ser Asp Thr Ala Ser Asn His
   645 650 655
   Asn Gly Phe Pro Val Val Glu Asp Val Gly Asp Thr Gln Pro Ala Arg
   660 665 670
   Leu Gln Gly Leu Ile Leu Arg Ser Gln Leu Ile Val Leu Leu Lys His
   675 680 685
   Lys Val Phe Val Glu Arg Ser Asn Met Gly Leu Val Gln Arg Arg Leu
   690 695 700
   Arg Leu Lys Asp Phe Arg Asp Ala Tyr Pro Arg Phe Pro Pro Ile Gln
   705 710 715 720
   Ser Ile His Val Ser Gln Asp Glu Arg Glu Cys Thr Met Asp Leu Ser
   725 730 735
   Glu Phe Met Asn Pro Ser Pro Tyr Thr Val Pro Gln Glu Ala Ser Leu
   740 745 750
   Pro Arg Val Phe Lys Leu Phe Arg Ala Leu Gly Leu Arg His Leu Val
   755 760 765
   Val Val Asp Asn His Asn Gln Val Val Gly Leu Val Thr Arg Lys Asp
   770 775 780
   Leu Ala Arg Tyr Arg Leu Gly Lys Gly Gly Leu Glu Glu Leu Ser Leu
   785 790 795 800
   Ala Gln Thr
<210> 93
   <211> 2495
   <212> DNA
   <213> Rattus norvegicus
<400> 93
   attgcctacc ccgggtggag accgtgctcg tccggccctc ttgcctcacg ttctgcagct 60
   ctgcagctcc gcaatcctac accatggcgg acagccggga cccagccagc gaccagatga 120
   agcagtggaa ggagcagcgg gcccctcaga aacccgatgt cctgaccacc ggaggcggga 180
   acccaatagg agataaactt aatatcatga ctgcggggcc ccgagggccc ctcctcgttc 240
   aagatgtggt tttcaccgac gagatggcac actttgacag agagcggatt cctgagagag 300
   tggtacatgc aaagggagca ggtgcttttg gatactttga ggtcacccac gatattacca 360
   gatactccaa ggcaaaggtg tttgagcata ttgggaagag gactcctatt gccgtccgat 420
   tctccacagt cgctggagag tcaggctcag ctgacacagt tcgtgaccct cgtgggtttg 480
   cagtgaaatt ctacactgaa gatggtaact gggacctcgt gggaaacaac acccctattt 540
   tcttcatcag ggatgccatg ttgtttccat cctttatcca tagccagaag agaaacccac 600
   aaactcacct gaaggaccct gacatggtct gggacttctg gagtctttgt ccagagtctc 660
   tccatcaggt tactttcttg ttcagcgacc gagggattcc agatggacat cggcacatga 720
   atggctatgg ctcacacacc ttcaagctgg ttaatgcgaa tggagaggca gtgtactgca 780
   agttccatta caagactgac cagggcatca aaaacttgcc tgttgaagag gcaggaagac 840
   ttgcacagga agacccggat tatggcctcc gagatctttt caatgccatc gccagtggca 900
   attacccatc ctggactttt tacatccagg tcatgacttt caaggaggca gaaaccttcc 960
   catttaatcc atttgacctg accaaggttt ggcctcacaa ggactaccct cttataccag 1020
   ttggcaaact ggtcttaaac agaaatcctg ctaattattt tgctgaagtt gaacagatgg 1080
   cttttgaccc aagcaacatg ccccctggca ttgagcccag cccggacaag atgctccagg 1140
   gccgcctttt tgcttaccca gacactcacc gccaccgcct gggaccaaac tatctgcaga 1200
   tacctgtgaa ctgtccctac cgtgctcgcg tggccaacta ccagcgcgat ggccccatgt 1260
   gcatgcatga caaccagggt ggtgctccca actactaccc caacagcttc agcgcaccag 1320
   agcagcaggg ctcggccctg gagcaccata gccagtgctc tgcagatgtg aagcgcttca 1380
   acagtgctaa tgaagacaac gtcactcagg tgcggacatt ctatacgaag gtgttgaatg 1440
   aggaggagag gaaacgcctg tgtgagaaca ttgccaacca cctgaaagat gctcagcttt 1500
   tcattcagag gaaagcggtc aagaatttca ctgacgtcca ccctgactac ggggcccgag 1560
   tccaggctct tctggaccag tacaactccc agaagcctaa gaatgcaatt cacacctacg 1620
   tacaggccgg ctctcacata gctgccaagg gaaaagctaa cctgtaaagc acgggtgctc 1680
   agcctcctca gcctgcactg aggagatccc tcatgaagca gggcacaagc ctcaccagta 1740
   atcatcgctg gatggagtct cccctgctga agcgcagact cacgctgacg tctttaaaac 1800
   gataatccaa gcttctagag tgaatgatag ccatgctttt gatgacattt cccgaggggg 1860
   aaattaaaga ttaggcttta gcaatcactt aacagaaaca tggatctgct taggacttct 1920
   gtttggatta ttcatttaaa atgattacaa gaaaggtttt ctagccagaa acatgatttg 1980
   attagatatg atatatgata aaatcttggt gattttacta tagtcttatg ttacctcaca 2040
   gcctggtata tatacaacac acacacacac acacacacac acacaccaaa acacacatac 2100
   actatacaca cacacacaca cacacactaa aacacacata cacaacacac acatacacta 2160
   cacacacaga acacacaaca caaacataca cacataggca cacacacaca cacacacaca 2220
   cacacacaca cacacacaca cacacatgaa tgaagggatt ataaagatgg cccacccaga 2280
   attttttttt atttttctaa ggtccttata agaaaaacca tacttggatc atgtcttcca 2340
   aaaataactt tagcactgtt gaaacttaat gtttattcct gtgtagttga ttggattcct 2400
   tttccccttg aaattatgtt tatgctgata cacagtgatt tcacataggg tgatttgtat 2460
   ttgcttacat ttttacaata aaatgatctt catgg 2495
<210> 94
   <211> 527
   <212> PRT
   <213> Rattus norvegicus
<400> 94
   Met Ala Asp Ser Arg Asp Pro Ala Ser Asp Gln Met Lys Gln Trp Lys
   1 5 10 15
   Glu Gln Arg Ala Pro Gln Lys Pro Asp Val Leu Thr Thr Gly Gly Gly
                   20                  25                  30
   Asn Pro Ile Gly Asp Lys Leu Asn Ile Met Thr Ala Gly Pro Arg Gly
               35                  40                  45
   Pro Leu Leu Val Gln Asp Val Val Phe Thr Asp Glu Met Ala His Phe
       50                  55                  60
   Asp Arg Glu Arg Ile Pro Glu Arg Val Val His Ala Lys Gly Ala Gly
   65                  70                  75                  80
   Ala Phe Gly Tyr Phe Glu Val Thr His Asp Ile Thr Arg Tyr Ser Lys
                   85                  90                  95
   Ala Lys Val Phe Glu His Ile Gly Lys Arg Thr Pro Ile Ala Val Arg
               100                 105                 110
   Phe Ser Thr Val Ala Gly Glu Ser Gly Ser Ala Asp Thr Val Arg Asp
           115                 120                 125
   Pro Arg Gly Phe Ala Val Lys Phe Tyr Thr Glu Asp Gly Asn Trp Asp
       130                 135                 140
   Leu Val Gly Asn Asn Thr Pro Ile Phe Phe Ile Arg Asp Ala Met Leu
   145                 150                 155                 160
   Phe Pro Ser Phe Ile His Ser Gln Lys Arg Asn Pro Gln Thr His Leu
                   165                 170                 175
   Lys Asp Pro Asp Met Val Trp Asp Phe Trp Ser Leu Cys Pro Glu Ser
               180                 185                 190
   Leu His Gln Val Thr Phe Leu Phe Ser Asp Arg Gly Ile Pro Asp Gly
           195                 200                 205
   His Arg His Met Asn Gly Tyr Gly Ser His Thr Phe Lys Leu Val Asn
       210                 215                 220
   Ala Asn Gly Glu Ala Val Tyr Cys Lys Phe His Tyr Lys Thr Asp Gln
   225                 230                 235                 240
   Gly Ile Lys Asn Leu Pro Val Glu Glu Ala Gly Arg Leu Ala Gln Glu
                   245                 250                 255
   Asp Pro Asp Tyr Gly Leu Arg Asp Leu Phe Asn Ala Ile Ala Ser Gly
               260                 265                 270
   Asn Tyr Pro Ser Trp Thr Phe Tyr Ile Gln Val Met Thr Phe Lys Glu
           275                 280                 285
   Ala Glu Thr Phe Pro Phe Asn Pro Phe Asp Leu Thr Lys Val Trp Pro
       290                 295                 300
   His Lys Asp Tyr Pro Leu Ile Pro Val Gly Lys Leu Val Leu Asn Arg
   305                 310                 315                 320
   Asn Pro Ala Asn Tyr Phe Ala Glu Val Glu Gln Met Ala Phe Asp Pro
                   325                 330                 335
   Ser Asn Met Pro Pro Gly Ile Glu Pro Ser Pro Asp Lys Met Leu Gln
               340                 345                 350
   Gly Arg Leu Phe Ala Tyr Pro Asp Thr His Arg His Arg Leu Gly Pro
   355 360 365
   Asn Tyr Leu Gln Ile Pro Val Asn Cys Pro Tyr Arg Ala Arg Val Ala
   370 375 380
   Asn Tyr Gln Arg Asp Gly Pro Met Cys Met His Asp Asn Gln Gly Gly
   385 390 395 400
   Ala Pro Asn Tyr Tyr Pro Asn Ser Phe Ser Ala Pro Glu Gln Gln Gly
   405 410 415
   Ser Ala Leu Glu His His Ser Gln Cys Ser Ala Asp Val Lys Arg Phe
   420 425 430
   Asn Ser Ala Asn Glu Asp Asn Val Thr Gln Val Arg Thr Phe Tyr Thr
   435 440 445
   Lys Val Leu Asn Glu Glu Glu Arg Lys Arg Leu Cys Glu Asn Ile Ala
   450 455 460
   Asn His Leu Lys Asp Ala Gln Leu Phe Ile Gln Arg Lys Ala Val Lys
   465 470 475 480
   Asn Phe Thr Asp Val His Pro Asp Tyr Gly Ala Arg Val Gln Ala Leu
   485 490 495
   Leu Asp Gln Tyr Asn Ser Gln Lys Pro Lys Asn Ala Ile His Thr Tyr
   500 505 510
   Val Gln Ala Gly Ser His Ile Ala Ala Lys Gly Lys Ala Asn Leu
   515 520 525
<210> 95
   <211> 2423
   <212> DNA
   <213> Mus musculus
<400> 95
   attgccttct ccgggtggag accagaccgc tgcgtccgtc cctgctgtct cacgttccgc 60
   agctctgcag ctccgcaatc ctacaccatg tcggacagtc gggacccagc cagcgaccag 120
   atgaagcagt ggaaggagca gcgggcctcg cagagacctg atgtcctgac caccggaggc 180
   gggaacccaa taggagataa acttaatatc atgaccgcgg ggtcccgagg gcccctcctc 240
   gttcaggatg tggttttcac tgacgagatg gcacactttg acagagagcg gattcctgag 300
   agagtggtac acgcaaaagg agcaggtgct tttggatact ttgaggtcac ccacgatatc 360
   accagatact ccaagggaaa ggtgtttgag catattggaa agaggacccc tattgccgtt 420
   cggttctcca cagtcgctgg agagtcaggc tcagctgaca cagttcgtga ccctcggggg 480
   tttgcagtga aattttacac tgaagatggt aactgggatc ttgtgggaaa caacacccct 540
   attttcttca tcagggatgc catattgttt ccatccttta tccatagcca gaagagaaac 600
   ccacagactc acctgaagga tcctgacatg gtctgggact tctggagtct tcgtcccgag 660
   tctctccatc aggtttcttt cttgttcagt gaccgaggga ttcccgatgg tcaccggcac 720
   atgaatggct atggatcaca caccttcaag ttggttaatg cagatggaga ggcagtctat 780
   tgcaagttcc attacaagac cgaccagggc atcaaaaact tgcctgttgg agaggcagga 840
   aggcttgctc aggaagatcc ggattatggc ctccgagatc ttttcaatgc catcgccaat 900
   ggcaattacc cgtcctggac gttttacatc caggtcatga cttttaagga ggcagaaact 960
   ttcccattta atccatttga tctgaccaag gtttggcctc acaaggacta ccctcttata 1020
   ccagttggca aagtggtttt aaacaaaaat ccagttaatt actttgctga agttgaacag 1080
   atggcttttg acccaagcaa tatgccccct ggcatcgagc ccagccctga caaaaagctt 1140
   cagggccgcc tttttgccta cccggacact caccgccacc gcctgggacc caactatctg 1200
   cagatacctg tgaactgtcc ctaccgcgct cgagtggcca actaccagcg tgatggcccc 1260
   atgtgcatgc atgacaacca gggtggtgcc cccaactatt accccaacag cttcagcgca 1320
   ccagagcagc agcgctcagc cctggagcac agcgtccagt gcgctgtaga tgtgaaacgc 1380
   ttcaacagtg ctaatgaaga caatgtcact caggtgcgga cattctacac aaaggtgttg 1440
   aatgaggagg agaggaaacg cctgtgtgag aacattgccg gccacctgaa ggacgctcag 1500
   cttttcattc agaagaaagc ggtcaagaat ttcactgacg tccaccctga ctatggggcc 1560
   cgcatccagg ctcttctgga caagtacaac gctgagaagc ctaagaacgc aattcacacc 1620
   tacacgcagg ccggctctca catggctgcg aagggaaaag ctaacctgta actccggtgc 1680
   tcagcctccg ctgaggagac ctctcgtgaa gccgagcctg aggatcacct gtaatcaacg 1740
   ctggatggat tctcccccgc cggagcgcag actcacgctg atgactttaa aacgataatc 1800
   cggcttctta gagtgaatga taaccatgct tttgatgccg tttcctgaag ggaaatgaaa 1860
   ggttagggct tagcaatcat ttaacagaaa catggatcta ataggacttc tgtttggatt 1920
   attcatttaa atgactacat ttaaaatgat tacaagaaag gtgttctagc cagaaacatg 1980
   acttgattag acaagataaa aatcttggcg agaatagtgt attctcctat tacctcatgg 2040
   tctggtatat atacaataca acacacatac cacacacaca cacacatgca atacacacac 2100
   tacacacaca tacacacact cacacacact catacacaca catgaagaga tgataaagat 2160
   ggcccactca gaattttttt tttatttttc taaggtcctt ataagcaaaa ccatacttgc 2220
   atcatgtctt ccaaaagtaa ctttagcact gttgaaactt aatgtttatt cctgtgctgt 2280
   gcggtgctgt gctgtgctgt gctgtgcagc taatcagatt cttgtttttt cccacttgga 2340
   ttatgttgat gctaatacgc agtgatttca cataggatga tttgtacttg cttacatttt 2400
   tacaataaaa tgatctacat gga                                         2423
<210> 96
   <211> 527
   <212> PRT
   <213> Mus musculus
<400> 96
   Met Ser Asp Ser Arg Asp Pro Ala Ser Asp Gln Met Lys Gln Trp Lys
   1 5 10 15
   Glu Gln Arg Ala Ser Gln Arg Pro Asp Val Leu Thr Thr Gly Gly Gly
   20 25 30
   Asn Pro Ile Gly Asp Lys Leu Asn Ile Met Thr Ala Gly Ser Arg Gly
   35 40 45
   Pro Leu Leu Val Gln Asp Val Val Phe Thr Asp Glu Met Ala His Phe
   50 55 60
   Asp Arg Glu Arg Ile Pro Glu Arg Val Val His Ala Lys Gly Ala Gly
   65 70 75 80
   Ala Phe Gly Tyr Phe Glu Val Thr His Asp Ile Thr Arg Tyr Ser Lys
   85 90 95
   Gly Lys Val Phe Glu His Ile Gly Lys Arg Thr Pro Ile Ala Val Arg
   100 105 110
   Phe Ser Thr Val Ala Gly Glu Ser Gly Ser Ala Asp Thr Val Arg Asp
   115 120 125
   Pro Arg Gly Phe Ala Val Lys Phe Tyr Thr Glu Asp Gly Asn Trp Asp
   130 135 140
   Leu Val Gly Asn Asn Thr Pro Ile Phe Phe Ile Arg Asp Ala Ile Leu
   145 150 155 160
   Phe Pro Ser Phe Ile His Ser Gln Lys Arg Asn Pro Gln Thr His Leu
   165 170 175
   Lys Asp Pro Asp Met Val Trp Asp Phe Trp Ser Leu Arg Pro Glu Ser
   180 185 190
   Leu His Gln Val Ser Phe Leu Phe Ser Asp Arg Gly Ile Pro Asp Gly
   195 200 205
   His Arg His Met Asn Gly Tyr Gly Ser His Thr Phe Lys Leu Val Asn
   210 215 220
   Ala Asp Gly Glu Ala Val Tyr Cys Lys Phe His Tyr Lys Thr Asp Gln
   225 230 235 240
   Gly Ile Lys Asn Leu Pro Val Gly Glu Ala Gly Arg Leu Ala Gln Glu
   245 250 255
   Asp Pro Asp Tyr Gly Leu Arg Asp Leu Phe Asn Ala Ile Ala Asn Gly
   260 265 270
   Asn Tyr Pro Ser Trp Thr Phe Tyr Ile Gln Val Met Thr Phe Lys Glu
   275 280 285
   Ala Glu Thr Phe Pro Phe Asn Pro Phe Asp Leu Thr Lys Val Trp Pro
   290 295 300
   His Lys Asp Tyr Pro Leu Ile Pro Val Gly Lys Val Val Leu Asn Lys
   305 310 315 320
   Asn Pro Val Asn Tyr Phe Ala Glu Val Glu Gln Met Ala Phe Asp Pro
   325 330 335
   Ser Asn Met Pro Pro Gly Ile Glu Pro Ser Pro Asp Lys Lys Leu Gln
   340 345 350
   Gly Arg Leu Phe Ala Tyr Pro Asp Thr His Arg His Arg Leu Gly Pro
   355 360 365
   Asn Tyr Leu Gln Ile Pro Val Asn Cys Pro Tyr Arg Ala Arg Val Ala
   370 375 380
   Asn Tyr Gln Arg Asp Gly Pro Met Cys Met His Asp Asn Gln Gly Gly
   385 390 395 400
   Ala Pro Asn Tyr Tyr Pro Asn Ser Phe Ser Ala Pro Glu Gln Gln Arg
   405 410 415
   Ser Ala Leu Glu His Ser Val Gln Cys Ala Val Asp Val Lys Arg Phe
   420 425 430
   Asn Ser Ala Asn Glu Asp Asn Val Thr Gln Val Arg Thr Phe Tyr Thr
   435 440 445
   Lys Val Leu Asn Glu Glu Glu Arg Lys Arg Leu Cys Glu Asn Ile Ala
   450 455 460
   Gly His Leu Lys Asp Ala Gln Leu Phe Ile Gln Lys Lys Ala Val Lys
   465 470 475 480
   Asn Phe Thr Asp Val His Pro Asp Tyr Gly Ala Arg Ile Gln Ala Leu
   485 490 495
   Leu Asp Lys Tyr Asn Ala Glu Lys Pro Lys Asn Ala Ile His Thr Tyr
   500 505 510
   Thr Gln Ala Gly Ser His Met Ala Ala Lys Gly Lys Ala Asn Leu
   515 520 525
<210> 97
   <211> 2279
   <212> DNA
   <213> Homo sapiens
<400> 97
   tgcctgctga gggtggagac ccacgagccg aggcctcctg cagtgttctg cacagcaaac 60
   cgcacgctat ggctgacagc cgggatcccg ccagcgacca gatgcagcac tggaaggagc 120
   agcgggccgc gcagaaagct gatgtcctga ccactggagc tggtaaccca gtaggagaca 180
   aacttaatgt tattacagta gggccccgtg ggccccttct tgttcaggat gtggttttca 240
   ctgatgaaat ggctcatttt gaccgagaga gaattcctga gagagttgtg catgctaaag 300
   gagcaggggc ctttggctac tttgaggtca cacatgacat taccaaatac tccaaggcaa 360
   aggtatttga gcatattgga aagaagactc ccatcgcagt tcggttctcc actgttgctg 420
   gagaatcggg ttcagctgac acagttcggg accctcgtgg gtttgcagtg aaattttaca 480
   cagaagatgg taactgggat ctcgttggaa ataacacccc cattttcttc atcagggatc 540
   ccatattgtt tccatctttt atccacagcc aaaagagaaa tcctcagaca catctgaagg 600
   atccggacat ggtctgggac ttctggagcc tacgtcctga gtctctgcat caggtttctt 660
   tcttgttcag tgatcggggg attccagatg gacatcgcca catgaatgga tatggatcac 720
   atactttcaa gctggttaat gcaaatgggg aggcagttta ttgcaaattc cattataaga 780
   ctgaccaggg catcaaaaac ctttctgttg aagatgcggc gagactttcc caggaagatc 840
   ctgactatgg catccgggat ctttttaacg ccattgccac aggaaagtac ccctcctgga 900
   ctttttacat ccaggtcatg acatttaatc aggcagaaac ttttccattt aatccattcg 960
   atctcaccaa ggtttggcct cacaaggact accctctcat cccagttggt aaactggtct 1020
   taaaccggaa tccagttaat tactttgctg aggttgaaca gatagccttc gacccaagca 1080
   acatgccacc tggcattgag gccagtcctg acaaaatgct tcagggccgc ctttttgcct 1140
   atcctgacac tcaccgccat cgcctgggac ccaattatct tcatatacct gtgaactgtc 1200
   cctaccgtgc tcgagtggcc aactaccagc gtgatggccc gatgtgcatg caggacaatc 1260
   agggtggtgc tccaaattac taccccaaca gctttggtgc tccggaacaa cagccttctg 1320
   ccctggagca cagcatccaa tattctggag aagtgcggag attcaacact gccaatgatg 1380
   ataacgttac tcaggtgcgg gcattctatg tgaacgtgct gaatgaggaa cagaggaaac 1440
   gtctgtgtga gaacattgcc ggccacctga aggatgcaca aattttcatc cagaagaaag 1500
   cggtcaagaa cttcactgag gtccaccctg actacgggag ccacatccag gctcttctgg 1560
   acaagtacaa tgctgagaag cctaagaatg cgattcacac ctttgtgcag tccggatctc 1620
   acttggcggc aagggagaag gcaaatctgt gaggccgggg ccctgcacct gtgcagcgaa 1680
   gcttagcgtt catccgtgta acccgctcat cactggatga agattctcct gtgctagatg 1740
   tgcaaatgca agctagtggc ttcaaaatag agaatcccac tttctatagc agattgtgta 1800
   acaattttaa tgctatttcc ccaggggaaa atgaaggtta ggatttaaca gtcatttaaa 1860
   aaaaaaattt gttttgacgg atgattggat tattcattta aaatgattag aaggcaagtt 1920
   tctagctaga aatatgattt tatttgacaa aatttgttga aattatgtat gtttacatat 1980
   cacctcatgg cctattatat taaaatatgg ctataaatat ataaaaagaa aagataaaga 2040
   tgatctactc agaaattttt atttttctaa ggttctcata ggaaaagtac atttaataca 2100
   gcagtgtcat cagaagataa cttgagcacc gtcatggctt aatgtttatt cctgataata 2160
   attgatcaaa ttcatttttt tcactggagt tacattaatg ttaattcagc actgatttca 2220
   caacagatca atttgtaatt gcttacattt ttacaataaa taatctgtac gtaagaaca  2279
<210> 98
   <211> 527
   <212> PRT
   <213> Homo sapiens
<400> 98
   Met Ala Asp Ser Arg Asp Pro Ala Ser Asp Gln Met Gln His Trp Lys
    1               5                  10                  15
   Glu Gln Arg Ala Ala Gln Lys Ala Asp Val Leu Thr Thr Gly Ala Gly
               20                  25                  30
   Asn Pro Val Gly Asp Lys Leu Asn Val Ile Thr Val Gly Pro Arg Gly
           35                  40                  45
   Pro Leu Leu Val Gln Asp Val Val Phe Thr Asp Glu Met Ala His Phe
   50 55 60
   Asp Arg Glu Arg Ile Pro Glu Arg Val Val His Ala Lys Gly Ala Gly
   65 70 75 80
   Ala Phe Gly Tyr Phe Glu Val Thr His Asp Ile Thr Lys Tyr Ser Lys
   85 90 95
   Ala Lys Val Phe Glu His Ile Gly Lys Lys Thr Pro Ile Ala Val Arg
   100 105 110
   Phe Ser Thr Val Ala Gly Glu Ser Gly Ser Ala Asp Thr Val Arg Asp
   115 120 125
   Pro Arg Gly Phe Ala Val Lys Phe Tyr Thr Glu Asp Gly Asn Trp Asp
   130 135 140
   Leu Val Gly Asn Asn Thr Pro Ile Phe Phe Ile Arg Asp Pro Ile Leu
   145 150 155 160
   Phe Pro Ser Phe Ile His Ser Gln Lys Arg Asn Pro Gln Thr His Leu
   165 170 175
   Lys Asp Pro Asp Met Val Trp Asp Phe Trp Ser Leu Arg Pro Glu Ser
   180 185 190
   Leu His Gln Val Ser Phe Leu Phe Ser Asp Arg Gly Ile Pro Asp Gly
   195 200 205
   His Arg His Met Asn Gly Tyr Gly Ser His Thr Phe Lys Leu Val Asn
   210 215 220
   Ala Asn Gly Glu Ala Val Tyr Cys Lys Phe His Tyr Lys Thr Asp Gln
   225 230 235 240
   Gly Ile Lys Asn Leu Ser Val Glu Asp Ala Ala Arg Leu Ser Gln Glu
   245 250 255
   Asp Pro Asp Tyr Gly Ile Arg Asp Leu Phe Asn Ala Ile Ala Thr Gly
   260 265 270
   Lys Tyr Pro Ser Trp Thr Phe Tyr Ile Gln Val Met Thr Phe Asn Gln
   275 280 285
   Ala Glu Thr Phe Pro Phe Asn Pro Phe Asp Leu Thr Lys Val Trp Pro
   290 295 300
   His Lys Asp Tyr Pro Leu Ile Pro Val Gly Lys Leu Val Leu Asn Arg
   305 310 315 320
   Asn Pro Val Asn Tyr Phe Ala Glu Val Glu Gln Ile Ala Phe Asp Pro
   325 330 335
   Ser Asn Met Pro Pro Gly Ile Glu Ala Ser Pro Asp Lys Met Leu Gln
   340 345 350
   Gly Arg Leu Phe Ala Tyr Pro Asp Thr His Arg His Arg Leu Gly Pro
   355 360 365
   Asn Tyr Leu His Ile Pro Val Asn Cys Pro Tyr Arg Ala Arg Val Ala
   370 375 380
   Asn Tyr Gln Arg Asp Gly Pro Met Cys Met Gln Asp Asn Gln Gly Gly
   385                 390                     395                    400
   Ala Pro Asn Tyr Tyr Pro Asn Ser Phe Gly Ala Pro Glu Gln Gln Pro
                   405                     410                     415
   Ser Ala Leu Glu His Ser Ile Gln Tyr Ser Gly Glu Val Arg Arg Phe
               420                     425                     430
   Asn Thr Ala Asn Asp Asp Asn Val Thr Gln Val Arg Ala Phe Tyr Val
           435                     440                     445
   Asn Val Leu Asn Glu Glu Gln Arg Lys Arg Leu Cys Glu Asn Ile Ala
       450                     455                     460
   Gly His Leu Lys Asp Ala Gln Ile Phe Ile Gln Lys Lys Ala Val Lys
   465                 470                     475                     480
   Asn Phe Thr Glu Val His Pro Asp Tyr Gly Ser His Ile Gln Ala Leu
                   485                     490                     495
   Leu Asp Lys Tyr Asn Ala Glu Lys Pro Lys Asn Ala Ile His Thr Phe
               500                     505                     510
   Val Gln Ser Gly Ser His Leu Ala Ala Arg Glu Lys Ala Asn Leu
           515                     520                     525
<210> 99
   <211> 978
   <212> DNA
   <213> Rattus norvegicus
<400> 99
   atggcgagca gcagcggctc caaggccgaa tttatcgtcg gtgggaaata caaactggtg 60
   cggaagatcg gatctggttc cttcggggac atttatctag cgatcaacat caccaatggc 120
   gaggaagtgg cagtgaaact agaatcccag aaggccaggc atccccagtt gctgtacgag 180
   agcaaactgt ataagattct tcaaggtggg gttggcatcc ctcacatacg gtggtatggg 240
   caaggaaaag actataatgt gctagtcatg gatcttctgg gacccagcct cgaagacctc 300
   ttcaatttct gttcaagaag gttcactatg aagactgtac ttatgttagc tgaccagatg 360
   atcagtagaa ttgaatatgt gcatacaaag aattttatac acagagacat taaaccagat 420
   aacttcctaa tgggtattgg gcgtcactgt aataagttat tccttattga ttttggtttg 480
   gccaaaaagt acagagacaa caggacaagg caacacatac catacaggga agataaaaac 540
   ctcactggca ctgcccggta tgccagcatc aatgcacatc ttggcattga gcagagtcgc 600
   cgagatgaca tggaatcttt aggatatgtt ttgatgtatt ttaatagaac cagtctgcca 660
   tggcaagggc taaaggctgc aacaaagaaa caaaaatatg aaaagattag tgaaaagaag 720
   atgtccactc ctgttgaagt tttgtgtaag gggtttcctg cagaatttgc catgtactta 780
   aattactgtc gtgggctgcg ctttgaggaa gctccggatt acatgtatct gaggcagctg 840
   ttccgcatcc tcttcaggac cctgaaccac cagtatgact acacgtttga ttggacgatg 900
   ttaaagcaga aagcagccca gcaggcagcc tcttccagtg ggcagggtca gcaggcccaa 960
   acccccacag gtttctaa                                               978
<210> 100
   <211> 325
   <212> PRT
   <213> Rattus norvegicus
<400> 100
   Met Ala Ser Ser Ser Gly Ser Lys Ala Glu Phe Ile Val Gly Gly Lys
   1               5                   10                  15
   Tyr Lys Leu Val Arg Lys Ile Gly Ser Gly Ser Phe Gly Asp Ile Tyr
                   20                  25                  30
   Leu Ala Ile Asn Ile Thr Asn Gly Glu Glu Val Ala Val Lys Leu Glu
               35                  40                  45
   Ser Gln Lys Ala Arg His Pro Gln Leu Leu Tyr Glu Ser Lys Leu Tyr
           50                  55                  60
   Lys Ile Leu Gln Gly Gly Val Gly Ile Pro His Ile Arg Trp Tyr Gly
    65                  70                  75                  80
   Gln Gly Lys Asp Tyr Asn Val Leu Val Met Asp Leu Leu Gly Pro Ser
                    85                  90                  95
   Leu Glu Asp Leu Phe Asn Phe Cys Ser Arg Arg Phe Thr Met Lys Thr
               100                 105                 110
   Val Leu Met Leu Ala Asp Gln Met Ile Ser Arg Ile Glu Tyr Val His
           115                 120                 125
   Thr Lys Asn Phe Ile His Arg Asp Ile Lys Pro Asp Asn Phe Leu Met
       130                 135                 140
   Gly Ile Gly Arg His Cys Asn Lys Leu Phe Leu Ile Asp Phe Gly Leu
   145                 150                 155                 160
   Ala Lys Lys Tyr Arg Asp Asn Arg Thr Arg Gln His Ile Pro Tyr Arg
                   165                 170                 175
   Glu Asp Lys Asn Leu Thr Gly Thr Ala Arg Tyr Ala Ser Ile Asn Ala
               180                 185                 190
   His Leu Gly Ile Glu Gln Ser Arg Arg Asp Asp Met Glu Ser Leu Gly
           195                 200                 205
   Tyr Val Leu Met Tyr Phe Asn Arg Thr Ser Leu Pro Trp Gln Gly Leu
       210                 215                 220
   Lys Ala Ala Thr Lys Lys Gln Lys Tyr Glu Lys Ile Ser Glu Lys Lys
   225                 230                 235                 240
   Met Ser Thr Pro Val Glu Val Leu Cys Lys Gly Phe Pro Ala Glu Phe
                   245                 250                 255
   Ala Met Tyr Leu Asn Tyr Cys Arg Gly Leu Arg Phe Glu Glu Ala Pro
               260                 265                 270
   Asp Tyr Met Tyr Leu Arg Gln Leu Phe Arg Ile Leu Phe Arg Thr Leu
           275                 280                 285
   Asn His Gln Tyr Asp Tyr Thr Phe Asp Trp Thr Met Leu Lys Gln Lys
       290                 295                 300
   Ala Ala Gln Gln Ala Ala Ser Ser Ser Gly Gln Gly Gln Gln Ala Gln
   305                 310                 315                 320
   Thr Pro Thr Gly Phe
                   325
<210> 101
   <211> 1259
   <212> DNA
   <213> Homo sapiens
<400> 101
   ccgcctccgt gttccgtttc ctgccgccct cctctcgtag ccttgcctag tgtggagccc 60
   caggcctccg tcctcttccc agaggtgtcg aggcttggcc ccagcctcca tcttcgtctc 120
   tcaggatggc gagtagcagc ggctccaagg ctgaattcat tgtcggtggg aaatataaac 180
   tggtacggaa gatcgggtct ggctccttcg gggacatcta tttggcgatc aacatcacca 240
   acggcgagga agtggcactg aagctagaat ctcagaaggc caggcatccc cagttgctgt 300
   acgagagcaa gctctataag attcttcaag gtggggttgg catcccccac atacggtggt 360
   atggtcagga aaaagactac aatgtactag tcatggatct tctgggacct agcctcgaag 420
   acctcttcaa tttctgttca agaaggttca caatgaaaac tgtacttatg ttagctgacc 480
   agatgatcag tagaattgaa tatgtgcata caaagaattt tatacacaga gacattaaac 540
   cagataactt cctaatgggt attgggcgtc actgtaataa gttattcctt attgattttg 600
   gtttggccaa aaagtacaga gacaacagga caaggcaaca cataccatac agagaagata 660
   aaaacctcac tggcactgcc cgatatgcta gcatcaatgc acatcttggt attgagcaga 720
   gtcgccgaga tgacatggaa tcattaggat atgttttgat gtattttaat agaaccagcc 780
   tgccatggca agggctaaag gctgcaacaa agaaacaaaa atatgaaaag attagtgaaa 840
   agaagatgtc cacgcctgtt gaagttttat gtaaggggtt tcctgcagaa tttgcgatgt 900
   acttaaacta ttgtcgtggg ctacgctttg aggaagcccc agattacatg tatctgaggc 960
   agctattccg cattcttttc aggaccctga accatcaata tgactacaca tttgattgga 1020
   caatgttaaa gcagaaagca gcacagcagg cagcctcttc aagtgggcag ggtcagcagg 1080
   cccaaacccc cacaggcaag caaactgaca aatccaagag taacatgaaa ggtttctaat 1140
   ttctaagcat gaattgagga acagaagaag cagacgagat gatcggagca gcatttgttt 1200
   ctccccaaat ctagaaattt tagttcatat gtacactagc cagtggttgt ggacaacca  1259
<210> 102
   <211> 337
   <212> PRT
   <213> Homo sapiens
<400> 102
   Met Ala Ser Ser Ser Gly Ser Lys Ala Glu Phe Ile Val Gly Gly Lys
    1               5                  10                  15
   Tyr Lys Leu Val Arg Lys Ile Gly Ser Gly Ser Phe Gly Asp Ile Tyr
               20                  25                  30
   Leu Ala Ile Asn Ile Thr Asn Gly Glu Glu Val Ala Leu Lys Leu Glu
           35                  40                  45
   Ser Gln Lys Ala Arg His Pro Gln Leu Leu Tyr Glu Ser Lys Leu Tyr
       50                  55                  60
   Lys Ile Leu Gln Gly Gly Val Gly Ile Pro His Ile Arg Trp Tyr Gly
   65                  70                  75                  80
   Gln Glu Lys Asp Tyr Asn Val Leu Val Met Asp Leu Leu Gly Pro Ser
                   85                  90                  95
   Leu Glu Asp Leu Phe Asn Phe Cys Ser Arg Arg Phe Thr Met Lys Thr
               100                 105                 110
   Val Leu Met Leu Ala Asp Gln Met Ile Ser Arg Ile Glu Tyr Val His
           115                 120                 125
   Thr Lys Asn Phe Ile His Arg Asp Ile Lys Pro Asp Asn Phe Leu Met
       130                 135                 140
   Gly Ile Gly Arg His Cys Asn Lys Leu Phe Leu Ile Asp Phe Gly Leu
   145                 150                 155                 160
   Ala Lys Lys Tyr Arg Asp Asn Arg Thr Arg Gln His Ile Pro Tyr Arg
                   165                 170                 175
   Glu Asp Lys Asn Leu Thr Gly Thr Ala Arg Tyr Ala Ser Ile Asn Ala
               180                 185                 190
   His Leu Gly Ile Glu Gln Ser Arg Arg Asp Asp Met Glu Ser Leu Gly
           195                 200                 205
   Tyr Val Leu Met Tyr Phe Asn Arg Thr Ser Leu Pro Trp Gln Gly Leu
       210                 215                 220
   Lys Ala Ala Thr Lys Lys Gln Lys Tyr Glu Lys Ile Ser Glu Lys Lys
   225                 230                 235                 240
   Met Ser Thr Pro Val Glu Val Leu Cys Lys Gly Phe Pro Ala Glu Phe
                   245                 250                 255
   Ala Met Tyr Leu Asn Tyr Cys Arg Gly Leu Arg Phe Glu Glu Ala Pro
               260                 265                 270
   Asp Tyr Met Tyr Leu Arg Gln Leu Phe Arg Ile Leu Phe Arg Thr Leu
           275                 280                 285
   Asn His Gln Tyr Asp Tyr Thr Phe Asp Trp Thr Met Leu Lys Gln Lys
       290                 295                 300
   Ala Ala Gln Gln Ala Ala Ser Ser Ser Gly Gln Gly Gln Gln Ala Gln
   305                 310                 315                 320
   Thr Pro Thr Gly Lys Gln Thr Asp Lys Ser Lys Ser Asn Met Lys Gly
                   325                 330                 335
   Phe
<210> 103
   <211> 1058
   <212> DNA
   <213> Mus musculus
<400> 103
   tggcccyysr rgscaagnan tcggcacgag gcggcacgag ggccgatcgc ccggtgcata 60
   ngcgcagggt ctccgggtag tcatggcgtc cccatctcgg aggctgcaga ctaagccggt 120
   cattacttgt ttgaaaagcg ttctcttgat ctacactttc atcttttgga tcactggtgt 180
   tatccttctt gccgttggca tttggggcaa ggtgagcctg gaaaattatt tttccctctt 240
   aaatgagaag gccaccaatg tgccttttgt gctcattggc acaggcactg tcattattct 300
   cttgggcacc tttggttgtt tcgctacctg tcgaacctcc gcctggatgc tgaaactgta 360
   cgcgatgttt ctgacactca tttttttggt cgaactagtt gccgccattg ttggatttgt 420
   tttcagacat gagattaaga acagctttaa aagtaactat gaaaacgctc tgaaggagta 480
   caactccaca ggagactata gaagtgaagc tgtagacaag atccaaagta cgttgcattg 540
   ttgcggtgtc accaattacg gagattggaa aggtacgaac tattactcag aaacaggatt 600
   tcccaagagc tgctgtaaac tggagggctg ttatccacag agagatgcag ataaagtcaa 660
   cgaagarggt tgtttcataa aggtaatgac aactatagag tcagaaatgg gagtcgttgc 720
   tggaatttct tttggagttg cttgctttca gctaattgga atctttcttg cctactgcct 780
   gtctcgtgcc ataacgaata accagtatga gatagtataa tcactacaca cgggctcata 840
   tctcctcagt tttaaggtta cctttgtgag ttataacagc gcctggatgg agactgacat 900
   ctgcttagcc tgaaaagcta caccagtagg ctgattcaat caagatactt gtgtattgta 960
   ttctgtgtcc actttctgtc ctattcttac acccctgtat ctctgaaaca ctggaagtgc 1020
   tagtaaattg taaatgaaat aaaaaaaaaa aaaaaana 1058
<210> 104
   <211> 245
   <212> PRT
   <213> Mus musculus
<400> 104
   Met Ala Ser Pro Ser Arg Arg Leu Gln Thr Lys Pro Val Ile Thr Cys
   1 5 10 15
   Leu Lys Ser Val Leu Leu Ile Tyr Thr Phe Ile Phe Trp Ile Thr Gly
   20 25 30
   Val Ile Leu Leu Ala Val Gly Ile Trp Gly Lys Val Ser Leu Glu Asn
   35 40 45
   Tyr Phe Ser Leu Leu Asn Glu Lys Ala Thr Asn Val Pro Phe Val Leu
   50 55 60
   Ile Gly Thr Gly Thr Val Ile Ile Leu Leu Gly Thr Phe Gly Cys Phe
   65 70 75 80
   Ala Thr Cys Arg Thr Ser Ala Trp Met Leu Lys Leu Tyr Ala Met Phe
   85 90 95
   Leu Thr Leu Ile Phe Leu Val Glu Leu Val Ala Ala Ile Val Gly Phe
   100 105 110
   Val Phe Arg His Glu Ile Lys Asn Ser Phe Lys Ser Asn Tyr Glu Asn
   115 120 125
   Ala Leu Lys Glu Tyr Asn Ser Thr Gly Asp Tyr Arg Ser Glu Ala Val
   130 135 140
   Asp Lys Ile Gln Ser Thr Leu His Cys Cys Gly Val Thr Asn Tyr Gly
   145 150 155 160
   Asp Trp Lys Gly Thr Asn Tyr Tyr Ser Glu Thr Gly Phe Pro Lys Ser
   165 170 175
   Cys Cys Lys Leu Glu Gly Cys Tyr Pro Gln Arg Asp Ala Asp Lys Val
   180 185 190
   Asn Glu Glu Gly Cys Phe Ile Lys Val Met Thr Thr Ile Glu Ser Glu
   195 200 205
   Met Gly Val Val Ala Gly Ile Ser Phe Gly Val Ala Cys Phe Gln Leu
   210 215 220
   Ile Gly Ile Phe Leu Ala Tyr Cys Leu Ser Arg Ala Ile Thr Asn Asn
   225 230 235 240
   Gln Tyr Glu Ile Val
   245
<210> 105
   <211> 1096
   <212> DNA
   <213> Homo sapiens
<400> 105
   ggcacgagca gggtctcggg ctagtcatgg cgtccccgtc tcggagactg cagactaaac 60
   cagtcattac ttgtttcaag agcgttctgc taatctacac ttttattttc tggatcactg 120
   gcgttatcct tcttgcagtt ggcatttggg gcaaggtgag cctggagaat tacttttctc 180
   ttttaaatga gaaggccacc aatgtcccct tcgtgctcat tgctactggt accgtcatta 240
   ttcttttggg cacctttggt tgttttgcta cctgccgagc ttctgcatgg atgctaaaac 300
   tgtatgcaat gtttctgact ctcgtttttt tggtcgaact ggtcgctgcc atcgtaggat 360
   ttgttttcag acatgagatt aagaacagct ttaagaataa ttatgagaag gctttgaagc 420
   agtataactc tacaggagat tatagaagcc atgcagtaga caagatccaa aatacgttgc 480
   attgttgtgg tgtcaccgat tatagagatt ggacagatac taattattac tcagaaaaag 540
   gatttcctaa gagttgctgt aaacttgaag attgtactcc acagagagat gcagacaaag 600
   taaacaatga aggttgtttt ataaaggtga tgaccattat agagtcagaa atgggagtcg 660
   ttgcaggaat ttcctttgga gttgcttgct tccaactgat tggaatcttt ctcgcctact 720
   gcctctctcg tgccataaca aataaccagt atgagatagt gtaacccaat gtatctgtgg 780
   gcctattcct ctctaccttt aaggacattt agggtccccc ctgtgaatta gaaagttgct 840
   tggctggaga actgacaaca ctacttactg atagaccaaa aaactacacc agtaggttga 900
   ttcaatcaag atgtatgtag acctaaaact acaccaatag gctgattcaa tcaagatccg 960
   tgctcgcagt gggctgattc aatcaagatg tatgtttgct atgttctaag tccaccttct 1020
   atcccattca tgttagatcg ttgaaaccct gtatccctct gaaacactgg aagagctagt 1080
   aaattgtaaa tgaagt 1096
<210> 106
   <211> 245
   <212> PRT
   <213> Homo sapiens
<400> 106
   Met Ala Ser Pro Ser Arg Arg Leu Gln Thr Lys Pro Val Ile Thr Cys
   1               5                   10                  15
   Phe Lys Ser Val Leu Leu Ile Tyr Thr Phe Ile Phe Trp Ile Thr Gly
               20                  25                  30
   Val Ile Leu Leu Ala Val Gly Ile Trp Gly Lys Val Ser Leu Glu Asn
           35                  40                  45
   Tyr Phe Ser Leu Leu Asn Glu Lys Ala Thr Asn Val Pro Phe Val Leu
       50                  55                  60
   Ile Ala Thr Gly Thr Val Ile Ile Leu Leu Gly Thr Phe Gly Cys Phe
   65                  70                  75                  80
   Ala Thr Cys Arg Ala Ser Ala Trp Met Leu Lys Leu Tyr Ala Met Phe
                   85                  90                  95
   Leu Thr Leu Val Phe Leu Val Glu Leu Val Ala Ala Ile Val Gly Phe
               100                 105                 110
   Val Phe Arg His Glu Ile Lys Asn Ser Phe Lys Asn Asn Tyr Glu Lys
           115                 120                 125
   Ala Leu Lys Gln Tyr Asn Ser Thr Gly Asp Tyr Arg Ser His Ala Val
       130                 135                 140
   Asp Lys Ile Gln Asn Thr Leu His Cys Cys Gly Val Thr Asp Tyr Arg
   145                 150                 155                 160
   Asp Trp Thr Asp Thr Asn Tyr Tyr Ser Glu Lys Gly Phe Pro Lys Ser
                   165                 170                 175
   Cys Cys Lys Leu Glu Asp Cys Thr Pro Gln Arg Asp Ala Asp Lys Val
               180                 185                 190
   Asn Asn Glu Gly Cys Phe Ile Lys Val Met Thr Ile Ile Glu Ser Glu
           195                 200                 205
   Met Gly Val Val Ala Gly Ile Ser Phe Gly Val Ala Cys Phe Gln Leu
       210                 215                 220
   Ile Gly Ile Phe Leu Ala Tyr Cys Leu Ser Arg Ala Ile Thr Asn Asn
   225                 230                 235                 240
   Gln Tyr Glu Ile Val
                   245
<210> 107
   <211> 2769
   <212> DNA
   <213> Homo sapiens
<400> 107
   ggacacggct gtggccgctg cctctacccc cgccacggat cgccgggtag taggactgcg 60
   cggctccagg ctgagggtcg gtccggaggc gggtgggcgc gggtctcacc cggattgtcc 120
   gggtggcacc gttcccggcc ccaccgggcg ccgcgaggga tcatgtctac agcctctgcc 180
   gcctcctcct cctcctcgtc ttcggccggt gagatgatcg aagccccttc ccaggtcctc 240
   aactttgaag agatcgacta caaggagatc gaggtggaag aggttgttgg aagaggagcc 300
   tttggagttg tttgcaaagc taagtggaga gcaaaagatg ttgctattaa acaaatagaa 360
   agtgaatctg agaggaaagc gtttattgta gagcttcggc agttatcccg tgtgaaccat 420
   cctaatattg taaagcttta tggagcctgc ttgaatccag tgtgtcttgt gatggaatat 480
   gctgaagggg gctctttata taatgtgctg catggtgctg aaccattgcc atattatact 540
   gctgcccacg caatgagttg gtgtttacag tgttcccaag gagtggctta tcttcacagc 600
   atgcaaccca aagcgctaat tcacagggac ctgaaaccac caaacttact gctggttgca 660
   ggggggacag ttctaaaaat ttgtgatttt ggtacagcct gtgacattca gacacacatg 720
   accaataaca aggggagtgc tgcttggatg gcacctgaag tttttgaagg tagtaattac 780
   agtgaaaaat gtgacgtctt cagctggggt attattcttt gggaagtgat aacgcgtcgg 840
   aaaccctttg atgagattgg tggcccagct ttccgaatca tgtgggctgt tcataatggt 900
   actcgaccac cactgataaa aaatttacct aagcccattg agagcctgat gactcgttgt 960
   tggtctaaag atccttccca gcgcccttca atggaggaaa ttgtgaaaat aatgactcac 1020
   ttgatgcggt actttccagg agcagatgag ccattacagt atccttgtca gtattcagat 1080
   gaaggacaga gcaactctgc caccagtaca ggctcattca tggacattgc ttctacaaat 1140
   acgagtaaca aaagtgacac taatatggag caagttcctg ccacaaatga tactattaag 1200
   cgcttagaat caaaattgtt gaaaaatcag gcaaagcaac agagtgaatc tggacgttta 1260
   agcttgggag cctcccgtgg gagcagtgtg gagagcttgc ccccaacctc tgagggcaag 1320
   aggatgagtg ctgacatgtc tgaaatagaa gctaggatcg ccgcaaccac aggcaacgga 1380
   cagccaagac gtagatccat ccaagacttg actgtaactg gaacagaacc tggtcaggtg 1440
   agcagtaggt catccagtcc cagtgtcaga atgattacta cctcaggacc aacctcagaa 1500
   aagccaactc gaagtcatcc atggacccct gatgattcca cagataccaa tggatcagat 1560
   aactccatcc caatggctta tcttacactg gatcaccaac tacagcctct agcaccgtgc 1620
   ccaaactcca aagaatctat ggcagtgttt gaacagcatt gtaaaatggc acaagaatat 1680
   atgaaagttc aaacagaaat tgcattgtta ttacagagaa agcaagaact agttgcagaa 1740
   ctggaccagg atgaaaagga ccagcaaaat acatctcgcc tggtacagga acataaaaag 1800
   cttttagatg aaaacaaaag cctttctact tactaccagc aatgcaaaaa acaactagag 1860
   gtcatcagaa gtcagcagca gaaacgacaa ggcacttcat gattctctgg gaccgttaca 1920
   ttttgaaata tgcaaagaaa gacttttttt ttaaggaaag gaaaacctta taatgacgat 1980
   tcatgagtgt tagctttttg gcgtgttctg aatgccaact gcctatattt gctgcatttt 2040
   tttcattgtt tattttcctt ttctcatggt ggacatacaa ttttactgtt tcattgcata 2100
   acatggtagc atctgtgact tgaatgagca gcactttgca acttcaaaac agatgcagtg 2160
   aactgtggct gtatatgcat gctcattgtg tgaaggctag cctaacagaa caggaggtat 2220
   caaactagct gctatgtgca aacagcgtcc attttttcat attagaggtg gaacctcaag 2280
   aatgacttta ttcttgtatc tcatctcaaa atattaataa tttttttccc aaaagatggt 2340
   atataccaag ttaaagacag ggtattataa atttagagtg attggtggta tattacggaa 2400
   atacggaacc tttagggata gttccgtgta agggctttga tgccagcatc cttggatcag 2460
   tactgaactc agttccatcc gtaaaatatg taaaggtaag tggcagctgc tctatttaat 2520
   gaaagcagtt ttaccggatt ttgttagact aaaatttgat tgtgatacat tgaacaaaat 2580
   ggaactcatt tttttttaag gagtaaagat ttttaattct gtgattgtgt gtatgtgtgt 2640
   tgaaactgta aagcttttat gactctaata ttaatctctt aaatgaaatt aaaaggcaaa 2700
   agaacatgat tgagcttaaa tgatcatttc ttcctgcagt gattcttgga ttgttttctc 2760
   atgtatttg                                                         2769
<210> 108
   <211> 579
   <212> PRT
   <213> Homo sapiens
<400> 108
   Met Ser Thr Ala Ser Ala Ala Ser Ser Ser Ser Ser Ser Ser Ala Gly
   1               5                   10                  15
   Glu Met Ile Glu Ala Pro Ser Gln Val Leu Asn Phe Glu Glu Ile Asp
               20                  25                  30
   Tyr Lys Glu Ile Glu Val Glu Glu Val Val Gly Arg Gly Ala Phe Gly
           35                  40                  45
   Val Val Cys Lys Ala Lys Trp Arg Ala Lys Asp Val Ala Ile Lys Gln
       50                  55                  60
   Ile Glu Ser Glu Ser Glu Arg Lys Ala Phe Ile Val Glu Leu Arg Gln
   65                  70                  75                  80
   Leu Ser Arg Val Asn His Pro Asn Ile Val Lys Leu Tyr Gly Ala Cys
                   85                  90                  95
   Leu Asn Pro Val Cys Leu Val Met Glu Tyr Ala Glu Gly Gly Ser Leu
               100                 105                 110
   Tyr Asn Val Leu His Gly Ala Glu Pro Leu Pro Tyr Tyr Thr Ala Ala
           115                 120                 125
   His Ala Met Ser Trp Cys Leu Gln Cys Ser Gln Gly Val Ala Tyr Leu
       130                 135                 140
   His Ser Met Gln Pro Lys Ala Leu Ile His Arg Asp Leu Lys Pro Pro
   145                 150                 155                 160
   Asn Leu Leu Leu Val Ala Gly Gly Thr Val Leu Lys Ile Cys Asp Phe
                   165                 170                 175
   Gly Thr Ala Cys Asp Ile Gln Thr His Met Thr Asn Asn Lys Gly Ser
               180                 185                 190
   Ala Ala Trp Met Ala Pro Glu Val Phe Glu Gly Ser Asn Tyr Ser Glu
           195                 200                 205
   Lys Cys Asp Val Phe Ser Trp Gly Ile Ile Leu Trp Glu Val Ile Thr
       210                 215                 220
   Arg Arg Lys Pro Phe Asp Glu Ile Gly Gly Pro Ala Phe Arg Ile Met
   225                 230                 235                 240
   Trp Ala Val His Asn Gly Thr Arg Pro Pro Leu Ile Lys Asn Leu Pro
                   245                 250                 255
   Lys Pro Ile Glu Ser Leu Met Thr Arg Cys Trp Ser Lys Asp Pro Ser
               260                 265                 270
   Gln Arg Pro Ser Met Glu Glu Ile Val Lys Ile Met Thr His Leu Met
           275                 280                 285
   Arg Tyr Phe Pro Gly Ala Asp Glu Pro Leu Gln Tyr Pro Cys Gln Tyr
       290                 295                 300
   Ser Asp Glu Gly Gln Ser Asn Ser Ala Thr Ser Thr Gly Ser Phe Met
   305                 310                 315                 320
   Asp Ile Ala Ser Thr Asn Thr Ser Asn Lys Ser Asp Thr Asn Met Glu
                   325                 330                 335
   Gln Val Pro Ala Thr Asn Asp Thr Ile Lys Arg Leu Glu Ser Lys Leu
               340                 345                 350
   Leu Lys Asn Gln Ala Lys Gln Gln Ser Glu Ser Gly Arg Leu Ser Leu
           355                 360                 365
   Gly Ala Ser Arg Gly Ser Ser Val Glu Ser Leu Pro Pro Thr Ser Glu
       370                 375                 380
   Gly Lys Arg Met Ser Ala Asp Met Ser Glu Ile Glu Ala Arg Ile Ala
   385                 390                 395                 400
   Ala Thr Thr Gly Asn Gly Gln Pro Arg Arg Arg Ser Ile Gln Asp Leu
                   405                 410                 415
   Thr Val Thr Gly Thr Glu Pro Gly Gln Val Ser Ser Arg Ser Ser Ser
               420                 425                 430
   Pro Ser Val Arg Met Ile Thr Thr Ser Gly Pro Thr Ser Glu Lys Pro
           435                 440                 445
   Thr Arg Ser His Pro Trp Thr Pro Asp Asp Ser Thr Asp Thr Asn Gly
       450                 455                 460
   Ser Asp Asn Ser Ile Pro Met Ala Tyr Leu Thr Leu Asp His Gln Leu
   465                 470                 475                 480
   Gln Pro Leu Ala Pro Cys Pro Asn Ser Lys Glu Ser Met Ala Val Phe
                   485                 490                 495
   Glu Gln His Cys Lys Met Ala Gln Glu Tyr Met Lys Val Gln Thr Glu
               500                 505                 510
   Ile Ala Leu Leu Leu Gln Arg Lys Gln Glu Leu Val Ala Glu Leu Asp
           515                 520                 525
   Gln Asp Glu Lys Asp Gln Gln Asn Thr Ser Arg Leu Val Gln Glu His
       530                 535                 540
   Lys Lys Leu Leu Asp Glu Asn Lys Ser Leu Ser Thr Tyr Tyr Gln Gln
   545                 550                 555                 560
   Cys Lys Lys Gln Leu Glu Val Ile Arg Ser Gln Gln Gln Lys Arg Gln
                   565                 570                 575
   Gly Thr Ser
<210> 109
   <211> 1268
   <212> DNA
   <213> Rattus norvegicus
<400> 109
   gaggttgtgc tggggctccg cgcggcctgc agtcccgggc ccgcgacgcg cgccgcccgc 60
   ccgccatgga gcctggcccc gacggcccag ccgcgcccgg ccccgccgcc atccgtgagg 120
   gttggttccg agagacctgc agcctgtggc ctggccaggc cctgtcgctg caagtggagc 180
   agctgctgca ccaccggcgc tcgcggtacc aagacatcct cgtcttccgc agtaaaacct 240
   acggcaacgt gctggttctg gatggcgtca tccagtgtac cgagagggac gagttctcct 300
   accaggagat gatagccaac ctgccgctct gcagccaccc caacccgcgg aaggtgctga 360
   tcatcggggg tggagatggg ggcgtcctac gggaagtggt gaagcacccc tctgtggagt 420
   ctgtggtcca gtgcgagatc gatgaggatg tcattgaggt ctctaagaag ttcctgcctg 480
   gcatggccgt tggctactcc agctcaaagc tgactctcca cgtgggcgat ggctttgagt 540
   tcatgaagca gaaccaagat gcctttgatg tcatcatcac cgactcctca gaccccatgg 600
   gccctgctga gagcctcttc aaggagtcct attaccagct catgaagaca gccctcaaag 660
   aagatggcat cctctgttgc cagggcgagt gccagtggct gcacctggac ctcatcaagg 720
   agatgaggca tttctgcaag tctctcttcc ctgtggtgag ctatgcctac tgtaccattc 780
   ccacctatcc cagtggccag atcggcttca tgctgtgtag caaaaacccg agcaccaact 840
   tccgggagcc cgtgcagcag ctgacacagg cccaggtaga gcagatgcag ctgaagtact 900
   ataactcaga catgcaccgc gcggccttcg tgctgccaga gttcacccga aaggccctga 960
   atgacataag ctgaatccag tgccactgta acaccacccg agacctcagt tggattggac 1020
   caaggagctt ccaagctgtc tgggaccacc agtcctggac caaactccca gatgactctt 1080
   gcccaccaac caagtgttac aggccccaga atgctgcctg gcctgccctg ctgggtggac 1140
   tcagtctctg tgtctgtcta tctctgtggc gttcagcccc acgcctatac cagctctgta 1200
   cagcaccacc tatgccctgt gacccaacaa aaaacacgtg tatttataac aaaaaaaaaa 1260
   aaaaaaaa                                                          1268
<210> 110
   <211> 302
   <212> PRT
   <213> Rattus norvegicus
<400> 110
   Met Glu Pro Gly Pro Asp Gly Pro Ala Ala Pro Gly Pro Ala Ala Ile
   1               5                   10                  15
   Arg Glu Gly Trp Phe Arg Glu Thr Cys Ser Leu Trp Pro Gly Gln Ala
               20                  25                  30
   Leu Ser Leu Gln Val Glu Gln Leu Leu His His Arg Arg Ser Arg Tyr
           35                  40                  45
   Gln Asp Ile Leu Val Phe Arg Ser Lys Thr Tyr Gly Asn Val Leu Val
       50                  55                  60
   Leu Asp Gly Val Ile Gln Cys Thr Glu Arg Asp Glu Phe Ser Tyr Gln
   65                  70                  75                  80
   Glu Met Ile Ala Asn Leu Pro Leu Cys Ser His Pro Asn Pro Arg Lys
                   85                  90                  95
   Val Leu Ile Ile Gly Gly Gly Asp Gly Gly Val Leu Arg Glu Val Val
               100                 105                 110
   Lys His Pro Ser Val Glu Ser Val Val Gln Cys Glu Ile Asp Glu Asp
           115                 120                 125
   Val Ile Glu Val Ser Lys Lys Phe Leu Pro Gly Met Ala Val Gly Tyr
       130                 135                 140
   Ser Ser Ser Lys Leu Thr Leu His Val Gly Asp Gly Phe Glu Phe Met
   145                 150                 155                 160
   Lys Gln Asn Gln Asp Ala Phe Asp Val Ile Ile Thr Asp Ser Ser Asp
                   165                 170                 175
   Pro Met Gly Pro Ala Glu Ser Leu Phe Lys Glu Ser Tyr Tyr Gln Leu
               180                 185                 190
   Met Lys Thr Ala Leu Lys Glu Asp Gly Ile Leu Cys Cys Gln Gly Glu
           195                 200                 205
   Cys Gln Trp Leu His Leu Asp Leu Ile Lys Glu Met Arg His Phe Cys
       210                 215                 220
   Lys Ser Leu Phe Pro Val Val Ser Tyr Ala Tyr Cys Thr Ile Pro Thr
   225                 230                 235                 240
   Tyr Pro Ser Gly Gln Ile Gly Phe Met Leu Cys Ser Lys Asn Pro Ser
                   245                 250                 255
   Thr Asn Phe Arg Glu Pro Val Gln Gln Leu Thr Gln Ala Gln Val Glu
               260                 265                 270
   Gln Met Gln Leu Lys Tyr Tyr Asn Ser Asp Met His Arg Ala Ala Phe
           275                 280                 285
   Val Leu Pro Glu Phe Thr Arg Lys Ala Leu Asn Asp Ile Ser
       290                 295                 300
<210> 111
   <211> 1282
   <212> DNA
   <213> Mus musculus
<400> 111
   cgggttgtgc tgggggggtc cgcgcgccct gcagtcccga acccgctacg cgctccgtcg 60
   gcccgcccgc ccgccatgga gcctggcccc gacggcccag ccgcgcccgg ccccgccgcc 120
   atccgtgagg gctggttccg agagacctgc agcctgtggc ccggccaggc cctgtcgctg 180
   caagtggagc agctgcttca ccaccggcga tcgcggtacc aagacatcct cgtcttccgc 240
   agtaaaacct acggcaacgt gctggttctg gatggcgtca tccagtgtac tgagagggat 300
   gagttctcct accaggagat gatcgccaac ctgccgctct gcagccaccc caacccgcgg 360
   aaggtgctga tcatcggggg tggagatggg ggcgtcctac gggaagtggt gaagcacccc 420
   tctgtggagt cggtggtcca gtgcgagatt gatgaggatg tcattgaagt ctctaagaag 480
   ttcctgcctg gcatggccgt tggcttctcc agctcaaagc tgactctcca cgtgggcgat 540
   ggctttgagt tcatgaaaca gaaccaagat gcctttgacg tcatcatcac cgactcctca 600
   gaccccatgg gccctgctga gagcctcttc aaggagtcct attaccagct catgaagaca 660
   gcactcaaag aagatggcat cctgtgctgc cagggtgagt gccagtggct gcacctggac 720
   ctcatcaagg agatgaggca cttctgcaaa tctctcttcc ccgtggtgga ctacgcctac 780
   tgtagcattc ctacctatcc cagcggccag atcggcttca tgctgtgtag caaaaacccg 840
   agcaccaact tccgggagcc agtgcagcag ttgacacagg cccaggtgga gcagatgcag 900
   ctgaaatact ataactcgga catgcaccgt gccgccttcg tactgcctga gttcacccgg 960
   aaggccctca atgacataag ctgaatccag gtgccactgt gacaccaccc gagacctcaa 1020
   tcggattgga ccaaggatct tccaagttgt ctggggacca ccagtcctgg accagactcc 1080
   cagatgactc ttgcccacca accaagtgtt acaggcccca tgatgctgcc tggcctggcc 1140
   tggcctggcc tggcctggcc tgccctgctg ggtggactca gtctctgtct gtctatctct 1200
   gtggcgttca gccccacgcc tataccagct ctgtacagca ccgcctatgc cctgtgaccc 1260
   aacaaaatac atgtgtattc cg                                          1282
<210> 112
   <211> 302
   <212> PRT
   <213> Mus musculus
<400> 112
   Met Glu Pro Gly Pro Asp Gly Pro Ala Ala Pro Gly Pro Ala Ala Ile
   1               5                   10                  15
   Arg Glu Gly Trp Phe Arg Glu Thr Cys Ser Leu Trp Pro Gly Gln Ala
               20                  25                  30
   Leu Ser Leu Gln Val Glu Gln Leu Leu His His Arg Arg Ser Arg Tyr
           35                  40                  45
   Gln Asp Ile Leu Val Phe Arg Ser Lys Thr Tyr Gly Asn Val Leu Val
       50                  55                  60
   Leu Asp Gly Val Ile Gln Cys Thr Glu Arg Asp Glu Phe Ser Tyr Gln
   65                  70                  75                  80
   Glu Met Ile Ala Asn Leu Pro Leu Cys Ser His Pro Asn Pro Arg Lys
                   85                  90                  95
   Val Leu Ile Ile Gly Gly Gly Asp Gly Gly Val Leu Arg Glu Val Val
               100                 105                 110
   Lys His Pro Ser Val Glu Ser Val Val Gln Cys Glu Ile Asp Glu Asp
           115                 120                 125
   Val Ile Glu Val Ser Lys Lys Phe Leu Pro Gly Met Ala Val Gly Phe
       130                 135                 140
   Ser Ser Ser Lys Leu Thr Leu His Val Gly Asp Gly Phe Glu Phe Met
   145                 150                 155                 160
   Lys Gln Asn Gln Asp Ala Phe Asp Val Ile Ile Thr Asp Ser Ser Asp
                   165                 170                 175
   Pro Met Gly Pro Ala Glu Ser Leu Phe Lys Glu Ser Tyr Tyr Gln Leu
               180                 185                 190
   Met Lys Thr Ala Leu Lys Glu Asp Gly Ile Leu Cys Cys Gln Gly Glu
           195                 200                 205
   Cys Gln Trp Leu His Leu Asp Leu Ile Lys Glu Met Arg His Phe Cys
       210                 215                 220
   Lys Ser Leu Phe Pro Val Val Asp Tyr Ala Tyr Cys Ser Ile Pro Thr
   225                 230                 235                 240
   Tyr Pro Ser Gly Gln Ile Gly Phe Met Leu Cys Ser Lys Asn Pro Ser
                   245                 250                 255
   Thr Asn Phe Arg Glu Pro Val Gln Gln Leu Thr Gln Ala Gln Val Glu
               260                 265                 270
   Gln Met Gln Leu Lys Tyr Tyr Asn Ser Asp Met His Arg Ala Ala Phe
           275                 280                 285
   Val Leu Pro Glu Phe Thr Arg Lys Ala Leu Asn Asp Ile Ser
       290                 295                 300
<210> 113
   <211> 1235
   <212> DNA
   <213> Homo sapiens
<400> 113
   ggcggagctg gtcccgttgt gctgcggcgc cgcgcggcct gcagtcccgg gcccgcgccc 60
   cgcgccgccc gcccgcccgc catggagccc ggccccgacg gccccgccgc ctccggcccc 120
   gccgccatcc gcgagggctg gttccgcgag acctgcagcc tgtggcccgg ccaggccctg 180
   tcactgcagg tggagcagct gctccaccac cggcgctcgc gctaccagga catcctcgtc 240
   ttccgcagta agacctatgg caacgtgctg gtgttggacg gtgtcatcca gtgcacggag 300
   agagacgagt tctcctacca ggagatgatc gccaacctgc ctctctgcag ccaccccaac 360
   ccgcgaaagg tgctgatcat cgggggcgga gatggaggtg tcctgcggga ggtggtgaag 420
   cacccctccg tggagtccgt ggtccagtgt gagatcgacg aggatgtcat ccaagtctcc 480
   aagaagttcc tgccaggcat ggccattggc tactctagct cgaagctgac cctacatgtg 540
   ggtgacggtt ttgagttcat gaaacagaat caggatgcct tcgacgtgat catcactgac 600
   tcctcagacc ccatgggccc cgccgaaagt ctcttcaagg agtcctatta ccagctcatg 660
   aagacagccc tcaaggaaga tggtgtcctc tgctgccagg gcgagtgcca gtggctgcac 720
   ctggacctca tcaaggagat gcggcagttc tgccagtccc tgttccccgt ggtggcctat 780
   gcctactgca ccatccccac ctaccccagc ggccagatcg gcttcatgct gtgcagcaag 840
   aacccgagca cgaacttcca ggagccggtg cagccgctga cacagcagca ggtggcgcag 900
   atgcagctga agtactacaa ctccgacgtg caccgcgccg cctttgtgct gcccgagttt 960
   gcccgcaagg ccctgaatga tgtgagctga gcccaggcgc caccactgat gccacccagg 1020
   acctcggacc ttggagcctg cggggtgcct cggcccctcc agccccgggc cggacctcct 1080
   gctggctctc gcccaccaac caagtgttac aagccccaga atgctgcccg gcctgccctg 1140
   ctgggcggac tgtctgtgtg tctgtctctc tggcgttcca cctccaagcc tataccagct 1200
   gtgtacagcg ccatctctct gccttctgtt gcccc                            1235
<210> 114
   <211> 302
   <212> PRT
   <213> Homo sapiens
<400> 114
   Met Glu Pro Gly Pro Asp Gly Pro Ala Ala Ser Gly Pro Ala Ala Ile
    1               5                  10                  15
   Arg Glu Gly Trp Phe Arg Glu Thr Cys Ser Leu Trp Pro Gly Gln Ala
               20                  25                  30
   Leu Ser Leu Gln Val Glu Gln Leu Leu His His Arg Arg Ser Arg Tyr
           35                  40                  45
   Gln Asp Ile Leu Val Phe Arg Ser Lys Thr Tyr Gly Asn Val Leu Val
       50                  55                  60
   Leu Asp Gly Val Ile Gln Cys Thr Glu Arg Asp Glu Phe Ser Tyr Gln
   65                  70                  75                  80
   Glu Met Ile Ala Asn Leu Pro Leu Cys Ser His Pro Asn Pro Arg Lys
                   85                  90                  95
   Val Leu Ile Ile Gly Gly Gly Asp Gly Gly Val Leu Arg Glu Val Val
               100                 105                 110
   Lys His Pro Ser Val Glu Ser Val Val Gln Cys Glu Ile Asp Glu Asp
           115                 120                 125
   Val Ile Gln Val Ser Lys Lys Phe Leu Pro Gly Met Ala Ile Gly Tyr
       130                 135                 140
   Ser Ser Ser Lys Leu Thr Leu His Val Gly Asp Gly Phe Glu Phe Met
   145                 150                 155                 160
   Lys Gln Asn Gln Asp Ala Phe Asp Val Ile Ile Thr Asp Ser Ser Asp
                   165                 170                 175
   Pro Met Gly Pro Ala Glu Ser Leu Phe Lys Glu Ser Tyr Tyr Gln Leu
               180                 185                 190
   Met Lys Thr Ala Leu Lys Glu Asp Gly Val Leu Cys Cys Gln Gly Glu
           195                 200                 205
   Cys Gln Trp Leu His Leu Asp Leu Ile Lys Glu Met Arg Gln Phe Cys
       210                 215                 220
   Gln Ser Leu Phe Pro Val Val Ala Tyr Ala Tyr Cys Thr Ile Pro Thr
   225                 230                 235                 240
   Tyr Pro Ser Gly Gln Ile Gly Phe Met Leu Cys Ser Lys Asn Pro Ser
                   245                 250                 255
   Thr Asn Phe Gln Glu Pro Val Gln Pro Leu Thr Gln Gln Gln Val Ala
               260                 265                 270
   Gln Met Gln Leu Lys Tyr Tyr Asn Ser Asp Val His Arg Ala Ala Phe
           275                 280                 285
   Val Leu Pro Glu Phe Ala Arg Lys Ala Leu Asn Asp Val Ser
       290                 295                 300
<210> 115
   <211> 2195
   <212> DNA
   <213> Homo sapiens
<400> 115
   atgaaatggg tcttggcaaa actattcaat caattacatt cctctatgaa atccttctga 60
   ctggtataag aggacctttc ctgattattg ctccactttc tactattgca aactgggaga 120
   gagaatttcg tacgtggact gatattaacg ttgtggttta tcatgggagc ctgattagca 180
   gacaaatgat acagcaatac gagatgtact tcagggattc acaggggcgt atcattcgag 240
   gagcttacag attccaagcc atcatcacca cttttgaaat gattcttgga ggctgtggag 300
   agcttaatgc aattgaatgg cgatgtgtga ttattgatga agcacatagg ttaaaaaata 360
   aaaattgtaa actcttagag ggcctgaaac tcatgaatct ggaacacaag gtgcttttga 420
   ctggcacccc tctccaaaat acagttgaag aactatttag tcttcttcac tttcttgaac 480
   ccttaaggtt tccttctgaa tcaacattta tgcaagaatt tggggatctg aaaacagagg 540
   aacaggtaca gaaacttcag gttatcctga aaccaatgat gttgagacga ttaaaagaag 600
   atgtggaaaa gaagttggca cctaaagaag aaaccatcat tgaagtagaa cttactaata 660
   ttcaaaagaa atactaccgg gctatcttgg aaaagaactt ttctttttta tccaaaggag 720
   caggacaaac taatgtacct aacttggtca ataccatgat ggagctcagg aaatgttgta 780
   atcatccata tcttataaaa ggtgctgagg agaaaatact tggagaattt agagatactt 840
   acaatccagc tgcttctgat tttcatcttc aagcaatgat ccagtctgct ggtaaattgg 900
   tccttattga taaattgctt cccaaaatga aagccggagg tcataaagtg ctcatcttct 960
   ctcaaatggt tcgttgcctt gacattctgg aggactatct catacataaa agatacttat 1020
   atgagcgaat tgatggcaga gtcagaggaa atcttcggca agctgctata gatagattta 1080
   gtaaacctga ttcagataga tttgtttttc tcctgtgtac ccgagctggt gggttgggca 1140
   tcaacttaac tgcagctgat acatgtataa tttttgattc tgattggaat cctcagaatg 1200
   atcttcaggc ccaagctcgt tgccacagaa ttggtcagaa caaagcagtt aaagtctaca 1260
   gactggtaac tcgtaactca tatgagagag agatgtttga ccgagccagt ttgaaactgg 1320
   gcctagataa agctgtgtta cagagcatga gtggaagaga aagtaatgtt ggtggtattc 1380
   agcagctttc caaaaaggaa atagaagatc tgcttcgaag aggtgcttat ggtgctatta 1440
   tggaggaaga agatgaaggc tctaaattct gcgaagagga tatcgatcag attttactac 1500
   gtcgtacaaa aactattaca attgaatcag aaggacgtgg gtcaacattt gccaaggcga 1560
   gttttgtggc atctggaaac cggacagata tttctttaga tgatcccaac ttctggcaaa 1620
   aatgggctaa aaaggcagaa atagatatag aggccatcag tggcagaaac ggcttggtta 1680
   ttgacactcc aagaattagg aagcaaacaa gaccttttag tgccacaaaa gatgaattgg 1740
   ctgaattatc tgaagctgaa agtgaaggag atgaaaagcc caaactccgg agaccctgtg 1800
   accgttccaa tggctatgga agaactgaat gctttagagt tgagaaaaac ctgctagttt 1860
   atgggtgggg ccgatggaga gagattctat ctcatggccg tttcaaaagg cagctaaatg 1920
   aacacgatgt agagataatt tgccgagctc tcttagcata ttgccttgtt cactaccgag 1980
   gagatgagaa gattaaaggt ttcatatggg atctcattac tccaactgaa gatggacaga 2040
   cacgagagct acagaatcat ctaggcctat cagctcctgt acccaggggt cgaaaaggga 2100
   agaaagtaaa aactcaaaca agctcatttg atatacaaaa agcagaatgg cttcgaaaat 2160
   ataatcccga gcagctcctt caagatgaag gctac                            2195
<210> 116
   <211> 730
   <212> PRT
   <213> Homo sapiens
<400> 116
   Met Gly Leu Gly Lys Thr Ile Gln Ser Ile Thr Phe Leu Tyr Glu Ile
    1               5                  10                  15
   Leu Leu Thr Gly Ile Arg Gly Pro Phe Leu Ile Ile Ala Pro Leu Ser
               20                  25                  30
   Thr Ile Ala Asn Trp Glu Arg Glu Phe Arg Thr Trp Thr Asp Ile Asn
           35                  40                  45
   Val Val Val Tyr His Gly Ser Leu Ile Ser Arg Gln Met Ile Gln Gln
       50                  55                  60
   Tyr Glu Met Tyr Phe Arg Asp Ser Gln Gly Arg Ile Ile Arg Gly Ala
   65                  70                  75                  80
   Tyr Arg Phe Gln Ala Ile Ile Thr Thr Phe Glu Met Ile Leu Gly Gly
                   85                  90                  95
   Cys Gly Glu Leu Asn Ala Ile Glu Trp Arg Cys Val Ile Ile Asp Glu
               100                 105                 110
   Ala His Arg Leu Lys Asn Lys Asn Cys Lys Leu Leu Glu Gly Leu Lys
           115                 120                 125
   Leu Met Asn Leu Glu His Lys Val Leu Leu Thr Gly Thr Pro Leu Gln
       130                 135                 140
   Asn Thr Val Glu Glu Leu Phe Ser Leu Leu His Phe Leu Glu Pro Leu
   145                 150                 155                 160
   Arg Phe Pro Ser Glu Ser Thr Phe Met Gln Glu Phe Gly Asp Leu Lys
                   165                 170                 175
   Thr Glu Glu Gln Val Gln Lys Leu Gln Val Ile Leu Lys Pro Met Met
               180                 185                 190
   Leu Arg Arg Leu Lys Glu Asp Val Glu Lys Lys Leu Ala Pro Lys Glu
           195                 200                 205
   Glu Thr Ile Ile Glu Val Glu Leu Thr Asn Ile Gln Lys Lys Tyr Tyr
       210                 215                 220
   Arg Ala Ile Leu Glu Lys Asn Phe Ser Phe Leu Ser Lys Gly Ala Gly
   225                 230                 235                 240
   Gln Thr Asn Val Pro Asn Leu Val Asn Thr Met Met Glu Leu Arg Lys
   245 250 255
   Cys Cys Asn His Pro Tyr Leu Ile Lys Gly Ala Glu Glu Lys Ile Leu
   260 265 270
   Gly Glu Phe Arg Asp Thr Tyr Asn Pro Ala Ala Ser Asp Phe His Leu
   275 280 285
   Gln Ala Met Ile Gln Ser Ala Gly Lys Leu Val Leu Ile Asp Lys Leu
   290 295 300
   Leu Pro Lys Met Lys Ala Gly Gly His Lys Val Leu Ile Phe Ser Gln
   305 310 315 320
   Met Val Arg Cys Leu Asp Ile Leu Glu Asp Tyr Leu Ile His Lys Arg
   325 330 335
   Tyr Leu Tyr Glu Arg Ile Asp Gly Arg Val Arg Gly Asn Leu Arg Gln
   340 345 350
   Ala Ala Ile Asp Arg Phe Ser Lys Pro Asp Ser Asp Arg Phe Val Phe
   355 360 365
   Leu Leu Cys Thr Arg Ala Gly Gly Leu Gly Ile Asn Leu Thr Ala Ala
   370 375 380
   Asp Thr Cys Ile Ile Phe Asp Ser Asp Trp Asn Pro Gln Asn Asp Leu
   385 390 395 400
   Gln Ala Gln Ala Arg Cys His Arg Ile Gly Gln Asn Lys Ala Val Lys
   405 410 415
   Val Tyr Arg Leu Val Thr Arg Asn Ser Tyr Glu Arg Glu Met Phe Asp
   420 425 430
   Arg Ala Ser Leu Lys Leu Gly Leu Asp Lys Ala Val Leu Gln Ser Met
   435 440 445
   Ser Gly Arg Glu Ser Asn Val Gly Gly Ile Gln Gln Leu Ser Lys Lys
   450 455 460
   Glu Ile Glu Asp Leu Leu Arg Arg Gly Ala Tyr Gly Ala Ile Met Glu
   465 470 475 480
   Glu Glu Asp Glu Gly Ser Lys Phe Cys Glu Glu Asp Ile Asp Gln Ile
   485 490 495
   Leu Leu Arg Arg Thr Lys Thr Ile Thr Ile Glu Ser Glu Gly Arg Gly
   500 505 510
   Ser Thr Phe Ala Lys Ala Ser Phe Val Ala Ser Gly Asn Arg Thr Asp
   515 520 525
   Ile Ser Leu Asp Asp Pro Asn Phe Trp Gln Lys Trp Ala Lys Lys Ala
   530 535 540
   Glu Ile Asp Ile Glu Ala Ile Ser Gly Arg Asn Gly Leu Val Ile Asp
   545 550 555 560
   Thr Pro Arg Ile Arg Lys Gln Thr Arg Pro Phe Ser Ala Thr Lys Asp
   565 570 575
   Glu Leu Ala Glu Leu Ser Glu Ala Glu Ser Glu Gly Asp Glu Lys Pro
               580                 585                 590
   Lys Leu Arg Arg Pro Cys Asp Arg Ser Asn Gly Tyr Gly Arg Thr Glu
           595                 600                 605
   Cys Phe Arg Val Glu Lys Asn Leu Leu Val Tyr Gly Trp Gly Arg Trp
       610                 615                 620
   Arg Glu Ile Leu Ser His Gly Arg Phe Lys Arg Gln Leu Asn Glu His
   625                 630                 635                     640
   Asp Val Glu Ile Ile Cys Arg Ala Leu Leu Ala Tyr Cys Leu Val His
                   645                 650                     655
   Tyr Arg Gly Asp Glu Lys Ile Lys Gly Phe Ile Trp Asp Leu Ile Thr
               660                 665                 670
   Pro Thr Glu Asp Gly Gln Thr Arg Glu Leu Gln Asn His Leu Gly Leu
           675                 680                 685
   Ser Ala Pro Val Pro Arg Gly Arg Lys Gly Lys Lys Val Lys Thr Gln
       690                 695                 700
   Thr Ser Ser Phe Asp Ile Gln Lys Ala Glu Trp Leu Arg Lys Tyr Asn
   705                 710                 715                     720
   Pro Glu Gln Leu Leu Gln Asp Glu Gly Tyr
                   725                 730

## Beschreibung

Die Erfindung betrifft ein Verfahren zur Auffindung schmerzrelevanter Substanzen.

Zur Therapie von Schmerzen stehen unterschiedliche Arzneimittel zur Verfügung wie z.B. Acetylsalicylsäure, Paracetamol, Dipyrone, Tramadol, Morphin und Fentanyl; aber auch Substanzen wie Amitryptilin-und Ketamin kommen zur Behandlung von Schmerzpatienten zum Einsatz. Trotz zunehmend verfeinerter Therapieschemata kann jedoch insbesondere bei chronischen Schmerzzuständen oft keine dauerhafte Verbesserung für die Patienten erzielt werden. Hierfür ist unter anderem auch die Tatsache verantwortlich, daß es beim chronischen Schmerz zu dauerhaften Veränderungen beteiligter Nervenzellen kommt.

Die Schmerzforschung der letzten Jahre erbrachte die grundlegende Erkenntnis, daß der Entwicklung gerade chronischer Schmerzzustände plastische Veränderungen des Nervensystems, insbesondere in den nozizeptiven Neuronen der Hinterwurzelganglien und der Neurone im Bereich der Dorsalhörner des Rückenmarks, zugrunde liegen (als Überblick siehe: Coderre et al. 1993; Zimmermann & Herdegen, 1996). Die neuronale Plastizität geht einher mit Veränderungen in der Expression bestimmter Gene und führt zur langanhaltenden Veränderung des Phänotyps der betroffenen Neuronen. Das Konzept der neuronalen Plastizität wurde bisher vor allem auf Entwicklungs-, Lem-und Regenerationsprozesse angewandt, doch die neueren Befunde aus der Schmerzforschung zeigen, daß dieses Konzept auch bei pathophysiologischen Vorgängen greift (Tölle, 1997).

Die Chronifizierung des Schmerzes ist tierexperimentell auf phänomenologischer Ebene bereits relativ gut charakterisiert. Die Induktion chronischer Schmerzzustände führt zu folgenden Veränderungen:
- Erhöhte Empfindlichkeit und verringerte Reizschwelle peripherer Nozizeptoren
- Aktivierung sogenannter stiller Nozizeptoren
- Reorganisation rezeptiver Felder
- Erregbarkeitszutiahme im Rückenmark.

Diese plastischen Veränderungen sind sowohl für die in den Ganglien vorkommenden primären Afferenzen, als auch für die im Rückenmark lokalisierten nachgeschalteten Neurone beschrieben worden und werden auch supraspinal z. B. im Thalamus vermutet. In Analogie zu den für Lern- und Gedächtnisprozesse beschriebenen Mechanismen ist anzunehmen, daß in den beteiligten Zellen ein spezifisches Genprogramm abläuft, das die koordinierte Regulation relevanter Gene beinhaltet, deren Expression dann maßgeblich zur pathophysiologischen Ausprägung chronischer Schmerzen beiträgt.

Ausgangspunkt der Erfindung war daher die Identifizierung derartiger schmerzregulierter Gene, die in ihrer Expression unter Schrnerzbedingungen verändert und deshalb wahrscheinlich an der Entstehung und Verarbeitung von insbesondere chronischen Schmerzen beteiligt sind, über ihre Regulationszusammenhänge.

Für eine Reihe bekannter Gene wurde bereits eine Regulation in verschiedenen Schmerzmodellen nachgewiesen (s. Tabelle 1), so zum Beispiel für Neurotransmitter (Substanz P, CGRP), Rezeptoren (Substanz P-Rezeptor, µ, κ, δ-Opiatrezeptoren, NMDA-Rezeptor) und Transkriptionsfaktoren (cJun, JunB, cFos oder Krox24). Die Tatsache, daß die genannten Rezeptoren bereits als molekulare Targets für die Entwicklung neuer Analgetika verwendet werden (Dickenson, 1995), gibt einen deutlichen Hinweis darauf, daß auch die Identifizierung neuer schmerzregulierter Gene für die Entwicklung von Analgetika, insbesondere für entsprechende Screeningverfahren, von großem Interesse ist. Die zentrale Idee ist hierbei, die Entstehung oder Persistenz von Schmerzen, insbesondere chronischer Art, zu unterbrechen, indem solche Proteine in ihrer Funktion beeinflußt werden, die in Schmerz-Zuständen verstärkt oder vermindert gebildet werden.

Verschiedene Veröffentlichungen beschäftigen sich mit der Idee, Modulatoren für bestimmte solcher Verbindungen aufzufinden.

Sargovi et al. (Trends Pharmacol. Sci. (2000), 21 (3), 93-98) beschäftigen sich beispielsweise mit der Entwicklung von pharmakologischen Agenzien, die die Neutrophin-Funktion modulieren. Mittels "high-througput screening"-Verfahren werden dazu Liganden von Neutrophinen oder deren Rezeptoren identifiziert, wobei die Untersuchung im wesentlichen auf den p75 Rezeptor sowie dessen Beteiligung in der Apoptose und der Einfluß auf den trk-Rezeptor ausgerichtet ist.

Pim-2 ist z.B. aus "Baytel et al. (1998): "The human Pim-2 proto-oncogene and its testicular expression", Biochimica et Biophysica Acta, Gene Structure and Expression, Bd. 1442, Nr. 2-3, Seiten 274-285" bekannt. Der Artikel beschreibt die Anschaltung der Expression bei Pim-1 defizienten Mäusen und die Relevanz dieses kompensatorischen "taggings" bei der Tumorgenese.

**Tabelle 1: Regulation bekannter Gene/Genprodukte in Schmerz-Tiermodellen**

| **Gen(produkt)** | **Reg** | **Gewebe/Zelle** | **Modell** | **Literatur** |
|---|---|---|---|---|
| **(a) Neurotransmitter** | | | | |
| CGRP | ⇑ | RM-Dorsalhom | UV-Bestrahlung der Haut | Gillardon F et al. (1992) Ann NY Acad Sci657: 493-96 |
| Preprotachykinin & CGRP-mRNA | ⇑ | DRG | Monoarthritis | Donaldson LF et al. (1992) Mol Brain Res 16: 143-49 |
| Preprotachykinin -mRNA | ⇑ | RM-Dorsalhom | Formalin | Noguchi &Ruda (1992) J Neurosci 12:2563-72 |
| Prodynorphin mRNA | ⇑ | Rückenmark | Exp Arthritis | Höllt et al (1987) Neurosci Lett 96:247-52 |
| Dynorphin Prot. | ⇑ | Rückenmark | Formalin | Ruda et al. (1988) PNAS 85: 622-26 |
| Substanz P | ⇑ | Nozizeptoren | Exp. Arthritis | Levine JD et al. (1984) Science 226:547-49 |

| **(b) Neurotrophine** | | | | |
|---|---|---|---|---|
| BDNF mRNA & Immunreaktivität | ⇑ | DRG: trkA+ Zellen | I. th. NGF Inj. | Michael GC et al. (1997) J Neurosci 17: 8476-90 |

| **(c) Rezeptoren** | | | | |
|---|---|---|---|---|
| µ, κ, δ-Bindung | ⇓ ⇑ | RM-Dorsalhom | Monoarthritis | Besse D et al. (1992) Eur J Pharmacol 223:123-31 |
| µ-Oplatrezeptor-Immunreaktivität | ⇑ | DRG | Carageenan ind. Entzündung | Ji R-R et al. (1995) J Neurosci 15: 8156-66 |
| κ & δ-Opiatrez.-mRNA | ⇓ | DRG | Carageenan ind. Entzündung | Ji R-R et al. (1995) J Neurosci 15: 8156-66 |
| κ & µ-Opiatrezeptor-mRNA | ⇑ | RM-Dorsalhom | Monoarthritis | Maekawa K et al. (1995) Pain 64:365-71 |
| CCKₐ-Rez. mRNA | ⇑ | DRG | Axotomie | Zhang X et al. (1993) Neuroscience 57: 227-233 |
| NMDA-R1-mRNA | ⇓ | RM-Dorsalhom Laminae I & II | CFA-induzierte Entzündung | Kus L et al. (1995) Neuroscience 68: 159-65 |

| **(d) Enzyme** | | | | |
|---|---|---|---|---|
| NADPH-Diaphorase Aktivität | ⇑ | RM-Dorsalhom | Ischiaticus-Transektion | Fiallos-Estrada et al. ('93) Neurosci Lett 150: 169-73 |
| NADPH-Diaphorase | ⇑ | Rückenmark | Formalin | Solodkin et al. 1992 Neurosci 51: 495-99 |
| NO-Synthetase mRNA | ⇑ | DRG | Axotomie | Verge VMK et al. (1992) PNAS 89: 11617-62 |
| NO-Synthetase Protein | ⇑ | RM-Dorsalhom | Formalin | Herdegen et al. (1994) Mol Brain Res 22:245-58 |
| NO-Synthetase-Immunreaktivität | ⇑ | DRG | Ischiaticus-Transektion | Fiallos-Estrada et al. ('93) Neurosci Lett 150: 169-73 |

| **(e) Signalkaskade** | | | | |
|---|---|---|---|---|
| rap1A, rap1B, H-ras mRNA | ⇑ | Rückenmark | Formalin | Urayama O et al. (1997) Mol Brain Res 45:331-34 |
| PKC-Bindung | ⇑ | RM-Dorsalhorn | CFA-induzierte Monoarthritis | Tölle TR et al (82) J Neurol 242(S2):135 |

| **(f)Transkriptionsf.** | | | | |
|---|---|---|---|---|
| cFOS | ⇑ | Rückenmark | Noxische Stimulierung | Hunt SP et al. (1987) Nature 328: 632-34 |
| cJun,JunB, cFOS Krox24 | ⇑ | RM-Dorsalhorn | Formalin | Herdegen T et al. (1994) Mol Brain Res 22: 245-48 |

| | | | | |
|---|---|---|---|---|
| RM, Rückenmark; DRG, Dorsal Root Ganglia; CFA, Complete Freund Adjunvans; NGF, Nerve Growth Factor. | | | | |

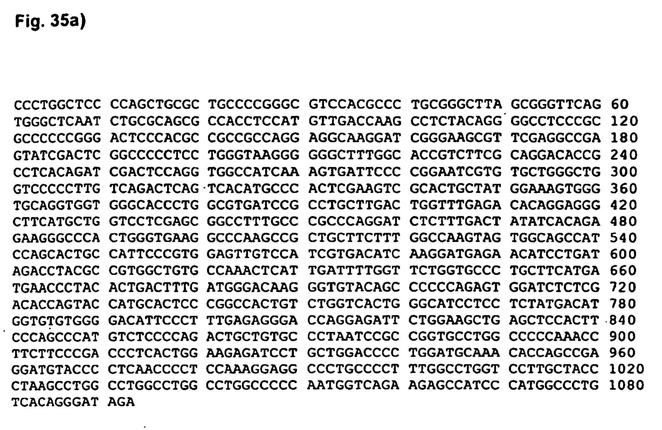

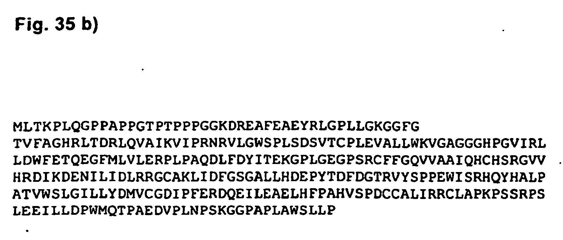

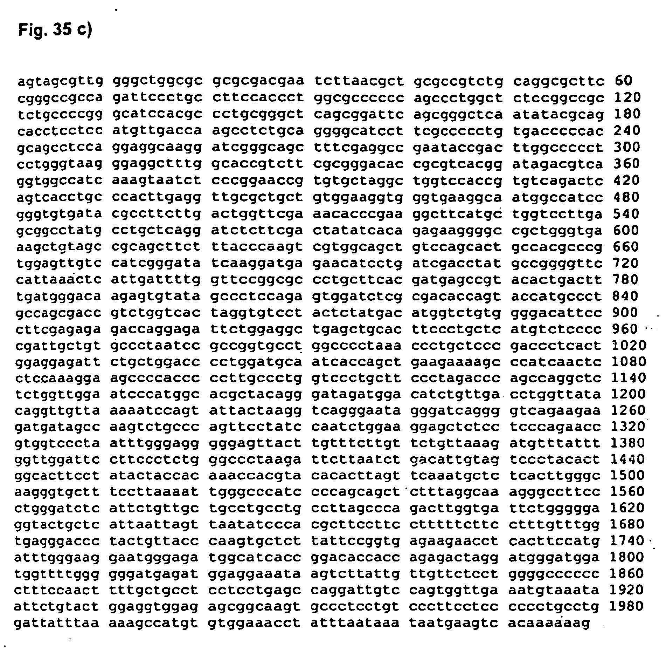

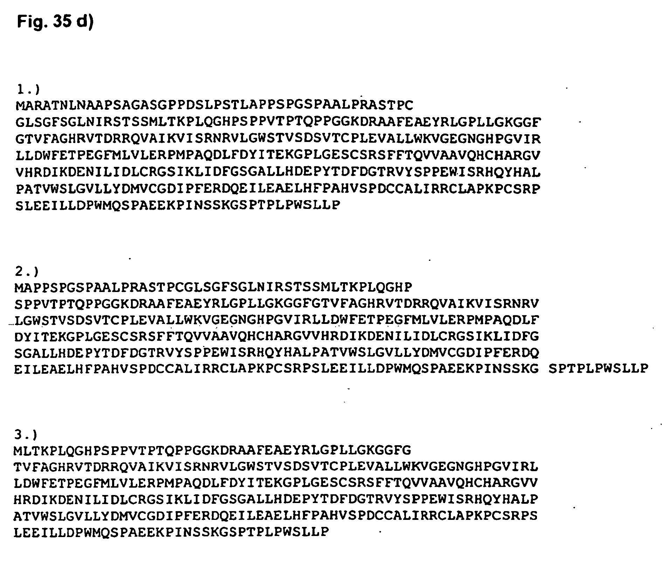

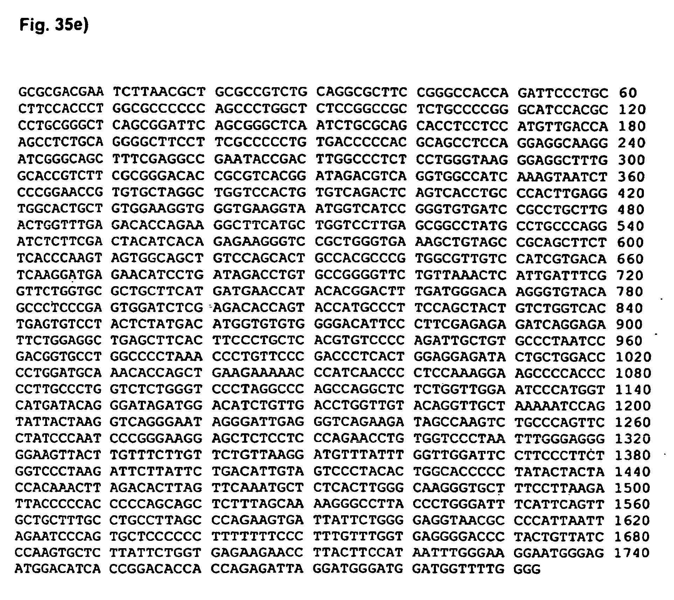

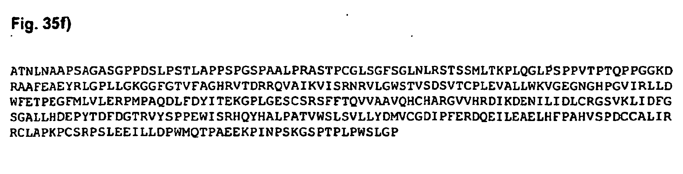

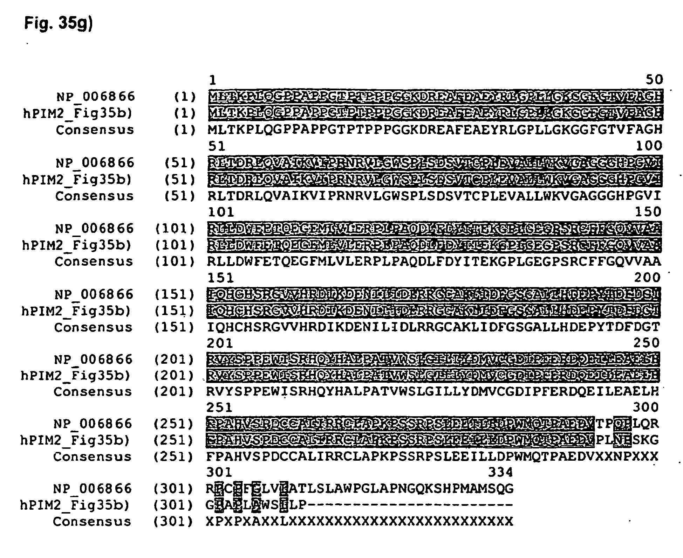

Daraus folgend war primäre Aufgabe der Erfindung, ein Screeningverfahren zur Identifizierung im Schmerz relevanter, insbesondere schmerzregulierender Substanzen zu entwickeln. Die Erfindung betrifft daher ein Verfahren zur Auffindung schmerzregulierender Substanzen umfassend folgende Verfahrenschritte:
(a) Inkubation einer zu testenden Substanz unter geeigneten Bedingungen mit einer Zelle und/oder einer Präparation aus einer solchen Zelle, die ein Peptid oder Protein synthetisiert hat, das ausgewählt ist aus folgender Gruppe:
   - PIM-2,
   - einem Peptid oder Protein, für das oder für einen Teil dessen ein Polynukleotid gemäß einer der Abbildungen 35a), 35c), oder 35e), oder ein dazu zu mindestens 90 % ähnliches Polynukleotid kodiert,
   - einem Peptid oder Protein mit einer Aminosäuresequenz gemäß einer der Abbildungen 35b), 35d), 35f), oder ein dazu zu mindestens 90 % ähnliches Peptid oder Protein
   - und/oder einem Protein, das durch eine Nukleinsäure kodiert wird, die unter stringenten Bedingungen an ein Polynukleotid gemäß einer der Abbildungen 35a), 35c) oder 35e), oder deren Antisense Polynukleotid bindet,
(b) Messung der Bindung der Testsubstanz an dem von der Zelle synthetisierten Peptid oder Protein oder Messung mindestens eines der durch die Bindung der Testsubstanz an das Peptid oder Protein veränderten funktionellen Parameters über Messung der Regulation, Hemmung und/oder Aktivierung von Rezeptoren, lonenkanälen und/oder Enzymen, insbesondere über Messung der Veränderung der Genexpression, des lonenmilieus, des pH oder des Membranpotentials, über Veränderung der Enzymaktivität oder der Konzentration der 2^{nd} messenger, oder Messung der Bindung über die Verdrängung eines bekannten markierten Liganden des Peptids oder Proteins und/oder über die daran gebundene Aktivität einer markierten Testsubstanz.

Dieses neuartige Screeningverfahren basiert darauf, daß hier eine potentielle Schmerz-Wirksamkeit einer Substanz über ihre Wechselwirkung mit einer schmerzregulierten Peptid- oder Proteinstruktur aufgefunden werden kann.

Dabei bezieht sich der Begriff schmerzregulierend auf einen potentiellen regulierenden Einfluß auf das physiologische Schmerzgeschehen, insbesondere auf eine analgetische Wirkung. Der Begriff Substanz umfaßt jede als Arzneimittel-Wirkstoff geeignete Verbindung, insbesondere also niedermolekulare Wirkstoffe, aber auch andere wie Nukleinsäuren, Fette, Zucker, Peptide oder Proteine wie Antikörper.

Die Inkubation unter geeigneten Bedingungen ist hier so zu verstehen, daß die zu untersuchende Substanz mit der Zelle oder der entsprechenden Präparation in einem wässrigen Medium eine definierte Zeit vor der Messung reagieren kann. Dabei kann das wässrige Medium temperiert werden, beispielsweise zwischen 4°C und 40°C, vorzugsweise bei Raumtemperatur oder bei 37°C. Die Inkubationszeit kann zwischen wenigen Sekunden und mehreren Stunden variiert werden, je nach der Wechselwirkung der Substanz mit dem Peptid oder Protein. Bevorzugt sind aber Zeiten zwischen 1 min und 60 min. Das wäßrige Medium kann geeignete Salze und/oder Puffersysteme enthalten, so daß bei der Inkubation beispielsweise ein pH zwischen 6 und 8, vorzugsweise pH 7,0 - 7,5 im Medium herrscht. Dem Medium können weiter geeignete Substanzen, wie Coenzyme, Nährstoffe etc. beigefügt werden. Die geeigneten Bedingungen können vom Fachmann in Abhängigkeit von der zu untersuchenden Wechselwirkung der Substanz mit dem Peptid oder Protein aufgrund seiner Erfahrung, der Literatur oder weniger, einfacher Vorversuche leicht festgelegt werden, um im Verfahren einen möglichst deutlichen Meßwert zu erhalten.

Eine Zelle, die ein bestimmtes Peptid oder Protein synthetisiert hat, ist ein Zelle, die dieses Peptid oder Protein bereits endogen exprimiert hat oder eine solche, die gentechnisch verändert wurden, so daß sie dieses Peptid oder Protein exprimiert und entsprechend vor Beginn des erfindungsgemäßen Verfahrens das Peptid oder Protein enthält. Die Zellen können Zellen aus evt. immortalisierten Zellinien sein oder native aus Geweben stammende und aus diesen isolierte Zellen sein, wobei der Zellverband meist aufgelöst ist. Die Präparation aus diesen Zellen umfaßt insbesondere Homogenate aus den Zellen, das Cytosol, eine Membranfraktion der Zellen mit Membranfragmenten, eine Suspension isolierter Zellorganellen etc.

Die hier aufgezählten Proteine und Peptid wurden im Rahmen dieser Erfindung als durch Schmerz reguliert identifiziert, in dem in einem Tier Schmerz ausgelöst wurde und nach angemessener Zeit das Expressionsmuster in bestimmten Geweben des Tieres mit denen eines Kontroll-Tieres ohne schmerzauslösende Maßnahmen verglichen. Die dabei gefundenen verändert exprimierten Peptide und Proteine umfassen auch bekannte Proteine, wie die Proteinkinase, PIM-2. Aus welcher Spezies diese Proteine stammen, ist für die Funktion des Verfahrens unerheblich, es ist aber bevorzugt, die humane, Maus- oder Ratten-Variante zu verwenden. Die genannten Proteine sind in Hinblick auf die kodierende DNA- und die Aminosäure-Sequenz bekannt und auch in ihrer generellen Funktion beschrieben. Sie wurden aber bisher im Stand der Technik nicht in einen Zusammenhang mit Schmerz und insbesondere der Schmerzregulation gebracht. Da hier die Identifizierung der Proteine über eine Veränderung der Expression in einem In-vivo-Schmerzmodell erfolgte, hat das daraus abgeleitete erfindungsgemäße Screening-Verfahren für zukünftige Arzneimittel unter Verwendung dieser Proteine den erheblichen Vorteil, nicht nur auf theoretischen Überlegungen aufzubauen, sondern vermutlich eine starke In-vivo-Relevanz zu besitzen. Da mit diesem Verfahren die Wechselwirkung von Substanzen mit im Schmerzbereich bisher nicht verwendeten Proteinen und Peptiden als Maßstab für das Auffinden schmerzregulierender Substanzen ermöglicht wird, sind mit diesem Verfahren jetzt möglicherweise schmerzrelevante Substanzen aufzufinden, die bei den im Stand der Technik bisher bekannten Verfahren mit anderen Peptiden oder Proteinen nicht aufgefallen wären. Auch dies ist ein erheblicher Vorteil des neuen erfindungsgemäßen Verfahrens.

Ausgewählt werden können die verwendeten Proteine oder Peptide auch aus solchen, für die ein Polynukleotid gemäß einer der Abbildungen 35a), 35c) oder 35e), kodiert. Abgebildet sind die meisten der bekannten kodierenden cDNA-Sequenzen für Maus, Ratte und Mensch von PIM-2, für das erfindungsgemäße verfahren. Verwendbar sind auch Peptide und Proteine, für die eine DNA-Sequenz kodiert, die einer der abgebildeten zu mindestens 90 % ähnlich ist, da so geringe Abweichungen die Wechselwirkung mit Peptid und Protein und damit die Funktion des Verfahrens meist nicht beeinflussen. Dabei versteht man unter 90% Ähnlichkeit eine 90%-ige Übereinstimmung der Basenfolge im kodierenden Bereich des Polynukleotids.

Die Proteine können auch ausgewählt sein aus solchen mit einer Aminosäuresequenz gemäß einer der Abbildungen 35b), 35d) oder 35f). Auch dies sind überwiegend aus dem Stand der Technik bekannte Sequenzen bis auf die Sequenz der PIM-2 Ratte, die im Rahmen der Erfindung ermittelt wurde. Verwendbar sind hier auch Peptide und Proteine, deren Aminosäure-Sequenz einer der abgebildeten zu mindestens 90 % ähnlich ist, da auch geringe Abweichungen in der Aminosäureabfolge die Wechselwirkung der Substanz mit Peptid und Protein und damit die Funktion des Verfahrens meist nicht beeinflussen. Dabei versteht man unter 90% Ähnlichkeit eine 90%-ige Übereinstimmung in der Aminosäureabfolge.

Die Proteine können auch ausgewählt sein aus solchen, die durch eine Nukleinsäure kodiert werden, die unter stringenten Bedingungen an ein Polynukleotid gemäß einer der Abbildungen 35a), 35c) oder 35e), oder deren Antisense Polynukleotid binden. Dabei versteht man unter stringenten Bedingungen solche unter denen nur perfekt basengepaarte Nukleinsäure-Stränge gebildet werden und stabil bleiben und unter Antisense Polynukleotid ein aus mehreren natürlichen oder modifizierten Nukleinsäuren bestehendes Molekül, dessen Basenabfolge komplementär zur Basenabfolge eines Teilbereiches einer in der Natur vorkommenden RNA ist.

Im Rahmen dieser Erfindung wurden durch Schmerz regulierte Genfragmente identifiziert und sequenziert, die im Stand der Technik noch keinem bekannten Peptid oder Protein zugeordnet sind.

Das jeweils ausreichend umfangreiche sequenzierte Genfragment definiert eindeutig das zugehörige Gen, das Polynukleotid, von dessen Sequenz das Genfragment ein Teil ist. Dadurch, daß das entsprechende Genfragment als schmerzreguliert identifiziert wurde, ist auch eine klar physiologische Funktion des Gens umrissen. Zum vollständigen Gen, das das Fragment umfaßt, gelangt der Fachmann über bekannte Methoden. So kann ein Polynukleotid als Sonde markiert, eine cDNA-Bank mit der Sonde hybridisiert und unter stringenten Bedingungen gewaschen werden und der cDNA-Klon, an den die Sonde gebunden hat, isoliert und gegebenenfalls sequenziert werden. Ebenso ist das Gen, bzw. Polynukleotid dadurch erhältlich, daß Abschnitte eines Polynukleotids als Gen-spezifische Oligonukleotid-Primer ermittelt und synthetisiert werden, mit denen dann durch PCR ausgehend von einzel- oder doppelsträngiger DNA, von cDNA-Bibliotheken oder genomischer DNA als Template das verlängerte Polynukleotid generiert und gegebenenfalls sequenziert wird. Das Vorgehen wird anhand eines Beispiels später genauer erläutert. Auch für diese Gruppe der Peptide und Proteine gilt der Vorteil gegenüber den bekannten Verfahren, daß bei ihrer Verwendung im erfindungsgemäßen Verfahren ein *in vivo-*Bezug sichergestellt ist und das Verfahren erlaubt, potentiell schmerzregulierende Substanzen zu identifizieren, die in den im Stand der Technik bisher bekannten Screening-Methoden möglicherweise nicht aufgefallen wären.

Der Maßstab über den das Verfahren die Auffindung interessanter Substanzen erlaubt, ist entweder die Bindung an das Protein oder Peptid, die z.B. durch Verdrängung eines bekannten Liganden oder das Ausmaß gebundener Substanz nachgewiesen werden kann, oder die Veränderung eines funktionellen Parameters durch die Wechselwirkung der Substanz mit dem Peptid oder Protein. Diese Wechselwirkung kann insbesondere in einer Regulation, Hemmung und/oder Aktivierung von Rezeptoren, lonenkanälen und/oder Enzymen liegen und veränderte funktionelle Parameter können beispielsweise die Genexpression, das Ionenmilieu, der pH oder das Membranpotential, bzw. die Veränderung der Enzymaktivität oder der Konzentration der 2^{nd} messenger sein.

Zur Erläuterung der Erfindung werden im folgenden neben den im allgemeinen Text zu Begriffen gegebenen Erklärungen weitere Definitionen angegeben, um klarzustellen, wie bestimmte, insbesondere in den Ansprüchen verwendete Begriffe im Sinne dieser Erfindung zu verstehen und auszulegen sind.
- Substanz: Damit ist eine chemische Verbindung gemeint. Hier handelt es sich im engeren Sinne um Verbindungen, die potentiell eine Wirkung im Körper entfalten können, niedermolekulare Wirkstoffe, Nukleinsäuren, Fette, Zucker, Peptide oder Proteine, insbesondere hier niedermolekulare Wirkstoffe.
- schmerzregulierend: Im Sinne der Erfindung heißt schmerzregulierend, daß die Substanz die Wahrnehmung von Schmerz direkt oder indirekt beeinflußt, insbesondere natürlich analgetisch wirkt.
- Inkubation: Unter Inkubation ist das Einbringen und Belassen eines biologischen Untersuchungsobjektes, beispielsweise einer Zelle oder eines Proteins, in einem temperierten Medium wie in einem Brutschrank oder auf einem Wasserbad zu verstehen. Dabei heiß hier unter geeigneten Bedingungen eine Inkubation unter physiologischen Bedingungen (z.B. 37°C pH7,2) oder bei den Bedingungen, bei denen eine optimale Messung im Verfahren möglich wird.
- Zelle: Die Zelle ist ein sich selbst regulierendes, offenes, mit seiner Umgebung durch permanenten Stoffaustausch in einem Fließgleichgewicht stehendes System mit eigenem Stoffwechsel, und Vermehrungsfähigkeit. Die Zelle kann separat kultiviert oder Teil eines Gewebes, insbesondere aus einem Organ, sein, und dort vereinzelt oder noch im Zellverband vorliegen.
- Präparation aus einer Zelle: Darunter versteht man Präparate, die mittels chemischer, biologischer, mechanischer oder physikalischer Methoden unter Änderung der Zellstruktur hergestellt werden, beispielsweise Membranfragmente, isolierte Zellkompartimente, isoliertes Cytosol, oder aus Gewebe gewonnenes Homogenat.
- Peptid: Verbindung aus über peptidische Bindungen zu Ketten verknüpften Aminosäuren. Ein Oligopeptid besteht aus zwischen 2 und 9 Aminosäuren, ein Polypeptid aus zwischen 10 und 100 Aminosäuren.
- Protein: Verbindung aus über peptidische Bindungen zu Ketten verknüpften mehr als 100 Aminosäuren u.U. mit einer definierten Raumstruktur.
- PIM-2: Ein Proto-Oncogen und eine Serin-Threonin-Kinase
- Polynukleotid: Das zugrundeliegende Nukleotid ist ein grundsätzlich aus Nucleinbase, Pentose und Phosphorsäure bestehender Grundbaustein der Nucleinsäuren. Diese entspricht einem hochmolekularen Polynucleotid aus mehreren Nukleotiden, die über Phosphorsäure-Pentose-Veresterung miteinander verknüpft sind. In dieser Erfindung werdes aber auch modifizierte Polynukleotide, verwendet die zwar die Basenabfolge beibehalten, aber über ein modifiziertes Rückgrat statt der Phosphorsäure-Pentose verfügen.
- zu mindestens 90 (95, 97)% ähnlich: Darunter ist zu verstehen, daß die mit erfaßten Polynucleotide in ihrem kodierenden Bereich bezüglich der Basenabfolge zu mindestens 90% (95%, 97%) identisch mit der Referenz (Abbildung etc.) sind und die mit erfaßten Peptide und - Proteine in ihrer Primärstruktur, der Abfolge der Aminosäuren zu mindestens 90% (95%, 97%) mit der Referenz identisch sind.
- Gen: Mit dem Begriff Gen wird ein Genomabschnitt mit einer definierten Nukleotidsequenz bezeichnet, der die Information zur Synthese einer m-oder prä-mRNA oder einer sonstigen RNA (z.B. tRNA, rRNA, snRNA etc.) enthält. Es besteht aus kodierenden und nicht kodierenden Abschnitten.
- Genfragment: Nukleinsäureabschnitt, der in seiner Basenabfolge einen Teilbereich eines Gens beinhaltet
- physiologisch verlängertes Genfragment: ein Genfragment, das durch molekularbiologische Verfahren, wie z.B. das Durchmustern einer cDNA-Bibliothek, das Herausziehen komplementärer DNA-Stränge aus einem Nukleinsäuregemisch oder PCR-vermittelten Verfahren (sog. RACE-Protokolle) so verlängert wird, daß es in seiner Sequenz der in dem entsprechenden Zielorgan (Gehirn, Rückenmark, Hinterwurzelganglion) exprimierten mRNA entspricht.
- Bindung an das Peptid oder Protein: Wechselwirkung zwischen Substanz und Peptid oder Protein, die zu Fixierung führt.
- funktionelle Parameter: darunter versteht man Meßgrößen eines Experimentes, die mit der Funktion eines Proteins (lonenkanal, Rezeptor, Enzym) korrelieren
- gentechnisch manipuliert: Manipulation von Zellen, Geweben oder Organismen derart, daß hier genetisches Material eingebracht wird
- endogen exprimiert: Expression eines Proteins, die eine Zelllinie unter geeigneten Kulturbedingungen aufweist, ohne das dieses entsprechende Protein durch gentechnische Manipulation zur Expression veranlasst wurde
- G-Protein: International übliche Abkürzung für ein Gunaosintriphosphat (GTP)-bindendes Protein, das als Signalprotein durch G-Protein gekoppelte Rezeptoren aktiviert wird
- Reportergen: Generelle Bezeichnung für Gene, deren Genprodukte sich mit Hilfe einfacher biochemischer Methoden oder histochemischer Methoden einfach nachweisen lassen, wie z.B. Luziferase, alkalische Phosphatase oder Green Fluorescent Protein (GFP).
- (rekombinantes) DNA-Konstrukt: Generelle Bezeichnung für jede Art von DNA-Molekülen, die durch die in vitro-Verknüpfung von DNA-Molekülen entstanden sind.
- Klonierunasvektor: Generelle Bezeichnung für Nukleinsäure-Moleküle, die beim Klonieren als Träger von Fremdgenen oder Teilen dieser Gene dienen.
- Expressionsvektor: Bezeichnung für speziell konstruierte Klonierungsvektoren, die nach Einbringen in eine geeignete Wirtszelle die Transcription und Translation des in den Vektor einklonierten Fremdgens erlauben.
- LTR-Sequenz: Abkürzung für Long terminal repeat. Generelle Bezeichnung für längere Sequenzbereiche, die an beiden Enden eines linearen Genoms zu finden sind. Derartige Sequenzbereiche kommen z.B. in den Genomen von Retroviren und an den Enden eukaryotischer Transposons vor.
- Poly-.A-Schwanz: die am 3'-Ende von messenger-RNAs durch Polyadenylierung angeheftenen Adenyl-Reste (ca. 20-250).
- Promotor-Sequenz: Bezeichnung für einen DNA-Sequenzbereich, von dem aus die Transkription eines Gens, d.h. die Synthese der mRNA, gesteuert wird.
- ORI-Sequenz: Abkürzung für Origin of replication. Die ORI-Sequenz erlaubt einem DNA-Molekül, sich als autonome Einheit in der Zelle zu vermehren.
- Enhancer-Sequenz: Bezeichung für relativ kurze, zum Teil als Repetitionen auftretende, genetische Elemente, die in der Regel die Expression mancher Gene in unterschiedlichem Maße verstärken.
- Transkriptionsfaktor: Bezeichnung für ein Protein, das über eine Bindung an spezifische DNA-Sequenzen, die Transkription, eines Gens beeinflußt.
- kultivieren: Zellen oder Gewebe unter geeigneten Kulturbedingungen halten
- Bedingungen die eine Expression erlauben, darunter versteht man die Auswahl und Anwendung von Kulturbedingungen die eine Expression des interessierenden Proteins erlauben, darunter gehören Temperaturänderung, Mediumwechsel, Zusatz von induzierenden Substanzen, Weglassen hemmender Substanzen.
- Inkubationszeit: Zeitdauer für die Zellen oder Gewebe inkubiert, d.h. einer definierten Temperatur ausgesetzt werden.
- Selektionsdruck: Anwendungen von Kulturbedingungen die Zellen mit einem bestimmtem Genprodukt, dem sog. Selektionsmarker, einen Wachstumsvorteil verschaffen.
- Amphibienzelle, Zelle aus einem Tier der Klasse der Amphibia
- Bakterienzelle, Zelle die dem Überreich der Eubacteria oder Archaebacteria zuzuordnen ist, oder von ihr abstammt.
- Hefezelle, Zelle die der Ordnung der Endomycetalse zuzuordnen ist, oder von ihr abstammt.
- Insektenzelle, Zelle die der Ordnung der Hexapoda zuzuordnen ist, oder von ihr abstammt.
- native Säugetierzelle, aus einem Säugetier stammende Zelle, die in ihren relevanten Merkmalen der im Organismus befindlichen Zelle' entspricht.
- immortalisierte Säugetierzelle: Zelle die durch die angewendeten Kulturbedingungen oder gentechnische Manipulation die Eigenschaft erlangt hat, sich über die normalerweise übliche Teilungshäufigkeit hinaus (ca. 100) in der Kultur zu teilen.
- markiert: durch entsprechende Modifizierung oder Derivatisierung für eine Nachweisreaktion zugänglich gemacht. Beispielsweise radioaktiv, fluoreszierend oder lumineszierend.
- Ligand: Substanz, die an ein im Körper oder einer Zelle befindliches Molekül, im speziellen einen Rezeptor, bindet
- Verdrängung: vollständiges oder partielles Entfernen eines Liganden von seiner Bindungsstelle
- gebundene Aktivität: Biochemisch oder physikalisch erfaßter Meßwert, der mit der an einem Rezptor gebundenen Ligandenmenge korreliert
- Regulation: die als Teil eines Regelprozesses erfolgte Hemmung oder Aktivierung eines Vorgangs
- Hemmung: als Sonderfall der Regulation die Verhinderung/Minderung eines Vorgangs
- Aktivierung: als Sonderfall der Regulation die Verstärkung eines Vorgangs
- Rezeptoren, Im weitesten Sinne alle im pro- oder eukaryotischen Organismus vorhandenen Moleküle, an die ein Wirkstoff binden kann. Im engeren Sinne membrangebundene Proteine oder Komplexe mehrerer Proteine, die durch Bindung eines Wirkstoffes eine Änderung in der Zelle hervorrufen.
- lonenkanäle: Membrangebundene Proteine oder Komplexe mehrerer Proteine, durch die Kationen oder Anionen durch die Membran hindurchgelangen können.
- Enzyme: Bezeichnung für Proteine oder Komplexe aus einer aktivierenden Nichteiweißkomponente mit einem Protein, die katalytische Eigenschaften besitzen.
- Genexpression (exprimieren/exprimierbar): das Übersetzen der genetischen Information eines Genes in RNA (RNA-Expression) oder in Protein (Proteinexpression).
- Ionenmilieu: Ionenkonzentration eines oder mehrerer Ionen in einem bestimmten Kompartiment.
- Membranpotential: Spannungsdifferenz über eine Membran aufgrund eines Überschusses an Kationen auf der einen Seite und Anionen auf der anderen Seite der Membran.
- Veränderung der Enzymaktivität: Hemmung oder Induktion der katalytischen Aktivität eines Enzyms.
- 2^{nd} messenger: kleines Molekül, das als Antwort auf ein extrazelluläres Signal entweder im Cytosol gebildet wird oder in das Cytosol hineinwandert und dabei hilft die Information an das Zellinnere weiterzugeben, wie zum Beispiel cAMP, IP₃.
- (Gen-)Sonde: Bezeichnung für jede Art von Nukleinsäuren, mit deren Hilfe man ein gesuchtes Gen oder eine bestimmte DNA-Sequenz nachweisen kann. Durch Derivatisierung der Gensonde (z.B. Biotin, magnetische Beads, Digoxinin) können zudem DNA-Moleküle aus einem Gemisch herausgezogen werden. Als Sonden werden klonierte Gene, Genfragmente, chemisch synthetisierte Oligonukleotide und auch. RNA verwendet, die meist radioaktiv markiert ist.
- DNA: Internationale Bezeichnung für Desoxyribonukleinsäure
- genomische DNA: Generelle Bezeichnung für die bei eukaryotischen Organismen aus dem Zellkern einer Zelle stammende DNA.
- cDNA: Abkürzung für complementary DNA. Bezeichnung für die einzel- bzw. doppelsträngige DNA-Kopie eines RNA-Moleküls.
- cDNA-Bank/Bibliothek: Bezeichung für eine Sammlung von willkürlich klonierten cDNA-Fragmenten, die zusammengenommen die Gesamtheit aller von einer Zelle oder einem Gewebe synthetisierten RNA repäsentieren.
- cDNA-Klon: Bezeichnung für eine Population genetisch einheitlicher Zellen, die sich von einer einzigen Zelle ableiten, derart, daß diese Zelle eine künstlich eingebrachtes cDNA-Fragment enthält.
- Hybridisierung: Durch Basenpaarung bewirkte Ausbildung eines doppelsträngigen Nukleinsäuremoleküls aus zwei getrennten Einzelsträngen.
- stringente Bedingungen: Bedingungen, unter denen nur perfekt basengepaarte Nukleinsäure-Stränge gebildet werden und stabil bleiben.
- isolieren: ein gesuchtes Molekül aus einem Gemisch herausfinden und abtrennen.
- DNA-Sequenzierung: Bestimmung der Abfolge der von Basen in einem DNA-Molekül.
- Nukleinsäuresequenz: Bezeichnung für die Primärstruktur eines DNA-Moleküls, d.h. die Abfolge der einzelnen Basen, aus denen sich eine DNA zusammensetzt.
- Genspezifische Oligonukleotid Primer: Oligonukleinsäuren, also 10-40 Basen lange Nukleinsäurefragmente, die in ihrer Basenzusammensetzung eine stringente Hybridisierung an das gesuchte Gen oder die gesuchte cDNA erlauben.
- Ermitteln von Oligonukleotid Primern: Die manuelle oder Computer-unterstützte Suche von Oligonukleotiden zu einer vorgegebenen DNA-Sequenz, die für eine Hybridisierung und/oder eine Polymerase-Ketten Reaktion optimal geeignet sind.
- PCR: Abkürzung für Polymerase-Kettenreaktion. In vitro-Verfahren zur selektiven Anreicherung von Nucleinsäure-Bereichen definierter Länge und definierter Sequenz aus einem Gemisch von NukleinsäureMolekülen.
- DNA-Template: Nukleinsäuremolekül oder ein Gemisch von Nukleinsäuremolekülen, aus denen ein DNA-Abschnitt mit Hilfe der PCR (s.o.) vervielfältigt wird.
- RNA: International gebräuchliche Abkürzung für Ribonukleinsäuren
- mRNA: International gebräuchliche Abkürzung für messenger-Ribonukleinsäuren, die am Transfer der genetischen Information aus dem Kern in die Zelle beteiligt sind und die Information für die Synthese eines Polypetids oder eines Proteins beinhalten.
- Antiserise Polynukleotid: Eine aus mehreren natürlichen oder modifizierten Nukleinsäuren bestehendes Molekül, deren Basenabfolge komplementär zur Basenabfolge eines Teilbereiches einer in der Natur vorkommenden RNA ist.
- PNA: International gebräuchliche Abkürzung für peptidic Nucleic Acids. Hierbei bilden peptidisch verknüpfte Aminosäuren eine Kette, wobei die Aminosäuren als Seitenkette eine für die Hybridisierung mit DNA oder RNA fähigen Base trägt.
- Sequenz: Abfolge von Nukleotiden oder Aminosäuren. Im spezifischen Sinne dieser Erfindung ist damit die Nukleinsäuresequenz gemeint.
- Ribozym: Bezeichnung für eine katalytisch aktive Ribonukleinsäure (z.B. Ligase, Endonuklease, Polymerase, Exonuklease)
- DNA-Enzym: Bezeichnung für ein DNA-Molekül, das katalytische Aktivität beinhaltet (z.B. Ligase, Endonuklease, Polymerase, Exonuklease)
- katalytische RNA/DNA: generelle Bezeichnung für Ribozyme bzw. DNA-Enzyme (s.o.).
- Adenovirus: bei Vertebraten vorkommender cytopathogener Virus
- Adenoassozüertes Virus (AAV): Gehört zur Familie der Parvoviren. Für eine effektive Vermehrung des AAV ist eine Coinfektion der Wirtszellen mit Helferviren (z.B. Herpes-, Vaccina- oder Adenoviren) erforderlich. Die Eigenschaft von AAV, stabil in das Wirtsgenom zu integrieren, macht es als Transduktionsvektor für Säugetierzellen besonders interessant.
- Herpesvirus: Viraler Erreger der Herpes-Infektion
- posttranslationale Modifikation: Veränderung an Proteinen oder Polypetiden, die nach der Translation durchgeführt wird, hierzu zählen z.B. Phosphorylierung, Glykosylierung, Amidierung, Acetylierung oder Proteolyse.
- glykosilieren: Bezeichnung für das Anhängen von einzelnen Zuckermolekülen oder ganzen Zuckerketten an Proteine.
- phosphorylieren: Bezeichnung für das Anhängen von einem oder mehreren Phosphatresten an ein Protein, bevorzugt an die OH-Gruppen der Aminosäuren Serin, Threonin oder Tyrosin.
- amidieren. Die Bezeichnung für das Umwandeln einer Carboxylfunktion in eine Amidfunktion, z.B. an den carboxyterminalen Aminosäurerest eines Peptides oder Proteins.
- mit Membrananker versehen: Posttranslationelle Modifikation eines Proteins, oder eines anderen organischen Moleküls, derart daß es durch Anhängen eines hydrophoben Moleküls, geeigneterweise eine Fettsäure oder ein Derivat derselben, an die Lipiddoppetschicht-Membran von Zellen verankert wird.
- spalten: in diesem spezifischen Fall die Spaltung eines Peptids oder Proteins in mehrere Untersequenzen.
- verkürzen: Ein aus mehreren Einzelteilen bestehendes Molekül um eine oder mehrere Teile zu verkürzen.
- Antikörper: Lösliche, oder an Zellmembranen gebundene, als Immunglobuline bezeichnete Proteine mit einer spezifischen Bindungsstelle für Antigene.
- monoklonaler Antikörper: sind gegen eine einzige antigene Determinante eines Antigens gerichtete Antikörper mit extrem hoher Selektivität
- polyklonaler Antikörper: Gemisch aus Antikörpern, die gegen mehrere Determinanten eines Antigens gerichtet sind.
- transgen: genetisch verändert
- nichthumanes Säugetier: Die Gesamtheit der Säugetiere (Klasse der Mammalia) mit Ausnahme der Spezies Mensch.
- Keim-Zelle: Zelle mit haploidem Genom, die durch Verschmelzung mit einer zweiten Keimzelle die Bildung eines neuen Organismus ermöglicht.
- somatische Zelle: diploide Zelle als Bestandteil eines Organismus
- chromosomale Einbringung: Eingriff in die Nukleotidsequenz auf chromosomaler Ebene
- Genom: Allgemeine Beschreibung für die Gesamtheit aller Gene in einem Organismus
- Vorfahr des Tieres: Ein Tier (der Vorfahr), das auf natürliche oder künstliche Weise durch Weitergabe an seinem genetischen Material in direkter Linie mit einem anderen Tier (dem Nachfahren) verwandt ist.
- exprimierbar: Ein Nukleinsäuremolekül ist dann exprimierbar, wenn es die Information zur Synthese eines Proteins oder Polypetids beinhaltet und mit ensprechenden regulatorischen Sequenzen versehen ist, die eine Synthese dieses Proteins oder Polypeptids in vitro oder in vivo erlauben. Wenn diese Voraussetzungen nicht mehr gegeben sind, beispielsweise durch Eingriff in die kodierende Sequenz, ist das Nukleinsäuremolekül nicht mehr exprimierbar.
- Nagetier: Tier aus der Ordnung der Rodentia, z.B. Ratte oder Maus
- als schmerzregulierende Substanz identifizierbar: Substanz die bei Einbringung in einen lebenden Organismus eine Verhaltensänderung bewirkt, die der Fachmann als schmerzhemmend bezeichnet (antinozizeptiv, antihyperalgetisch oder antiallodynisch). Im Falle des Screeningverfahrens bezieht sich das darauf, daß die Substanz beim Screening durch stärkere Bindung oder Auslösung einer Änderung eines funktionellen Parameters deutlich, beispielsweise 100 %, die Bindung oder Wechselwirkung des Durchschnitts der getesteten Substanzen übertrifft.
- Verbindung: ein anderer Name für Molekül, als aus mehreren Atomen bestehend, hier ein durch das erfindungsgemäße Verfahren identifiziertes Molekül.
- Wirkstoff: Eine Verbindung, die bei Anwendung an einem Organismus eine Veränderung in diesem Organismus hervorruft. Im Besonderen werden darunter organisch-chemisch synthetisierte Moleküle verstanden, die auf den Organismus eine heilende Wirkung ausüben. Hier insbesondere Moleküle, die an die erfindungsgemäßen Proteine und Peptide binden.
- niedermolekular: Molekül mit einem Molekutargewicht < 2kDa
- Arzneimittel: ein Stoff entsprechend der Definition im Artikel 1 §2 des Gesetzes über den Verkehr mit Arzneimitteln
- Diagnostikum: Verbindung oder Verfahren, das verwendet werden kann, um eine Krankheit zu diagnostizieren
- Behandlung von Schmerz: Verfahren, mit dem Ziel Schmerzen zu lindern oder aufzuheben, oder das zu erwartende Auftreten von Schmerzen zu hemmen (preemptive Analgesie).
- chronischer Schmerz: eine Schmerzempfindung von länger anhaltender Dauer, oft dadurch gekennzeichnet, daß sie über Zeitpunkt und Ort des initialen Stimulus hinausreicht, die Schmerzempfindlichkeit des Körpers steigert.
- Gentherapie: Unter Gentherapie versteht man alle Verfahren, die das Ziel haben, genetische Erkrankungen durch geeignete Veränderungen des Genoms kausal zu behandeln.
- In-vivo-Gentherapie: Einbringen von genetischem Material in den lebenden Organismus mit dem Ziel der Gentherapie. Man kann zwischen somatischem und Keimbahn-Eingriff unterscheiden, der einmal an diploiden Zellen und einmal an haploiden Zellen stattfindet.
- In-vitro-Gentherapie: Einbringen von genetischem Material in Zellen außerhalb des menschlichen Körpers mit dem Ziel diese nachher wieder durch Einbringen in den menschlichen Körper zur Gentherapie zur verwenden.
- Diagnostik: Verfahren, um eine Krankheit zu identifizieren.
- Wirksamkeitsuntersuchung: Untersuchung mit dem Ziel die Wirksamkeit einer Verbindung nach Einwirkung auf einen lebenden Organismus zu untersuchen.

In einer bevorzugten Ausführungsform des Verfahrens wird die Zelle vor dem Schritt (a) gentechnisch manipuliert. Dabei wird genetisches Material in die Zelle eingebracht, insbesondere eine oder mehrere Polynukleotidsequenzen. In einer weiter bevorzugten Variante dieser Ausführungsform erlaubt die gentechnische Manipulation die Messung mindestens eines der durch die Testsubstanz veränderten funktionellen Parameter. In dieser Ausführungsform werden durch gentechnische Manipulation Voraussetzungen geschaffen unter denen die Veränderung eines funktionellen Parameters überhaupt oder verbessert gemessen werden kann. Dabei ist es insbesondere bevorzugt, daß durch die gentechnische Manipulion eine in der Zelle nicht endogen exprimierte Form eines G-Proteins exprimiert oder ein Reportergen eingeführt wird. Darunter ist insbesondere die gentechnische Einführung eines endogen nicht vorhandenen oder physiologisch nicht exprimierten G-Proteins (GTPbindenden Proteins) in die Zelle zu verstehen, beispielsweise die Einführung eines chimären G-Proteins, das eine Veränderung des Signalweges erlaubt oder eines promiskuitiven G-Proteins, das sehr bindungsfreudig ist. Die Einführung eines Reportergens wiederum erlaubt die Messung einer (extrazellulär ausgelösten) induzierten Expression des Genproduktes.

In einer weiteren bevorzugten Ausführungsform wird die Zelle gentechnisch so manipuliert, daß die Zelle mindestens ein Polynukleotid gemäß einer der Abbildungen 35a), 35c) oder 35e), oder ein dazu zu mindestens 90 % ähnliches Polynukleotid enthält. Damit kann beispielsweise erreicht werden, daß ein Peptid oder Protein, das in der im Verfahren verwendeten Zelle oder Präparation nicht endogen exprimiert wird, von der Zelle synthetisiert wird. Dabei ist es insbesondere bevorzugt, wenn das Polynukleotid in einem rekombinanten DNA-Konstrukt enthalten ist. Unter einem (rekombinanten) DNA-Konstrukt versteht man ein in-vitro hergestelltes DNA-Molekül.

Wenn beim Verfahren vor dem Schritt a) die Zelle gentechnisch manipuliert wird, ist es bevorzugt, daß die Zelle nach der gentechnischen Manipulation und vor dem Schritt (a) unter Bedingungen, die eine Expression erlauben, kultiviert wird, gegebenenfalls unter Selektionsdruck. Unter kultivieren versteht man, Zellen oder Gewebe bei Bedingungen, die ein Überleben der Zellen, bzw. deren Nachfolgegeneration sichern, zu halten. Dabei sollten die Bedingungen hier so gewählt werden, daß eine Expression des durch die gentechnische Manipulation eingefügten Materials ermöglicht wird. Dazu sollten pH, Sauerstoffgehalt, und Temperatur physiologisch gehalten sein und ausreichend Nährstoffe und notwendige Cofaktoren beigefügt sein. Der Selektionsdruck erlaubt, nur die Zellen weiter zu kultivieren, bei denen die gentechnische Manipulation zumindest teilweise erfolgreich war. Dazu gehört beispielsweise die Einführung einer Antibiotikaresistenz über das DNA-Konstrukt.

Es ist bei dem erfindungsgemäßen Verfahren besonders bevorzugt, wenn die verwendete Zelle eine Amphibienzelle, Bakterienzelle, Hefezelle, Insektenzelle oder eine immortalisierte oder native Säugetierzelle ist. Beispiele für Amphibienzellen sind Xenopus Oocyten, für Bakterienzellen E-coli-Zellen, für Hefezellen Saccharomyces cerevisiae, für Insektenzellen Sf9-Zellen, für immortalisierte Säugetierzelle HeLa-Zellen und für native Säugetierzellen die CHO (Chinese Hamster Ovary)-Zelle.

Bei einer bevorzugten Meßmethode zur Feststellung der Bindung der Substanz an Peptid oder Protein im erfindungsgemäßen Verfahren erfolgt die Messung der Bindung über die Verdrängung eines bekannten markierten Liganden vom Peptid oder Protein und/oder über die daran gebundene Aktivität einer markierten Testsubstanz. Dabei ist ein Ligand ein mit hoher Spezifität an das Protein oder Peptid bindendes Molekül, das durch eine ebenfalls bindende, zu testende Substanz aus der Bindungsstelle verdrängt wird. Unter Markierung ist eine den Nachweis erleichternde künstliche Modifikation am Molekül zu verstehen. Beispiele sind radioaktive, fluoreszierende oder lumineszierende Markierungen.

Bei einer anderen bevorzugten Meßmethode zur Feststellung der durch die Bindung der Substanz an Peptid oder Protein im erfindungsgemäßen Verfahren ausgelösten Veränderung der funktionellen Parameter, erfolgt die Messung mindestens eines der durch die Testsubstanz veränderten funktionellen Parameter über Messung der Regulation, Hemmung und/oder Aktivierung von Rezeptoren, lonenkanälen und/oder Enzymen, insbesondere über Messung der Veränderung der Genexpression, des lonenmilieus, des pH oder des Membranpotentials, über Veränderung der Enzymaktivität oder der Konzentration der 2^{nd} messenger. Damit ist auf der einen Seite direkt die Messung der Wirkung der Substanz über die Beeinflußung von Rezeptoren, lonenkanälen und/oder Enzymen erfaßt, auf der anderen Seite als bevorzugt zu messende Beispiele sich ändernder Parameter wie Genexpression, lonenmilieu, pH, Membranpotential, Enzymaktivität oder Konzentration der 2^{nd} messenger. Dabei versteht man unter lonenmilieu insbesondere die Konzentration eines oder mehrer Ionen in einem Zellkompartiment, insbesondere dem Cytosol, unter Membranpotential die Ladungsdiffferenz zwischen zwei Seiten einer Biomembran und unter 2^{nd} messenger Botenstoffe des intrazellulären Signalwegs wie z.B. zyklisches AMP (cAMP), Inosotoltriphosphat (IP3) oder Diacylglycerol (DAG).

Im Verfahren wird das Peptid oder Protein in den Schritten (a) und (b) aus folgenden Gruppen ausgewählt:
- ein Peptid oder Protein, für das oder für einen Teil dessen ein Polynukleotid gemäß einer der Abbildungen 35a), 35c) oder 35e), oder ein dazu zu mindestens 95 %, vorzugsweise 97%, ähnliches Polynukleotid kodiert, oder
- ein Peptid oder Protein mit einer Aminosäuresequenz gemäß einer der Abbildungen 35b), 35d) oder 35f) oder ein dazu zu mindestens 95 %, vorzugsweise 97%, ähnliches Peptid oder Protein
- und/oder ein Protein, das durch eine Nukleinsäure - kodiert wird, die unter stringenten Bedingungen an ein Polynukleotid gemäß einer der Abbildungen 35a), 35c), 35e) oder deren Antisense Polynukleotid bindet.

Darunter erfaßt ist die Verwendung von Peptiden und insbesondere Proteinen mit bekannter Sequenz und Funktion, ohne daß für diese im Stand der Technik eine Funktion im Schmerz bekannt war.

Hybridisierung bedeutet die Bindung zweier einzelsträngiger Nukleinsäuremoleküle, wobei das Waschen unter stringenten Bedingungen gewährleisten soll, daß nur über exakt gepaarte Basen hybridisierte Nukleinsäuremoleküle gebunden bleiben.

Es ist weiter bevorzugt, daß es sich bei dem erfindungsgemäß verwendeten Polynukleotid um RNA bzw. ein- oder doppelstängige DNA, insbesondere mRNA oder cDNA handelt.

Ebenso ist es bevorzugt, daß es sich bei dem verwendeten Polynukleotid um ein Antisense-Polynukleotid oder PNA handelt, das eine Sequenz aufweist, die in der Lage ist, spezifisch an ein erfindungsgemäßes Polynukleotid zu binden. Dabei versteht man unter PNA "peptidic nucleic acid" (peptidische Nukleinsäure), die zwar die Basenpaare trägt, aber dessen Rückrat peptidisch gebunden ist. Ein Antisense-Polynukleotid zeigt die komplementäre Basenabfolge zu mindestens einem Teil einer Basis-Nukleinsäure. Ebenfalls bevorzugt ist es, daß das verwendete Polynukleotid Teil eines Ribozym oder sonstigen DNA-Enzyms oder einer katalytischen RNA bzw. DNA ist. Unter Ribozym ist eine katalytisch aktive Ribonukleinsäure zu verstehen, unter DNA-Enzym ein entsprechende Desoxyribonukleinsäure, also katalytische RNA beziehungsweise DNA.

Es ist auch Gegenstand dieser Erfindung, wenn das verwendete Peptid oder Protein posttransiational modifiziert wurde, es insbesondere glykosiliert, phosphoryliert, amidiert, methyliert, acetyliert, ADP-ribosyliert, hydroxyliert, mit einem Membrananker versehen, gespalten oder verkürzt wurde. Posttranslationale Modifikationen sind beispielsweise dem Voet/Voet, Biochemistry, 1^{st} Edition, 1990, S. 935-938 zu entnehmen.

Eine durch ein erfindungsgemäßes Verfahren aufgefund Verbindung als schmerzregutierende Substanz identifizierbar. Hierbei bezieht sich Verbindung insbesondere auf niedermolekulare Wirkstoffe, aber auch auf Peptide, Proteine und Nukleinsäuren. Dabei bedeutet identifizierbar, daß die Verbindung das Merkmal aufweist, daß es beim erfindungsgemäßen Screeningverfahren bezüglich der Bindung deutlich stärker, vorzugsweise doppelt so stark bindet wie der Durchschnitt der zu testenden Substanzen oder bezüglich der Änderung der funktionellen Parameter deutlich vom Durchschnitt der zu testenden Substanzen abweicht.

Ein weiterer Gegenstand der Erfindung ist die Verwendung eines Peptids oder Proteins ausgewählt aus einer der folgenden Gruppen:
- PIM-2,
- einem Peptid oder Protein, für das oder für einen Teil dessen ein Polynukleotid gemäß einer der Abbildungen (35a), 35c) oder 35e), oder ein dazu zu mindestens 90 % ähnliches Polynukleotid kodiert,
- einem Peptid oder Protein mit einer Aminosäuresequenz gemäß einer der Abbildungen 35b), 35d) oder 35f), oder ein dazu zu mindestens 90 % ähnliches Peptid oder Protein
- und/oder einem Protein, das durch eine Nukleinsäure kodiert wird, die unter stringenten Bedingungen an ein Polynukleotid gemäß einer der Abbildungen 35a), 35c) oder 35e), oder deren Antisense Polynukleotid bindet,
in einem Verfahren zur Auffindung schmerzregulierender Substanzen.

Insgesamt ist eine wichtige Grundlage der Erfindung die Identifizierung schmerzregulierter Gene und Genfragmente. Darauf basiert das Screeningverfahren.

### 1. Therapie chronischer Schmerzen

Die Sequenzen wurden aus Rückenmarksgewebe isoliert. Im Rückenmark projizieren die primären sensorischen Neuronen auf nachgeschaltete zentralnervöse Neurone, es handelt sich hierbei neben supraspinalen Vorgängen um die zentrale Umschaltstelle für nozizeptive Information. Zahlreiche Experimente konnten zeigen, daß der Entwicklung chronischer Schmerzzustände plastische Veränderungen des Nervensystems zugrundeliegen (als Überblick siehe Corderre et al., 1993; Zimmermann und Herdegen, 1996). Insbesondere in den Neuronen der dorsalen Wurzelganglien und des Rückenmarks sind plastische Veränderungen beschrieben worden, die mit der Regulation schmerzrelevanter Gene einhergeht. So ist für eine Reihe von Neurotransmitter-Rezeptoren, die für die Schmerztherapie von Bedeutung sind, eine Gen-Regulation im Rückenmark beschrieben worden (siehe Tabelle 1). Auf dieser Grundlage könnten die gefundenenen, unter Schmerz regulierten cDNA-Sequenzen zur Therapie (Gentherapie, Antisense, Ribozyme) und Diagnose chronischer Schmerzzustände verwendet werden.

### 1.1 Antisense-Strategien

Hierbei werden, abgeleitet von der Nukleinsäuresequenz der vollständigen cDNA oder von Teilbereichen Konstrukte erstellt, die die mRNA oder Proteinkonzentration herabsetzen können. Dies können z.B. antisense-Oligonukleotide (DNA oder RNA) sein, die eventuell unter Verwendung modifizierter Nukleotidbausteine (z.B. O-Altyl-Ribose) eine erhöhte Stabilität gegenüber Nukleasen aufweisen. Zudem ist die Verwendung von Ribozymen denkbar, die als enzymatisch aktive RNA-Moleküle eine spezifische Spaltung der RNA katalysieren. Daneben könnten auch Vektoren eingesetzt werden, die die erfindungsgemäß verwendeten Sequenzen oder Teilbereiche dieser Nukleotidsequenzen unter Kontrolle eines geeigneten Promotors exprimieren und somit für eine in-vivo oder ex-vivo Therapie geeignet sind. Zusätzlich sind auch Antisense-Konstrukte möglich, die unter Austausch des Phosphatrückgrats von Nukleotidsequenzen (z.B. PNAs, d.h. Peptide Nucleic Acids) oder Verwendung nichttraditioneller Basen wie Inosine, Queosine oder Wybutosine sowohl wie Acetyl-, Methyl-, Thio- und ähnlich modifizierte Formen von Adenin, Cytidin, Guanosin u, Thymidin und Uridin nicht oder in geringerem Masse durch endogene Nukleasen abgebaut werden können.

### 1.2. Antagonisten/ Agonisten bzw. Inhibitoren/Aktivatoren der von den erfindungsgemäßen Nukleotidsequenzen kodierten Genprodukte.

Dies umfaßt Substanzen, die durch eine Bindung an das Genprodukt dessen Funktion verändern: Dies können sein:
1.2.1. Organisch-chemische Moleküle, die im Rahmen eines Wirkstoffscreenings unter Verwendung der Genprodukte der erfindungsgemäßen cDNA ats Bindungspartner gefunden werden.
1.2.2. Antikörper, seien es polyklonale, chimäre, single-chain, F_{ab-}Fragmente oder Fragmente aus Phagen-Banken, die bevorzugt als neutralisierende Antikörper über eine Bindung an die Genprodukte spezifisch die Funktion beeinflussen.
1.2.3. Aptamere, d.h. Nukleinsäuren oder Nukleinsäurederivate mit Proteinbindenden Eigenschaften. Dazu gehören auch sog. Spiegelmere, die durch Spiegelevolution gewonnene spiegelbildliche und damit stabile Oligonukleotide darstellen, die hochaffin und hochspezifisch ein Zielmolekül binden können (Klußmann et al., 1996).

### 1.3. Gentherapie

Die beschriebenen Sequenzen können zur Therapie neurologischer Erkrankungen insbesondere chronischer Schmerzustände eingesetzt werden, indem sie nach Klonierung in geeignete Vektoren (z. B. Adenovirus-Vektoren oder adeno-assozierter Virus-Vektoren) zur in vivo oder ex-vivo Therapie verwendet werden, um dort z.B. einer Überexpression oder Unterexpression des endogenen Genproduktes entgegenzusteuem, die Sequenz des defekten Genproduktes zu korrigieren (z. B. durch Transsplicing mit dem exogenen Konstrukt) oder ein funktionelles Genprodukt zur Verfügung zu stellen.

### 2. Diagnose

Polynukleotidsequenzen (Oligonukleotide, antisense -DNA & RNA-Moleküle, PNAs), die von den erfindungsgemäßen Nukleotidsequenzen abgeleitet sind, könnten zur Diagnose von Zuständen oder Erkrankungen eingesetzt werden, die mit einer Expression dieser Gensequenzen assoziiert sind. Beispiele dieser Zustände oder Erkrankungen beinhalten neurologische Erkrankungen inklusive chronischer Schmerzen oder neuropathischer Schmerzen (hervorgerufen z.B. durch Diabetes, Krebs oder AIDS) oder neurodegenerativer Erkrankungen wie Alzheimer, Parkinson, Chorea Huntington, Jacob-Creutzfeld, amyotrophe Lateralsklerose und Demenzen. Die Nukleotidsequenzen können auf vielfältige Weise (Northemblot, Southernblot, FISH-Analyse, PRINS-Analyse, PCR) entweder zur Identifizierung der Genproduktes oder abweichender diagnostisch relevanter Genprodukte oder zur Quantifizierung des Genproduktes dienen. Neben der Nukleinsäurediagnostik können auch Antikörper oder Aptamere gegen das von den erfindungsgemäßen Nukleinsäuren kodierte Protein zur Diagnostik eingesetzt werden (z. B. mittels ELISA, RIA, immuncytochemische oder immunhistochemische Verfahren), um das Protein oder abweichende Formen zu identifizieren und das Protein zu quantifizieren.
Im Hinblick auf eine Gendiagnostik könnten Nukleinsäure-Sonden abgeleitet von den erfindungsgemäßen Nukleotidsequenzen zur Bestimmung des Gen-Lokus eingesetzt werden (z.B. durch FISH, FACS. artifizielle Chromosomen wie YACs, BACs oder P1-Konstrukte).

Die folgenden Beispiele und Abbildungen sollen die Erfindung erläutern, ohne sie darauf zu beschränken.

### Abbildungen und Beispiele

**Abbildungen:**

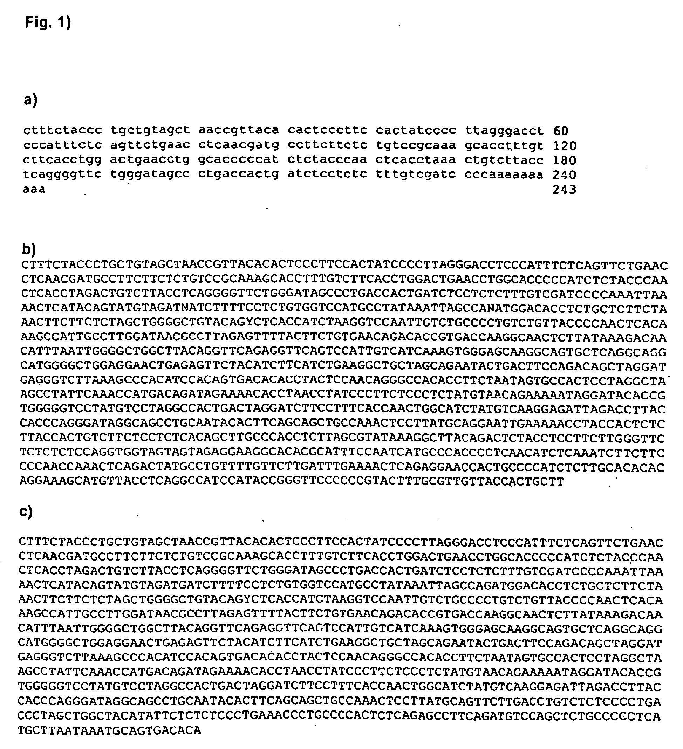

- Fig. 1): Genfragment 111_16 [a) ursprünglich sequenziertes Genfragment aus DDRT-PCR, b) entsprechendes 1. verlängertes Genfragment, c) entsprechendes 2. verlängertes Genfragment]

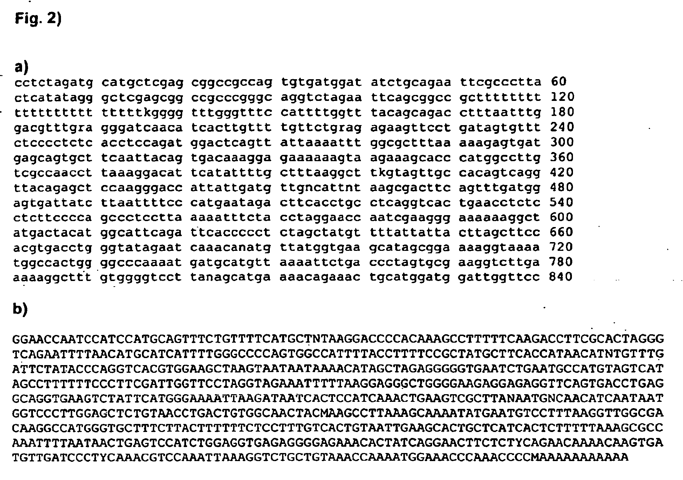

- Fig. 2): Genfragment 111_33 [a) ursprünglich sequenziertes Genfragment aus DDRT-PCR, b) entsprechendes neues Genfragment]

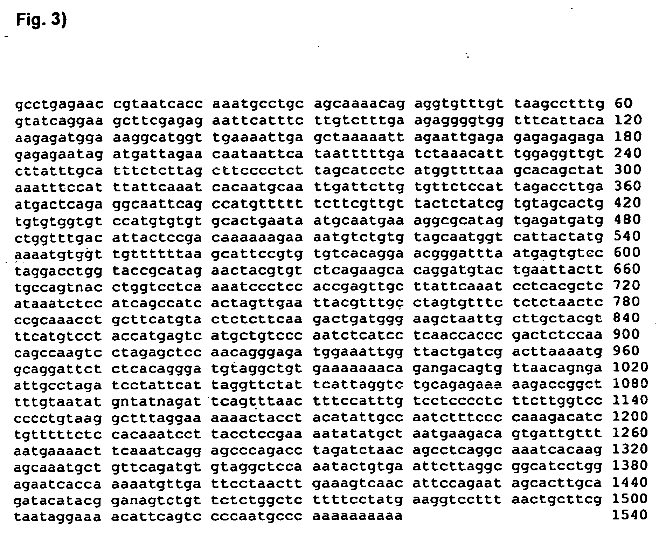

- Fig. 3): Genfragment 111_39

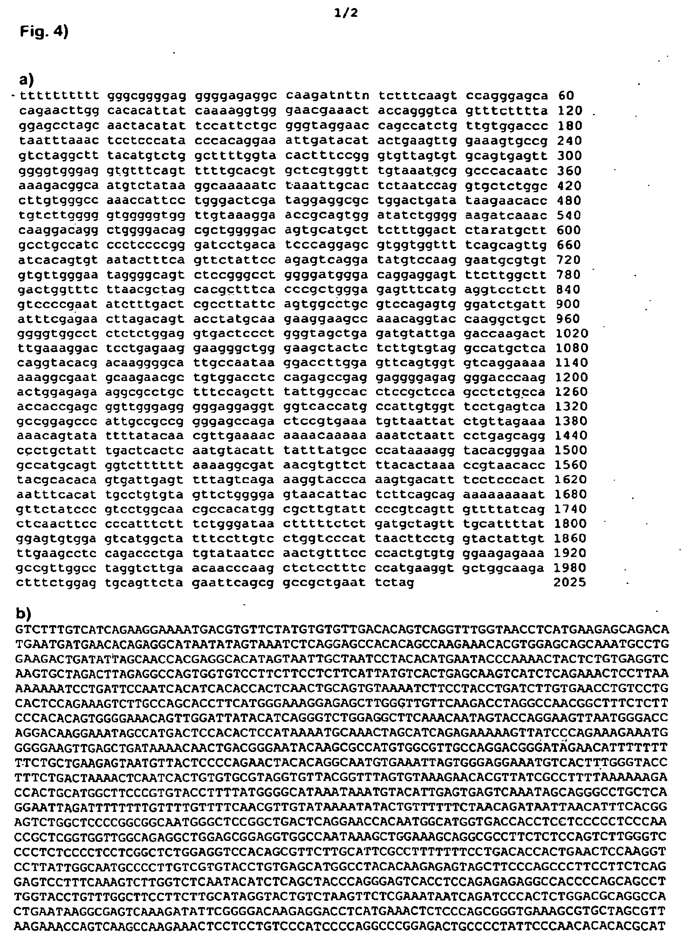

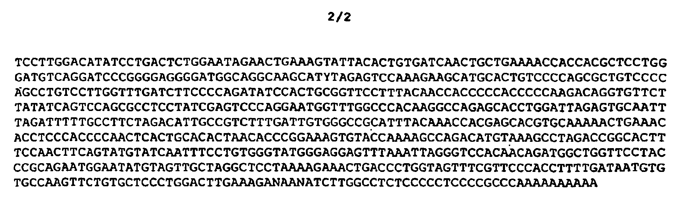

- Fig. 4): Genfragment 111_45 [a) ursprünglich sequenziertes Genfragment aus DDRT-PCR, b) entsprechendes verlängertes Genfragment]

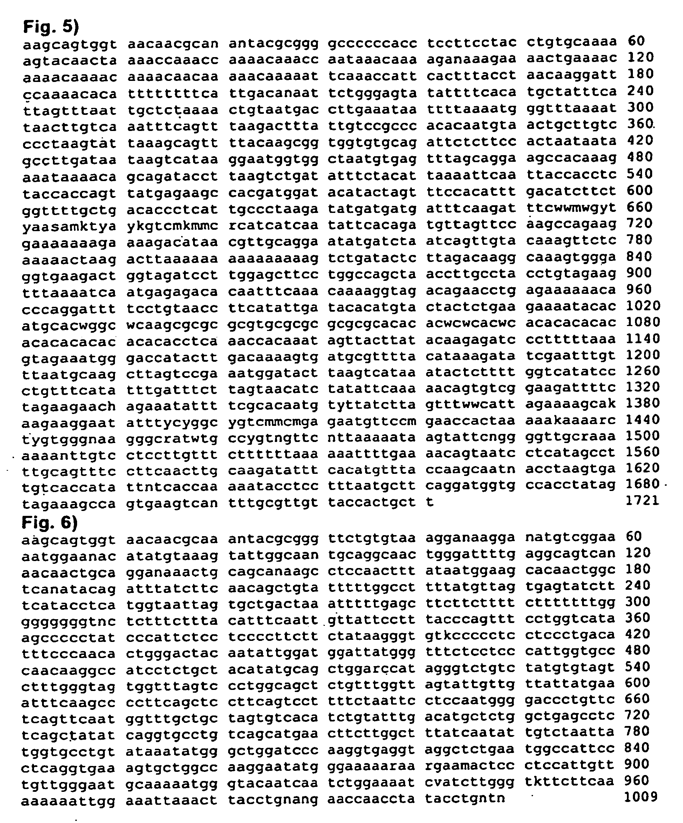

- Fig. 5): Teil des Genfragments 113_15

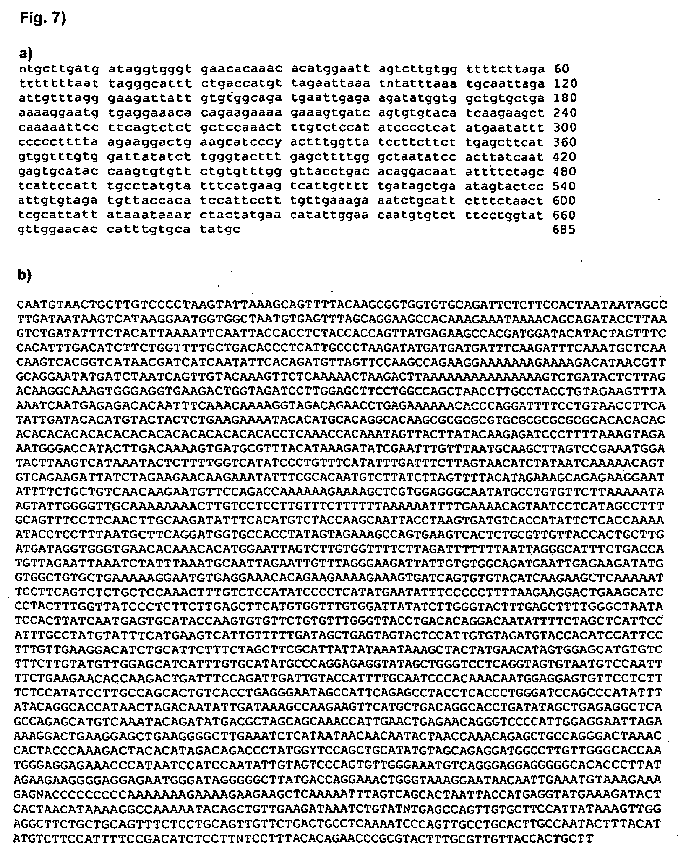

- Fig. 6): Teil des Genfragments 113_15
- Fig. 7): Teil des Genfragments 113_15 [a) ursprünglich sequenziertes Genfragment aus ODRT-PCR, b) entsprechendes verlängertes Genfragment]

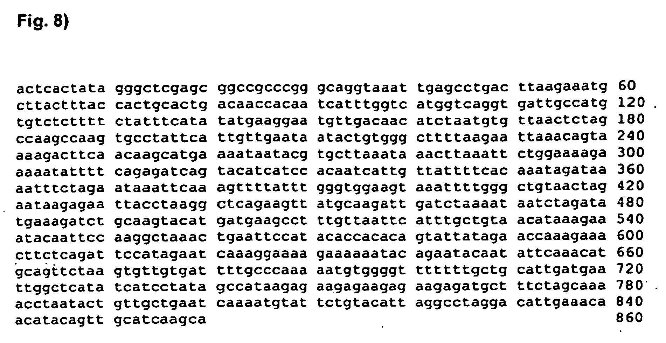

- Fig. 8): Genfragment 113_20

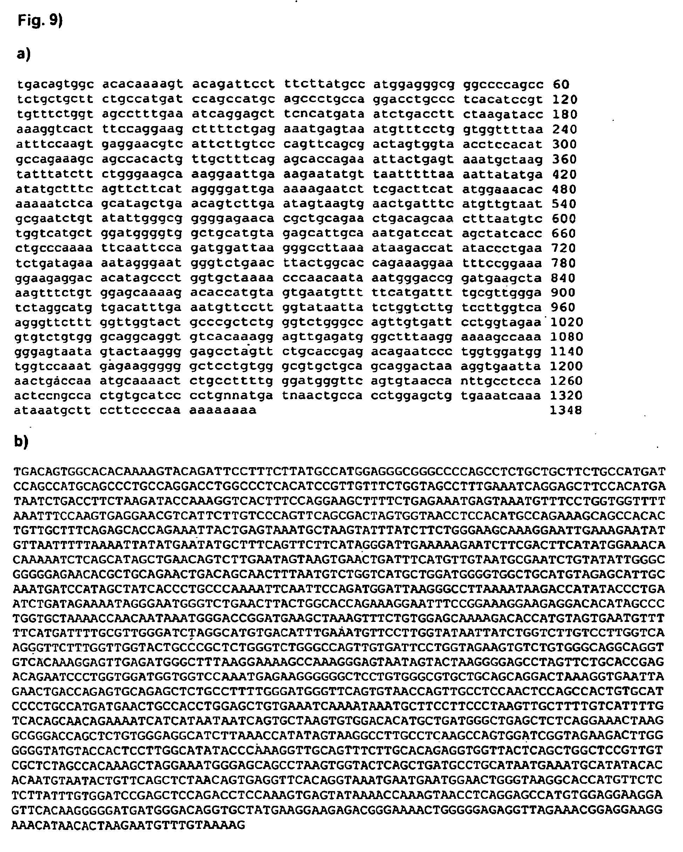

- Fig. 9): Genfragment 124_29 [a) ursprünglich sequenziertes Genfragment aus DDRT-PCR, b) entsprechendes verlängertes Genfragment]

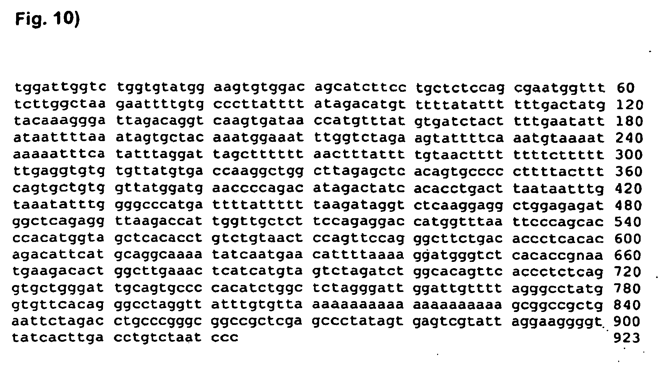

- Fig. 10): Genfragment 124_4

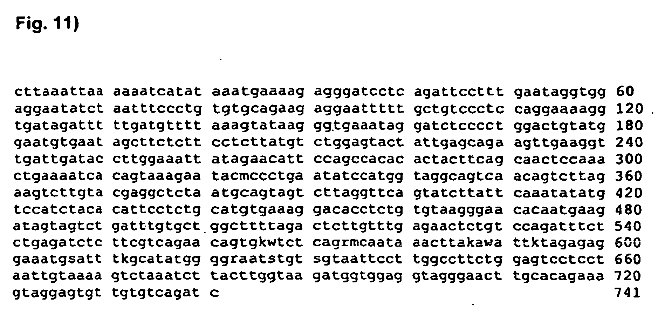

- Fig. 11): Genfragment 127_3

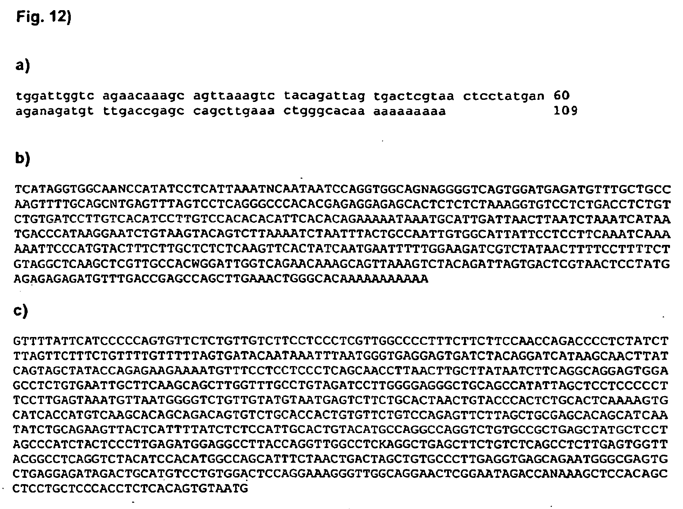

- Fig. 12): Genfragment 127_34 [a) ursprünglich sequenziertes Genfragment aus DDRT-PCR, b) entsprechendes 1. verlängertes Genfragment, c) entsprechendes 2. verlängertes Genfragment]

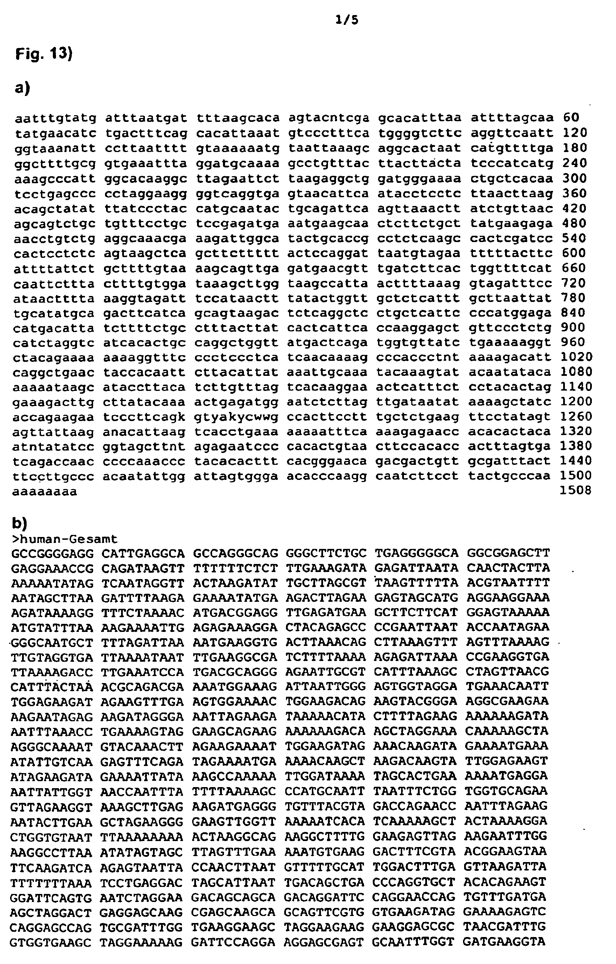

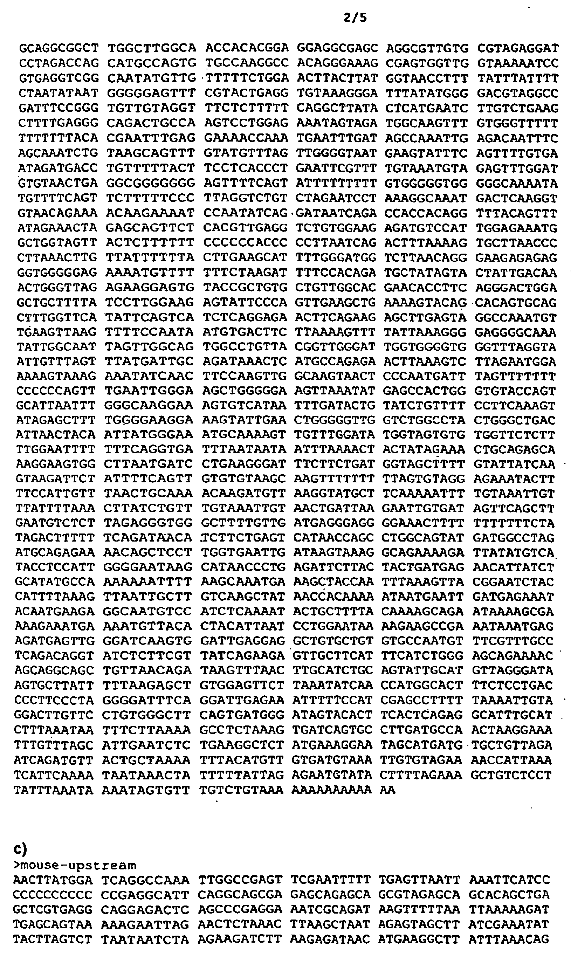

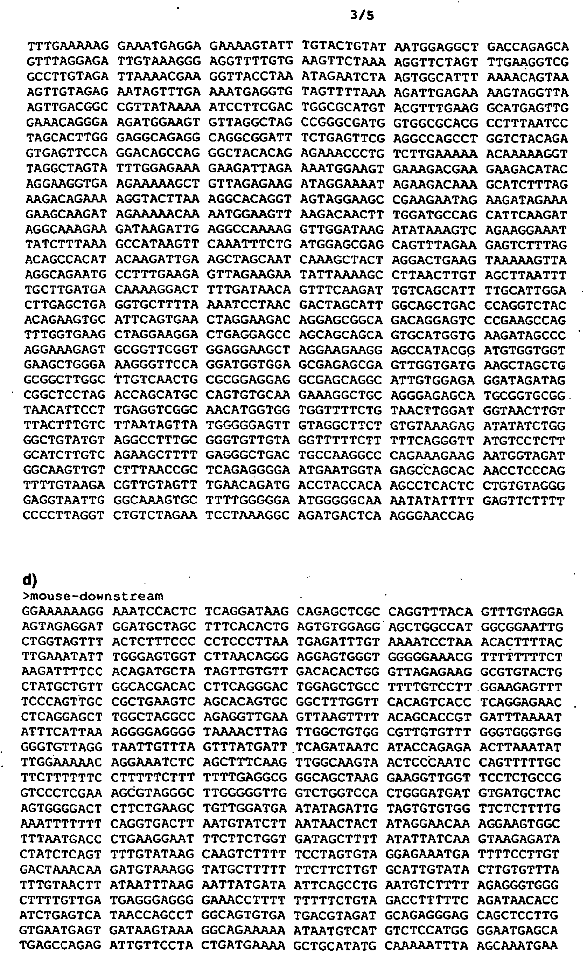

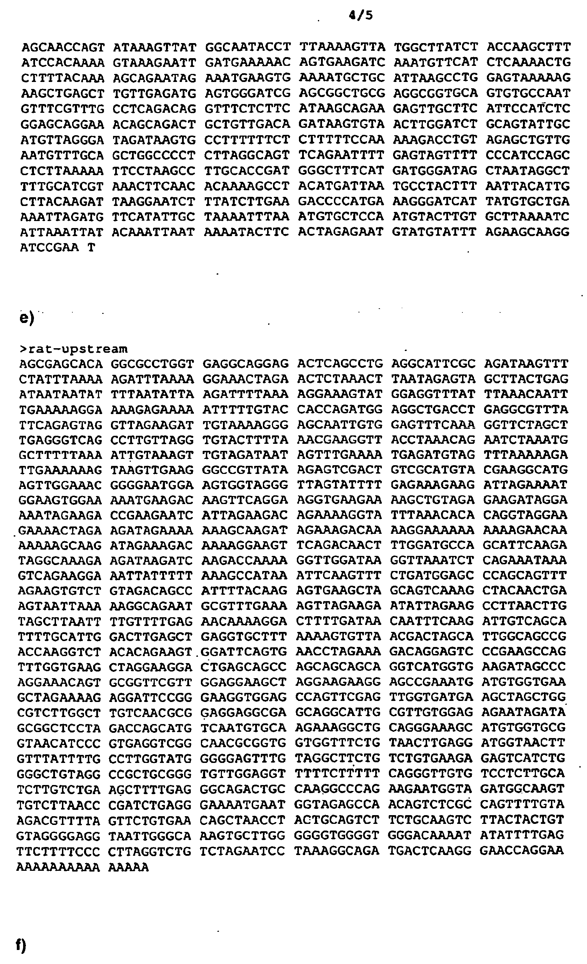

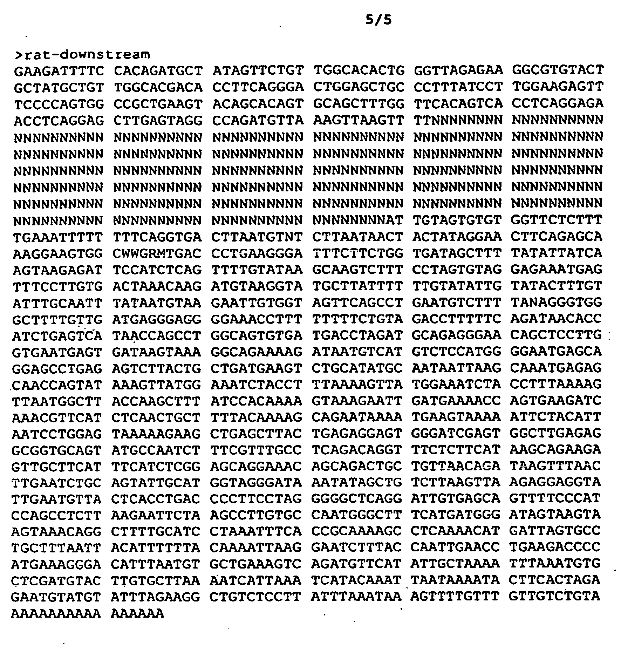

- Fig. 13): Genfragment 127_8 [a) ursprünglich sequenziertes Genfragment aus DDRT-PCR, b) entsprechende umfangreichere humane Nukleotidsequenz (RUP), c) upstream Teil der entsprechenden Maus Nukleotidsequenz,d) downstream Teil der entsprechenden Maus Nukleotidsequenz, e) upstream Teil der entsprechenden Ratten Nukleotidsequenz, f) downstream Teil der entsprechenden Ratten Nuklleotidsequenz].

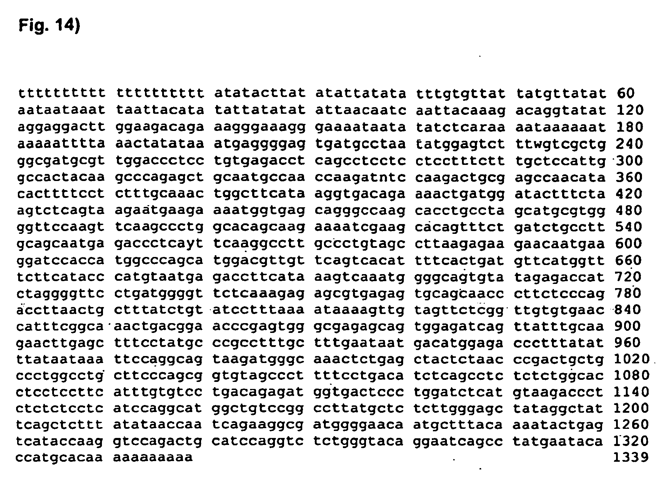

- Fig. 14): Genfragment 135_18

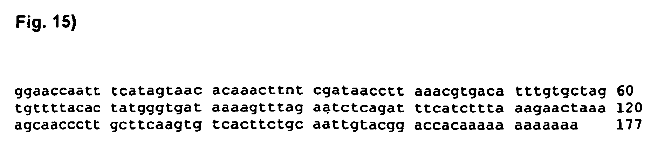

- Fig. 15): Genfragment 135_2

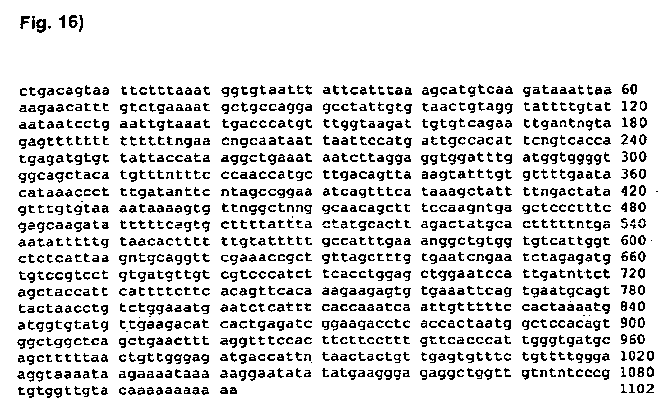

- Fig. 16): Genfragment 135_7

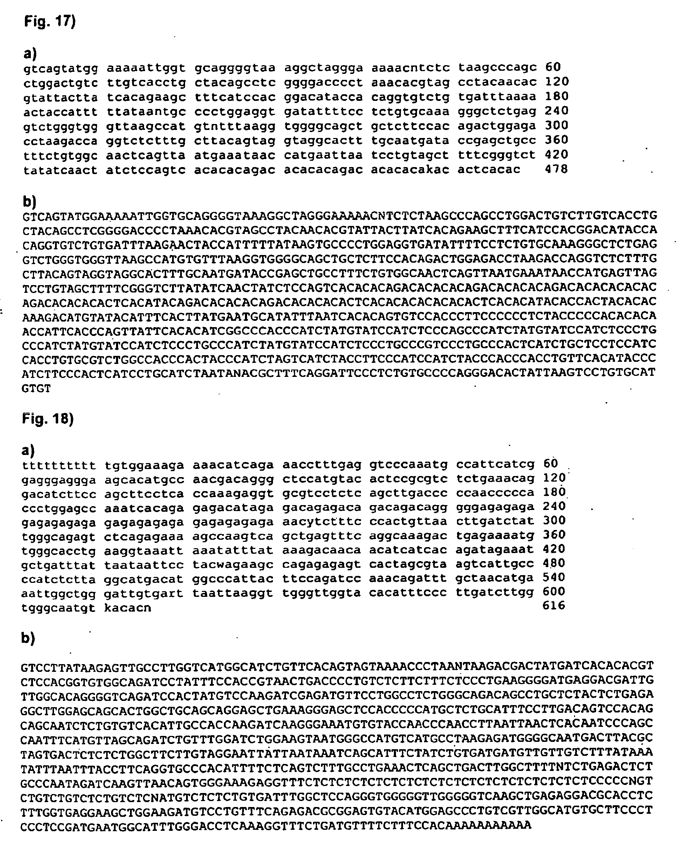

- Fig. 17): Teil des Genfragments 135_9 [a) ursprünglich sequenziertes Genfragment aus DDRT-PCR, b) entsprechendes verlängertes Genfragment]
- Fig. 18): Teil des Genfragments 135_9 [a) ursprünglich sequenziertes, Genfragment aus DDRT-PCR, b) entsprechendes verlängertes Genfragment]

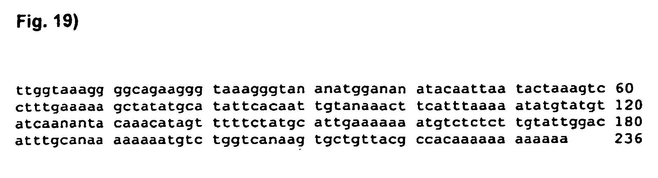

- Fig. 19): Genfragment 141_1
- Fig. 20): Teil des Genfragments 141_13 [a) ursprünglich sequenziertes Genfragment aus DDRT-PCR, b) entsprechende umfangreichere humane Nukleotidsequenz (verwandt mit ABLIM und KIAA 0843), c) entsprechende Maus Nukleotidsequenz, d) entsprechende Ratten Nuklieotidsequenz].
- Fig. 21): Teil des Genfragments 141_13 [a) ursprünglich sequenziertes Genfragment aus DDRT-PCR, b) entsprechendes verlängertes Genfragment]

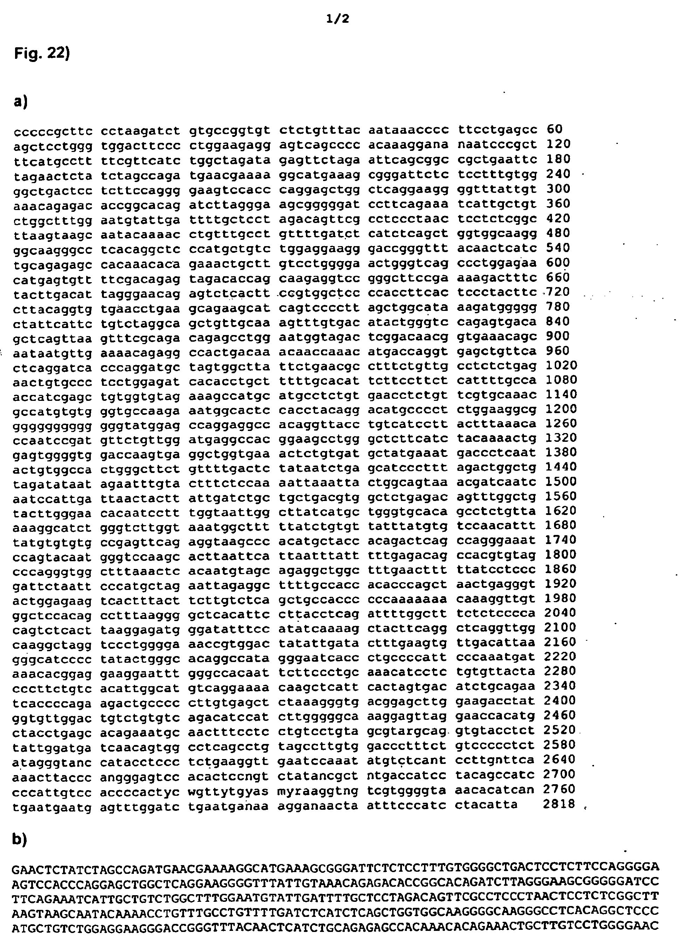

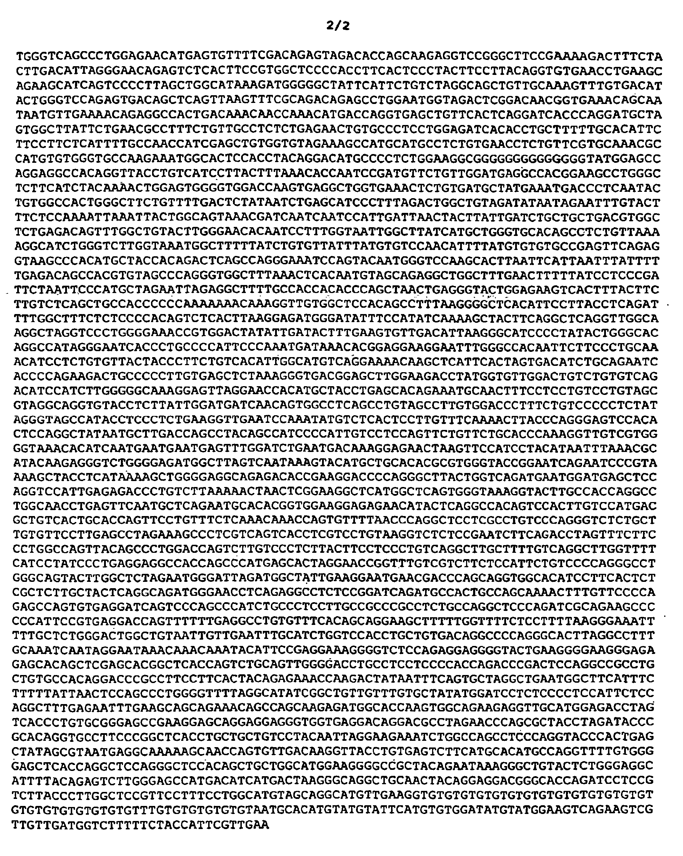

- Fig. 22): Genfragment 141_24 [a) ursprünglich sequenziertes Genfragment aus DDRT-PCR, b) entsprechendes verlängertes Genfragment]

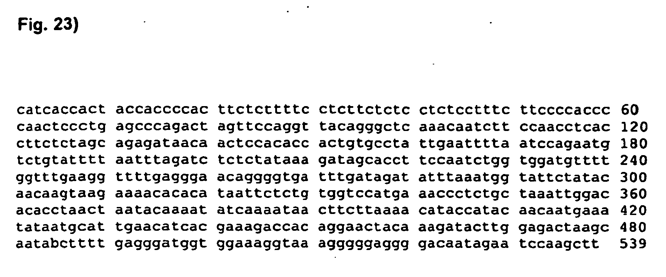

- Fig. 23): Genfragment 141_6

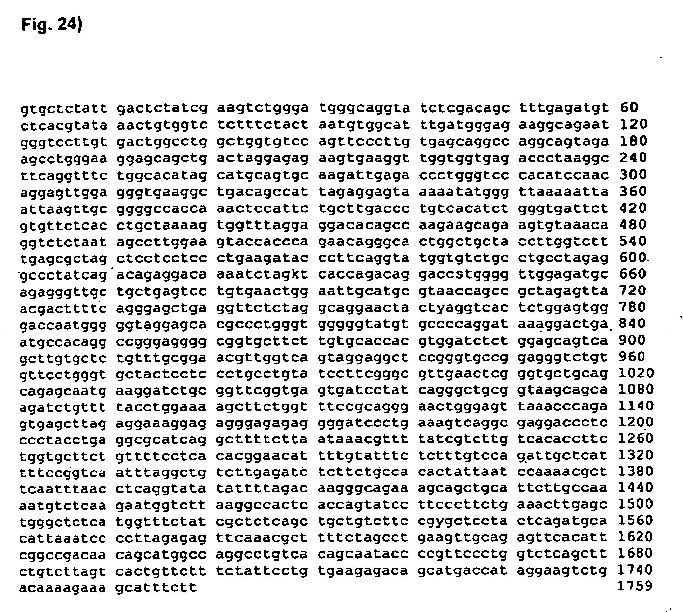

- Fig. 24): Genfragment 146_10
- Fig. 25): Genfragment 154_26

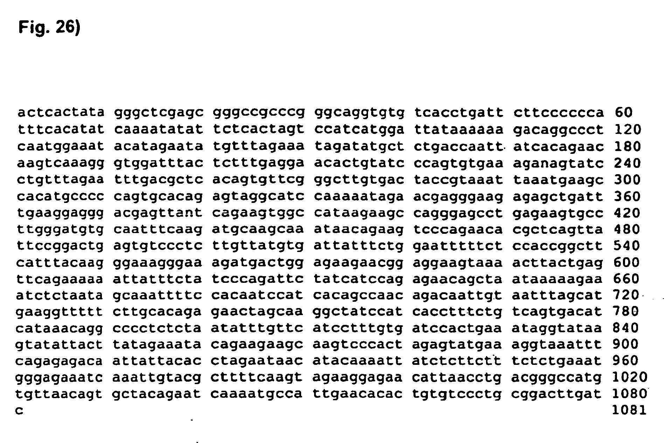

- Fig. 26): Genfragment 154_29

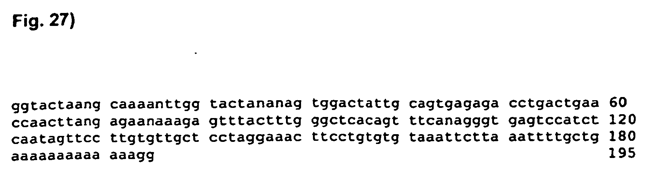

- Fig. 27): Genfragment 164_12

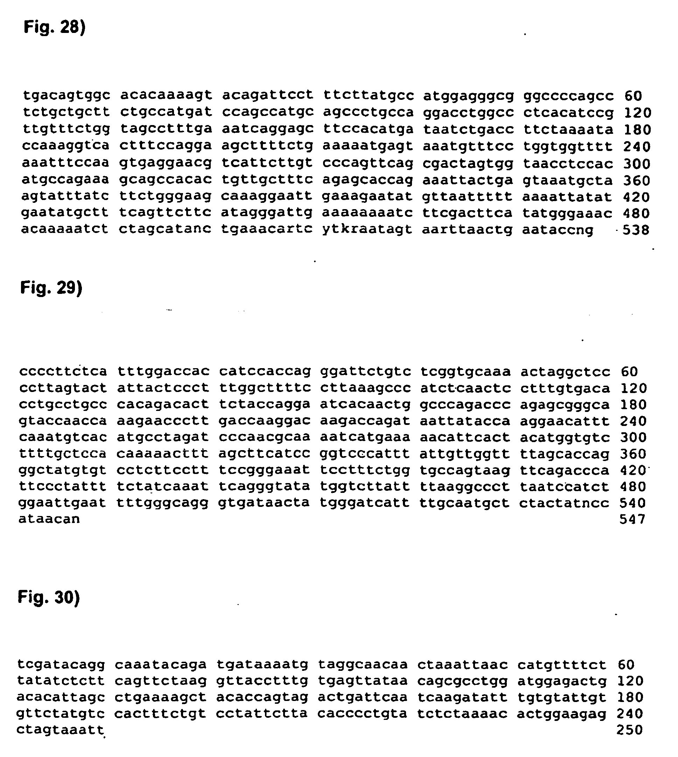

- Fig. 28): Teil des Genfragments 164_24
- Fig. 29): Teil des Genfragments 164_24
- Fig. 30): Teil des Genfragments 164_24

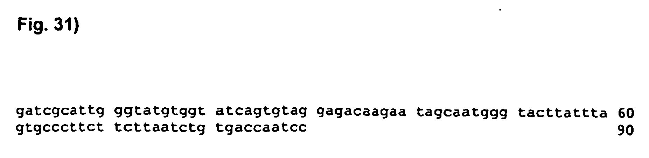

- Fig. 31): Genfragment 180_12

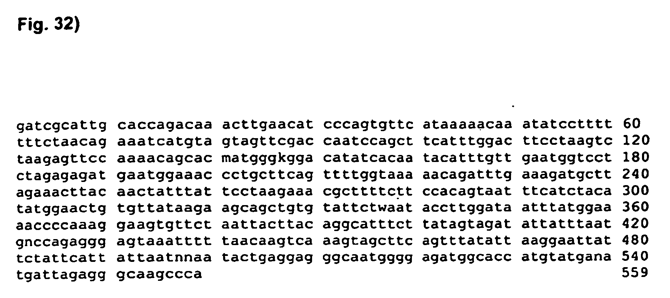

- Fig. 32): Genfragment 180_8

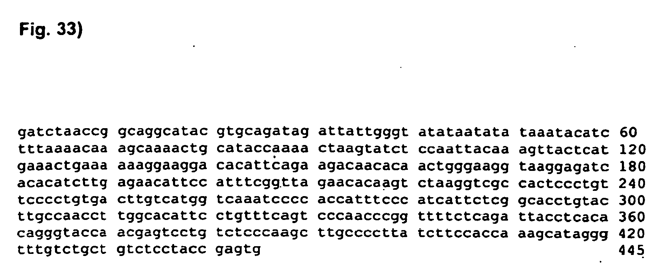

- Fig. 33): Genfragment 89_28

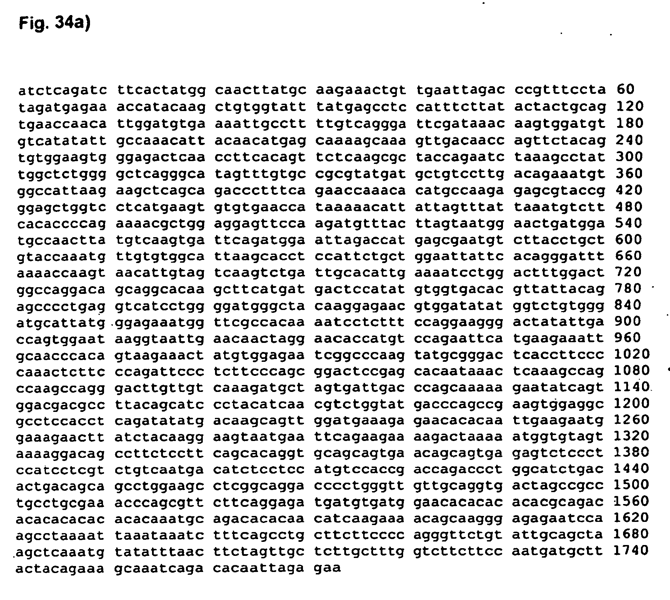

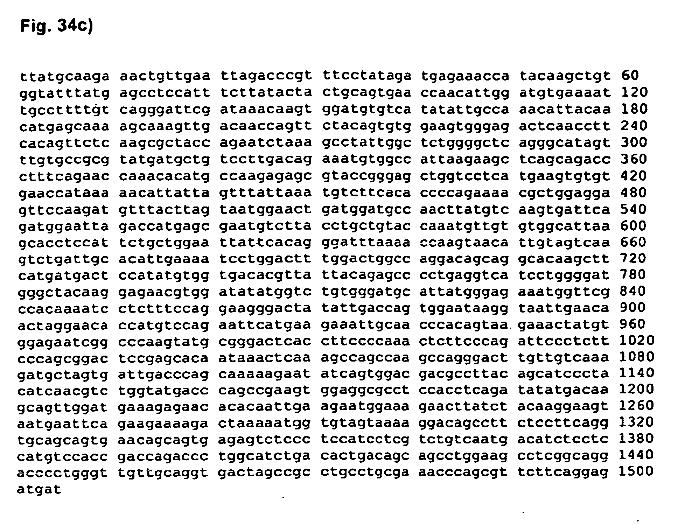

- Fig. 34a): cDNA-Sequenz von JNK3, human, α1-Subtyp; AN: U34820

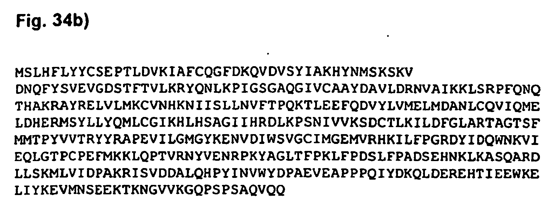

- Fig. 34b): Aminosäure-Sequenz von JNK3, human, α1-Subtyp; AN: U34820
- Fig. 34c): cDNA-Sequenz von JNK3, human, α2-Subtyp; AN: U34819

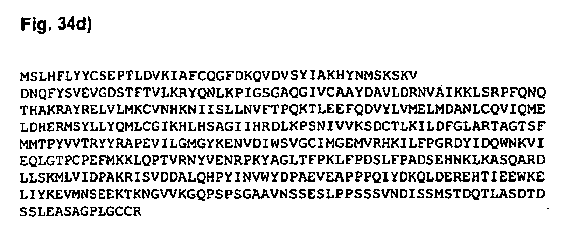

- Fig. 34d): Aminosäure-Sequenz von JNK3, human, α1-Subtyp; AN: U34819

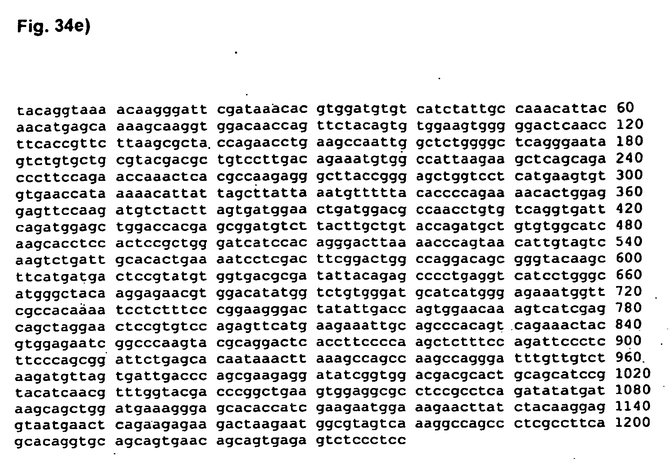

- Fig. 34e): cDNA-Sequenz von JNK3, Maus; AN: AB005665

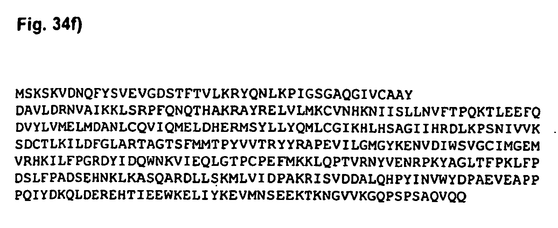

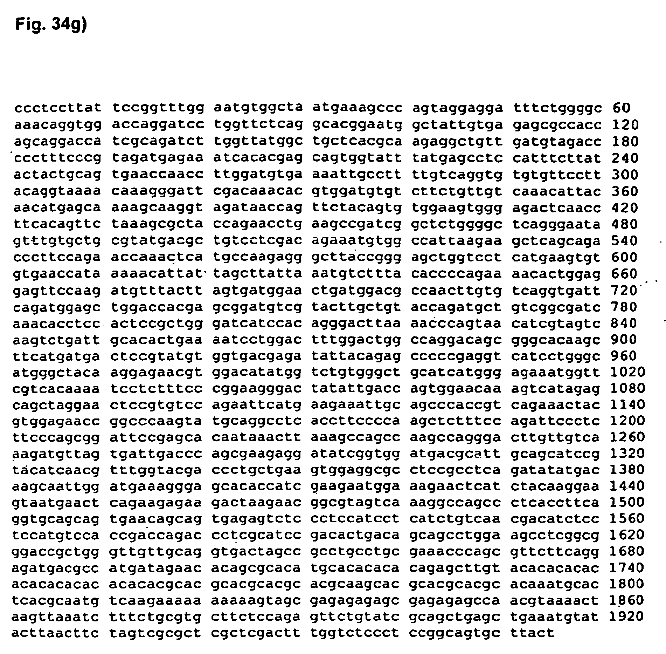

- Fig. 34f): Aminosäure-Sequenz von JNK3, Maus; AN: AB005665
- Fig. 34g): cDNA-Sequenz von JNK3, Ratte; AN: NM_012806

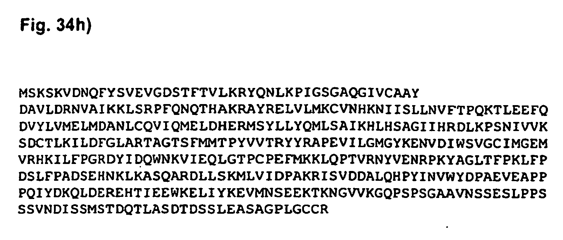

- Fig. 34h): Aminosäure-Sequenz von JNK3, Ratte; AN: NM_012806
- Fig. 35a): cDNA-Sequenz von PIM-2, human; eigene Klonierung
- Fig. 35b): Aminosäure-Sequenz von PIM-2, human; eigene Klonierung
- Fig. 35c): cDNA-Sequenz von PIM-2, Maus; AN: L41495
- Fig. 35d): Aminosäure-Sequenzen von PIM-2, Maus; AN: L41495 unterschiedliche Proteinlängen:
1.) 40 kDa
2.) 37 kDa
3.) 34 kDa
- Fig. 35e): cDNA-Sequenz von PIM-2, Ratte; eigene Klonierung
- Fig. 35f): Aminosäure-Sequenz von PIM-2, Ratte; eigene Klonierung
- Fig. 35g): Vergleich der Proteinsequenzen der in 35b) dargestellten PIM-2. Sequenz mit der in der Gendatenbank niedergelegten humanen PIM-2 Sequenz NM_006875

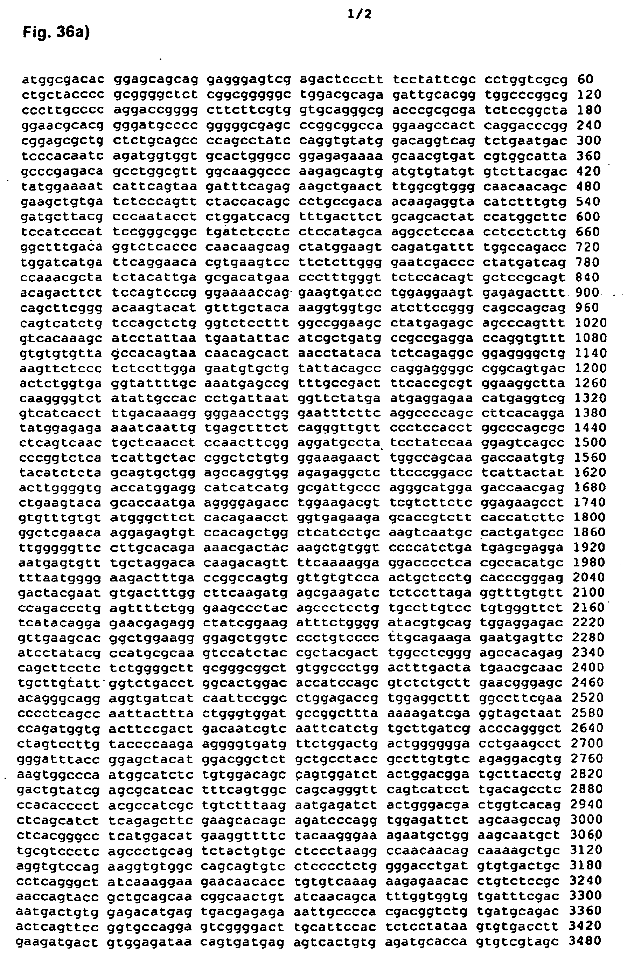

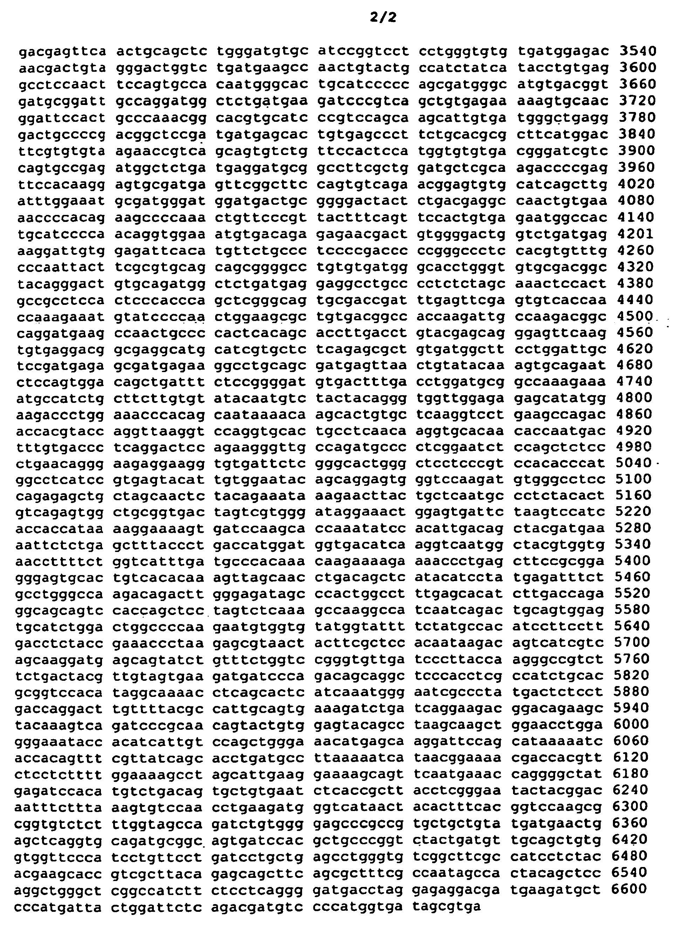

- Fig. 36a): cDNA-Sequenz von LR11, Maus; AN: AB015790

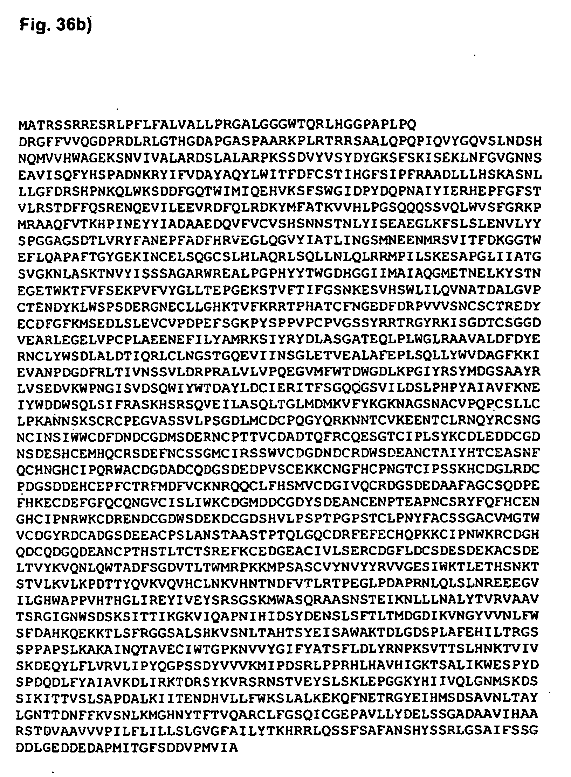

- Fig. 36b): Aminosäure -Sequenz von LR11, Maus; AN: AB015790

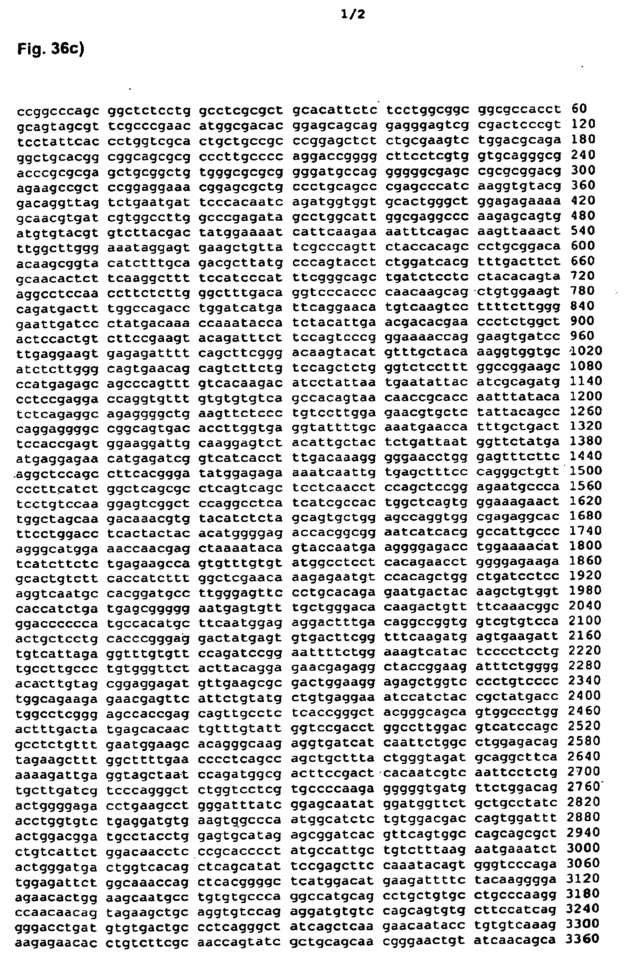

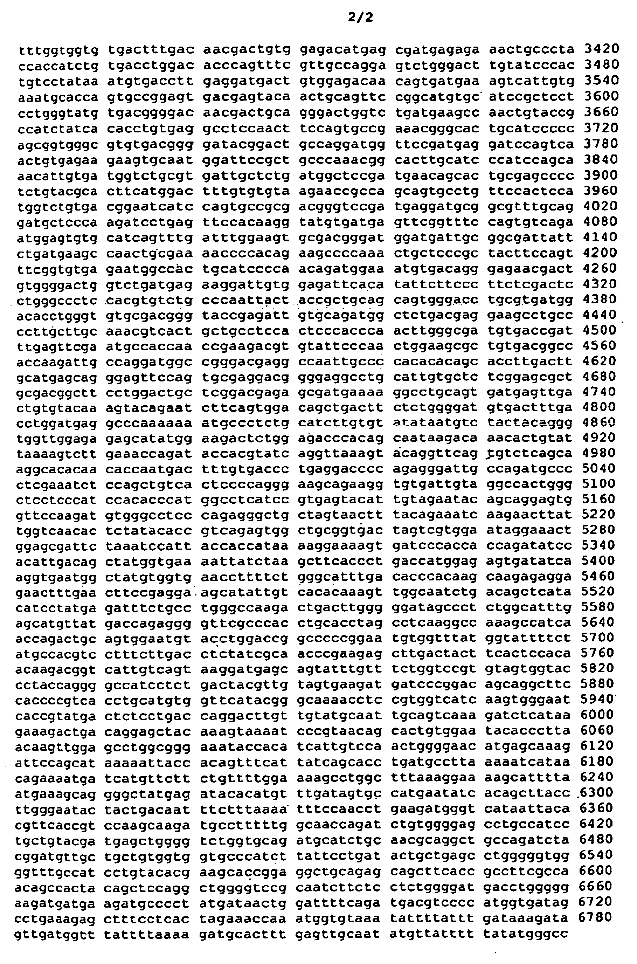

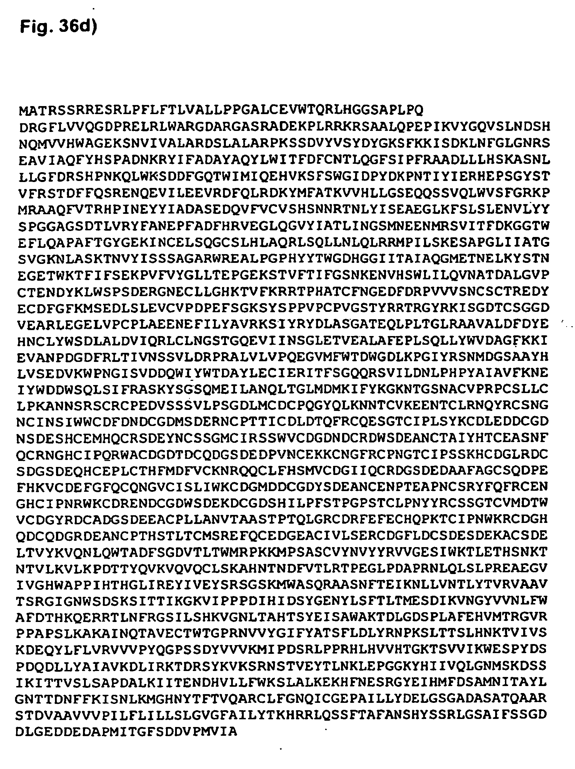

- Fig. 36c): cDNA-Sequenz von LR11, human; AN: Y08110
- Fig. 36d): Aminosäure -Sequenz von LR11, human; AN: Y08110

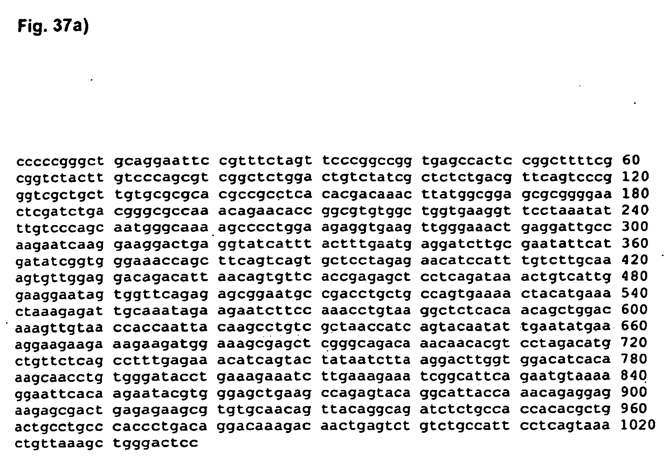

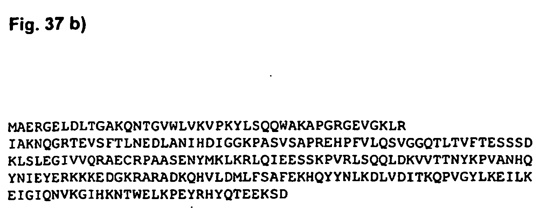

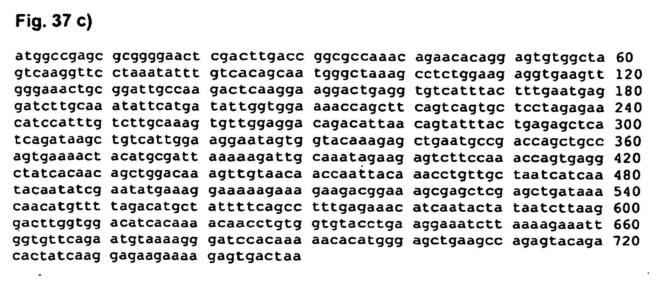

- Fig. 37a): cDNA-Sequenz von TFIIFβ, Ratte; AN: D10665
- Fig. 37b): Aminosäure -Sequenz von TFIIFβ, Ratte; AN: D10665
- Fig. 37c): cDNA-Sequenz von TFilFβ, human; AN: X59745

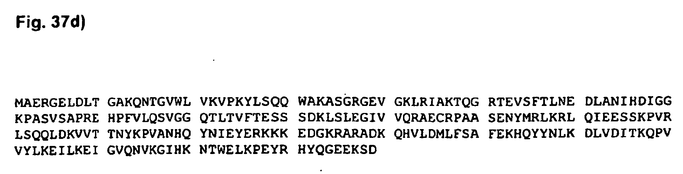

- Fig. 37d): Aminosäure -Sequenz von TFIIFβ, human; AN: X59745

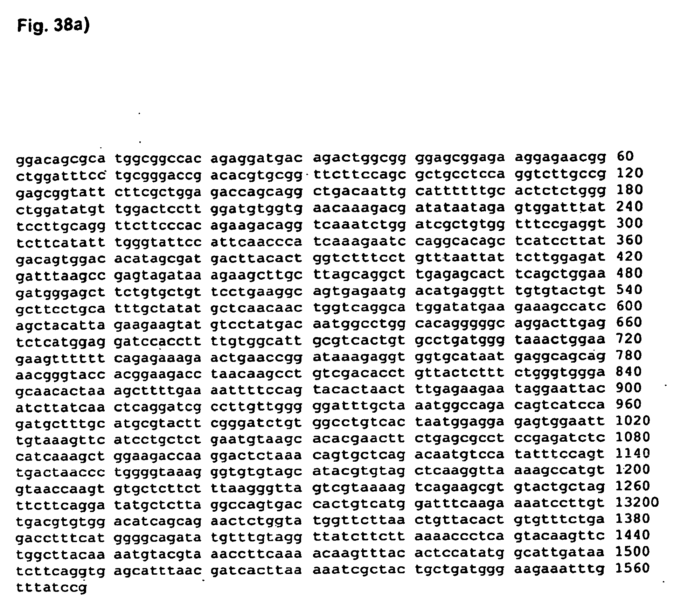

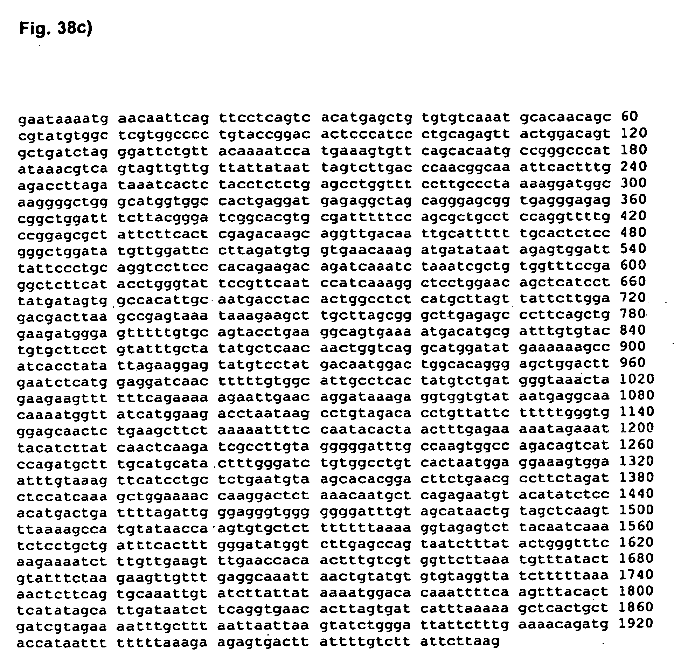

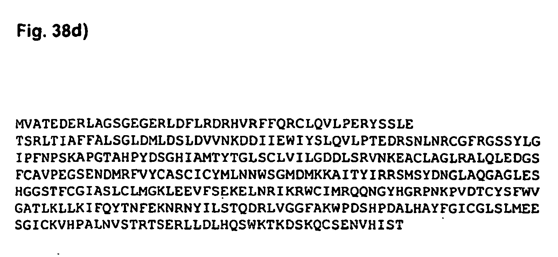

- Fig. 38a): cDNA-Sequenz von GGT, Ratte; AN: L24116

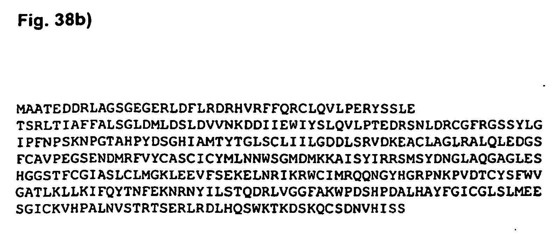

- Fig. 38b): Aminosäure -Sequenz von GGT, Ratte; AN: L24116
- Fig. 38c): cDNA-Sequenz von GGT, human; AN: L25441
- Fig. 38d): Aminosäure -Sequenz von GGT, human; AN: L25411

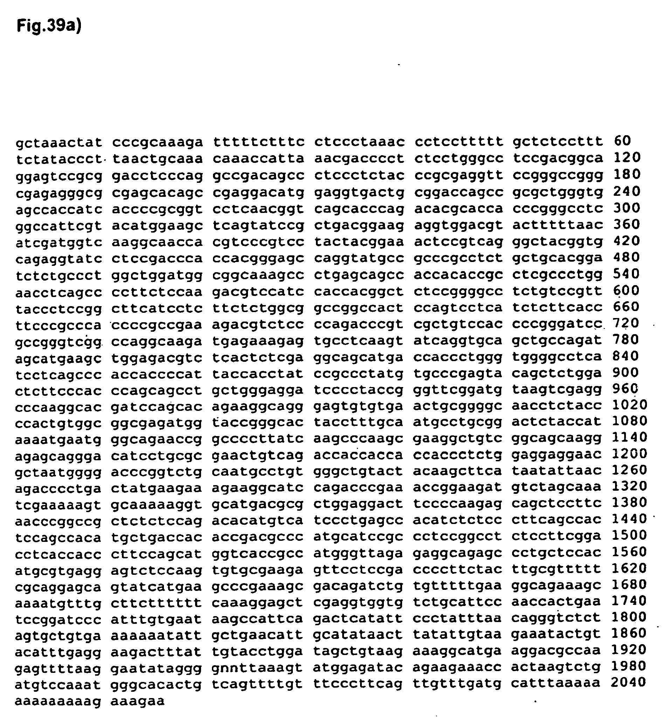

- Fig. 39a): cDNA-Sequenz von GATA3, Maus; AN: NM_008091

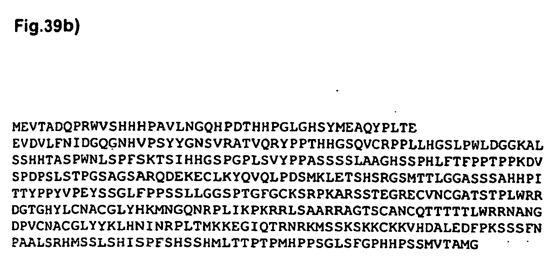

- Fig. 39b): Aminosäure -Sequenz von GATA3, Maus; AN: NM_008091

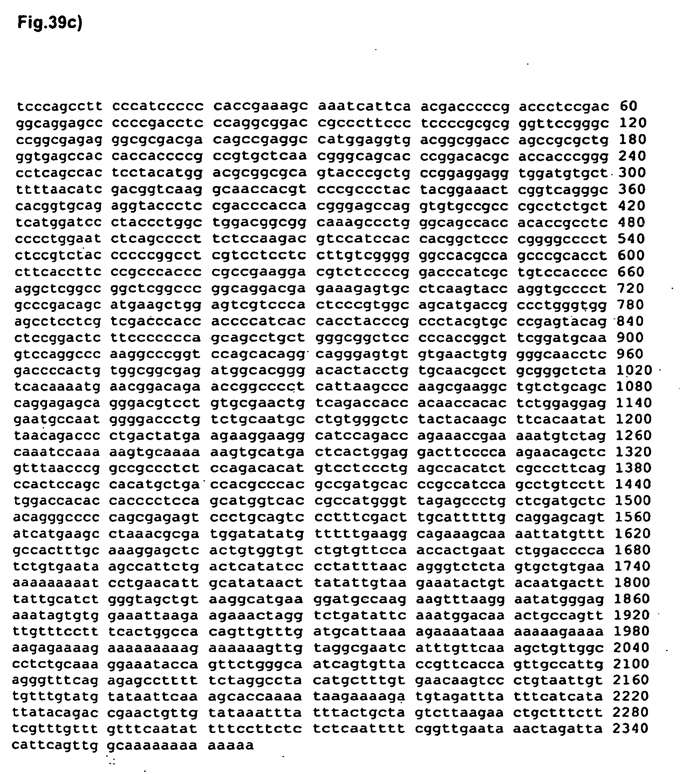

- Fig. 39c): cDNA-Sequenz von GATA3, human; AN: NM_002051

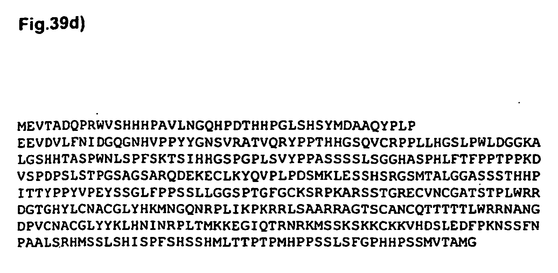

- Fig. 39d): Aminosäure -Sequenz von GATA3, human; AN: NM_002051

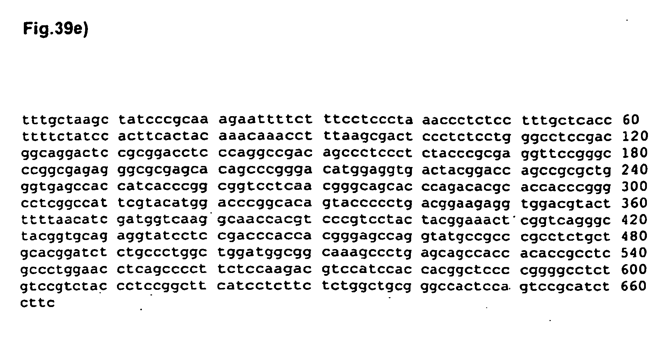

- Fig. 39e): Teil-cDNA-Sequenz von GATA3, Ratte; AN: AB000217

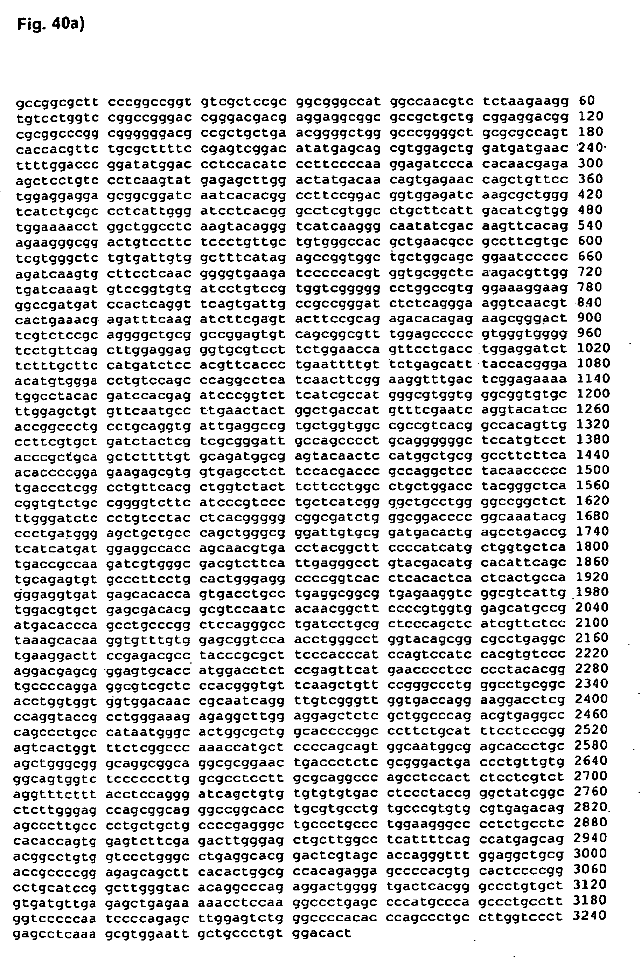

- Fig. 40a): cDNA-Sequenz von CCL-7, human; AN: AF224741

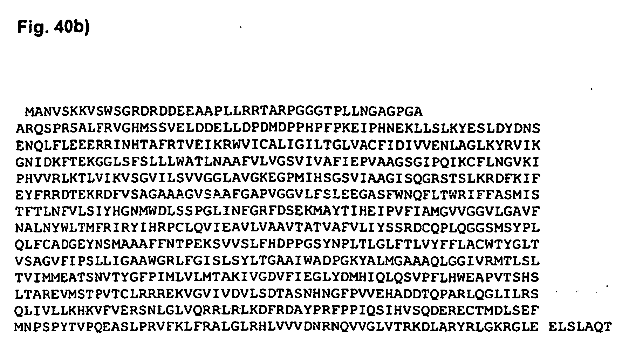

- Fig. 40b): Aminosäure -Sequenz von CCL-7, human; AN: AF224741

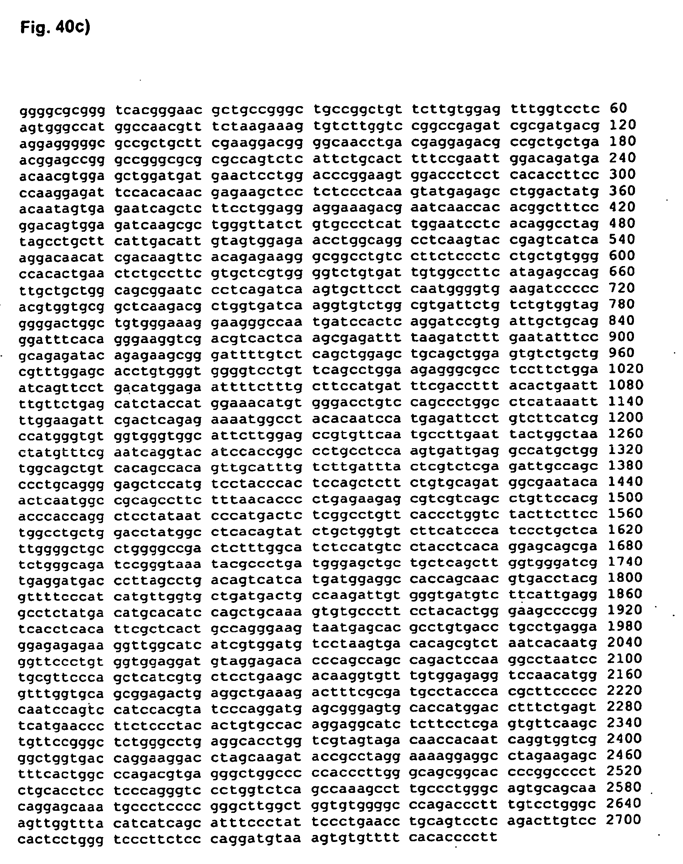

- Fig. 40c): cDNA-Sequenz von CCL-7, Ratte; AN: Z67744

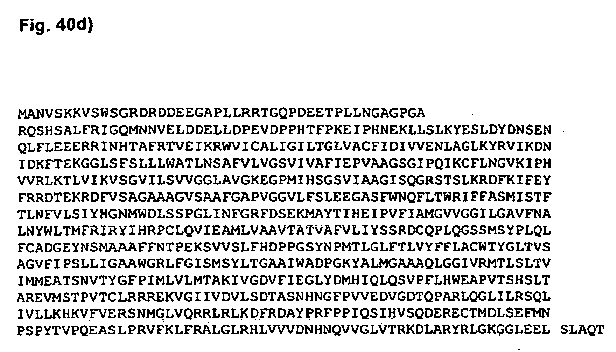

- Fig. 40d): Aminosäure -Sequenz von CCL-7, Ratte; AN: Z67744

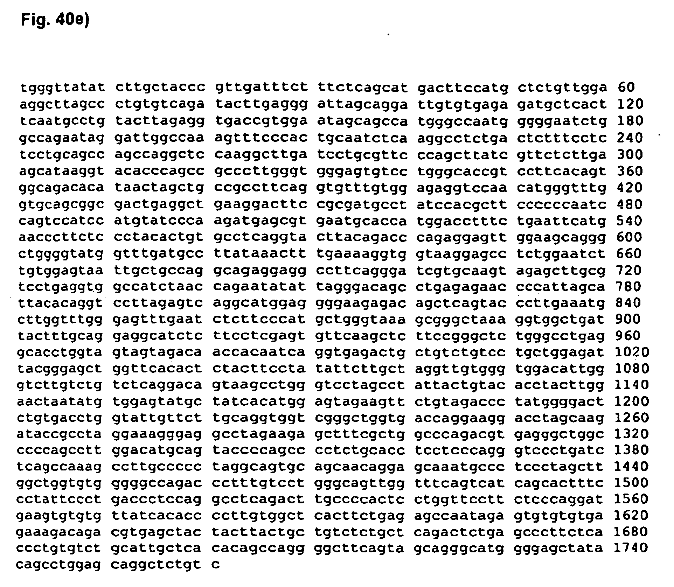

- Fig. 40e): genomische DNA-Sequenz von CCL-7, Maus; AN: AH063101
- Fig. 40f): Aminosäure -Sequenz von CCL-7, Maus; AN: AH063101

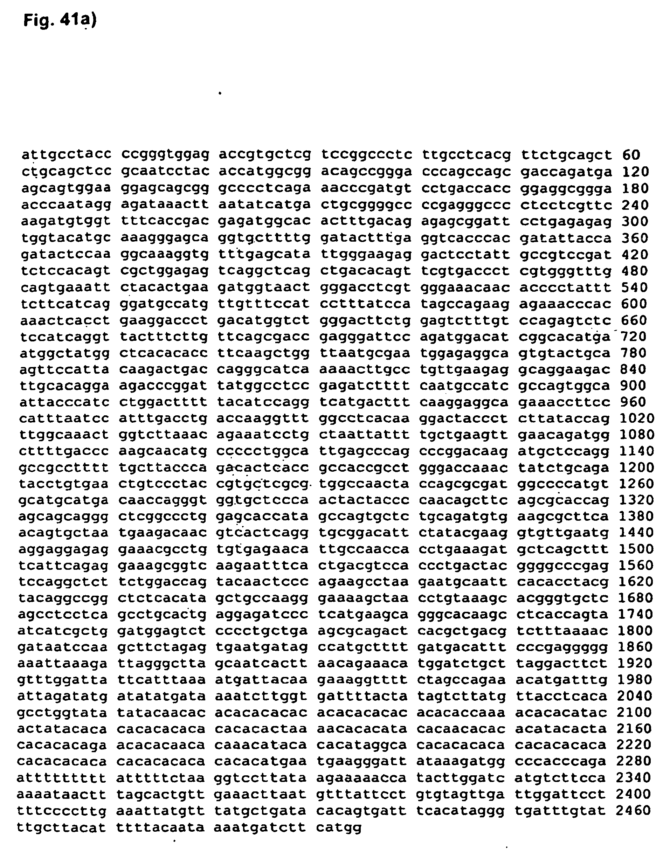

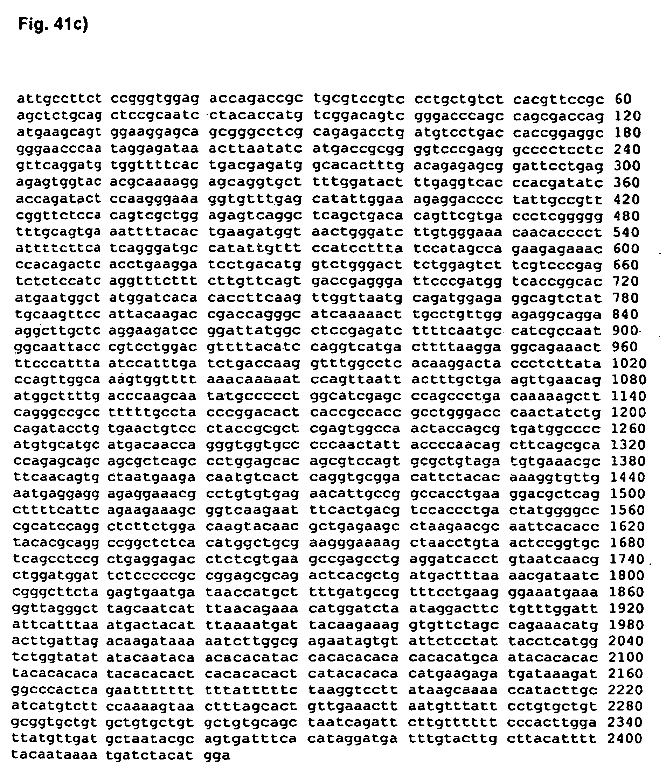

- Fig. 41a): cDNA-Sequenz von Catalase, Ratte; AN: NM_012520

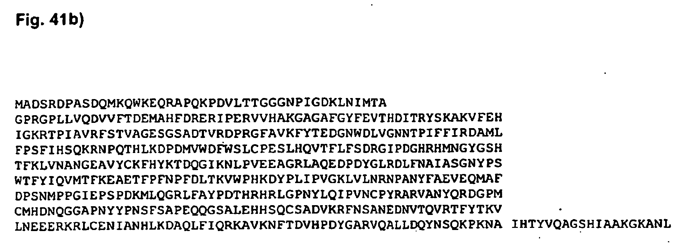

- Fig. 41b): Aminosäure -Sequenz von Catalase, Ratte; AN: NM_012520
- Fig. 41c): cDNA-Sequenz von Catalase, Maus; AN: NM_009804

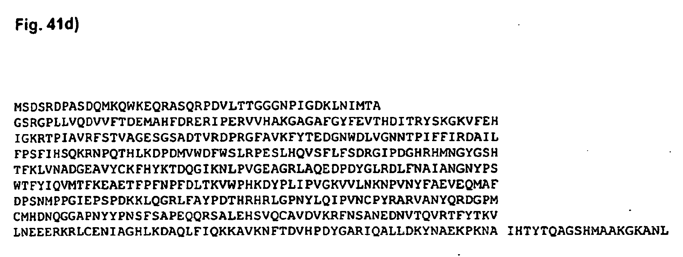

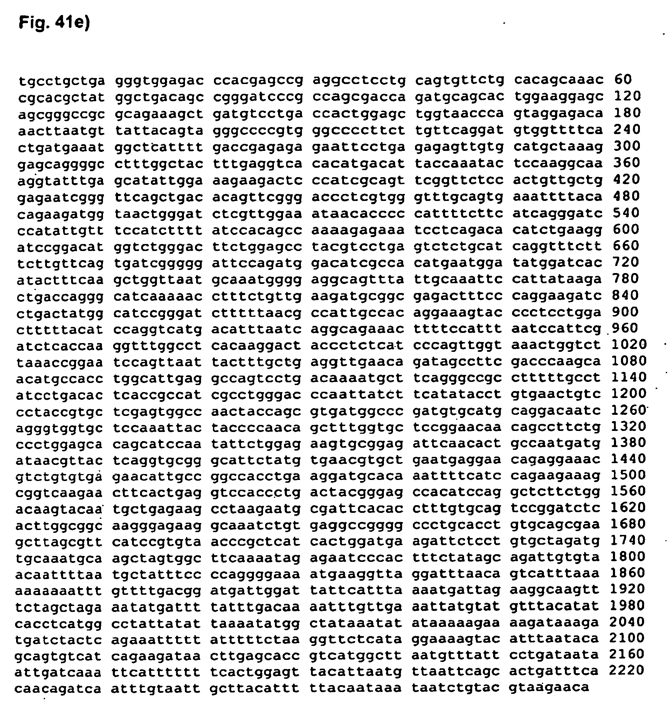

- Fig. 41d): Aminosäure -Sequenz von Catalase, Maus; AN: NM_009804
- Fig. 41e): cDNA-Sequenz von Catalase, human; AN: NM_001752

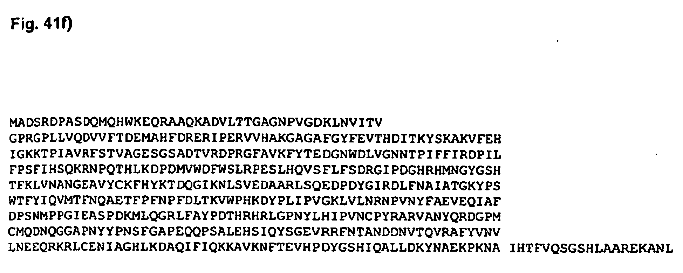

- Fig. 41f): Aminosäure -Sequenz von Catalase, human; AN: NM_001752

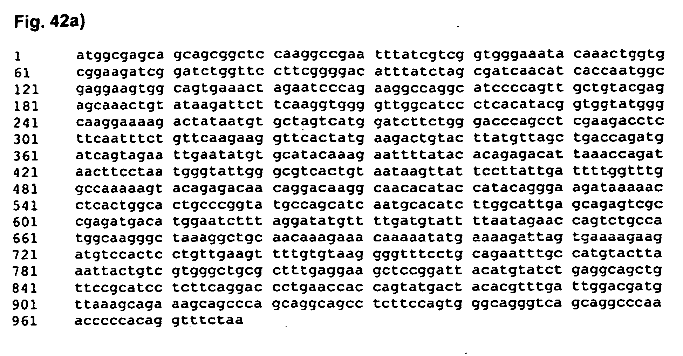

- Fig. 42a): cDNA-Sequenz von Caseinkinase 1a, Ratte; AN: U77582

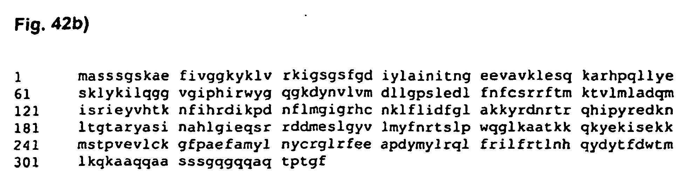

- Fig. 42b): Aminosäure -Sequenz von Caseinkinase 1α, Ratte; AN: AAB 19227

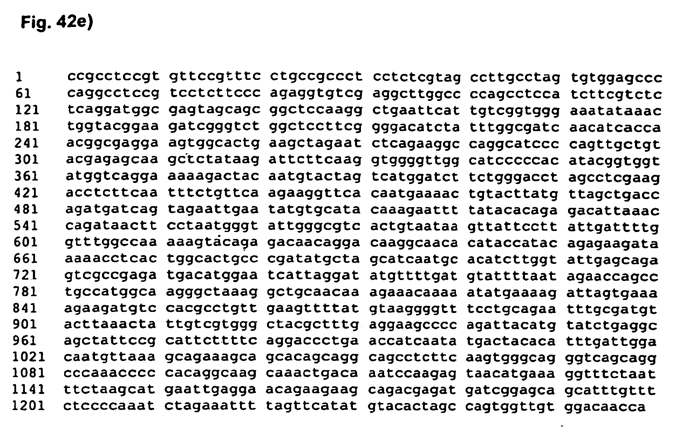

- Fig. 42e): cDNA-Sequenz von Caseinkinase 1α, human; AN: NM_001892

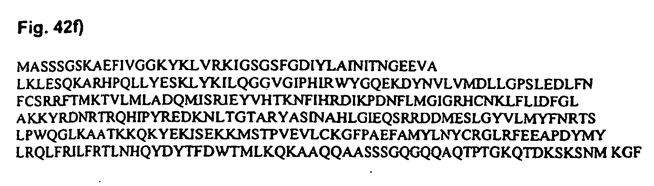

- Fig. 42f): Aminosäure -Sequenz von Caseinkinase 1α, human; AN: NM_001892

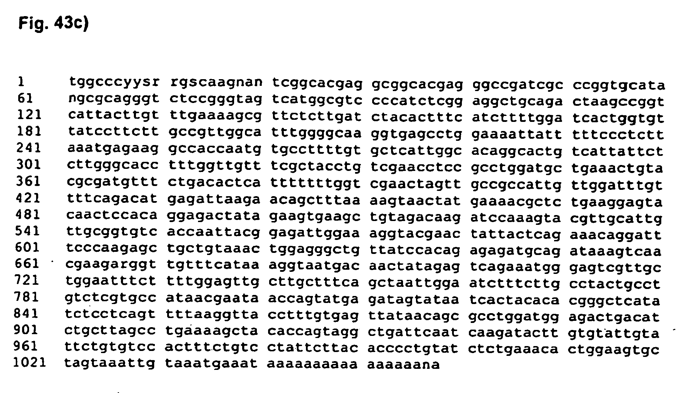

- Fig. 43c): cDNA-Sequenz von Tetraspanin-6, Maus; AN: AF053454

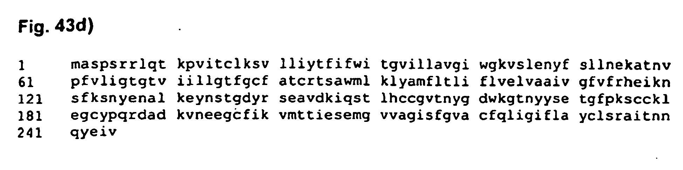

- Fig. 43d): Aminosäure-Sequenz von Tetraspanin-6, Maus; AN: AAC69711

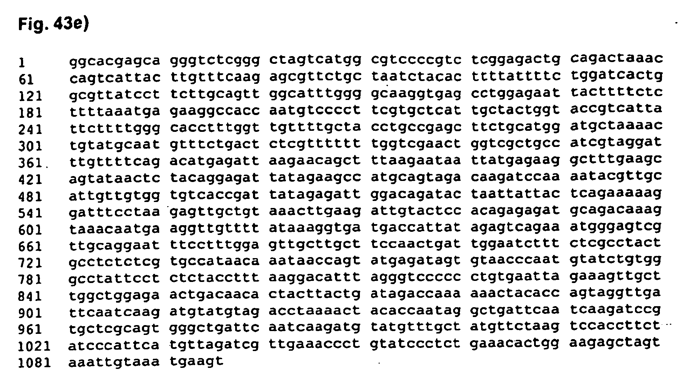

- Fig. 43e): cDNA-Sequenz von Tetraspanin TM4-D, human; AN: AF133426
- Fig. 43f): Aminosäure -Sequenz von Tetraspanin TM4-D, human; AN: AF133426

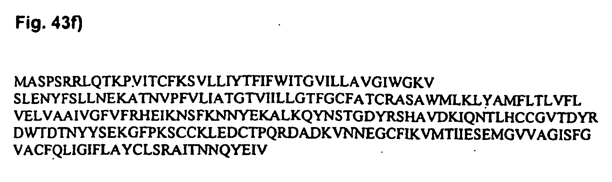

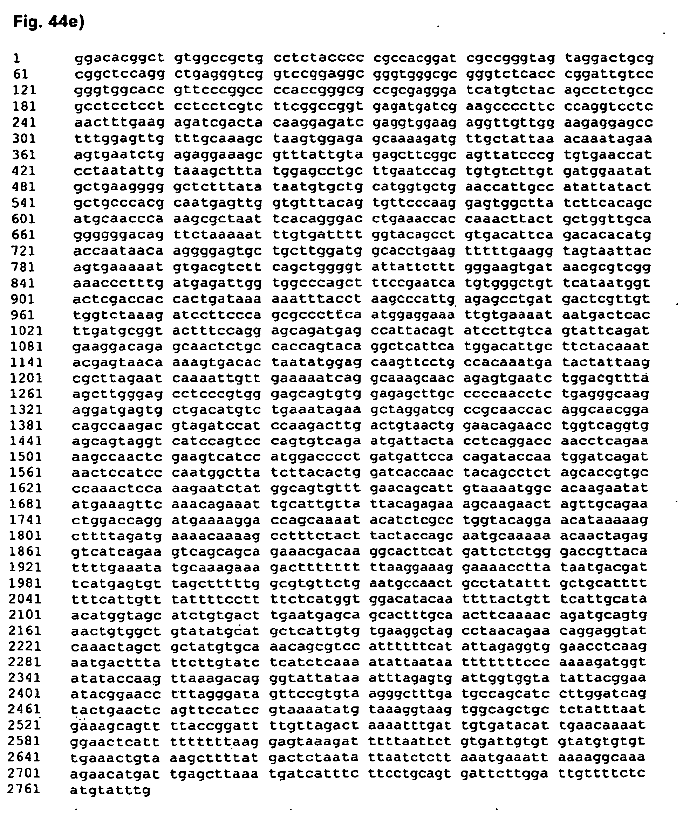

- Fig. 44e): cDNA-Sequenz von MAP3K7/TAK-1, human; AN: NM_003188

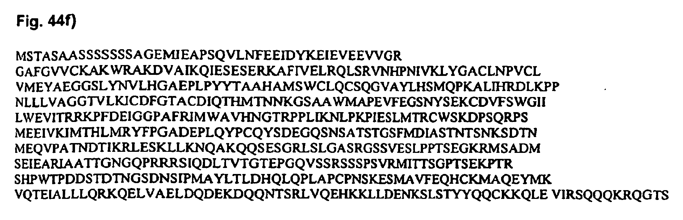

- Fig. 44f): Aminosäure -Sequenz von MAP3K7/TAK-1, human; AN: NM_003188

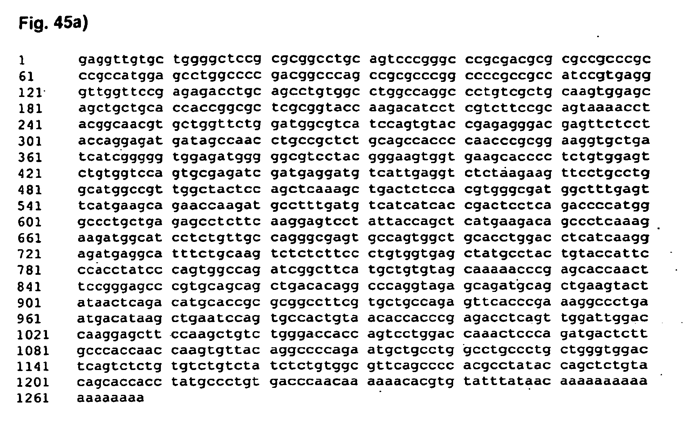

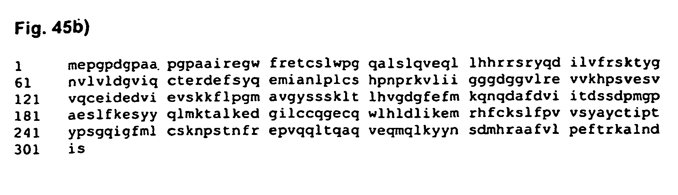

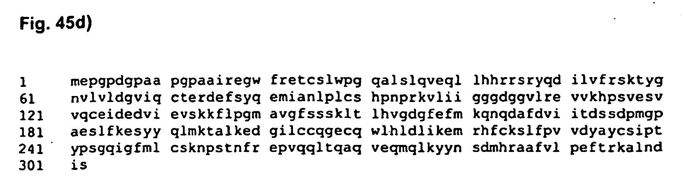

- Fig. 45a): cDNA-Sequenz von Spermidin Synthase, Ratte; AN: AF337636
- Fig. 45b): Aminosäure -Sequenz von Spermidin Synthase, Ratte; AN: AAK21288

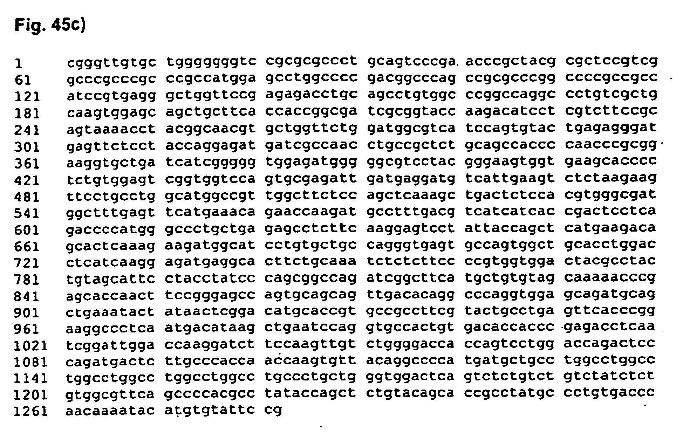

- Fig. 45c): cDNA-Sequenz von Spermidin Synthase, Maus; AN: L19311
- Fig. 45d): Aminosäure -Sequenz von Spermidin Synthase, Maus; AN: AAC37666

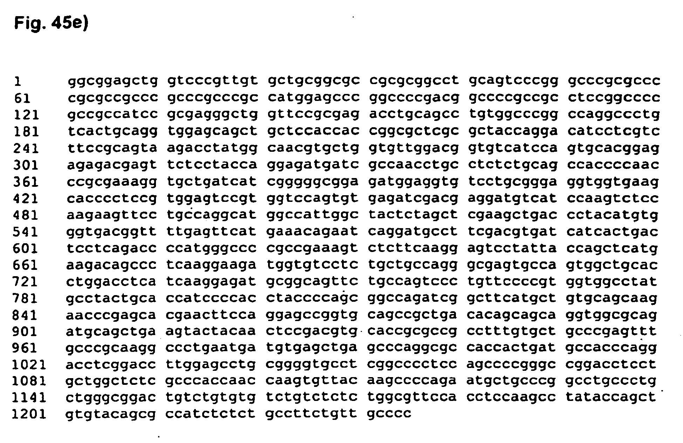

- Fig. 45e): cDNA-Sequenz von Spermidin Synthase, human; AN: XM_042276

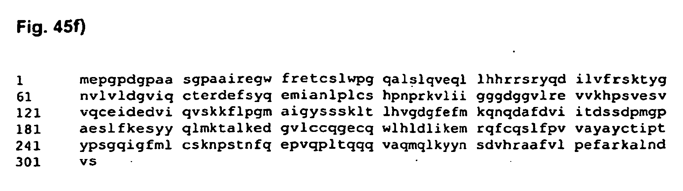

- Fig. 45f): Aminosäure-Sequenz von Spermidin Synthase, human; AN: XP_042276

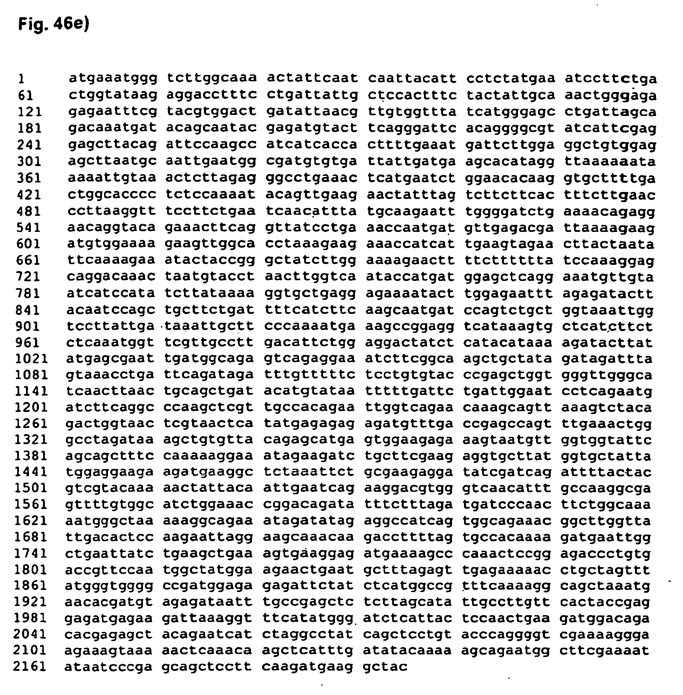

- Fig. 46e): cDNA-Sequenz von BAB14112, human; AN: AK022582

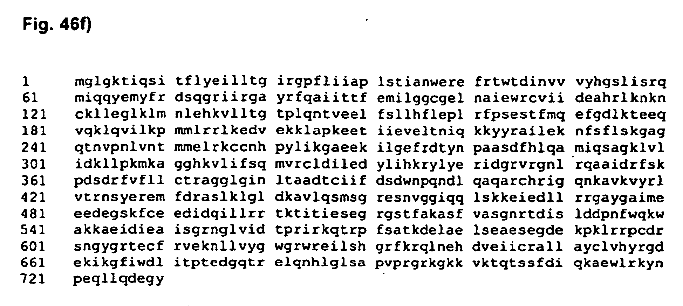

- Fig. 46f): Aminosäure -Sequenz von BAB14112, human; AN: BAB14112
- Fig. 47): Casein-Kinase in verschiedednen Schmerzmodellen
- Fig. 48): Tetrapanin (TSPAN) in verschiedednen Schmerzmodellen
- Fig. 49): M3K7-Kinase in verschiedednen Schmerzmodellen
- Fig. 50): Überblick über die angewandte generelle Klonierungsstrategie
- Fig. 51): Überblick über die Regulation der identifizierten Gene und Genfragmente

### Beispiele:

### Beispiel 1

### Identifizierung, Isolierung und Sequenzierung schmerzregulierter Gene

### 1.) Vorgehen

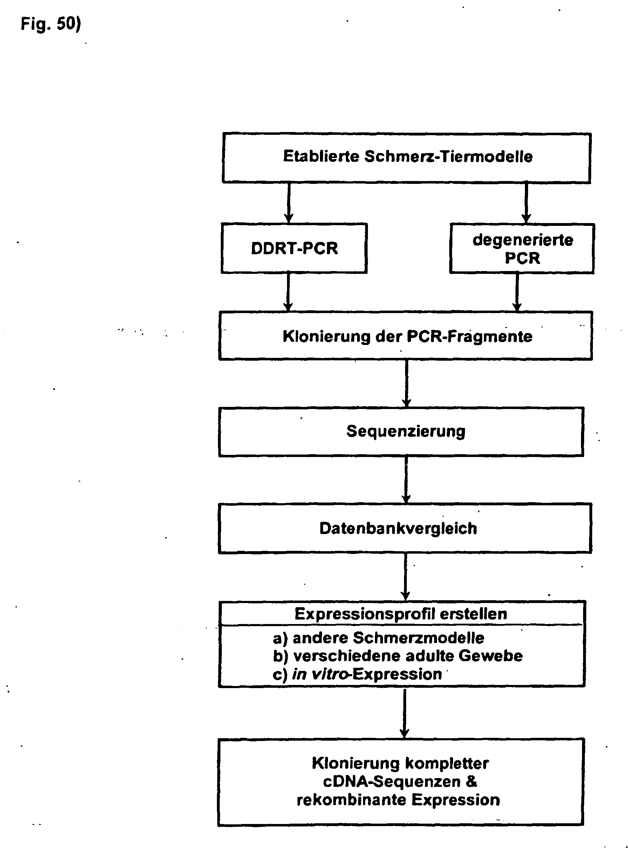

Es wurde folgendes Vorgehen gewählt (zur Erläuterung s. Abbildung 50):

Als Ausgangspunkt für die Isolierung schmerzregulierter Gene wurde das sogenannte Formalinmodell an der Ratte gewählt, bei dem Formalin in die Rattenpfote injiziert wird. Das Zielgewebe, in dem die schmerzregulierte Expression der erfindungsgemäßen Gene nachgewiesen wurde, war der dorsale Teil des Rückenmarks der Ratte in den Segmenten L3-L6. Zur Isolierung differentiell regulierter Gene stehen vier Methoden zur Verfügung:
- cDNA-RDA (cDNA-representational difference analysis; Hubank & Schatz, 1994)
- DDRT-PCR (Differential Display RT-PCR; Liang & Pardee 1992, Bauer et al., 1994),
- Subtraktive Hybridisierung (Watson & Margulies, 1993)
- SAGE (Serial Analysis of Gene expression, Velculescu et al., 1995).

Eine vergleichende Bewertung der genannten Methoden führte zur Auswahl der DDRT-PCR, da diese Methode im Gegensatz zur subtraktiven Hybridisierung und der SAGE in der Lage ist, sowohl hoch- und herunterreguiierte Gene ais auch seitene Transkripte zu erfassen und darüberhinaus innerhalb kurzer Zeitspannen eine Fülle von Ergebnissen liefert.

### 2.) MATERIAL UND METHODEN

### Isolierung und Charakterisierung schmerzregulierter cDNA-Sequenzen

Methode nach Liang & Pardee (1992) / U.S.Patent 5.262.311
Modifikationen:
1. Dekamer-Primer nach Bauer at al. 1993
2. oligoDT-Primergemische nach Sompayrac et al. 1995
3. TAE-Polyacrylamid-Gelektrophorese

**Tabelle 2: Verwendete Oligonukleotidprimer**

| **Name** | **Sequenz (in 5' => 3'-Orientierung)** |
|---|---|
| Deka1 | TACAACGAGG |
| Deka2 | TGGATTGGTC |
| Deka3 | CTTTCTACCC |
| Deka4 | TTTTGGCTCC |
| Deka5 | GGAACCAATC |
| Deka6 | AAACTCCGTC |
| Deka7 | TCGATACAGG |
| Deka8 | TGGTAAAGGG |
| Deka9 | TCGGTCATAG |
| Deka10 | GGTACTAAGG |
| Deka11 | TACCTAAGCG |
| Deka12 | CTGCTTGATG |
| Deka13 | GTTTTCGCAG |
| Deka14 | GATCAAGTCC |
| Deka15 | GATCCAGTAC |
| Deka16 | GATCACGTAC |
| Deka17 | GATCTGACAC |
| Deka18 | GATCTCAGAC |
| Deka19 | GATCATAGCC |
| Deka20 | GATCAATCGC |
| Deka21 | GATCTAACCG |
| Deka22 | GATCGCATTG |
| Deka23 | GATCTGACTG |
| Deka24 | GATCATGGTC |
| Deka25 | GATCATAGCG |
| Deka26 | GATCTAAGGC |
| T7-PCR | ACGACTCACTATAGGGCGAATTGG |
| M13R-PCR | ACACAGGAAACAGCTATGACCATG |
| T7(dT)₁₅ | AAACGACGGCCAGTGAATTGTAATACGACTCACTATAGGCGCT₁₅ |

| **oligodT-Primergemische (äquimolar gemischt)** | |
|---|---|
| 3P1 | T₁₂AA + T₁₂AT + T₁₁AC + T₁₁AG |
| 3P2 | T₁₁GA + T₁₁GT + T₁₁CA + T₁₁CT |
| 3P3 | T₁₀GC + T₁₀GG + T₁₀CC + T₁₀CG |

**Tiermodell:** Der Formalin-Test stellt ein geeignetes Modell für den Bereich des inflammatorischen/persistenten Schmerz dar (Duibisson et al., 1997). Hierbei wurde 50µl 5%ige Formalinlösung unliateral in die Hinterpfote adulter Wistar-Ratten injiziert und die Tiere 24Std. nach der Injektion zur Gewebeentnahme getötet. Parallel wurden bei den Kontrolltieren isotonische Kochsalzlösung in die Hinterpfote injiziert.

**Gewebeentnahme.** Die Tier werden dekapitiert, die Rückenwirbelsäule herauspräpariert und das Rückenmark durch kräftige Injektion einer eiskalten isotonischen Kochsalzlösung aus dem Rückenmarkskanal herausgespült. Nach Entfernen der Dura wurde die dorsale Hälfte des Bereiches von L3 bis L6 herausgeschnitten und sofort in flüssigem Stickstoff eingefroren.

**RNA-Isolierung.** Aus den Gewebeproben wurde die Gesamt-RNÄ mit dem Trizol-Kit (Life Technologies) nach Angaben des Herstellers isoliert. Die RNA wurde UV-spektrometrisch quantifiziert (Extinktion bei 260nm) und durch denaturierende Geielektropherese in einem Formaldehyd-Agarosegel (Sambrook et al.. 1989) auf Integrität überprüft.

**DNase-Verdau.** Vor Einsatz in die DDRT-PCR werden eventuelle Spuren an genomischer DNA durch DNase-Verdau entfernt. Hierbei wurden je 6µg RNA in einem Gesamtvolumen von 100µl in 1X First-Strandbuffer (Life Techn.) und 10 Units RNase-freie DNasel (Boehringer Mannheim) für 15 Minuten bei 37°c inkubiert. Nach Phenol-Chloroform-Extraktion wurde die RNA durch Zugabe an 1/10 Vol. Natriumacetat pH5.2 und 2.5 Vol. Ethanol präzipitiert, in DEPC-Wasser gelöst, UV-spektrometrisch quantifiziert und durch erneute Formaldehyd-Agarose-Gefelektrophorese charakterisiert.

**Reverse Transkription.** Je 200ng DNasel-verdaute RNA wurden zunächst mit 2.5µM oligodT-Primergemisch (3P1, 3P2 oder 3P3) durch 5minütige Inkubation bei 70°C denaturiert, auf Eis abgeschreckt und dann in einem Gesamtvolumen von 20µl Reaktionsgemisch für 60 Min. bei 37°C inkubiert. Das Reaktionsgemisch enthielt 1X First-Strandbuffer (Life Techn.), 10 mM DTT, je 18.75 µM dATP, dCTP, dGTP, dTTP, 20 Units RNasin (Fa. Promega) und 200 Units Superscript II RNaseH⁻ - reverse Transkriptase (Life Techn.). Anschließend wurde das Enzym durch 5minütiges Erhitzen auf 99°C inaktiviert und der cDNA-Ansatz bei -20°C gelagert.

**DDRT-PCR.** Je 2µl der cDNA wurden in einem Volumen von 20µl der Polymerase-Kettenreaktion (PCR) unterworfen. Das Reaktionsgemisch enthielt 1X PCRII-Puffer (Fa. Perkin-Elmer), 2.5mM MgCl₂, 2µM je dATP, dCTP, dGTP, dTTP, 0.2µM Dekamer-Primer (Deka1-26), 0.8µM öligodT-Primergemisch (3P1, 3P2 oder 3P3) und 2µCi [α-³³P]dATP (≥2000mCi/mmol, Fa. Amersham) Die PCR-Ansätze wurden in einem PTC200-Thermocycler (Fa. MJ Research) folgendem Temperaturprofil unterworfen: 3 Min. bei 94°C; 40 Zyklen mit 15 Sek. 94°C, 1 min 40°C, innerhalb von 70 Sek. Hochheizen-auf 72°C, 30 Sek. 72°C; 10 Min. bei 72°C und Halten bei 4°C.

**Gelelektrophorese und Auswertung.** Die PCR-Proben wurden im Vakuum bis zur Trockne eingeengt, in 4µl Probenpuffer (0.25% Bromphenolblau, 0.25% Xylenecyanol FF, 30 % Glycerin) gelöst und je 2 µl in einem 6% igen Tris-Taurin-EDTA-Polyacrylamidgel (Multiphor-System, Fa. Promega) für 2.5 Std. bei 50 Watt elektrophoretisch aufgetrennt. Das Gel wurde anschließend für eine Stunde bei 80°C getrocknet und über Nacht auf einem BASIII-Detektionsscxeen (Fa. Fuji) exponiert. Zur Auswertung wurde der STORM-Phosphor-Imager (Fa. Molecular Dynämics) unter Verwendung der ImageQuant-Software verwendet. Die Autoradiographie-Daten wurden im gleichen Größenmaßstab auf Folie gedruckt, die dann zum Auschneiden der Fragmente verwendet wurde.

**Reamplifikation der DDRT-PCR-Fragmente.** Differentiell regulierte PCR-Banden wurden mit einem Skalpell aus dem Gel ausgeschnitten und durch 10minütiges Kochen in 100µl bidest. Wasser aus dem Gelstück eluiert. 25µl hiervon wurden in einem Gesamtvolumen von 50µl mittels PCR erneut amplifiziert. Das PCR-Reaktionsgemisch enthielt 1 X PCR-Pufferll (Fa. Perkin-Elmer); 1.5mM MgCl₂; 50µM je dATP, dCTP, DGTP, dTTP;M 0.2µM des entsprechenden Dekameres; 1µM 1PX oligodT-Primergemisch und 2.5µl Units AmpliTAQ-DNA-Polymerase (Perkin-Elmer). Das PCR-Temperaturprofil entsprach der originalen PCR-Reaktion (s.o.). Die PCR-Ansätze anschließend mit 10µl Probenpuffer (0.25% Bromphenolblau, 0.25% Xylenecyanol FF, 30 % Glycerin) versetzt und in einem 3%igen TAE-Agarosegel mit 10µg/ml Ethidiumbromid gelelektrophoretisch aufgetrennt und PCR-Produkte der erwarteten Größe aus dem Gel ausgeschnitten.

**Klonierung in TA-Klonierungsvektoren.** Die ausgeschnittenen Fragmente wurden mit dem Qiaquick-Gel-Extraktionskit (Fa. Qiagen) nach Angaben des Herstellers aufgereinigt, bis zur Trockne eingeengt und in 5µl bidest. Wasser aufgenommen. Anschließend wurden sie in den pCRII-TOPO-Vektor mittels des TOPO TA Cloning Kits (Fa. Invitrogen) nach Angaben des Herstellers ligiert und in TOP1OF'-E.coli-Zellen transformiert. Der Transformationsansatz wurde auf LB-Agar-Platten mit 100µg/ml Ampicillin ausgestrichen, die vorher mit 50µl 2% X-Gal (Fa. Sigma) und 50µl Isopropylthiogalactosid (Fa. Sigma) behandelt wurden. Die nach 15 stündiger Inkubation bei 37°C erhaltenen weißen Bakterien-Kione wurden in 5 ml LB-Flüssigmedium mit 100µg/ml Ampicillin (100µg/ml) überführt und über Nacht bei 37°C unter Schütteln inkubiert. Aus diesen Kulturen wurde Plasmid-DNA unter Verwendung des Qiagen-Spin-Miniprep-Kits (Fa. Qiagen) nach Angaben des Herstellers isoliert und je 5µl der Plasmid-DNA durch EcoRI-Restriktionsverdau und anschließende TAE-Agarose-Gelelektrophorese charakterisiert.

**Sequenzanalyse.** Hierbei wurden je 500 ng der Plasmid-DNA mit dem T7-PCR-Primer unter Verwendung des Dye Terminator Cycle Sequencing Kits (Fa. Perkin-Elmer) nach Angaben des Herstellers sequenziert und die Reaktionen mittels des automatischen Sequencers ABI 370 (Fa. Applied Biosystems Inc.) analysiert. Die DNA-Sequenzen wurden unter Verwendung der bioSCOUT-Software (Fa. LION, Heidelberg) mit den Gendatenbanken abgeglichen.

### Bestätigung der differentiellen Regulation der klonierten cDNA-Sequenzen.

Hierbei wurde die Technik des sog. reversen Northems angewendet, bei der die cDNA-Fragmente nach PCR-Amplifikation auf Replikafilter gebunden und mit radioaktiv markierter cDNA aus Kontroll-Rückenmärk versus Formalin-Rückenmark hybridisiert wurden.

**PCR-Amplifikation der klonierten cDNA-Fragmente.** Hierbei wurden je 50-100 ng der Plasmid-DNA in einem Volumen von 100µl der Polymerase-Kettenreaktion (PCR) unterworfen. Das Reaktionsgemisch enthielt 1X PCRII-Puffer (Fa. Perkin-Elmer); 1.25 mM MgCl₂; 10% DMSO; je 250µM dATP, dCTP, dGTP, dTTP; 0.8 µM T7-PCR-Primer, 0.8 µM M13R-PCR-Primer und 5 Units Ampli-TAQ-DNA-Polymerase (Fa. Perkin-Elmer). Die PCR-Ansätze wurden in einem PTC200-Thermocycler (Fa. MJ Research) folgendem Temperaturprofil unterworfen: 3 Min. bei 94°C; 40 Zyklen mit 15 Sek. 94°C, 15 Sek. 55°C, 30 Sek. 72°C; 10 Min. bei 72°C und Halten bei 4°C. Ein Aliquot von 5 µl wurde auf einem 3%igen TAE-Agarose-Gel aufgetrennt und bei erfolgreicher PCR-Rektion die PCR-Ansätze mittels des Quiaquick PCR-Purfication-Kits (Fa. Qiagen) nach Angaben des Herstellers aufgereinigt und UV-spektrometrisch quantifiziert.

**Herstellung der Slotblot-DNA-Filter.** Die HybondN-Filter wurden zunächst für 10 Min. in 6 X SSC-Puffer gebadet und in die Slotblotapparatur (Fa. Schleicher und Schüll) eingespannt. Je 1µg der DNA-Fragmente wurden in 6 X SSC -Puffer verdünnt, durch 10minütiges Kochen und anschließendes Abschrecken auf Eis denaturiert.und auf die Filter gesaugt. Die Filter wurden anschließend 10min in 1.5M NaCl/0.5M NaoH-Lösung und 10 min in 1 M NaCl/0.5M Tris-HCl pH=7.0 gewaschen, 30 min bei Raumtemperatur getrocknet und durch 5minütige UV-Bestrahlung (302nm, Transilluminator) an den Filter gebunden.

**Herstellung der amplifizierten RNA.** (modifiziert nach Poirier et al., 1997 & Superscript Choice System -Fa. Life Techn.). Hierbei wurden 5 µg Gesamt-RNA mit 100 ng T7(dT)15-Primer in einem Volumen von 11µl 5 min bei 70°C denaturiert und auf Eis abgeschreckt. Die denaturierte RNA wurde dann in einem Gesamtvolumen von 20µl mit 1 x First-Strandbuffer (Life Techn.), 10 mM Dtt, je 500µM dATP, dCTP, dGTP, dTTP und 400 Units Superscriptll reverse Transkriptase (Life Techn.) für 1 Std. bei 42°C inkubiert, auf 4°C abgekühlt. Zur Zweitstrangsynthese wurde dazugegeben: 91µl DEPC-H₂O, 30 µl 2nd-Strandbuffer (Life Tech.), 3 µl 10 mM dNTPs (Boehringer Mannheim), 1 µl E.coli DNA-Ligase (10U/µl; Life Tech.), 4 µl E.coli DNA-Polymerase. I (10U/µl; Life Tech.), 1 µl Rnase H (2U/µl; Life Tech.), der Ansatz dann für 2 Std. bei 16°C inkubiert und nach Zugabe von 2 µl T4 DNA-Polymerase (5U/µl; Life Tech.) weitere 5 Min. bei 16°C inkubiert. Die Inaktivierung erfolgte durch Zugabe von 10µl 0.5M EDTA und 10 minütiges Erhitzen auf 65°C. Nach Phenol-Chloroform-Extraktion und Fällung mit 70µl 7.5 M NH₄OAc und 500µl 100 %igem, -20°C kaltem Ethanol wurde der Niederschlag abzentrifugiert (5 Min. 14000g, RT), mit 1 ml 70%igem Ethanol gewaschen, in 50µl DEPC-H₂O gelöst, über eine S200-Sephacrylsäule entsalzen (Microspin; Fa. Pharmacia). Ein Aliqout (2µl) wird UV-spektrometrisch quantifiziert. Von dieser doppelsträngigen cDNA wird durch in vitro Transkription RNA hergestellt. Dabei werden 50µl cDNA (5-10µg) in einem Gesamtvolumen von 100µl mit 1 x Transcriptionspuffer (Fa. Promega), 7.5 mM rNTPs, 10µl T7 RNA-Polymerase-Mix (Ribomax-Kit; Fa. Promega) für 3-4 Std. bei 37°C inkubiert und danach auf 4 °C abgekühlt. Nach Zugabe von 3 µl RQ1-DNase (Rnasefrei; Fa. Promega) wird der Ansatz für 15 min bei 37°C inkubiert. Nach Phenol-Chloroform-Extraktion und Entsalzen über eine S200-Sephacrylsäule (Microspin; Fa. Pharmacia) wird ein Aliquot (2µl) UV-spektrometrisch vermessen.

**Herstellung der radioaktiven cDNA-Sonde.** Je 2µg der amplifizierten RNA werden mit 0.8 µl Hexamerprimergemisch (=750ng; Fa. Boehringer Mannheim) durch 5 minütiges Erhitzen auf 70°C und anschliessendes Abschrecken auf Eis denaturiert und in einem Gesamtvolumen von 25 µl revers transkribiert unter Zugabe von 2.5 µl PCR-Puffer (Life Tech.); 2.5 µl 25mM MgCl₂; 2.5µl 0.1 M DTT; 1µl je 20mM dATP/dTTP/dGTP; 1µl 120µM dCTP und 5µl [α-³²P]dCTP. Nach 5minütiger Inkubation bei Raumtemperatur wurde 2µl Superscriptll-reverse Transkriptase (200U/µl, Fa. Life Tech.9 hinzugegeben, für 10 min bei 25°C und anschließend für 50 min bei 42°C inkubiert. Nach Hitzeinaktivierung (15 min. 70°C) wurde die Probe mit DEPC-Wasser auf 50µl aufgefüllt und die nichtinkorporierte Radioaktivität mittels einer S200-Sephaaylsäule (Microspin; Fa. Pharmacia) entfernt. Von je 0.5µl des Eluats wurden in einem β-Counter (Fa. LKB) über Tscherenkow-Streuung die Aktivität bestimmt. Im restlichen Ansatz wurde die RNA durch Zugabe an 6µl 1 M NaOH/10mM EDTA-Lösung und 20minütige Inkubation bei 68°C hydrolysiert, danach durch Zugabe an 60µl 1 M Phosphatpuffer pH=7.0. neutralisiert und zu den jeweiligen prähybridisierten Replikafiltern hinzugegeben.

**Prähybridisierung, Hybridisierung, Waschen und Auswertung der Filter.** Die Filter werden in Hybridisierungsflaschen mit 10 ml Expresshyb-Lösung (Fa. Clontech), und 100µg/ml hitze-denaturierte SalmonSperm-DNA (Fa. Sigma) für 20 min bei 68°C inkubiert. Nach Abschütten des Prähybridisierungmixes und Zugabe von 5 ml der gleichen, auf 68°C Lösung wird die oben beschriebene radioaktive cDNA-Sonde hinzupippetiert und die Filter im Drehrohrofen Ober Nacht bei 68°C inkubiert. Danach folgen mehrere Waschschritte mit 0.2 X SSC / 0.1% SDS für 10 min bei Raumtemperatur; 0.2 X SSC / 0.1% SDS für 15 min bei 42°C und zuletzt in 0.2 X SSC / 0.1% SDS für 15 min bei 65°C. Die Filter werden feucht in Folie eingeschweißt und auf einem BASIII-Detektionsscreen (Fa. Fuji) exponiert. Zur Auswertung wurde der STORM-Phosphor-Imager (Fa. Molecular Dynamics) unter Verwendung der ImageQuant-Software verwendet.

### 3.) Ergebnisse

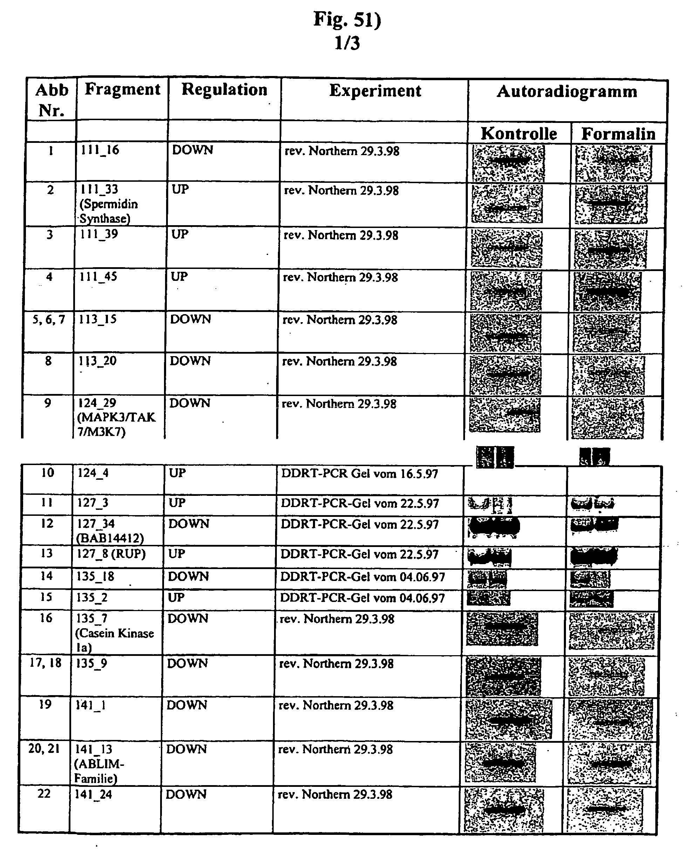

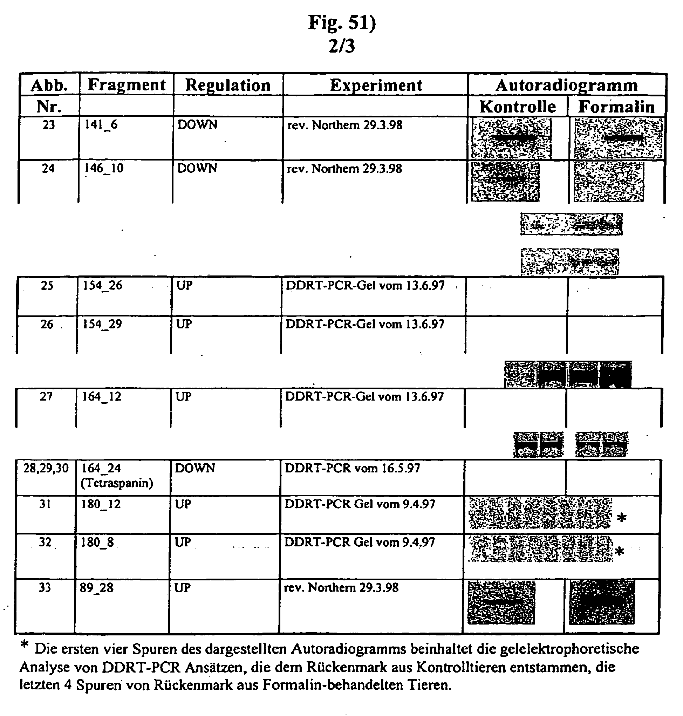

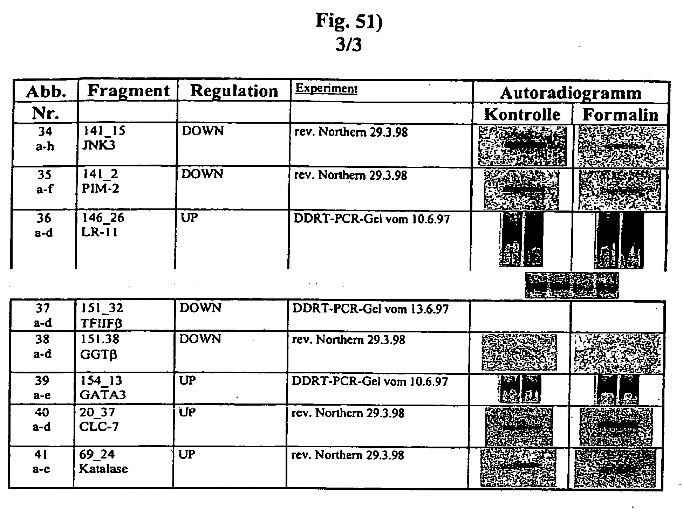

Unter Anwendung dieser Methode (s. Abbildung 50) wurden 35 schmerzregulierte Gene (siehe Tabelle 3 und Abbildung 51) zunächst als Partialsequenz, im weiteren Verlauf des Arbeitsprozesses zum Teil als verlängerte Sequenz kloniert.

Ausgehend vom ausgewählten Tiermodell wurden mit der angewandten Klonierungsstrategie cDNA-Sequenzen aus der Ratte kloniert.

Insgesamt wurden 27 aus dem Stand der Technik bisher unbekannte, nicht zugeordnete und 8 bekannte cDNA-Sequenzen bzw. Genfragmente aus dem Rückenmark von Ratten kloniert, die in ihrer Expression durch Bedingungen chronischer Schmerzen reguliert werden (s. Tabelle 4 ― bisher unbekannte, nicht zugeordnete Genfragmente - und Tabelle 5 ― bekannte cDNAs). Von den 27 unbekannten, nicht zugeordneten cDNA-Sequenzen ist über die Funktion im Stand der Technik nichts bekannt bzw. wurde erst durch eine tiefere Analyse in einigen Fällen etwas bekannt. Unsere Untersuchungen belegen nun eine Rolle im Schmerzgeschehen. Die Analyse des Expressionsmusters in verschiedenen Geweben der Ratte zeigte aber für 4 cDNAs (111.39, 111.45, 141.13, 154.29; siehe Tabelle 5) eine Fokussierung auf neuronale Gewebe. Damit sind diese cDNAs für die Entwicklung neuer Diagnose- und Therapieansätze im chronischen Schmerz besonders interessant. Durch die Experimente mit Antisense-Oligos oder Ribozymen kann die Rolle der neuartigen cDNAs weiter untersucht werden.

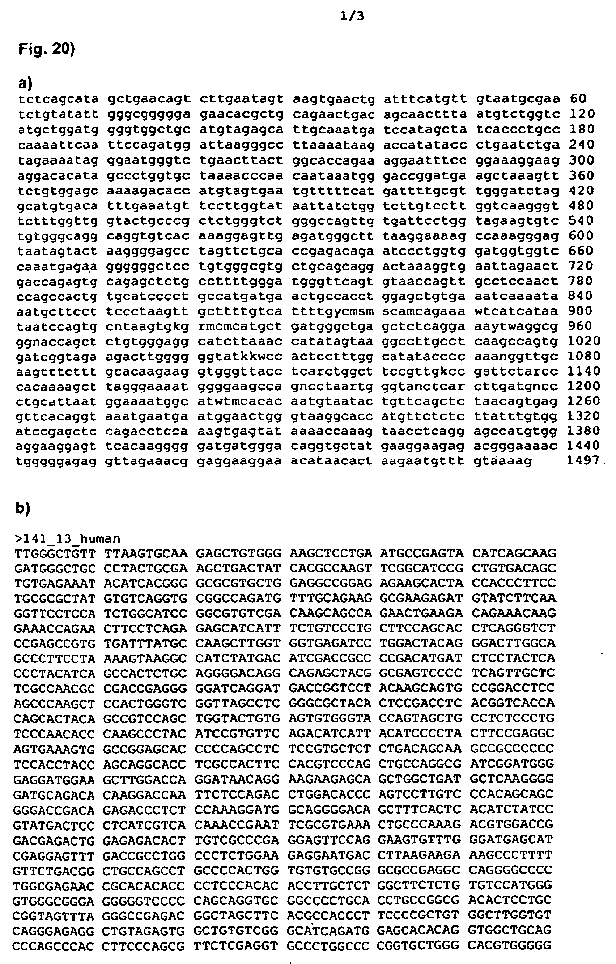

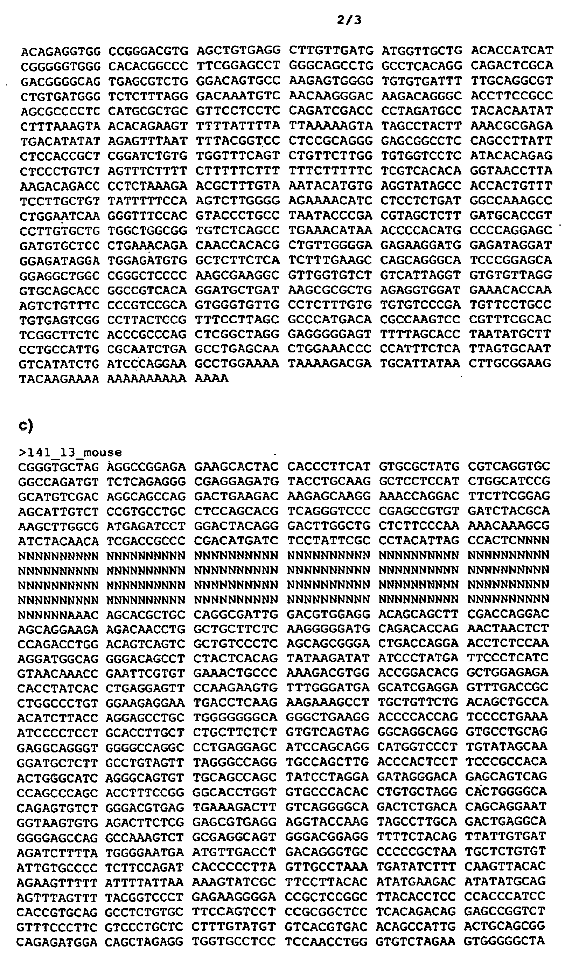

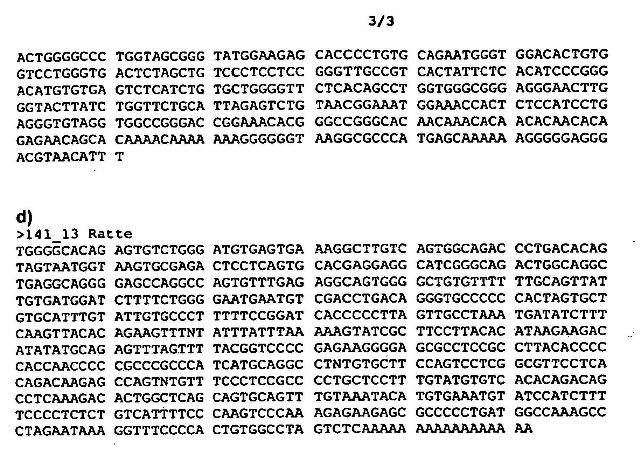

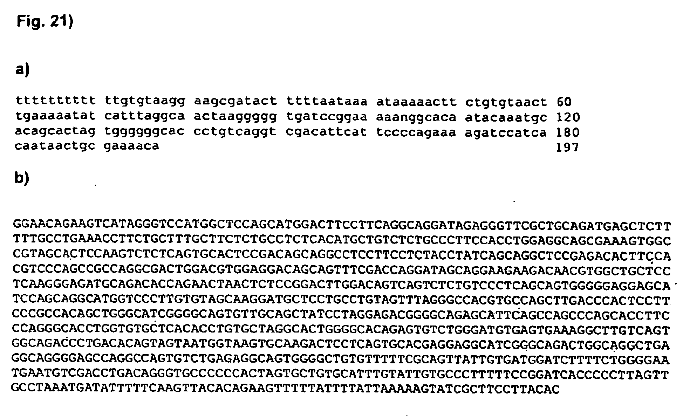

Besonders interessant ist das Genfragment 141-13. Die in den Abbildungen 20 und 21 gezeigte Verlängerung des Genfragments hat in der Analyse ergeben, das hier ein neues Gen vorliegt, das zu UNC-115 und anderen Mitgliedern der abLIM-Familie homolog, aber bisher unbekannt ist. Zusamen mit dem Expressionsmuster und in Anbetracht der interessanten Rolle von UNC-115 in der neuronalen Entwicklung ist daher dieses Genfragment bzw. Gen von besonderem Interesse.

Interessant ist auch das Genfragment 127-8, da dieses stark homolog zu einem offenbar stark exprimierten Teil des humanen Genoms ist (AN: NT_009379.1 auf Chromosom 11q13.

Für die 8 bekannten cDNAs sind die Funktionen im Prinzip bekannt:
cDNAs mit verstärkter Expression unter Schmerz:
   - Geranylgeranyltransferase
   - Katalase
   - Transkriptionsfaktor TFIIFβ
   - Transkriptionsfaktor GATA-3
   - LDL-like Receptor LR-11
   - Cl - Kanal CIC7
cDNAs mit verringerter Expression unter Schmerz:
   - MAP-Kinase/JNK3/SAPKβ
   - PIM-2 Kinase

Bei den durch die genauere Analyse zu den Genfragmenten (GF) aufgefundenen cDNAs handelt es sich um

| | | |
|---|---|---|
| GF111.33 = | Spermidin Synthase | (hochreguliert), |
| GF124-29 = | MAPK3/TAK-1 | (runterreguliert), |
| GF127-34 = | BAB14112 | (runterreguliert), |
| GF135-7 = | Casein Kinase 1a | (runterreguliert) und |
| GF 164-24 = | Tetraspanin (TM4-D/TSPAN-6) | (runterreguliert). |

Besonders hervorzuheben ist hierbei die PIM-2 Kinase. Für andere Isotypen dieses Enzyms, PIM-1 und -3, konnte gezeigt werden, dass diese Signalling-Kinasen an Lem- und Gedächtnisprozessen im Hippocampus beteiligt ist (Konietzko et al. (1999) EMBO J., 18: 3359-3369).
Im Rahmen der Erfindung wurde eine Nukleinsäuresequenz isoliert und charakterisiert, die für das vollständige Ratten PIM-2 Protein kodiert (Fig.35e, 35f). Die Nukleinsäuresequenz und die Proteinsequenz für Pim-2 aus der Ratte sind bisher nicht bekannt. Desweiteren wurde eine vollständige, d.h. die gesamte kodierende Region enthaltende, cDNA sioliert, die für das humane Pim-2 kodiert (35 a, b). Ein Sequenzvergleich mit der publizierten humanen PIM-2 Sequenz (Acc.nr. U77735) ergab folgende Unterschiede:

| **Sequenzunterschied** | **Auswirkung** | **Interpretation** |
|---|---|---|
| *Nt 230: C → T | Thr → Thr | stille Mutation, |
| | | evtl. Polymorphismus |
| Nt 515: A → G | Glu → Glu | stille Mutation, |
| | | evtl. Polymorphismus |
| nt 1064: Deletion T | Änderung des Leserasters führt zu veränderter C-terminaler Proteinsequenz | neuer C-Terminus ist der Maus und Rattensequenz ähnlicher, als die publizierte humane Pim-2 Sequenz |
| Nt 1175: A → C | 3'UTR, keine Auswirkung auf Proteinsequenz | |

| | | |
|---|---|---|
| *Die Postionsbezeichnungen beziehen sich auf die publizerte Pim-2 Sequenz (Accnr. U77735). | | |

Von diesen Unterschieden ist die bei Position 1064 gefundene Feletioneines Nukleotids von funktioneller Relevanz, da sie zu einer Änderung des Leserasters führt und damit ein Protein mit einer vollständig anderen C-terminalen Proteinsequenz ergibt, die im folgenden aufgeführt ist.

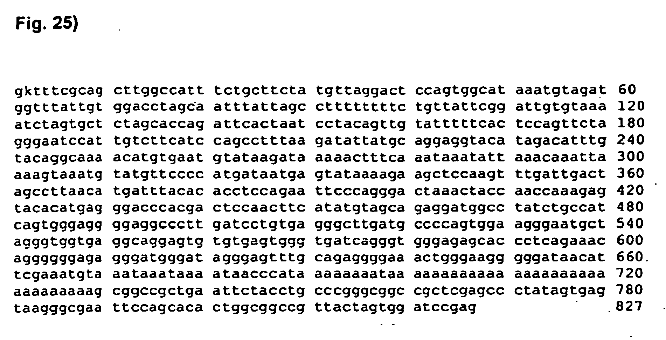

Vergleich der C-terimnalen Enden der beiden deduzierten PIM-2 Proteine (vollständiger Vergleich siehe Fig. 35g).
hPIM-2 (Acnr. U77735) TPQPLORRPCPFGLULATLSLAWPGLAPNGQKSHPNAMSQG
hPIM-2 (Patent Abb. 25b) PLNPSKGGPAPLAWSLLP

Ein weiteres Beispiel bildet die Katalase, die das für die Zelle schädliche H₂O₂ durch Umwandlung in O₂ und H₂O beseitigt.

Die Proteinkinase JNK-3 ist spezifisch in Nervenzellen des zentralen Nervensytems exprimiert (Mohit et al., 1995) und es ist für eine Reihe von Opiatrezeptoren (wie zB. µ-Opiat-Rezeptor und ORL-1) die nachgeschältete Aktivierung von Proteinkinasen aus dieser Familie der Mitogen-aktivierten Proteinkinasen beschrieben worden (Li & Chang, 1996; Hawes et al., 1998). Das Protein LR11 ist ein Mosaikprotein der Low density lipoprotein receptor (LDLR)" Familie (Möwald et al., 1997). Die stärkste Expression an LR11-mRNA konnte im Gehirn nachgewiesen werden, die zudem entwicklungsabhängig in einer Art reguliert wird, die eine Funktion bei der Synaptogenese oder Neuritenwachstum nahelegt (Hermann-Borgmeyer et al.; 1998).

**Tabelle 3. Expression der 27 (meist) unbekannten cDNAs in der adulten Ratte**

| Klon/ Abb.Nr. | Regulation | Expressionsprofil der neuartigen cDNAs |
|---|---|---|
| | | in Geweben der adulten Ratte |
| 180.12/31 | up | noch offen |
| 180.8/32 | up | noch offen |
| 111.16/1 | down | Milz, Magen, Rückenmark |
| 111.33/2 | up | Niere, Lunge, Dickdarm, Magen, Gehirn, Rückenmark |
| 111.39/3 | up | Gehirn, Rückenmark |
| 111.45/4 | up | Gehirn, Rückenmark (ubiquitär) |
| 113.15/5,6,7 | down | ubiquitär |
| 113.20/8 | down | Gehirn, Herz, Milz, Rückenmark |
| 124.29/9 | down | Dickdarm, Gehirn, Lunge, Testis |
| 124.4/10 | up | ubiquitär |
| 127.3/11 | up | spezifisch Hoden |
| 127.34/12 | down | noch offen |
| 127.8/13 | up | Milz, Rückenmark (ubiquitär) |
| 135.18/14 | down | noch offen |
| 135.2/15 | up | noch offen |
| 135.7/16 | down | Niere (ubiquitär) |
| 135.9/17,18 | down | noch offen |
| 141.1/19 | down | nicht erfolgreich |
| 141:13/19,20 | down | Rückenmark (ubiquitär) |
| 141.24/22 | down | Leber, Rückenmark, Skelettmuskel |
| 141.6/23 | down | ubiquitär |
| 146.10124 | down | Dickdarm, Herz, Magen, Rückenmark (ubiquitär) |
| 154.26/25 | up | Niere, Leber, Lunge |
| 154.29/26 | up | spezifisch Rückenmark |
| 164.12/27 | up | noch offen |
| 164.24/28,29,30 | down | noch offen |
| 89.28/33 | up | ubiquitär (Dickdarm, Gehirn, Lunge, Rückenmark, Testis) |

### Sequenzdaten der regulierten Klone

Die folgende Tabelle erlaubt die Zuordnung der Abbildungen zu den tabellarisch aufgelisteten cDNA-Klonen/Gen-Fragmenten.

**Tabelle 4: Bisher unbekannte, nicht zugeordnete Genfragmente**

| **Abbildung Nr.** | **Name der cDNA** | **Länge (bp)** |
|---|---|---|
| | | **(bp)** |
| 1 | 111 16 | 1268 |
| 2 | 111 33 | 840 |
| 3 | 111 39 | 1540 |
| 4 | 111 45 | 2025 |
| 5 | 113 15 | 1721 |
| 6 | 113 15a | 1009 |
| 7 | 113 15b | 685 |
| 8 | 113 20 | 860 |
| 9 | 124 29 | 1348 |
| 10 | 124 4 | 923 |
| 11 | 127 3 | 741 |
| 12 | 127 34 | 109 |
| 13 | 127 8 | 1508 |
| 14 | 135 18 | 1339 |
| 15 | 135 2 | 177 |
| 16 | 135 7 | 1102 |
| 17 | 135 9a | 478 |
| 18 | 135 9b | 616 |
| 19 | 141 1 | 236 |
| 20 | 141 13a | 1497 |
| 21 | 141 13b | 197 |
| 22 | 141 24 | 2818 |
| 23 | 141 6 | 539 |
| 24 | 146 10 | 1759 |
| 25 | 154 26 | 827 |
| 26 | 154 29 | 1081 |
| 27 | 164 12 | 195 |
| 28 | 164 24a | 538 |
| 29 | 164 24b | 547 |
| 30 | 164 24d | 250 |
| 31 | 180 12 | 90 |
| 32 | 180 8 | 559 |
| 33 | 89 28 | 445 |

**Tabelle 5: Genfragmente aus bekannten cDNA-Sequenzen**

| **Abbildung Nr. der bekannten DNA-Seq.** | **Name der cDNA** | **Länge des gefundenen Genfragments (bp)** |
|---|---|---|
| 34 a, c, e, g | b141 15 | 2733 |
| 35 a, c, e | b141 2 | 314 |
| 36 a, c | b141 26 | 338 |
| 37 a, c | b151 32 | 633 |
| 38 a, c | b151 38 | 2201 |
| 39 a, c, e | b154 13 | 190 |
| 40 a, c, e | b20 37a | 389 |
| 40 a, c, e | b20 37b | 109 |
| 41 a, c, e | b69 24 | 720 |

### Beispiel 2a)

### In-situ-Hybridisierung verschiedener aufgefundener Genfragmente oder bekannter Gene.

Ratten wurden verschiedenen Schmerzmodellen unterworfen, einige Zeit nach Auslösung des Schmerzes Rückenmarksschnitte gewonnen und durch in-situ-Hybridisierung mit DNA-Sonden oder mit spezifischen Antikörpern die Expression des jeweiligen Gens oder Genfragments untersucht und mit Kontrolltieren verglichen.

Die Modelle waren CFA induzierte Arthritis (CFA), Collagen induzierte Arthritis (CIA) und der Formalin-Test (Formalin), s. D. Dubuisson, S.G. Dennis, Pain 4, 161 - 174 (1977).

Tabelle 5a zeigt die Ergebnisse

**Tabelle 5a**

| **Name** | **Lokalisation** | **Expressions-Level** | **Regulation** | | |
|---|---|---|---|---|---|
| | | | **CFA** | **CIA** | **Formalin** |
| JNK3 | Grey- Matter | hoch | **hoch** | **hoch** | **hoch** |
| | DH + VH | | VH, tiefes DH | | |
| PIM-2 | Grey- Matter | relativ hoch | **Hoch** | **Hoch** | **Hoch** |
| | DH + VH | | DH runter | DH runter | DH runter |
| GF135.7 | Grey- Matter | niedrig | | | |
| | VH > DH | | DH runter | | |
| GF111.39 | Grey- Matter | sehr niedrig | 0 | | 0 |
| | DH ≥ VH | | | | |
| GF111.45 | Grey- Matter | sehr niedrig | **Hoch** | | **Hoch** |
| | DH >> VH | | DH>VH | | DH>VH |
| GF141.24 | Grey- Matter | sehr niedrig | | | **Hoch** |
| | DH >> VH | | | | DH>VH |

| | | | | | |
|---|---|---|---|---|---|
| DH = Dorsalhorn VH = Ventralhorn | | | | | |

### Beispiel 2b)

### Expressionsniveau-Bestimmung über RT-PCR verschiedener bekannter Gene (TSPAN, Casein-Kinase und M3K7-Kinase) nach verschiedenen Schmerzmodellen.

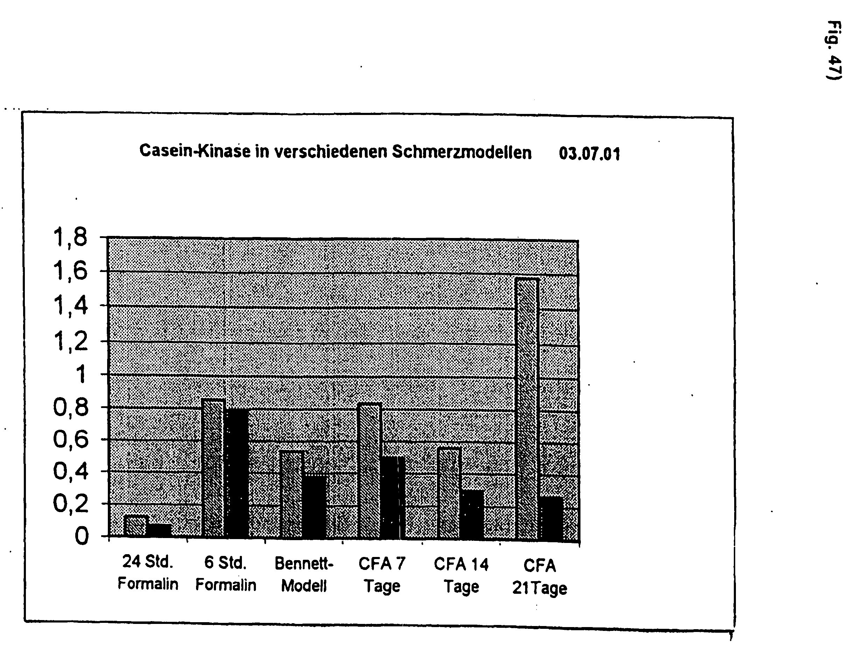

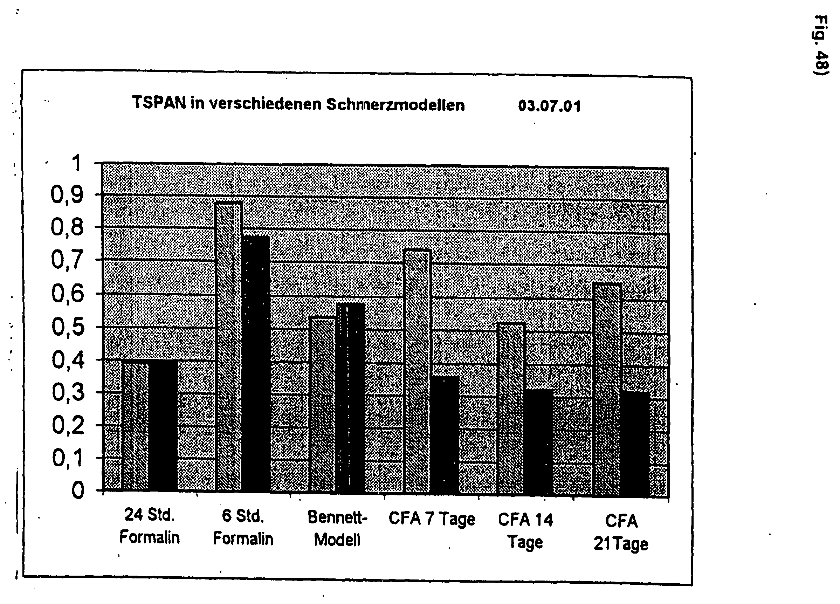

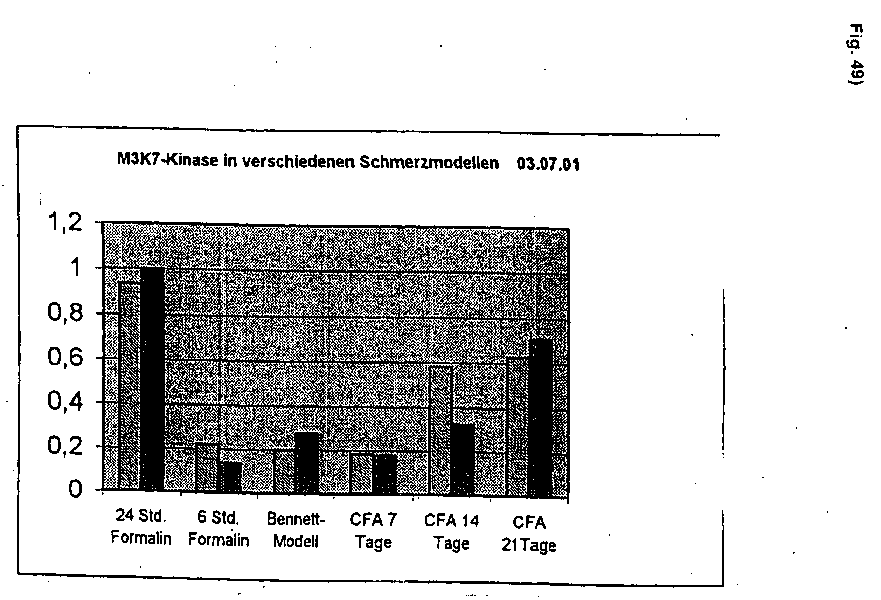

Der Umfang der Expression nach verschiedenen Schmwerzmodellen im Vergleich zu Kontrollen in Ratten wurde durch RT-PCR (s.o.) untersucht. Die Ergebnisse sind den Abbildungen 47, 48 und 49 zu entnehmen, wobei die hellen Balken den Kontrolltieren entsprechen und die dunklen Balken den behandelten Tieren. Die Y-Achse sind relative Einheiten (normalisiertes Expressionsniveau).

### Beispiel 2c)

### Klonierung der vollständigen humanen bzw. Ratten-Sequenzen, bzw. des entsprechenden vollständigen Gens zu den in Beispiel 1 gefundenen Genfragmenten gem. Abildungen 1-33

### 1.) Klonierung der full-length-Ratten-cDNA-Sequenzen

Die Klonierung der vollständigen Ratten-cDNA-Sequenzen erfolgt entweder mittels Durchscreenen von cDNA-Banken oder PCR-vermittelten Methoden.

### a) Durchscreenen

Die cDNA-Banken wurden mit RNA eines Gewebes erstellt, das die mRNA des gesuchten Genes exprimiert. Als Sonden finden einzel- oder doppelsträngige DNA oder RNA-Moleküle Verwendung, die entweder radioaktiv (z.B. mit [α³²P]dCTP) oder nicht radioaktiv markiert sind (z.B. mit Digoxigenin markiertes UTP). Die in Bakterien oder Phagen vorhandene cDNA-Bank wird auf Agar-Platten aufgebracht, nach Übertragen auf geeigneten Filtermembranen mit den Nukleinsäuresonden hybridisiert und nach stringenter Waschprozedur detektiert. Die durch diese Verfahren identifizierten cDNA-Klone werden weiter vereinzelt und durch Sequenzierung analysiert (siehe hierzu Sambrooks et al., 1989; Ausubel et al., 1990).

### b) PCR-vermittelte Methode

Bei den PCR-vermittelten Methoden werden ausgehend von den regulierten cDNA-Sequenzen Oligonukleotid-Primer in sense oder antisense- Orienterung ermittelt und synthetisiert. Bei dem herkömmlichen 5'-RACE Verfahren (Rapid amplification of 5'-cDNA Ends) wird der antisense-orientierte Primer zur cDNA-Synthese verwendet, danach die entstandene einzelsträngige cDNA durch Ligation eines Oligonukleotides mit RNA-Ligase oder Tailing-Reaktion mit terminaler Transferase verlängert und dann einer PCR-Reaktion mit einem weiter innen gelegenen (sog. nested) Primer und einem Tail-spezifischen Primer unterzogen. Die PCR-Amplifikate werden kloniert und durch Sequenzierung analysiert. Eine Abwandlung dieser Methode nutzt den Effekt der sogenannten Supression-PCR, um ausgehend von einem cDNA-Template sowohl in 5 als auch in 3'-Richtung die cDNA-Sequenz zu verlängern (Marathon cDNA Amplification Kit, Fa. Clontech). Hierbei wird eine doppelsträngige cDNA ausgehend von RNA aus Ratten-Rückenmark erstellt, und mit speziell modifizierten Linkern ligiert. Diese cDNA wird dann mit einem Gen-spezifischen Primer und einem Linker-spezifischen Primer mittels PCR amplifiziert, die Amplifikationsprodukte kloniert und sequenziert.

### 2.) Klonierung der homologen humanen cDNA-Sequenzen

Die homologen humanen cDNA-Sequenzen werden durch Modifikation der oben erwähnten Methoden isoliert. Beim herkömmlichen Screenen von cDNA-Banken werden Banken humanen Ursprungs verwendet und die Hybridisierung unter weniger stringenten Bedingungen durchgeführt, um auch cDNA-Klone geringerer Homologie zu erfassen.

Bei den PCR-vermittelten Strategien versucht man zunächst mit einer Vielzahl verschiedener Primer, die bevorzugt aus der kodierenden Region stammen, eine humane cDNA zu amplifizieren. Gelingt dies nicht, so kann man degenerierte PCR-Primer verwenden oder man wählt' weniger stringente PCR-Bedingungen.

### Beispiel 3:

### Durchführung des Screeningverfahrens mit Messung der Bindung über die Verdrängung eines radioaktiv markierten Liganden

Ein Nukleinsäureabschnitt, der für den Chloridkanal CLC-7 kodiert, wird in einem Expressionsvektor kloniert, der eine konstitutive Expression (z.B. CMV-Promoter) oder eine induzierbare Expression in eukaryonten Zellen erlaubt. Die DNA wird mit geeignetem Transfektionsverfahren, z.B. mit Lipofectamin (Fa. Roche Diagnostics) in eukaryontischen Zellen (z.B. CHO-Zellen, HEK293-Zellen oder NIH-3T3-Zellen) hineingebracht. Die Zellen werden in Anwesenheit eines Setektionsreagenzen (z.B. Zeocin, Hygromycin oder Neomycin) kultiviert so daß nur die Zellen überleben, die das DNA-Konstrukt aufgenommen haben, und bei länger andauernder Selektion, auch in das Genom inkorporiert haben.
Ausgehend von diesen Zellen werden Membranfraktionen gewonnen, die den CLC-7 Kanal in großer Menge enthalten und für einen Bindungsassay verwendet werden können. Dieser besteht aus 1.) dem CLC-7 enthaltenden Membranen 2.) einem radioaktiv markierten Liganden (z.B. Tritium markierten 4,4'-Diisothiocyanostilbene-2,2'-disuffonsäure (DIDS) oder [³H] markiertem 4-Acetamido-4'-isothiocyanatostilbene-2,2'-disutfonsäure (SITS)); 3.) einem Bindungspuffer (z.B. 50 mM HEPES pH 7.4, 1 mM EDTA) und dem auf Bindung zu untersuchenden Liganden. Die Substanzen SITS und DIDS waren in funktionellen Untersuchungen an CLC-7 exprimierenden Oozyten in der Lage, CLC-7 vermittelte Chloridströme zu inhibieren (unveröffentliche Ergebnisse Fr. Dr. Diewald, Universität Mainz). Nach Inkubation der oben genannten Reaktionsgemische (für z.B. 30-60 min) bei einer geeigneten Temperatur (meistens Raumtemperatur) werden die nichtgebundenen radioaktiven Ligandenmoleküle abfiltriert. Die Restmenge an gebundenem [³H]-DIDS bzw. [³H]-SITS wird nach Zugabe eines Szintillationscocktails in einem β-Counter (z.B. Trilux, Fa. Wallac) vermessen. Zeigt die Testsubstanz eine Bindung an den CLC-7-Kanal, so wird dies als verringerter radioaktiver Einbau detektiert. Dieses Verfahren wird geeignetermaßen so miniaturisiert, daß es in (96-, 384- oder 1536-well) Mikrotiterplatten durchgeführt werden kann, um dieses Verfahren mittels eines Roboters im sogenannten Hightroughput-Screening (HTS)-Verfahren durchzuführen:

### Beispiel 4:

### Durchführung des erfindungsgemäßen Screeningverfahrens mit Messung der durch die Bindung der Substanz veränderten funktionellen Parameter

Ein Nukleinsäureabschnitt, der für die Proteinkinase PIM-2 kodiert, wird in einem Expressionsvektor kloniert, der eine induzierbare Expression in Prokaryonten, wie z.B. E.coli erlaubt. Hierbei wird der Nukleinsäureabechnitt so modifiziert, daß er als Fusionsprotein mit einer zusätzlichen N- oder C-terminalen Aminosäuresequenz exprimiert wird. Diese Sequenz sollte bei unveränderter Funktion der PIM-2 Kinase eine Aufreinigung über ein spezifisches Verfahren erlauben, z.B. Glutathion S-Transferasefragment, das über Bindung an Glutathion eine Isolierung aus dem Proteingemisch erlaubt. Nach Transfektion der Bakterien, Induktion des Genes (z.B. mit IPTG beim lac-Promoter) und Aufschließen der Bakterien werden die Fusionsproteine aufgereinigt und in einem in vitro-Kinase Experiment eingesetzt. Hierbei werden 5 µg Protein bei 30°C für 30 Minuten in 50 µl Kinasepuffer (20 mM PIPES, pH 7.0, 5 mM MnCl₂, 7 mM β-Mercaptoethanol, 0.4 mM Spermin, 10 mMrATP) ergänzt mit 10 µCi [γ³²P] ATP. Als Substrate werden gereinigtes Histon H1-Protein (Fa. Sigma) oder bakteriell exprimiertes GST-NFATc1-Fusionsprotein hinzugegeben. Nach der Inkubationszeit wird das nicht-inkorpierte [γ-³²P] ATP abfiltriert und die Menge an eingebautem ³²Phosphat durch β-Szintillation (Trilux, Fa. Wallac) bestimmt. In einem Experiment zum Aufspüren neuer PIM-2 Kinase-Inhibitoren werden in diesem Ansatz die Testsubstanzen mitinkubiert und eine Abnahme der ³²P-Inkorporation als Indikator für einen Inhibitor benutzt. Dieses Verfahren wird geeigneter-maßen so miniaturisiert, daß es in (96-, 384- oder 1536-well) Mikrotiterplatten durchgeführt werden kann, um dieses Verfahren mittels eines Roboters im sogenannten Hightroughput-Screening (HTS)-Verfahren durchzuführen.

### Beispiel 5

### Beispiel für ein Arzneimittel enthaltend einen erfridtingsgemäßen Wirkstoff - Tablettenformulierung

Tabletten können durch direktes Verpressen von Mischungen des erfindungsgemäßen Wirkstoffes mit entprechenden Hilfsstoffen oder durch Verpressen von wirkstoffhaltigen Granulaten (mit gegebenenfalls weiteren Hilfsstoffen) hergestellt werden. Die Granulate können dabei entweder durch Feuchtgranulation mit z.B. wäßrigen Granulierflüssigkeiten und anschließender Trocknung dieser Granulate oder durch Trockengranulation z.B. über Kompaktierung hergestellt werden.

**■ Direktverpressung**

| | | |
|---|---|---|
| z.B. pro Tablette: | 25 mg | erfindungsgemäßer Wirkstoff |
| | 271 mg | LudipressTM (Granulat zur Direkttablettierung aus Lactose monohydrat, Povidon K30 und Crospovidon) |
| | 4 mg | Magnesiumstearat |
| | 300 mg | Gesamt |

Homogene Mischung des Wirkstoffes mit den Hilfsstoffen herstellen und diese auf einer Tablettenpresse zu Tabletten mit einem 0 von 10 mm verpressen.

**■ Trockengranulation**

| | | |
|---|---|---|
| z.B. pro Tablette: | 25 mg | erfindungsgemäßer Wirkstoff |
| | 166 mg | Microcristalline Cellulose |
| | 80 mg | Niedrig substituierte Hydroxypropylcellulose (I-HPC LH 11^{™}) |
| | 5 mg | Hochdisperses Siliziumdioxid |
| | 4 mg | Magnesiumstearat |
| | 280 mg | Gesamt |

Homogene Mischung des Wirkstoffes mit der Mikrokristallinen Cellulose und der I-HPC herstellen und diese Kopaktieren. Nach dem Sieben der Komprimate wird das entstandene Granulat mit Magnesiumstearat und Siliziumdioxid gemischt und auf einer Tablettenpresse zu Tabletten mit einem Ø von 9 mm verpreßt.

**■ Feuchtgranulation**

| | | |
|---|---|---|
| z.B. pro Tablette: | 25 mg | erfindungsgemäßer Wirkstoff |
| | 205 mg | Mikrokristalline Cellulose |
| | 6 mg | Povidon K30 |
| | 10 mg | Crospovidon |
| | 4 mg | Magnesiumstearat |
| | 250 mg | Gesamt |

Homogene Mischung des Wirkstoffes mit der Mikrokristallinen Cellulose und dem Crospovidon herstellen und diese in einem Granulator mit einer wäßrigen Lösung des Povidons granulieren. Das feuchte Granulat wird anschließend nachgranuliert und nach der Trocknung im Trockenschrank (50°C) 10 h getrocknet. Das trockene Granulat wird mit dem Magnesiumstearat zusammen gesiebt, endgemischt und auf einer Tablettenpresse zu Tabletten mit einem ∅ von 8 mm verpreßt.

### Beispiel 6

### Beispiel für ein Arzneimittel enthaltend einen erfindungsgemäßen Wirkstoff - parenterale Lösung

1 g eines erfindungsgemäßen Wirkstoffes wird in 1 I Wasser für Injektionszwecke bei Raumtemperatur gelöst und anschließend durch Zugabe von NaCl (Natriumchlorid) auf isotone Bedingungen eingestellt.

### Literatur:

Akopian AN, Sivilotti L & Wood JN (1995) Nature 379: 257-262
Ausubel FM, Brent R, Kingdton RE, Moore DD, Seidman JG, Smith JA & Struhl K eds.(1190) Current protocols in molecular biology. John Wiley &Sons, Inc. New York, NY.
Baba H, Doubell TP, Woolf CJ 1999: Peripheral inflammation facilitates Aβ fiber-mediated synaptic input to the substantia gelatinosa of the adult rat spinal cord. J Neurosci 19: 859-867
Bauer D, Müller H, Reich J, Riedel H, Ahrenkiel V, Warthoe P & Strauss M (1993): Identification of differentially expressed mRNA species by an improved display technique (DDRT-PCR) Nucl Acids Res 21: 4272-4280.
Bonini A, Anderson SM, Steiner DF (1997) Molecular cloning and tissue expression of a novel orphan G Protein-coupled receptor from rat lung. Biochem Biophys Res Comm 234: 190-193.
Chih-Cheng et al., (1995): A P2X prinoceptor expressed by a subset of sensory neurons. Nature 377:428-432
Corderre TJ, Katz J, Vaccarino AL, Melzack R (1993): Contribution of central plasticity to pathological pain: review of clinical and experimental evidence. Pain 52: 259-285.
Dickenson (1995) Novel pharmacological targets in the treatment of pain. Pain Rev., 2, 1-12.
Dubuisson et al., 1997 Pain, 4:161-174.
Feng Y & Gregor P (1997) Cloning of a novel member of the G Protein-coupled receptor family related to peptide receptors. Biochem Biophys Res Comm 231: 651-654.
Furukawa T, Yang Y, Nakamoto B, Stamatoyannopoulos G, Papayannopoulou T (1996): Identification of new genes expressed in a human erythroleukemia cell line. Bloods Cell Mol & Dis 22:11-22.
Gunasekar PG, Kanthasamy, AG, Borowitz JL, Isom GE 1995: NMDA receptor activation produces concurrent generation of nitric oxide and reactive oxygeh species: implication for cell death. J Neurochem 65: 2016-2021.
Hawes BE, Fried S, Yao X, Weig B, Graziano MP 1998: Nociceptin (ORL1) and µ-Opioid receptors mediate mitogen-activated protein kinase activation in CHO cells through a Gi-coupled signaling pathway: evidence for distinct mechanisms of agonist-mediated desensitization. J Neurochem 71: 1024-1033.
Hubank M & Schatz DG (1994): ldentifying differences in mRNA expression by representational difference analysis of cDNA. Nucl Acids Res 22: 5640-5648.
Klußmann S et al., 1996: Nature Biotechnology 14: 1112-1115.
Li L-Y & Chang K-J 1996: The stimulatory effect of opioids on mitogen-activated protein kinase in chinese hamster ovary cells transfected to express µ-opioid receptors. Mol Pharm 50:599-602.
Liang P & Pardee AB 1992: Differential Display of eukaryotic messenger RNA by means of the polymerase chain reaction. Science 257:967-971.
Methner A, Hermey G, Schinke B, Hermanns-Borgmeyer I (1997): A novel G Protein-coupled receptor with homology to neuropeptide and chemoattractant receptors expressed during bone development. Biochem Biophys Res Comm 233:336-342.
Mohit AA, Martin JH & Miller CA 1995: p493F12 Kinase: a novel MAP kinase expressed in a subset of neurons in the human nervous system. Neuron 14: 67-78.
Poirier GM-C, Pyati J, Wan JS, Erlander MG 1997: Screening differentially expressed cDNA clones obtained by differential display using amplified RNA. Nucleic Acids Research 25: 913-914.
Sambrook J, Fritsch EF & Maniatis T 1989: Molecular Cloning: A Laboratory Manual. Second Edition. Cold Spring Harbor Laboratory Press, Cold Spring Harbor.
Sompayrac L, Jane S, Burn TC, Tenen DG & Danna KJ 1995: Overcoming limitations of the mRNA differential display technique. Nucleic Acids Research 23: 4738-4739.
Tal M 1996: A novel antioxidant alleviates heat hyperalgesia in rats with an experimental painful neuropathy. Neurreport 7: 1382-1384.
Tölle TR (1997): Chronischer Schmerz. In: Klinische Neurobiologie, Herdergen T, Tölle TR, Bähr M (Hrsg.): S. 307-336; Spektrum Verlag, Heidelberg.
U.S.Patent 5.262.311
Velculescu VE, Zhang L, Vogelstein B, Kinzler KW (1995): Serial analysis of gene expression. Science 270: 484-487.
Wan JS, Sharp JS et al. (1996): Cloning differentially expressed mRNAs Nature Biotech 14:1685-1691.
Watson JB & Margulies JE (1993) Differential cDNA screening strategies to identify novel stage-specific proteins in the developing mammalian brain. Dev Neurosci 15: 77-86.
Wilks AF (1989) Two putative protein-tyrosine kinases identified by application of the polymerase chain reaction. Poc Natl Acad Sci USA 86: 1603-1607.
Woolf CJ, Shortland P, Coggeshall RE 1992: Peripheral nerve injury triggers central sprouting of myelinated afferents. Nature 355:75-78.
Zimmermann, M & Herdegen, T (1996): Plasticity of the nervous system at the systemic, cellular and molecular levels: a mechanism of chronic pain and hyperalgesia. Progr Brain Res 110: 233-259

## Patentansprüche

1. Verfahren zur Auffindung schmerzregulierender Substanzen, d.h. Substanzen, die die Wahrnehmung von Schmerz direkt oder indirekt beeinflussen, mit folgenden Verfahrensschritten:
(a) Inkubation einer zu testenden Substanz unter geeigneten Bedingungen mit einer Zelle und/oder einer Präparation aus einer solchen Zelle, die ein Peptid oder Protein synthetisiert hat, das ausgewählt ist aus folgender Gruppe:
- PIM-2,
- einem Peptid oder Protein, für das oder für einen Teil dessen ein Polynukleotid gemäss einer der Abbildungen 35a), 35c) oder 35e) oder ein dazu zu mindestens 90 % ähnliches Polynukleotid kodiert,
- einem Peptid oder Protein mit einer Aminosäuresequenz gemäss einer der Abbildungen 35b), 35d) oder 35f), oder ein dazu zu mindestens 90 % ähnliches Peptid oder Protein
- und/oder einem Protein, das durch eine Nukleinsäure kodiert wird, die unter stringenten Bedingungen an ein Polynukleotid gemäss einer der Abbildungen 35a), 35c) oder 35e) oder deren Antisense Polynukleotid bindet,
(b) Messung der Bindung der Testsubstanz an dem von der Zelle synthetisierten Peptid oder Protein oder Messung mindestens eines der durch die Bindung der Testsubstanz an das Peptid oder Protein veränderten funktionellen Parameters über Messung der Regulation, Hemmung und/oder Aktivierung von Rezeptoren, lonenkanälen und/oder Enzymen, insbesondere über Messung der Veränderung der Genexpression, des lonenmilieus, des pH oder des Membranpotentials, über Veränderung der Enzymaktivität oder der Konzentration der 2^{nd} messenger, oder Messung der Bindung über die Verdrängung eines bekannten markierten Liganden des Peptids oder Proteins und/oder über die daran gebundene Aktivität einer markierten Testsubstanz.

2. Verfahren gemäss Anspruch 1, **dadurch gekennzeichnet, dass** die Zelle vor dem Schritt (a) gentechnisch manipuliert wird.

3. Verfahren gemäss Anspruch 2, **dadurch gekennzeichnet, dass** die gentechnische Manipulation die Messung mindestens eines der durch die Testsubstanz veränderten funktionellen Parameter erlaubt.

4. Verfahren gemäss Anspruch 3, **dadurch gekennzeichnet, dass** durch die gentechnische Manipulation eine in der Zelle nicht endogen exprimierte Form eines G-Proteins exprimiert oder ein Reportergen eingeführt wird.

5. Verfahren gemäss einem der Ansprüche 2-4, **dadurch gekennzeichnet, dass** die Zelle gentechnisch so manipuliert wird, dass die Zelle mindestens ein Polynukleotid gemäss einer der Abbildungen 35a), 35c) oder 35e) oder ein dazu zu mindestens 90 % ähnliches Polynukleotid enthält.

6. Verfahren gemäss Anspruch 5, **dadurch gekennzeichnet, dass** das Polynukleotid in einem rekombinanten DNA-Konstrukt enthalten ist.

7. Verfahren gemäss einem der Ansprüche 2 bis 6, **dadurch gekennzeichnet, dass** die Zelle nach der gentechnischen Manipulation gemäss Anspruch 2 und vor dem Schritt (a) gemäss Anspruch 1 unter Bedingungen, die eine Expression erlauben, kultiviert wird, gegebenenfalls unter Selektionsdruck.

8. Verfahren gemäss einem der Ansprüche 1 bis 7, **dadurch gekennzeichnet, dass** die Zelle eine Amphibienzelle, Bakterienzelle, Hefezelle, lnsektenzelle oder eine immortalisierte oder native Säugetierzelle ist.

9. Verfahren gemäss einem der Ansprüche 1-8, **dadurch gekennzeichnet, dass** das Peptid oder Protein in den Schritten (a) und (b) ausgewählt ist aus folgenden Gruppen:
- ein Peptid oder Protein, für das oder für einen Teil dessen ein Polynukleotid gemäss einer der Abbildungen 35a), 35c) oder 35e) oder ein dazu zu mindestens 95%, insbesondere 97%, ähnliches Polynukleotid kodiert, oder
- ein Peptid oder Protein mit einer Aminosäuresequenz gemäss einer der Abbildungen 35b), 35d) oder 35f) oder ein dazu zu mindestens 97%, ähnliches Peptid oder Protein
- und/oder einem Protein, das durch eine Nukleinsäure kodiert wird, die unter stringenten Bedingungen an ein Polynukleotid gemäss einer der Abbildungen 35a), 35c) oder 35e) oder deren Antisense Polynukleotid bindet.

10. Verwendung eines Peptids oder Proteins ausgewählt aus einer der folgenden Gruppen:
- PIM-2,
- einem Peptid oder Protein, für das oder für einen Teil dessen ein Polynukleotid gemäss einer der Abbildungen 35a), 35c) oder 35e) oder ein dazu zu mindestens 90 % ähnliches Polynukleotid kodiert,
- einem Peptid oder Protein mit einer Aminosäuresequenz gemäss einer der Abbildungen 35b), 35d) oder 35f) oder 46f) oder ein dazu zu mindestens 90 % ähnliches Peptid oder Protein
- und/oder einem Protein, das durch eine Nukleinsäure kodiert wird, die unter stringenten Bedingungen an ein Polynukleotid gemäss einer der Abbildungen 35a), 35c) oder 35e) oder deren Antisense Polynukleotid bindet,
in einem Verfahren zur Auffindung schmerzregulierender Substanzen, d.h. Substanzen, die die Wahrnehmung von Schmerz direkt oder indirekt beeinflussen.

## Claims

1. Process for the discovery of pain regulating substances, i.e. substances that directly or indirectly influence the perception of pain, comprising the following process steps:
(a) incubation of a test substance under suitable conditions with a cell and/or a preparation of such a cell which has synthesized a peptide or protein selected from the following group:
- PIM-2,
- a peptide or protein which, or part of which, is encoded by a polynucleotide according to one of Figures 35a), 35c) and 35e) or by a polynucleotide that is at least 90% similar thereto,
- a peptide or protein having an amino acid sequence according to one of Figures 35b), 35d) and 35f), or a peptide or protein that is at least 90% similar thereto,
- and/or a protein which is encoded by a nucleic acid that binds under stringent conditions to a polynucleotide according to one of Figures 35a), 35c) and 35e) or to their antisense polynucleotide;
(b) measurement of the binding of the test substance to the peptide or protein synthesized by the cell, or measurement of at least one of the functional parameters changed by the binding of the test substance to the peptide or protein, via measurement of the regulation, inhibition and/or activation of receptors, ion channels and/or enzymes, especially via measurement of the change in the gene expression, the ionic medium, the pH or the membrane potential, via the change in the enzyme activity or the concentration of the 2nd messenger, or measurement of the binding via the displacement of a known labelled ligand of the peptide or protein and/or via the activity of a labelled test substance that is bound thereto.

2. Process according to Claim 1, **characterized in that** the cell is genetically manipulated before step (a).

3. Process according to Claim 2, **characterized in that** the genetic manipulation allows the measurement of at least one of the functional parameters changed by the test substance.

4. Process according to Claim 3, **characterized in that**, due to the genetic manipulation, a form of a G protein that is not endogenously expressed in the cell is expressed or a reporter gene is introduced.

5. Process according to one of Claims 2-4, **characterized in that** the cell is genetically manipulated so that it contains at least one polynucleotide according to one of Figures 35a), 35c) and 35e) or a polynucleotide that is at least 90% similar thereto.

6. Process according to Claim 5, **characterized in that** the polynucleotide is contained in a recombinant DNA construct.

7. Process according to one of Claims 2 to 6, **characterized in that**, after the genetic manipulation according to Claim 2 and before step (a) according to Claim 1, the cell is cultivated under conditions that allow expression, optionally under selection pressure.

8. Process according to one of Claims 1 to 7, **characterized in that** the cell is an amphibian cell, a bacterial cell, a yeast cell, an insect cell or an immortalized or native mammalian cell.

9. Process according to one of Claims 1-8, **characterized in that** the peptide or protein in steps (a) and (b) is selected from the following groups:
- a peptide or protein which, or part of which, is encoded by a polynucleotide according to one of Figures 35a), 35c) and 35e) or by a polynucleotide that is at least 95%, especially 97%, similar thereto, or
- a peptide or protein having an amino acid sequence according to one of Figures 35b), 35d) and 35f), or a peptide or protein that is at least 97% similar thereto,
- and/or a protein which is encoded by a nucleic acid that binds under stringent conditions to a polynucleotide according to one of Figures 35a), 35c) and 35e) or to their antisense polynucleotide.

10. Use of a peptide or protein selected from one of the following groups:
- PIM-2,
- a peptide or protein which, or part of which, is encoded by a polynucleotide according to one of Figures 35a), 35c) and 35e) or by a polynucleotide that is at least 90% similar thereto,
- a peptide or protein having an amino acid sequence according to one of Figures 35b), 35d) and 35f), or a peptide or protein that is at least 90% similar thereto,
- and/or a protein which is encoded by a nucleic acid that binds under stringent conditions to a polynucleotide according to one of Figures 35a), 35c) and 35e) or to their antisense polynucleotide,
in a process for the discovery of pain regulating substances, i.e. substances that directly or indirectly influence the perception of pain.

## Revendications

1. Méthode de détection de substances régulatrices de la douleur, c'est-à-dire de substances qui influent directement ou indirectement sur la perception de la douleur, comprenant les étapes suivantes ;
(a) incubation, dans des conditions convenables, d'une substance à tester avec une cellule et/ou une préparation obtenue à partir d'une cellule du type ayant synthétisé un peptide ou une protéine qui est choisi(e) dans le groupe suivant :
- PIM-2,
- un peptide ou une protéine qui est codé(e) ou dont une partie est codée par un polynucléotide selon l'une des représentations 35a), 35c) ou 35e) ou par un polynucléotide qui y est semblable à 90% au moins,
- un peptide ou une protéine ayant une séquence d'acides aminés selon l'une des représentations 35b), 35d) ou 35f) ou bien un peptide ou une protéine qui y est semblable à 90% au moins,
- et/ou une protéine codée par un acide nucléique qui se lie dans des conditions stringentes à un polynucléotide selon l'une des représentations 35a), 35c) ou 35e) ou au polynucléotide antisens correspondant,
(b) mesure de la liaison de la substance à tester au peptide ou à la protéine synthétisé(e) par la cellule ou mesure d'au moins l'un des paramètres fonctionnels modifiés par la liaison de la substance à tester au peptide ou à la protéine, par l'intermédiaire de la mesure de la régulation, de l'inhibition et/ou de l'activation de récepteurs, de canaux ioniques et/ou d'enzymes, en particulier par l'intermédiaire de la mesure de la modification de l'expression génique, du milieu ionique, du pH ou du potentiel de membrane, par l'intermédiaire de la modification de l'activité enzymatique ou de la concentration des seconds messagers, ou mesure de la liaison par l'intermédiaire du déplacement d'un ligand marqué connu du peptide ou de la protéine et/ou par l'intermédiaire de l'activité, liée à ce ligand, d'une substance marquée à tester.

2. Méthode selon la revendication 1, **caractérisée en ce que** la cellule est soumise à une technique de manipulation génétique préalablement à l'étape (a).

3. Méthode selon la revendication 2, **caractérisée en ce que** la technique de manipulation génétique permet la mesure de l'un au moins des paramètres fonctionnels modifiés par la substance à tester.

4. Méthode selon la revendication 3, **caractérisée en ce qu'**une forme d'expression non endogène d'une protéine G est exprimée dans la cellule ou un gène reporter est introduit par la technique de manipulation génétique.

5. Méthode selon l'une des revendications 2-4, **caractérisée en ce que** la cellule est traitée par une technique de manipulation génétique de façon telle qu'elle contienne au moins un polynucléotide conforme à l'une des représentations 35a), 35c) ou 35e) ou un polynucléotide qui y soit semblable à 90% au moins.

6. Méthode selon la revendication 5, **caractérisée en ce que** le polynucléotide est contenu dans un produit d'assemblage d'ADN recombiné.

7. Méthode selon l'une des revendications 2 à 6, **caractérisée en ce que** la cellule est cultivée après la manipulation génétique suivant la revendication 2 et avant l'étape (a) suivant la revendication 1 dans des conditions qui permettent une expression, éventuellement sous pression de sélection.

8. Méthode selon l'une des revendications 1 à 7, **caractérisée en ce que** la cellule est une cellule d'amphibien, une cellule bactérienne, une cellule de levure, une cellule d'insecte ou une cellule de mammifère immortalisée ou native.

9. Méthode selon l'une des revendications 1-8, **caractérisée en ce que** dans les étapes (a) et (b), le peptide ou la protéine est choisi(e) dans les groupes suivants :
- un peptide ou une protéine qui est codé(e) ou dont une partie est codée par un polynucléotide selon l'une des représentations 35a), 35c) ou 35e) ou par un polynuclotide qui y est semblable au moins à 95%, en particulier à 97%, ou
- un peptide ou une protéine ayant une séquence d'acides aminés selon l'une des représentations 35b, 35d) ou 35f) ou bien un peptide ou une protéine qui y est semblable à 97% au moins
- et/ou une protéine codée par un acide nucléique qui se lie dans des conditions stringentes à un polynucléotide selon l'une des représentations 35a), 35c) ou 35e) ou le polynucléotide antisens correspondant.

10. Utilisation d'un peptide ou d'une protéine choisi dans l'un des groupes suivants :
- PIM-2,
- un peptide ou une protéine qui est codé(e) ou dont une partie est codée par un polynucléotide selon l'une des représentations 35a), 35c) ou 35e) ou par un polynucléotide qui y est semblable à 90% au moins,
- un peptide ou une protéine ayant une séquence d'acides aminés selon l'une des représentations 35b), 35d) ou 35f) ou 46f) ou bien un peptide ou une protéine qui y est semblable à 90% au moins
- et/ou une protéine codée par un acide nucléique qui se lie dans des conditions stringentes à un polynucléotide selon l'une des représentations 35a), 35c) ou 35e) ou au polynucléotide antisens correspondant,
dans une méthode de détection de substances régulatrices de la douleur, c'est-à-dire de substances qui influent directement ou indirectement sur la perception de la douleur.
